# EUROPEAN PATENT SPECIFICATION

(11) **EP 4 087 583 B1**
(45) Date of publication and mention of the grant of the patent: **22.04.2026**
(21) Application number: 20911563.3
(22) Date of filing: 18.12.2020
(51) Int. Cl.: A61K 31/69, A61P 35/00, C07F 5/02, A61K 45/06

(54) **ARGINASE INHIBITORS AND METHODS OF USE**
ARGINASE-INHIBITOREN UND VERFAHREN ZUR VERWENDUNG
INHIBITEURS D'ARGINASE ET MÉTHODES D'UTILISATION

(30) Priority: 07.01.2020 US 202062958056 P
(43) Date of publication of application: 16.11.2022
(73) Proprietor: Merck Sharp & Dohme LLC, Rahway, New Jersey 07065 (US)
(72) Inventor: ACHAB, Abdelghani Abe, Boston, Massachusetts 02115-5727 (US); CHILDERS, Matthew, L., Boston, Massachusetts 02115-5727 (US); FISCHER, Christian, Boston, Massachusetts 02115-5727 (US); GATHIAKA, Symon, Boston, Massachusetts 02115-5727 (US); LI, Derun, Boston, Massachusetts 02115-5727 (US); LU, Min, Boston, Massachusetts 02115-5727 (US); PALANI, Anandan, Boston, Massachusetts 02115-5727 (US); PALTE, Rachel, L., Boston, Massachusetts 02115-5727 (US); PU, Qinglin, Boston, Massachusetts 02115-5727 (US); SLOMAN, David, L., Boston, Massachusetts 02115-5727 (US); ZHANG, Hongjun, Boston, Massachusetts 02132 (US)
(74) Representative: Merck Sharp & Dohme LLC
(86) International application number: PCT/US2020/065798
(87) International publication number: WO 2021/141751

(56) References cited:
- EP-A1- 3 034 509
- EP-A1- 3 897 622
- WO-A1-2017/075363
- WO-A1-2019/177873
- WO-A1-2019/177873
- WO-A1-2019/245890
- US-A1- 2017 121 352
- US-A1- 2017 319 536
- DATABASE PubChem Compound U.S. National Library of Medicine; 12 February 2012 (2012-02-12), "1,2-Dioxaspiro[4.5]dec-7-en-4-ylboronic acid", XP055840762, retrieved from ncbi Database accession no. CID 87392280
- ERB ET AL.: "An Easy Route to (Hetero)arylboronic Acids", CHEMISTRY A EUROPEAN JOURNAL, vol. 20, no. 22, 26 May 2014 (2014-05-26), pages 6608 - 6612, XP055840759

## Description

### TECHNICAL FIELD

The present invention is directed to arginase inhibitors. Specifically, the arginase inhibitors described herein can be useful in preventing, treating or acting as a remedial agent for arginase-related diseases.

### BACKGROUND

Arginase is an enzyme that metabolizes L-arginine to L-ornithine and urea. There are two types of arginase, and they are both products of distinct genes that are regulated independently and located on different chromosomes. Arginase I is a cytosolic protein (34.7 kDa) and is dominant in the liver, but also expressed extrahepatically. Arginase II is a mitochondrial protein and is expressed in kidney, small intestine, brain, monocytes and macrophages.

In addition to its fundamental role in the hepatic urea cycle, arginase also influences the immune systems in humans and mice. Arginase participates in many inflammatory disorders by decreasing the synthesis of nitric oxide and inducing fibrosis and tissue regeneration. L-Arginine deficiency, which is modulated by myeloid cell arginase, suppresses T-cell immune response. This mechanism plays a fundamental role in inflammation-associated immunosuppression.

Arginase expression and L-arginine depletion is also a known immune-suppressive pathway of the mammalian immune system. The depletion of arginine in the tumor microenvironment renders cytotoxic T-cells unable to proliferate and therefore unable to effectively mount an anti-tumor attack. Similarly, M2 macrophages and polymorphonuclear cells (PMNs) express high levels of arginase and may contribute to the local suppression of immune responses. Restoration of arginine levels in the tumor microenvironment via arginase inhibition would be expected to allow T-cell activation and proliferation to occur and result in T-cell mediated anti-tumor responses.

Small-molecule arginase inhibitors are currently described as promising therapeutics for the treatment of several diseases, including allergic asthma, inflammatory bowel disease, ulcerative colitis, cardiovascular diseases (atherosclerosis and hypertension), diseases associated with pathogens (e.g., Helicobacter pylori, Trypanosoma cruzi, Leishmania, Mycobacterium tuberculosis and Salmonella), cancer and induced or spontaneous immune disorders. Development of potent and specific inhibitors of arginase would be useful for the treatment of diseases where depletion of L-arginine from the microenvironment and/or induction of arginase pathway is involved in the evasion of anti-tumor immunity, especially for immuno-oncology indications. WO2019/245890 discloses arginase inhibitors and methods of use.

### SUMMARY

The technical information set out below may in some respects go beyond the scope of the presently claimed invention, which is defined by the appended set of claims. The additional technical information is provided to place the actual invention in a broader technical context and to illustrate possible related technical developments.

References herein to methods of treatment by therapy or surgery are to be interpreted as references to compounds, pharmaceutical compositions, combinations and/or medicaments for use in those methods.

In a first aspect of the invention there is provided compound of Formula I: 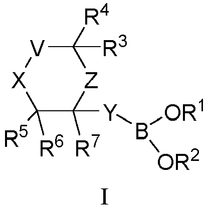 or a pharmaceutically acceptable salt thereof, wherein:
Y is a straight or branched (C1-C5)alkylene or cycloalkyl(C1-C5)alkylene, wherein one or more - CH2- groups in Y are optionally and independently replaced with a moiety selected from the group consisting of O, S and NH;
Z is a bond, or CH2;
V is O, NR8 or CR9R10, wherein X and V cannot be simultaneously O or S and O or NR8, respectively;
X is O, S, NR11, CH2, or CR12R13, wherein V and X cannot be simultaneously O and S, O or NR11, respectively;
R1 is hydrogen, C3-C6cycloalkyl or C1-C6alkyl or, taken with R2 forms a C3-C8cycloalkyl, wherein the C3-C8cycloalkyl is unsubstituted or substituted with one to four substituents selected from the group consisting of halogen, C1-C6alkyl or OH;
R2 is hydrogen, C3-C6cycloalkyl or C1-C6alkyl or, taken with R1 forms a C3-C8cycloalkyl, wherein the C3-C8cycloalkyl is unsubstituted or substituted with one to four substituents selected from the group consisting of halogen, C1-C6alkyl or OH;
R3 is hydrogen, halogen, C1-C6alkyl, haloC1-C6alkyl, OH, C1-C6alkylOH, C1-C6alkylOC1-C6alkyl, C1-C6alkoxy, COOH, NH2 or COOC1-C6alkyl;
R4 is taken with R7to form a heterocycle, wherein the heterocycle is pyrrolidine, azetidine, thiolane, thietane, oxetane, tetrahyrdrofurane or piperidine;
R5 is COOH;
R6 is hydrogen, halogen, C1-C6alkyl, haloC1-C6alkyl, OH, C1-C6alkylOH, C1-C6alkylOC1-C6alkyl, COOH, C1-C6alkoxy, COOH or COOC1-C6alkyl;
R8 is hydrogen, C3-C6cycloalkyl, C1-C6alkyl, C1-C6alkylaryl, C1-C6alkylNH2, COC1-C6alkylNH2, COC1-C6alkylNH(C1-C6alkyl), COC1-C6alkylN(C1-C6alkyl)2, C1-C6haloalkyl, C1-C6alkylOH or COC1-C6alkyl;
R9 is hydrogen, halogen, C1-C6alkyl, haloC1-C6alkyl, OH, C1-C6alkylOH, C1-C6alkylOC1-C6alkyl, C1-C6alkoxy, COOH or COOC1-C6alkyl;
R10 is hydrogen, halogen, C1-C6alkyl, haloC1-C6alkyl, OH, C1-C6alkylOH, C1-C6alkylOC1-C6alkyl, COOH, C1-C6alkoxy, COOH or COOC1-C6alkyl or is taken with R12 forms a C3-C6cycloalkyl or heterocycle, wherein the C3-C6cycloalkyl or heterocycle is unsubstituted or substituted with 1 to 3 substituents selected from the group consisting of halogen, OH, C1-C6alkylOH, oxo, C1-C6alkyl, C1-C6haloalkyl, COC1-C6alkyl, C1-C6alkoxy, COOH, NH2, C1-C6alkylNH2, C1-C6alkylphenyl, CO(C1-C6alkyl)NH2 and COOC1-C6alkyl , or taken with R6 forms an (C1-C5)alkylene bridge, wherein one or more -CH2- groups are optionally and independently replaced with a moiety selected from the group consisting of O, S and NH;
R11 is hydrogen, C3-C6cycloalkyl, C1-C6alkyl, C1-C6alkylaryl, C1-C6alkylNH2, COC1-C6alkylNH2, COC1-C6alkylNH(C1-C6alkyl), COC1-C6alkylN(C1-C6alkyl)2, C1-C6haloalkyl, C1-C6alkylOH or COC1-C6alkyl;
R12 is halogen, C1-C6alkyl, haloC1-C6alkyl, OH, C1-C6alkylOH, C1-C6alkylOC1-C6alkyl, COOH, C1-C6alkoxy, COOH or COOC1-C6alkyl or is taken with R6 or R10 to form a C3-C6cycloalkyl or heterocycle, wherein the C3-C6cycloalkyl or heterocycle is unsubstituted or substituted with 1 to 3 substituents selected from the group consisting of halogen, OH, C1-C6alkylOH, oxo, C1-C6alkyl, C1-C6haloalkyl, COC1-C6alkyl, C1-C6alkoxy, COOH, NH2, C1-C6alkylNH2, C1-C6alkylphenyl, CO(C1-C6alkyl)NH2 and COOC1-C6alkyl; and
R13 is halogen, C1-C6alkyl, haloC1-C6alkyl, OH, C1-C6alkylOH, C1-C6alkylOC1-C6alkyl, C1-C6alkoxy, COOH or COOC1-C6alkyl.

In a second aspect of the invention there is provided a compound of the invention, or a pharmaceutically acceptable salt thereof for use in treating cancer.

In a further aspect of the invention there is provided a combination comprising a compound of the invention, or a pharmaceutically acceptable salt thereof and other therapeutic agents for use in treating cancer.

In a further aspect of the invention there is provided a pharmaceutical composition comprising a compound of the invention, or a pharmaceutically acceptable salt thereof, and a pharmaceutically acceptable carrier.

In a further aspect of the invention there is provided a compound of the invention, or a pharmaceutically acceptable salt thereof for use in therapy.

Compounds of Formula I:

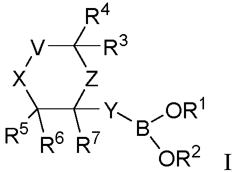

Described herein are arginase inhibitors, which can be useful in the prevention, treatment or amelioration of diseases where depletion of L-arginine from the microenvironment and/or induction of arginase pathway is involved in the evasion of anti-tumor immunity, especially for immuno-oncology indications.

Also described herein are methods of treating cancer comprising administering to a patient in need thereof a compound described herein, or a pharmaceutically acceptable salt thereof.

Also described herein are methods of treating fibrosis related diseases such as nonalcoholic fatty liver disease comprising administering to a patient in need thereof a compound described herein, or a pharmaceutically acceptable salt thereof.

Also described herein are uses of a compound described herein, or a pharmaceutically acceptable salt thereof, to treat cancer in a patient in need thereof.

Also described herein are pharmaceutical compositions comprising a compound described herein, or a pharmaceutically acceptable salt thereof and a pharmaceutically acceptable carrier. Also described herein are pharmaceutical compositions comprising a compound described herein and a pharmaceutically acceptable carrier.

### DETAILED DESCRIPTION

Described herein are compounds of Formula I: 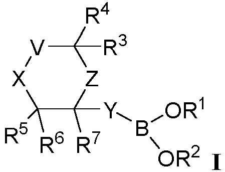 or a pharmaceutically acceptable salt thereof, wherein:
Y is a straight or branched (C₁-C₅)alkylene or cycloalkyl(C₁-C₅)alkylene, wherein one or more -CH₂- groups in Y are optionally and independently replaced with a moiety selected from the group consisting of O, S and NH;
Z is a bond, or CH₂;
V is O, NR⁸, or CR⁹R¹⁰, wherein X and V cannot be simultaneously O or S and O or NR⁸, respectively;
X is O, S, NR¹¹, CH₂, or CR¹²R¹³, wherein V and X cannot be simultaneously O and S, O or NR¹¹, respectively;
R¹ is hydrogen, C₃-C₆cycloalkyl or C₁-C₆alkyl or, taken with R² forms a C₃-C₈cycloalkyl, wherein the C₃-C₈cycloalkyl is unsubstituted or substituted with one to four substituents selected from the group consisting of halogen, C₁-C₆alkyl or OH;
R² is hydrogen, C₃-C₆cycloalkyl or C₁-C₆alkyl or, taken with R¹ forms a C₃-C₈cycloalkyl, wherein the C₃-C₈cycloalkyl is unsubstituted or substituted with one to four substituents selected from the group consisting of halogen, C₁-C₆alkyl or OH;
R³ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH, NH₂ or COOC₁-C₆alkyl, or taken with R⁴ forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl;
R⁴ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH, NH₂ or COOC₁-C₆alkyl or when taken with R³, R⁷ or R¹⁰, forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁶, R⁷ or R¹² forms an (C₁-C₅)alkylene bridge, wherein one or more -CH₂- groups are optionally and independently replaced with a moiety selected from the group consisting of O, S and NR¹⁴;
R⁵ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl or when taken with R⁶ forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl;
R⁶ is hydrogen, halogen, C₁-C₆alkyl, haloC₁-C₆alkyl, OH, C₁-C₆alkylOH, C₁-C₆alkylOC₁-C₆alkyl, COOH, C1-C6alkoxy, COOH or COOC₁-C₆alkyl or when taken with R⁵*,* R⁷ or R¹² forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁴ or R¹⁰ forms an (C₁-C₅)alkylene bridge, wherein one or more -CH₂- groups are optionally and independently replaced with a moiety selected from the group consisting of O, S and NR¹⁴;
R⁷ is hydrogen, halogen, C₁-C₆alkyl, C₃-C₆cycloalkyl, haloC₁-C₆alkyl, OH, C₁-C₆alkylOH, C₁-C₆alkylOC₁-C₆alkyl, COOH, C₁-C₆alkoxy or COOC₁-C₆alkyl or when taken with R⁴, or R⁶ forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, C₃-C₆cycloalkyl, oxo, C₁-C₆alkyl, C₁-C₆haloalkyl, COC₁-C₆alkyl, NH₂, C₁-C₆alkylNH₂, C₁-C₆alkylphenyl, CO(C₁-C₆alkyl)NH₂ and COOH, or taken with R⁴, R¹⁰ or R¹² forms an (C₁-C₅)alkylene bridge, wherein one or more -CH₂- groups are optionally and independently replaced with a moiety selected from the group consisting of O, S and NR¹⁴;
R⁸ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH or COC₁-C₆alkyl;
R⁹ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl;
R¹⁰ is hydrogen, halogen, C₁-C₆alkyl, haloC₁-C₆alkyl, OH, C₁-C₆alkylOH, C₁-C₆alkylOC₁-C₆alkyl, COOH, C1-C6alkoxy, COOH or COOC₁-C₆alkyl or when taken with R⁴ or R¹² forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl , or taken with R⁶ or R⁷ forms an (C₁-C₅)alkylene bridge, wherein one or more -CH₂- groups are optionally and independently replaced with a moiety selected from the group consisting of O, S and NH;
R¹¹ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH or COC₁-C₆alkyl;
R¹² is halogen, C₁-C₆alkyl, haloC₁-C₆alkyl, OH, C₁-C₆alkylOH, C₁-C₆alkylOC₁-C₆alkyl, COOH, C₁-C₆alkoxy, COOH or COOC₁-C₆alkyl or when taken with R⁶ or R¹⁰, forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁴ or R⁷ forms an (C₁-C₅)alkylene bridge, wherein one or more -CH₂- groups are optionally and independently replaced with a moiety selected from the group consisting of O, S and NH;
R¹³ is halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl; and
R¹⁴ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH, COC₁-C₆alkyl or COheterocycle wherein the heterocycle is a 3-7 membered nitrogen containing ring.

With regard to the compounds described herein, Y is a straight or branched (C₁-C₅)alkylene or cycloalkyl(C₁-C₅)alkylene, wherein one or more -CH₂- groups in Y are optionally and independently replaced with a moiety selected from the group consisting of O, S and NH.

In certain embodiments, Y is selected from the group consisting of straight or branched (C₁-C₅)alkylene, wherein one or more -CH₂- groups in Y are optionally and independently replaced with a moiety selected from the group consisting of O, S or NH. In certain embodiments, Y is selected from the group consisting of straight or branched (C₁-C₅)alkylene, wherein, when there is atleast two -CH₂- groups, one or more -CH₂- groups in Y are optionally and independently replaced with a moiety selected from the group consisting of O, S or NH. In certain embodiments, Y is methenyl, ethelenyl, propylenyl, butylenyl or pentylenyl. In certain embodiments Y is methenyl. In certain embodiments Y is ethelenyl. In certain embodiments Y is propylenyl. In other embodiments, one or more -CH₂- groups in Y are optionally and independently replaced with a moiety selected from the group consisting of O, S or NH. In certain embodiments, Y is (C₁-C₅)alkylene or O-C₁-C₄alkylene,

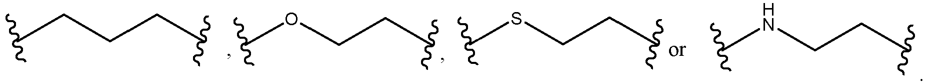

In certain embodiments, Y is a straight or branched cycloalkyl(C₁-C₅)alkylene, wherein one or more -CH₂- groups in Y are optionally and independently replaced with a moiety selected from the group consisting of O, S and NH. In certain embodiments, Y is 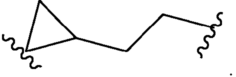 In certain embodiments, Y is 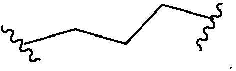 In certain embodiments, Y is 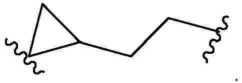 In certain embodiments, Y is

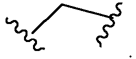

With regard to the compounds described herein, Z is a bond or CH₂. In certain embodiments, Z is a bond. In certain embodiments, Z is CH₂.

With regard to the compounds described herein, V is O, NR⁸, or CR⁹R¹⁰. In certain embodiments, V is O. In certain embodiments, V is NR⁸. In certain embodiments, V is CR⁹R¹⁰. In certain embodiments, V is CH₂.

With regard to the compounds described herein, X is O, S, NR¹¹, CH₂, or CR¹²R¹³. In certain embodiments, X is O. In certain embodiments, X is S. In certain embodiments, X is CH₂. In certain embodiments, X is NR¹¹. In certain embodiments, X is CR¹²R¹³.

In embodiments of the compounds described herein, V and X cannot be simultaneously O or S and O or NR¹¹. In embodiments of the compounds described herein, X and V cannot be simultaneously O or S and O or NR⁸.

With regard to the compounds described herein, R¹ is hydrogen, C₃-C₆cycloalkyl or C₁-C₆alkyl, or when taken with R² forms a C₃-C₈cycloalkyl, wherein the C₃-C₈cycloalkyl is unsubstituted or substituted with one to four substituents selected from the group consisting of halogen, C₁-C₆alkyl or -OH. In certain embodiments, R¹ is hydrogen. In certain embodiments, R¹ is C₃-C₆cycloalkyl. Suitable cycloalkyl groups include, but are not limited to, cyclopropyl, cyclobutyl, cyclopentyl, cyclohexyl. In certain embodiments, R¹ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R¹ and R² taken together form a C₃-C₈cycloalkyl, wherein the C₃-C₈cycloalkyl is unsubstituted or substituted with one to four substituents selected from the group consisting of halogen, C₁-C₆alkyl or -OH. In certain embodiments, the C₃-C₈cycloalkyl is a six or seven-membered carbon ring. In certain embodiments, the C₃-C₈cycloalkyl is a six or seven-membered, saturated carbon ring. In certain embodiments, the C₃-C₈cycloalkyl is a bridged ring. In certain embodiments, the C₃-C₈cycloalkyl is substituted with one substituent selected from the group consisting of halogen, C₁-C₆alkyl or -OH. In certain embodiments, the C₃-C₈cycloalkyl is substituted with two substituents selected from the group consisting of halogen, C₁-C₆alkyl or -OH. In certain embodiments, the C₃-C₈cycloalkyl is substituted with three substituents selected from the group consisting of halogen, C₁-C₆alkyl or -OH. In certain embodiments, the C₃-C₈cycloalkyl is substituted with four substituents selected from the group consisting of halogen, C₁-C₆alkyl or -OH. In certain embodiments, the C₃-C₈cycloalkyl is substituted with three substituents, wherein all the substituents are C₁-C₆alkyl groups. In certain embodiments, the C₃-C₈cycloalkyl is substituted with three substituents, wherein all the substituents are methyl. In certain embodiments, the C₃-C₈cycloalkyl is substituted with four substituents, wherein all the substituents are C₁-C₆alkyl groups. In certain embodiments, the C₃-C₈cycloalkyl is substituted with four substituents, wherein all the substituents are methyl.

In certain embodiments, R¹ and R², when taken together form a C₃-C₈cycloalkyl selected from the group consisting of:

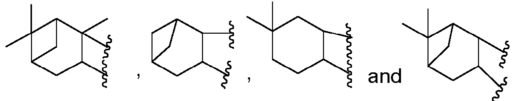

In certain embodiments, R¹ and R² when taken together form the following C₃-C₈cycloalkyl

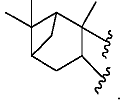

In certain embodiments, R¹ and R² when taken together along with the oxygens to which R¹ and R² are attached and the boron to which the oxygens are attached from a fused or multicyclic ring. Such an embodiment is shown in Example 9. With regard to the compounds described herein, R² is hydrogen, C₃-C₆cycloalkyl or C₁-C₆alkyl, or when taken with R¹ forms a C₃-C₈cycloalkyl, wherein the C₃-C₈cycloalkyl is unsubstituted or substituted with one to four substituents selected from the group consisting of halogen, C₁-C₆alkyl or -OH. In certain embodiments, R² is hydrogen. In certain embodiments, R² is C₃-C₆cycloalkyl. Suitable cycloalkyl groups include, but are not limited to, cyclopropyl, cyclobutyl, cyclopentyl, cyclohexyl. In certain embodiments, R² is C₁-C₆alkyl. Examples of suitable C₁-C₆alkyl groups can include but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R² when taken with R¹ forms a C₃-C₈cycloalkyl, wherein the C₃-C₈cycloalkyl is unsubstituted or substituted with one to four substituents selected from the group consisting of halogen, C₁-C₆alkyl or -OH. In certain embodiments, the C₃-C₈cycloalkyl is a six or seven carbon ring. In certain embodiments, the C₃-C₈cycloalkyl is a six or seven-membered saturated carbon ring. In certain embodiments, the C₃-C₈cycloalkyl is a bridged ring. In certain embodiments, the C₃-C₈cycloalkyl is substituted with one substituent selected from the group consisting of halogen, C₁-C₆alkyl or -OH. In certain embodiments, the C₃-C₈cycloalkyl is substituted with two substituents selected from the group consisting of halogen, C₁-C₆alkyl or -OH. In certain embodiments, the C₃-C₈cycloalkyl is substituted with three substituents selected from the group consisting of halogen, C₁-C₆alkyl or -OH. In certain embodiments, the C₃-C₈cycloalkyl is substituted with four substituents selected from the group consisting of halogen, C₁-C₆alkyl or -OH. In certain embodiments, the C₃-C₈cycloalkyl is substituted with three substituents, wherein all the substituents are C₁-C₆alkyl groups. In certain embodiments, the C₃-C₈cycloalkyl is substituted with three substituents, wherein all the substituents are methyl. In certain embodiments, the C₃-C₈cycloalkyl is substituted with four substituents, wherein all the substituents are C₁-C₆alkyl groups. In certain embodiments, the C₃-C₈cycloalkyl is substituted with four substituents, wherein all the substituents are methyl.

In certain embodiments, R¹ and R², when taken together form a C₃-C₈cycloalkyl selected from the group consisting of:

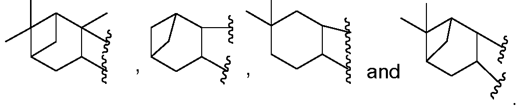

In certain embodiments, R¹ and R² when taken together form the following C₃-C₈cycloalkyl

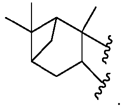

In certain embodiments, R¹ and R² are both hydrogen. In certain embodiments, R¹ and R² are each hydrogen or taken together form a pinane.

With regard to the compounds described herein, R³ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH, NH₂ or COOC₁-C₆alkyl, or taken with R⁴ forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R³ is hydrogen. In certain embodiments described herein, R³ is halogen. Examples of suitable halogens include, but are not limited to, chlorine, bromine, fluorine and iodine. In certain embodiments described herein, R³ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R³ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R³ is OH. In certain embodiments, R³ is C₁-C₆alkylOH. Examples of suitable alcohols, include, but are not limited to, methanol, ethanol, propanol, butanol and iso-butanol. In certain embodiments, R³ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R³ is COOH.

In certain embodiments, R³ is C₁-C₆alkoxy. Examples of suitable alkoxys, include but are not limited to, methoxy, ethoxy, butoxy and propoxy. In certain embodiments, R³ is COOC₁-C₆alkyl. Examples of suitable COOC₁-C₆alkyl groups include, but are not limited to, methoxycarbonyl, ethoxycarbonyl and butoxycarbonyl.

In certain embodiments, R³ is hydrogen or COOH. In certain embodiments, R³ is NH₂. In certain embodiments, R³ is hydrogen, NH₂ or COOH.

In certain embodiments, R³ is taken with R⁴ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R³ is taken with R⁴ and forms a C₃-C₆cycloalkyl. Suitable cycloalkyl groups include, but are not limited to, cyclopropyl, cyclobutyl, cyclopentyl, cyclohexyl. In certain embodiments, R³ is taken with R⁴ and forms a heterocycle. Suitable heterocycles include but are not limited to, nitrogen-containing heterocycles such as pyrrolidine and piperidine. In certain embodiments, R³ is taken with R⁴ to form a C₃-C₆cycloalkyl or heterocycle, wherein the C₃-C₆cycloalkyl or heterocycle is unsubstituted. In certain embodiment, R³ is taken with R⁴ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R³ is taken with R⁴ to form a pyrrolidine, wherein the pyrrolidine is substituted with COOH.

With regard to the compounds described herein, R⁴ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH, NH₂ or COOC₁-C₆alkyl or when taken with R³, R⁷ or R¹⁰, forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁶, R⁷ or R¹² forms an (C₁-C₅)alkylene bridge, wherein one or more -CH₂-groups are optionally and independently replaced with a moiety selected from the group consisting of O, S and NR¹⁴.

In certain embodiments, R⁴ is hydrogen. In certain embodiments described herein, R⁴ is halogen. Examples of suitable halogens include, but are not limited to, chlorine, bromine, fluorine and iodine. In certain embodiments described herein, R⁴ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R⁴ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R⁴ is OH. In certain embodiments, R⁴ is C₁-C₆alkylOH. Examples of suitable alcohols, include, but are not limited to, methanol, ethanol, propanol, butanol and iso-butanol. In certain embodiments, R⁴ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R⁴ is COOH.

In certain embodiments, R⁴ is C₁-C₆alkoxy. Examples of suitable alkoxys, include but are not limited to, methoxy, ethoxy, butoxy and propoxy. In certain embodiments, R⁴ is COOC₁-C₆alkyl. Examples of suitable COOC₁-C₆alkyl groups include, but are not limited to, methoxycarbonyl, ethoxycarbonyl and butoxycarbonyl.

In certain embodiments, R⁴ is hydrogen or COOH. In certain embodiments, R⁴ is NH₂. In certain embodiments, R⁴ is hydrogen, NH₂ or COOH.

In certain embodiments, R⁴ is taken with R³, R⁷ or R¹⁰ and forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁴ is taken with R³ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁴ is taken with R³ and forms a C₃-C₆cycloalkyl. Suitable cycloalkyl groups include, but are not limited to, cyclopropyl, cyclobutyl, cyclopentyl, cyclohexyl. In certain embodiments, R⁴ is taken with R³ and forms a heterocycle. Suitable heterocycles include but are not limited to, nitrogen-containing heterocycles such as pyrrolidine and piperidine. In certain embodiments, R⁴ is taken with R³ to form a C₃-C₆cycloalkyl or heterocycle, wherein the C₃-C₆cycloalkyl or heterocycle is unsubstituted. In certain embodiment, R⁴ is taken with R³ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁴ is taken with R³ to form a pyrrolidine, wherein the pyrrolidine is substituted with COOH.

In certain embodiments, R⁴ is taken with R⁷ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁴ is taken with R⁷ and forms a C₃-C₆cycloalkyl. Suitable cycloalkyl groups include, but are not limited to, cyclopropyl, cyclobutyl, cyclopentyl, cyclohexyl. In certain embodiments, R⁴ is taken with R⁷ and forms a heterocycle. Suitable heterocycles include, but are not limited to, pyrrolidine, azetidine, thiolane, thietane, oxetane, tetrahyrdrofurane and piperidine. Suitable heterocycles include but are not limited to, nitrogen-containing heterocycles such as pyrrolidine and piperidine. In certain embodiments, R⁴ is taken with R⁷ to form a C₃-C₆cycloalkyl or heterocycle, wherein the C₃-C₆cycloalkyl or heterocycle is unsubstituted. In certain embodiment, R⁴ is taken with R⁷ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁴ is taken with R⁷ to form a pyrrolidine, wherein the pyrrolidine is unsubstituted or substituted with 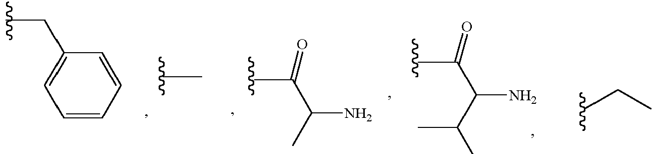 or

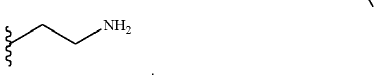

In certain embodiments, R⁴ is taken with R⁷ to form a piperidine, wherein the piperidine is unsubstituted or substituted with

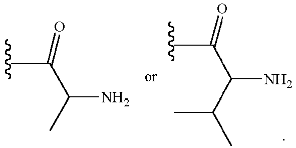

In certain embodiments, R⁴ is taken with R⁷ to form thietane. In certain embodiments, R⁴ is taken with R⁷ to form oxetane. In certain embodiments, R⁴ is taken with R⁷ to form thiolane. In certain embodiments, R⁴ is taken with R⁷ to form tetrahydrofurane.

In certain embodiments, R⁴ is taken with R⁷ to form: 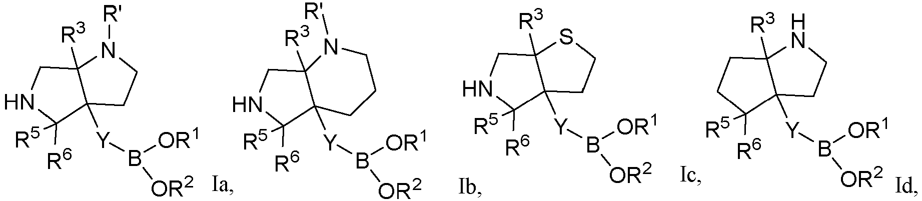 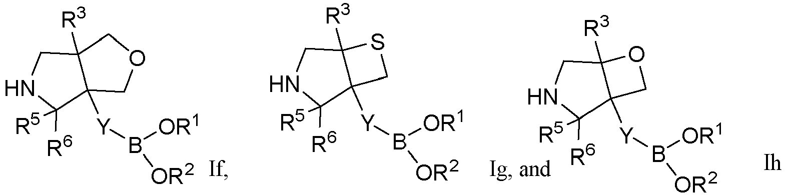 wherein, R' is halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁴ is taken with R¹⁰ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁴ is taken with R¹⁰ and forms a C₃-C₆cycloalkyl. Suitable cycloalkyl groups include, but are not limited to, cyclopropyl, cyclobutyl, cyclopentyl, cyclohexyl. In certain embodiments, R⁴ is taken with R¹⁰ and forms a heterocycle. Suitable heterocycles include but are not limited to, nitrogen-containing heterocycles such as pyrrolidine and piperidine. In certain embodiments, R⁴ is taken with R¹⁰ to form a C₃-C₆cycloalkyl or heterocycle, wherein the C₃-C₆cycloalkyl or heterocycle is unsubstituted. In certain embodiment, R⁴ is taken with R¹⁰ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁴ is taken with R¹⁰ to form a cyclopropyl or cyclobutyl, wherein the cyclopropyl or cyclobutyl is unsubstituted. In certain embodiments, R¹⁰ is taken with R⁴ to form a cyclopropyl or cyclobutyl, wherein the cyclopropyl or cyclobutyl is substituted with OH.

In certain embodiments, R⁴ is taken with R⁶, R⁷ or R¹² to form an (C₁-C₅)alkylene bridge, wherein one or more -CH₂- groups are optionally and independently replaced with a moiety selected from the group consisting of O, S and NR¹⁴. In certain embodiments, R⁴, when taken with R⁶ forms a (C₁-C₅)alkylene bridge. Examples of suitable bridges include, but are not limited to, 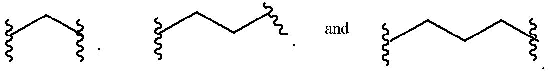 In certain embodiments, R⁴, when taken with R⁶ forms a (C₁-C₅)alkylene bridge, wherein the bridge is

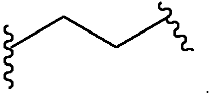

In certain embodiments, R⁴, when taken with R⁶ forms a (C₁-C₅)alkylene bridge, wherein one or more -CH₂- groups are optionally and independently replaced with NR¹⁴. Examples of suitable bridges include, but are not limited to,

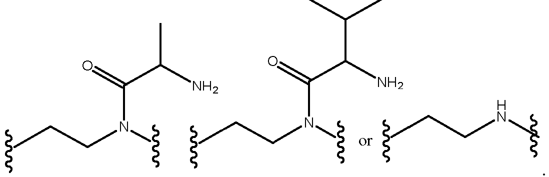

In certain embodiments, R⁴ is taken with R⁷ to form an (C₁-C₅)alkylene bridge, wherein one or more -CH₂- groups are optionally and independently replaced with a moiety selected from the group consisting of O, S and NR¹⁴.

In certain embodiments, R⁴ is taken with R¹² to form an (C₁-C₅)alkylene bridge, wherein one or more -CH₂- groups are optionally and independently replaced with a moiety selected from the group consisting of O, S and NR¹⁴. In certain embodiments, R⁴, when taken with R¹² forms a (C₁-C₅)alkylene bridge. Examples of suitable bridges include, but are not limited to, 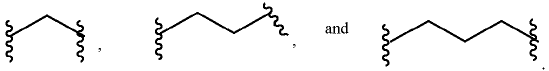 In certain embodiments, R⁴, when taken with R¹² forms a (C₁-C₅)alkylene bridge, wherein the bridge is

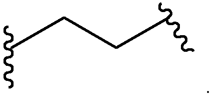

In certain embodiments, R⁴, when taken with R¹² forms a (C₁-C₅)alkylene bridge, wherein one or more -CH₂- groups are optionally and independently replaced with NR¹⁴. Examples of suitable bridges include, but are not limited to,

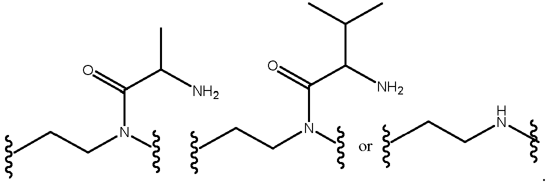

With regard to the compounds described herein, R⁵ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl, or taken with R⁶ forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁵ is hydrogen. In certain embodiments described herein, R⁵ is halogen. Examples of suitable halogens include, but are not limited to, chlorine, bromine, fluorine and iodine. In certain embodiments described herein, R⁵ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R⁵ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R⁵ is OH. In certain embodiments, R⁵ is C₁-C₆alkylOH. Examples of suitable alcohols, include, but are not limited to, methanol, ethanol, propanol, butanol and iso-butanol. In certain embodiments, R⁵ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R⁵ is COOH.

In certain embodiments, R⁵ is C₁-C₆alkoxy. Examples of suitable alkoxys, include but are not limited to, methoxy, ethoxy, butoxy and propoxy. In certain embodiments, R⁵ is COOC₁-C₆alkyl. Examples of suitable COOC₁-C₆alkyl groups include, but are not limited to, methoxycarbonyl, ethoxycarbonyl and butoxycarbonyl.

In certain embodiments, R⁵ is taken with R⁶ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁵ is taken with R⁶ and forms a C₃-C₆cycloalkyl. Suitable cycloalkyl groups include, but are not limited to, cyclopropyl, cyclobutyl, cyclopentyl, cyclohexyl. In certain embodiments, R⁵ is taken with R⁶ and forms a heterocycle. Suitable heterocycles include but are not limited to, nitrogen-containing heterocycles such as pyrrolidine and piperidine. In certain embodiments, R⁵ is taken with R⁶ to form a C₃-C₆cycloalkyl or heterocycle, wherein the C₃-C₆cycloalkyl or heterocycle is unsubstituted. In certain embodiment, R⁵ is taken with R⁶ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁵ is taken with R⁶ to form a pyrrolidine, wherein the pyrrolidine is substituted with COOH.

With regard to the compounds described herein, R⁶ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl or when taken with R⁵*,* R⁷ or R¹², forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁴ or R¹⁰ forms an (C₁-C₅)alkylene bridge, wherein one or more -CH₂-groups are optionally and independently replaced with a moiety selected from the group consisting of O, S and NR¹⁴.

In certain embodiments, R⁶ is hydrogen. In certain embodiments described herein, R⁶ is halogen. Examples of suitable halogens include, but are not limited to, chlorine, bromine, fluorine and iodine. In certain embodiments described herein, R⁶ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R⁶ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R⁶ is OH. In certain embodiments, R⁶ is C₁-C₆alkylOH. Examples of suitable alcohols, include, but are not limited to, methanol, ethanol, propanol, butanol and iso-butanol. In certain embodiments, R⁶ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R⁶ is COOH.

In certain embodiments, R⁶ is C₁-C₆alkoxy. Examples of suitable alkoxys, include but are not limited to, methoxy, ethoxy, butoxy and propoxy. In certain embodiments, R⁶ is COOC₁-C₆alkyl. Examples of suitable COOC₁-C₆alkyl groups include, but are not limited to, methoxycarbonyl, ethoxycarbonyl and butoxycarbonyl.

In certain embodiments, R⁶ is taken with R⁵*,* R⁷ or R¹² and forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁶ is taken with R⁵ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁶ is taken with R⁵ and forms a C₃-C₆cycloalkyl. Suitable cycloalkyl groups include, but are not limited to, cyclopropyl, cyclobutyl, cyclopentyl, cyclohexyl. In certain embodiments, R⁶ is taken with R⁵ and forms a heterocycle. Suitable heterocycles include but are not limited to, nitrogen-containing heterocycles such as pyrrolidine and piperidine. In certain embodiments, R⁶ is taken with R⁵ to form a C₃-C₆cycloalkyl or heterocycle, wherein the C₃-C₆cycloalkyl or heterocycle is unsubstituted. In certain embodiment, R⁶ is taken with R⁵ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁶ is taken with R⁵ to form a pyrrolidine, wherein the pyrrolidine is substituted with COOH.

In certain embodiments, R⁶ is taken with R⁷ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁶ is taken with R⁷ and forms a C₃-C₆cycloalkyl. Suitable cycloalkyl groups include, but are not limited to, cyclopropyl, cyclobutyl, cyclopentyl, cyclohexyl. In certain embodiments, R⁶ is taken with R⁷ and forms a heterocycle. Suitable heterocycles include, but are not limited to, pyrrolidine, azetidine, thiolane, thietane, oxetane, tetrahyrdrofurane and piperidine. Suitable heterocycles include but are not limited to, nitrogen-containing heterocycles such as pyrrolidine and piperidine. In certain embodiments, R⁶ is taken with R⁷ to form a cyclopentyl ring. In certain embodiments, R⁶ is taken with R⁷ to form a C₃-C₆cycloalkyl or heterocycle, wherein the C₃-C₆cycloalkyl or heterocycle is unsubstituted. In certain embodiment, R⁶ is taken with R⁷ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁶ is taken with R¹² to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁶ is taken with R¹² and forms a C₃-C₆cycloalkyl. Suitable cycloalkyl groups include, but are not limited to, cyclopropyl, cyclobutyl, cyclopentyl, cyclohexyl. In certain embodiments, R⁶ is taken with R¹² and forms a heterocycle. Suitable heterocycles include but are not limited to, nitrogen-containing heterocycles such as pyrrolidine and piperidine. In certain embodiments, R⁶ is taken with R¹² to form a piperidine. In certain embodiments, R⁶ is taken with R¹² to form a C₃-C₆cycloalkyl or heterocycle, wherein the C₃-C₆cycloalkyl or heterocycle is unsubstituted. In certain embodiment, R⁶ is taken with R¹² to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁶ is taken with R⁴ or R¹⁰ to form an (C₁-C₅)alkylene bridge, wherein one or more -CH₂- groups are optionally and independently replaced with a moiety selected from the group consisting of O, S and NR¹⁴. In certain embodiments, R⁶, when taken with R⁴ forms a (C₁-C₅)alkylene bridge. Examples of suitable bridges include, but are not limited to, 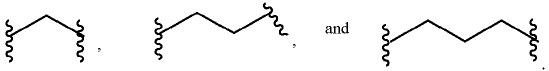 In certain embodiments, R⁶, when taken with R⁴ forms a (C₁-C₅)alkylene bridge, wherein one or more -CH₂- groups is independently replaced with NR¹⁴.

In certain embodiments, R⁶, when taken with R⁴ forms a (C₁-C₅)alkylene bridge, wherein the bridge is

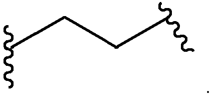

In certain embodiments, R⁶, when taken with R⁴ forms a (C₁-C₅)alkylene bridge, wherein one or more -CH₂- groups are optionally and independently replaced with NR¹⁴. Examples of suitable bridges include, but are not limited to,

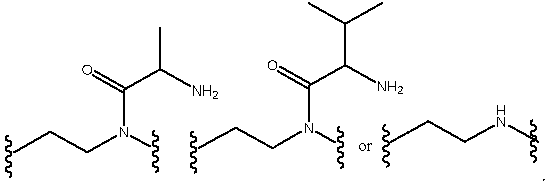

In certain embodiments, R⁶ is taken with R¹⁰ to form an (C₂-C₅)alkylene bridge, wherein one or more -CH₂- groups are optionally and independently replaced with a moiety selected from the group consisting of O, S and NR¹⁴. In certain embodiments, R⁶, when taken with R¹⁰ forms a (C₁-C₅)alkylene bridge. Examples of suitable bridges include, but are not limited to, 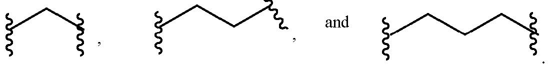 In certain embodiments, R⁶, when taken with R¹⁰ forms a (C₁-C₅)alkylene bridge, wherein the bridge is

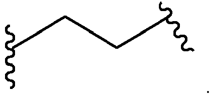

With regard to the compounds described herein, R⁷ is hydrogen, halogen, C₁-C₆alkyl, C₃-C₆cycloalkyl, haloC₁-C₆alkyl, OH, C₁-C₆alkylOH, C₁-C₆alkylOC₁-C₆alkyl, C₁-C₆alkoxy, COOH or COOC₁-C₆alkyl or when taken with R⁴ or R⁶, forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁴, R¹⁰ or R¹² forms an (C₁-C₅)alkylene bridge, wherein one or more -CH₂- groups are optionally and independently replaced with a moiety selected from the group consisting of O, S and NR¹⁴.

In certain embodiments, R⁷ is hydrogen. In certain embodiments described herein, R⁷ is halogen. Examples of suitable halogens include, but are not limited to, chlorine, bromine, fluorine and iodine. In certain embodiments described herein, R⁷ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R⁷ is C₃-C₆cycloalkyl. Suitable cycloalkyl groups include, but are not limited to, cyclopropyl, cyclobutyl, cyclopentyl, cyclohexyl. In certain embodiments, R⁷ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R⁷ is OH. In certain embodiments, R⁷ is C₁-C₆alkylOH. Examples of suitable alcohols, include, but are not limited to, methanol, ethanol, propanol, butanol and iso-butanol. In certain embodiments, R⁷ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R⁷ is COOH.

In certain embodiments, R⁷ is C₁-C₆alkoxy. Examples of suitable alkoxys, include but are not limited to, methoxy, ethoxy, butoxy and propoxy. In certain embodiments, R⁷ is COOC₁-C₆alkyl. Examples of suitable COOC₁-C₆alkyl groups include, but are not limited to, methoxycarbonyl, ethoxycarbonyl and butoxycarbonyl.

In certain embodiments, R⁷ is methyl.

In certain embodiments, R⁷ is taken with R⁴ or R⁶ and forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁷ is taken with R⁴ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁷ is taken with R⁴ and forms a C₃-C₆cycloalkyl. Suitable cycloalkyl groups include, but are not limited to, cyclopropyl, cyclobutyl, cyclopentyl, cyclohexyl. In certain embodiments, R⁷ is taken with R⁴ and forms a heterocycle. Suitable heterocycles include, but are not limited to, pyrrolidine, azetidine, thiolane, thietane, oxetane, tetrahyrdrofurane and piperidine. Suitable heterocycles include but are not limited to, nitrogen-containing heterocycles such as pyrrolidine and piperidine. In certain embodiments, R⁴ is taken with R⁷ to form a C₃-C₆cycloalkyl or heterocycle, wherein the C₃-C₆cycloalkyl or heterocycle is unsubstituted. In certain embodiment, R⁷ is taken with R⁴ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁷ is taken with R⁴ to form a pyrrolidine, wherein the pyrrolidine is unsubstituted or substituted with 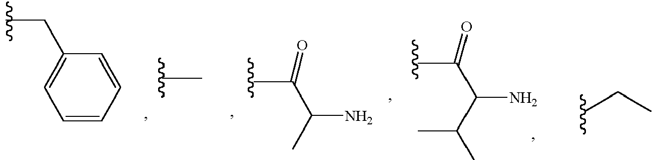 or

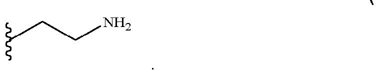

In certain embodiments, R⁷ is taken with R⁴ to form a piperidine, wherein the piperidine is unsubstituted or substituted with

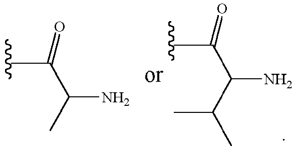

In certain embodiments, R⁷ is taken with R⁴ to form thietane. In certain embodiments, R⁷ is taken with R⁴ to form oxetane. In certain embodiments, R⁷ is taken with R⁴ to form thiolane. In certain embodiments, R⁷ is taken with R⁴ to form tetrahydrofurane.

In certain embodiments, R⁷ is taken with R⁴ to form: 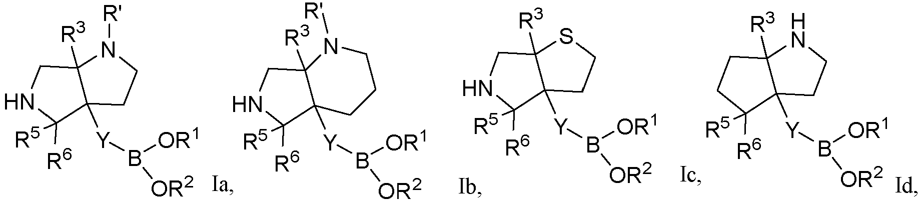 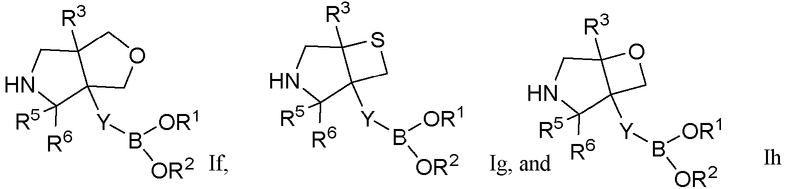 wherein, R' is halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R⁷ is taken with R⁶ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁷ is taken with R⁶ and forms a C₃-C₆cycloalkyl. Suitable cycloalkyl groups include, but are not limited to, cyclopropyl, cyclobutyl, cyclopentyl, cyclohexyl. In certain embodiments, R⁷ is taken with R⁶ and forms a heterocycle. Suitable heterocycles include, but are not limited to, pyrrolidine, azetidine, thiolane, thietane, oxetane, tetrahyrdrofurane and piperidine. Suitable heterocycles include but are not limited to, nitrogen-containing heterocycles such as pyrrolidine and piperidine. In certain embodiments, R⁷ is taken with R⁶ to form a C₃-C₆cycloalkyl or heterocycle, wherein the C₃-C₆cycloalkyl or heterocycle is unsubstituted. In certain embodiment, R⁷ is taken with R⁶ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R⁷ is taken with R⁶ to form a cyclopentyl.

In certain embodiments, R⁷ is taken with R⁴, R¹⁰ or R¹² to form an (C₁-C₅)alkylene bridge, wherein one or more -CH₂- groups are optionally and independently replaced with a moiety selected from the group consisting of O, S and NR¹⁴. In certain embodiments, R⁷, when taken with R⁴ forms a (C₁-C₅)alkylene bridge. Examples of suitable bridges include, but are not limited to, 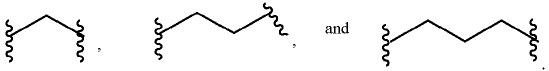 In certain embodiments, R⁷, when taken with R⁴ forms a (C₁-C₅)alkylene bridge, wherein one or more -CH₂- groups is independently replaced with NR¹⁴. Examples of suitable bridges include, but are not limited to, 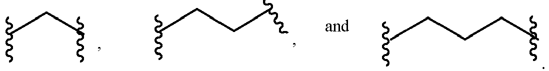 In certain embodiments, R⁷, when taken with R⁴ forms a (C₁-C₅)alkylene bridge, wherein the bridge is

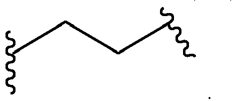

In certain embodiments, R⁷ is taken with R¹⁰ to form an (C₁-C₅)alkylene bridge, wherein one or more -CH₂- groups are optionally and independently replaced with a moiety selected from the group consisting of O, S and NR¹⁴. In certain embodiments, R⁷, when taken with R¹⁰ forms a (C₁-C₅)alkylene bridge. Examples of suitable bridges include, but are not limited to, 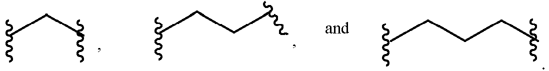 In certain embodiments, R⁷, when taken with R¹⁰ forms a (C₁-C₅)alkylene bridge, wherein the bridge is

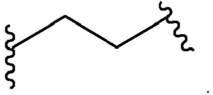

In certain embodiments, R⁷ is taken with R¹² to form an (C₁-C₅)alkylene bridge, wherein one or more -CH₂- groups are optionally and independently replaced with a moiety selected from the group consisting of O, S and NR¹⁴. In certain embodiments, R⁷, when taken with R¹² forms a (C₁-C₅)alkylene bridge. Examples of suitable bridges include, but are not limited to, 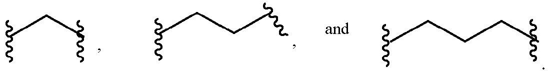 In certain embodiments, R⁷, when taken with R¹² forms a (C₁-C₅)alkylene bridge, wherein the bridge is

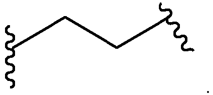

With regard to the compounds described herein, R⁸ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH or COC₁-C₆alkyl.

In certain embodiments described herein, R⁸ is hydrogen. In certain embodiments, R⁸ is C₃-C₆cycloalkyl. Suitable cycloalkyl groups include, but are not limited to, cyclopropyl, cyclobutyl, cyclopentyl, cyclohexyl. In certain embodiments described herein, R⁸ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R⁸ is C₁-C₆aryl. Suitable examples include, but are not limited to, CH₂phenyl. In certain embodiments, R⁸ is C₁-C₆alkylNH₂. Examples of suitable C₁-C₆alkylNH₂ groups include, but are not limited to, CH₂NH₂, CH₂ CH₂NH₂ and CH₂CH₂CH₂NH₂. In certain embodiments, R⁸ is COC₁-C₆alkylNH₂. In certain embodiments, R⁸ is COC₁-C₆alkylNH(C₁-C₆alkyl). In certain embodiments, R⁸ is COC₁-C₆alkylN(C₁-C₆alkyl)₂.

In certain embodiments, R⁸ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R⁸ is C₁-C₆alkylOH. Examples of suitable alcohols, include, but are not limited to, methanol, ethanol, propanol, butanol and iso-butanol. In certain embodiments, R⁸ is COC₁-C₆alkyl.

With regard to the compounds described herein, R⁹ is halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl.

In certain embodiments described herein, R⁹ is halogen. Examples of suitable halogens include, but are not limited to, chlorine, bromine, fluorine and iodine. In certain embodiments described herein, R⁹ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R⁹ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R⁹ is OH. In certain embodiments, R⁹ is C₁-C₆alkylOH. Examples of suitable alcohols, include, but are not limited to, methanol, ethanol, propanol, butanol and iso-butanol. In certain embodiments, R⁹ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R⁹ is COOH.

In certain embodiments, R⁹ is C₁-C₆alkoxy. Examples of suitable alkoxys, include but are not limited to, methoxy, ethoxy, butoxy and propoxy. In certain embodiments, R⁹ is COOC₁-C₆alkyl. Examples of suitable COOC₁-C₆alkyl groups include, but are not limited to, methoxycarbonyl, ethoxycarbonyl and butoxycarbonyl.

With regard to the compounds described herein, R¹⁰ is halogen, C₁-C₆alkyl, haloC₁-C₆alkyl, OH, C₁-C₆alkylOH, C₁-C₆alkylOC₁-C₆alkyl, COOH, C₁-C₆alkoxy, or COOC₁-C₆alkyl or when taken with R⁴ or R¹², forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁶ or R⁷ forms an (C₁-C₅)alkylene bridge, wherein one or more -CH₂- groups are optionally and independently replaced with a moiety selected from the group consisting of O, S and NR¹⁴.

In certain embodiments described herein, R¹⁰ is halogen. Examples of suitable halogens include, but are not limited to, chlorine, bromine, fluorine and iodine. In certain embodiments described herein, R¹⁰ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R¹⁰ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R¹⁰ is OH. In certain embodiments, R¹⁰ is C₁-C₆alkylOH. Examples of suitable alcohols, include, but are not limited to, methanol, ethanol, propanol, butanol and iso-butanol. In certain embodiments, R¹⁰ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R¹⁰ is COOH.

In certain embodiments, R¹⁰ is C₁-C₆alkoxy. Examples of suitable alkoxys, include but are not limited to, methoxy, ethoxy, butoxy and propoxy. In certain embodiments, R¹⁰ is COOC₁-C₆alkyl. Examples of suitable COOC₁-C₆alkyl groups include, but are not limited to, methoxycarbonyl, ethoxycarbonyl and butoxycarbonyl.

In certain embodiments, R¹⁰ is taken with R⁴ or R¹² and forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R¹⁰ is taken with R⁴ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R¹⁰ is taken with R⁴ and forms a C₃-C₆cycloalkyl. Suitable cycloalkyl groups include, but are not limited to, cyclopropyl, cyclobutyl, cyclopentyl, cyclohexyl. In certain embodiments, R¹⁰ is taken with R⁴ and forms a heterocycle. Suitable heterocycles include but are not limited to, nitrogen-containing heterocycles such as pyrrolidine and piperidine. In certain embodiments, R¹⁰ is taken with R⁴ to form a C₃-C₆cycloalkyl or heterocycle, wherein the C₃-C₆cycloalkyl or heterocycle is unsubstituted. In certain embodiment, R¹⁰ is taken with R⁴ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R¹⁰ is taken with R⁴ to form a cyclopropyl or cyclobutyl, wherein the cyclopropyl or cyclobutyl is unsubstituted. In certain embodiments, R¹⁰ is taken with R⁴ to form a cyclopropyl or cyclobutyl, wherein the cyclopropyl or cyclobutyl is substituted with OH.

In certain embodiments, R¹⁰ is taken with R¹² to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. Suitable cycloalkyl groups include, but are not limited to, cyclopropyl, cyclobutyl, cyclopentyl, cyclohexyl. In certain embodiments, R¹⁰ is taken with R¹² and forms a heterocycle. Suitable heterocycles include, but are not limited to, pyrrolidine, azetidine, thiolane, thietane, oxetane, tetrahyrdrofurane and piperidine. Suitable heterocycles include but are not limited to, nitrogen-containing heterocycles such as pyrrolidine and piperidine. In certain embodiments, R¹⁰ is taken with R¹² to form a C₃-C₆cycloalkyl or heterocycle, wherein the C₃-C₆cycloalkyl or heterocycle is unsubstituted. In certain embodiment, R¹⁰ is taken with R¹² to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R¹⁰ is taken with R¹² to form a cyclopropyl or cyclobutyl, wherein the cyclopropyl or cyclobutyl is unsubstituted. In certain embodiments, R¹⁰ is taken with R¹² to form a cyclopropyl or cyclobutyl, wherein the cyclopropyl or cyclobutyl is substituted with OH.

In certain embodiments, R¹⁰ is taken with R⁶ or R⁷ to form an (C₁-C₅)alkylene bridge, wherein one or more -CH₂- groups are optionally and independently replaced with a moiety selected from the group consisting of O, S and NR¹⁴. In certain embodiments, R¹⁰, when taken with R⁶ forms a (C₁-C₅)alkylene bridge. Examples of suitable bridges include, but are not limited to, 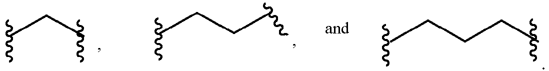 In certain embodiments, R¹⁰, when taken with R⁶ forms a (C₁-C₅)alkylene bridge, wherein the bridge is

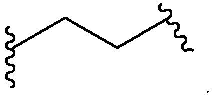

In certain embodiments, R¹⁰ is taken with R⁷ to form an (C₁-C₅)alkylene bridge, wherein one or more -CH₂- groups are optionally and independently replaced with a moiety selected from the group consisting of O, S and NR¹⁴. In certain embodiments, R¹⁰, when taken with R⁷ forms a (C₁-C₅)alkylene bridge. Examples of suitable bridges include, but are not limited to, 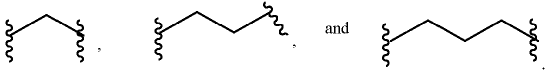 In certain embodiments, R¹⁰, when taken with R⁷ forms a (C₁-C₅)alkylene bridge, wherein the bridge is

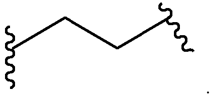

With regard to the compounds described herein, R¹¹ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH or COC₁-C₆alkyl.

In certain embodiments described herein, R¹¹ is hydrogen. In certain embodiments, R¹¹ is C₃-C₆cycloalkyl. Suitable cycloalkyl groups include, but are not limited to, cyclopropyl, cyclobutyl, cyclopentyl, cyclohexyl. In certain embodiments described herein, R¹¹ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R¹¹ is C₁-C₆aryl. Suitable examples include, but are not limited to, CH₂phenyl. In certain embodiments, R¹¹ is C₁-C₆alkylNH₂. Examples of suitable C₁-C₆alkylNH₂ groups include, but are not limited to, CH₂NH₂, CH₂ CH₂NH₂ and CH₂CH₂CH₂NH₂. In certain embodiments, R¹¹ is COC₁-C₆alkylNH₂. In certain embodiments, R¹¹ is COC₁-C₆alkylNH(C₁-C₆alkyl). In certain embodiments, R¹¹ is COC₁-C₆alkylN(C₁-C₆alkyl)₂.

In certain embodiments, R¹¹ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R¹¹ is C₁-C₆alkylOH. Examples of suitable alcohols, include, but are not limited to, methanol, ethanol, propanol, butanol and iso-butanol. In certain embodiments, R¹¹ is COC₁-C₆alkyl.

With regard to the compounds described herein, R¹² is halogen, C₁-C₆alkyl, haloC₁-C₆alkyl, OH, C₁-C₆alkylOH, C₁-C₆alkylOC₁-C₆alkyl, COOH, C₁-C₆alkoxy, or COOC₁-C₆alkyl or when taken with R⁶ or R¹⁰, forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl, or taken with R⁶ or R⁷ forms an (C₁-C₅)alkylene bridge, wherein one or more -CH₂- groups are optionally and independently replaced with a moiety selected from the group consisting of O, S and NR¹⁴.

In certain embodiments described herein, R¹² is halogen. Examples of suitable halogens include, but are not limited to, chlorine, bromine, fluorine and iodine. In certain embodiments described herein, R¹² is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R¹² is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R¹² is OH. In certain embodiments, R¹² is C₁-C₆alkylOH. Examples of suitable alcohols, include, but are not limited to, methanol, ethanol, propanol, butanol and iso-butanol. In certain embodiments, R¹² is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R¹² is COOH.

In certain embodiments, R¹² is C₁-C₆alkoxy. Examples of suitable alkoxys, include but are not limited to, methoxy, ethoxy, butoxy and propoxy. In certain embodiments, R¹² is COOC₁-C₆alkyl. Examples of suitable COOC₁-C₆alkyl groups include, but are not limited to, methoxycarbonyl, ethoxycarbonyl and butoxycarbonyl.

In certain embodiments, R¹² is taken with R⁶ or R¹⁰ and forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R¹² is taken with R⁶ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. Suitable cycloalkyl groups include, but are not limited to, cyclopropyl, cyclobutyl, cyclopentyl, cyclohexyl. In certain embodiments, R¹² is taken with R⁶ and forms a heterocycle. Suitable heterocycles include but are not limited to, nitrogen-containing heterocycles such as pyrrolidine and piperidine. In certain embodiments, R¹² is taken with R⁶ to form a C₃-C₆cycloalhkyl or heterocycle, wherein the C₃-C₆cycloalkyl or heterocycle is unsubstituted. In certain embodiment, R¹² is taken with R⁶ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl.

In certain embodiments, R¹² is taken with R¹⁰ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R¹² is taken with R¹⁰ and forms a C₃-C₆cycloalkyl. Suitable cycloalkyl groups include, but are not limited to, cyclopropyl, cyclobutyl, cyclopentyl, cyclohexyl. In certain embodiments, R¹² is taken with R¹⁰ and forms a heterocycle. Suitable heterocycles include, but are not limited to, pyrrolidine, azetidine, thiolane, thietane, oxetane, tetrahyrdrofurane and piperidine. Suitable heterocycles include but are not limited to, nitrogen-containing heterocycles such as pyrrolidine and piperidine. In certain embodiments, R¹² is taken with R¹⁰ to form a C₃-C₆cycloalkyl or heterocycle, wherein the C₃-C₆cycloalkyl or heterocycle is unsubstituted. In certain embodiment, R¹² is taken with R¹⁰ to form a C₃-C₆cycloalkyl or heterocycle, wherein the C₃-C₆cycloalkyl or heterocycle is substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl. In certain embodiments, R¹² is taken with R¹⁰ to form a cyclopentyl.

In certain embodiments, R¹² is taken with R⁴ or R⁷ to form an (C₁-C₅)alkylene bridge, wherein one or more -CH₂- groups are optionally and independently replaced with a moiety selected from the group consisting of O, S and NR¹⁴. In certain embodiments, R¹², when taken with R⁴ forms a (C₁-C₅)alkylene bridge. Examples of suitable bridges include, but are not limited to, 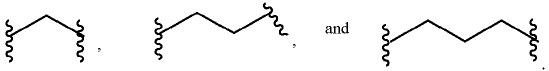 In certain embodiments, R¹², when taken with R⁴ forms a (C₁-C₅)alkylene bridge, wherein the bridge is

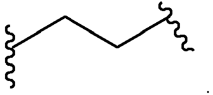

In certain embodiments, R¹² is taken with R⁷ to form an (C₁-C₅)alkylene bridge, wherein one or more -CH₂- groups are optionally and independently replaced with a moiety selected from the group consisting of O, S and NR¹⁴. In certain embodiments, R¹², when taken with R⁷ forms a (C₁-C₅)alkylene bridge. Examples of suitable bridges include, but are not limited to, 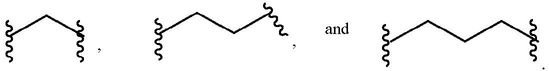 In certain embodiments, R¹², when taken with R⁷ forms a (C₁-C₅)alkylene bridge, wherein the bridge is

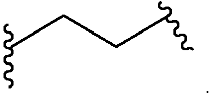

With regard to the compounds described herein, R¹³ is halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl.

In certain embodiments described herein, R¹³ is halogen. Examples of suitable halogens include, but are not limited to, chlorine, bromine, fluorine and iodine. In certain embodiments described herein, R¹³ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R¹³ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R¹³ is OH. In certain embodiments, R¹³ is C₁-C₆alkylOH. Examples of suitable alcohols, include, but are not limited to, methanol, ethanol, propanol, butanol and iso-butanol. In certain embodiments, R¹³ is C₁-C₆alkylOC₁-C₆alkyl. Suitable C₁-C₆alkylOC₁-C₆alkyls include but are not limited to, dimethyl ether, ethyl methyl ether, diethyl ether, dipropyl ether, dibutyl ether and diisopropyl ether. In certain embodiments, R¹³ is COOH.

In certain embodiments, R¹³ is C₁-C₆alkoxy. Examples of suitable alkoxys, include but are not limited to, methoxy, ethoxy, butoxy and propoxy. In certain embodiments, R¹³ is COOC₁-C₆alkyl. Examples of suitable COOC₁-C₆alkyl groups include, but are not limited to, methoxycarbonyl, ethoxycarbonyl and butoxycarbonyl.

With regard to the compounds described herein, R¹⁴ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH, COC₁-C₆alkyl or COheterocycle wherein the heterocycle is a 3-7 membered nitrogen containing ring.

In certain embodiments described herein, R¹⁴ is hydrogen. In certain embodiments, R¹⁴ is C₃-C₆cycloalkyl. Suitable cycloalkyl groups include, but are not limited to, cyclopropyl, cyclobutyl, cyclopentyl, cyclohexyl. In certain embodiments described herein, R¹⁴ is C₁-C₆alkyl. Examples of suitable C₁-C₆alkyl groups can include, but are not limited to, methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl and 1-ethyl-1-methylpropyl.

In certain embodiments, R¹⁴ is C₁-C₆aryl. Suitable examples include, but are not limited to, CH₂phenyl. In certain embodiments, R¹⁴ is C₁-C₆alkylNH₂. Examples of suitable C₁-C₆alkylNH₂ groups include, but are not limited to, CH₂NH₂, CH₂ CH₂NH₂ and CH₂CH₂CH₂NH₂. In certain embodiments, R¹⁴ is COC₁-C₆alkylNH₂. In certain embodiments, R¹⁴ is COC₁-C₆alkylNH(C₁-C₆alkyl). In certain embodiments, R¹⁴ is COC₁-C₆alkylN(C₁-C₆alkyl)₂.

In certain embodiments, R¹⁴ is haloC₁-C₆alkyl. Suitable haloC₁-C₆alkyls include but are not limited to, fluoromethyl, difluoromethyl, trifluoromethyl, 2-fluoroethyl, 1,2-difluoroethyl and 2,2-difluoroethyl. In certain embodiments, R¹⁴ is C₁-C₆alkylOH. Examples of suitable alcohols, include, but are not limited to, methanol, ethanol, propanol, butanol and iso-butanol. In certain embodiments, R¹⁴ is COC₁-C₆alkyl.

In certain embodiments, R¹⁴ is COheterocycle wherein the heterocycle is a 3-7 membered nitrogen containing ring. Examples include but are not limited to,

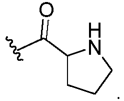

In certain embodiments, described herein are the following compounds: 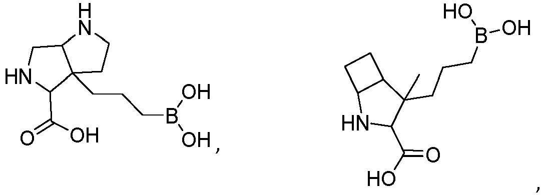 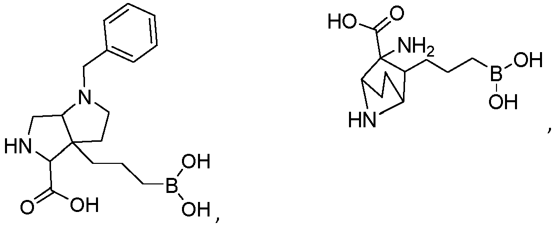 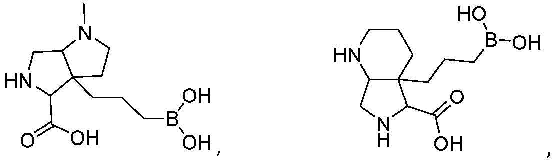 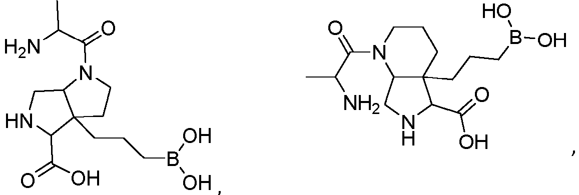 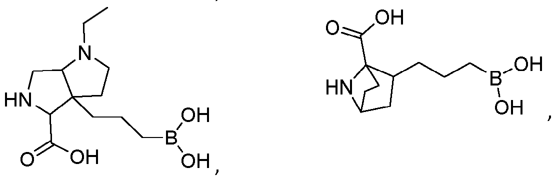 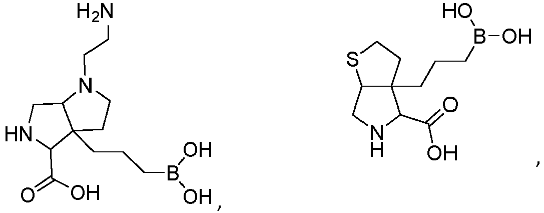 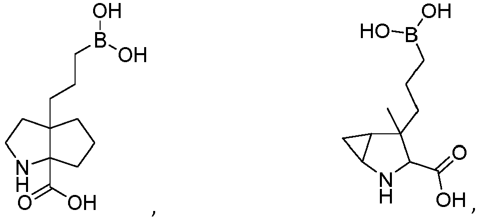 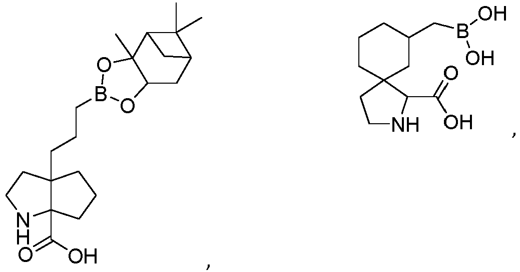 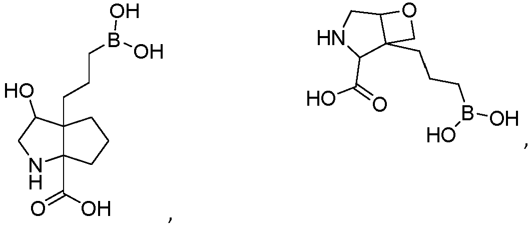 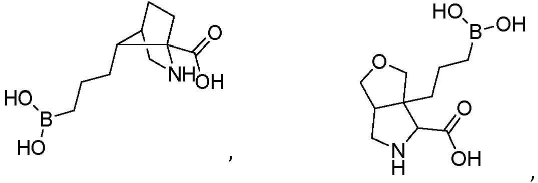 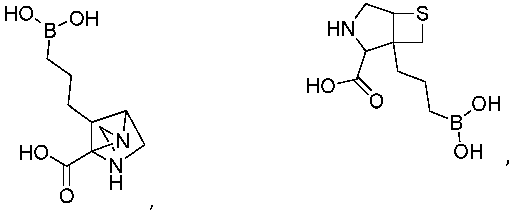 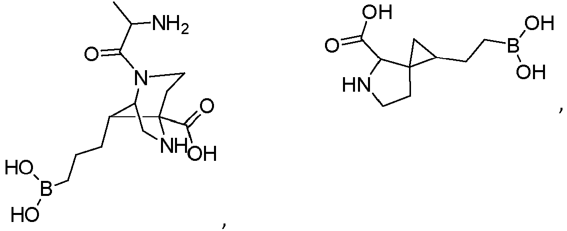 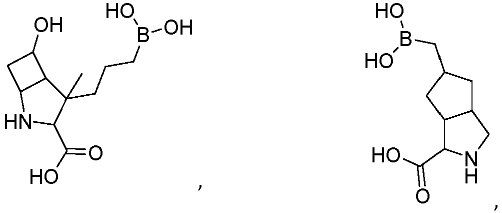 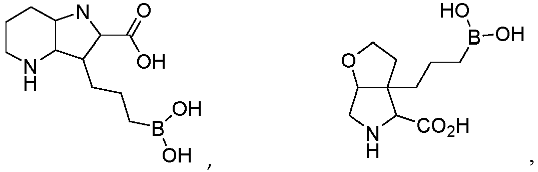 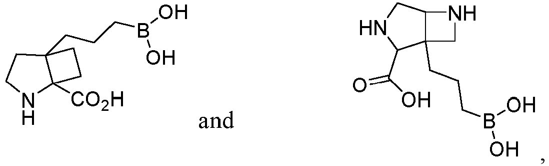 or a pharmaceutically acceptable salt thereof.

In other embodiments, described herein are the following compounds: 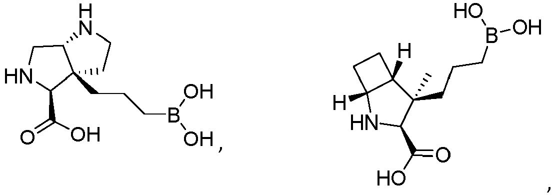 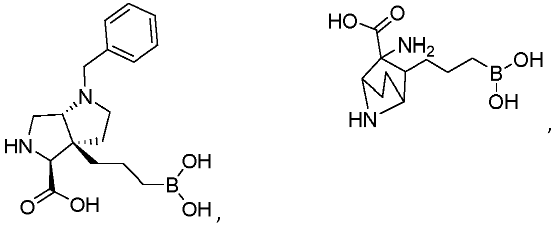 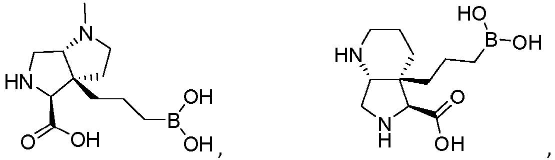 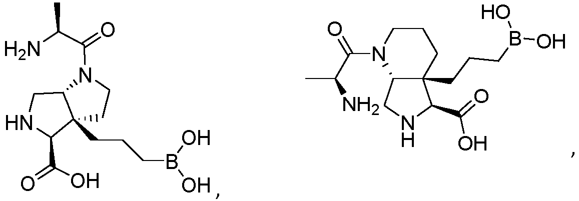 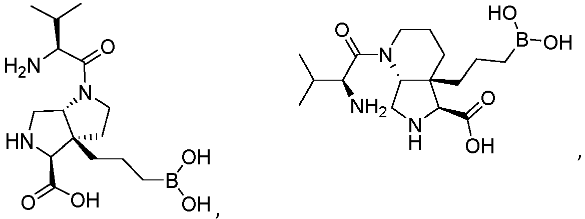 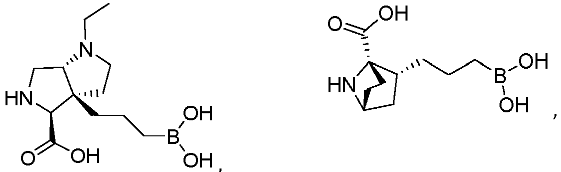 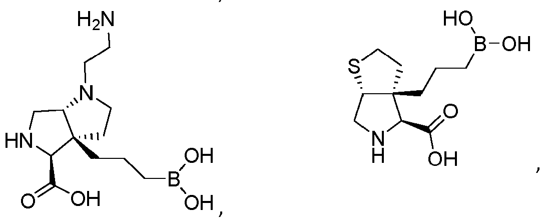 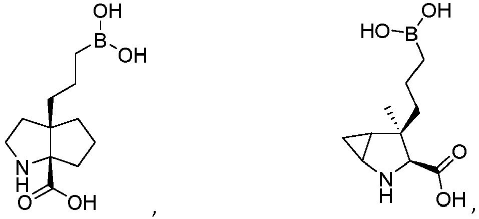 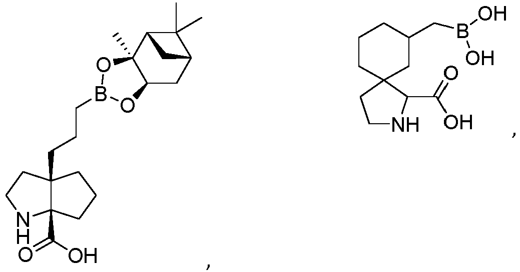 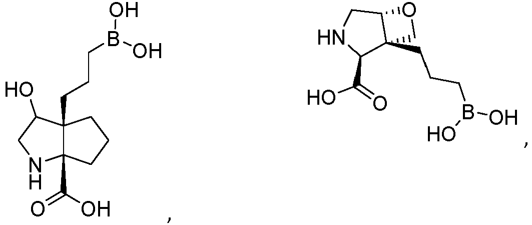 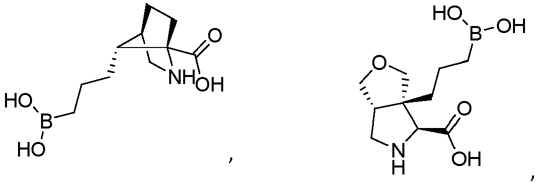 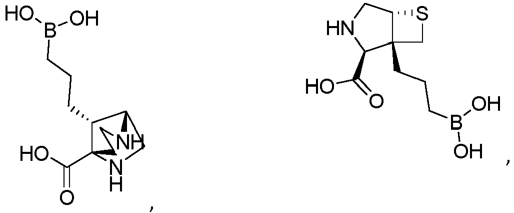 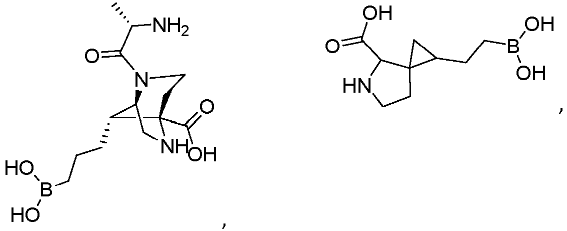 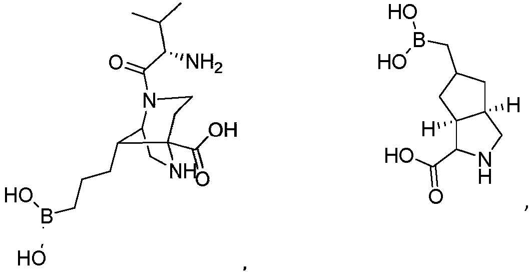 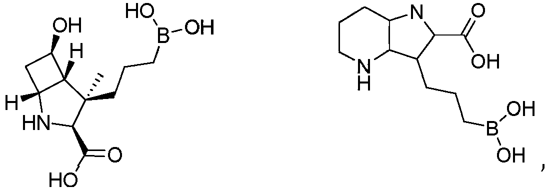 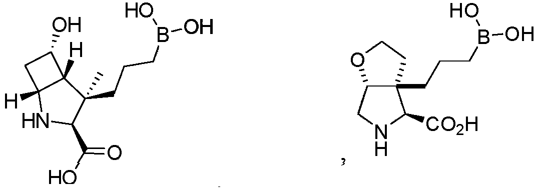 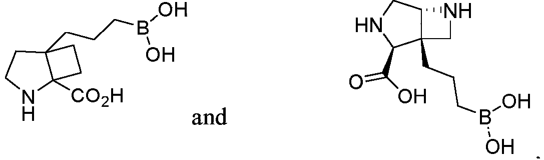 or pharmaceutically acceptable salts thereof.

### Definitions

The term "alkylene," or "alkylenyl" or "alkyl" by itself or as part of another substituent means a divalent straight, branched hydrocarbon radical having the stated number of carbon atoms. For example, -(C₁-C₅) alkylene, would include, *e.g*., -CH₂-, -CH₂CH₂-, -CH₂CH₂CH₂-, -CH₂CH₂CH₂CH₂-, -CH₂CH(CH₃)CH₂- or -CH₂CH₂CH₂CH₂CH₂-.

The term "halogen" includes a fluorine, a chlorine, a bromine or an iodine radical.

The term "C₁-C₆alkyl" encompasses straight alkyl having a carbon number of 1 to 6 and branched alkyl having a carbon number of 3 to 6. Specific examples thereof include methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1,2-dimethylpropyl, 1-ethylpropyl, n-hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl, 1-ethyl-1-methylpropyl, and the like.

The term "C₁-C₆haloalkyl" means an C₁-C₆alkyl as defined above wherein one or more hydrogen atoms on the alkyl is replaced by a halo group defined above.

The term "C₁-C₆alkoxy " refers to an alkyl group having 1 to 6 carbons linked to oxygen. Examples include methoxy, ethoxy, butoxy, isopropoxy and propoxy.

The term "COOC₁-C₆alkyl" refers to a -COOH group wherein the -OH is replaced with an alkoxy group as defined above. Examples include methoxycarbonyl, ethoxycarbonyl, isopropylcarbonyl and butoxycarbonyl.

The term "COC₁-C₆alkylNH₂ refers to a CO attached to a linear or branched alkyl group any hydrogen on the alkyl group is replaced with a NH₂ group. Examples include, but are not limited to:

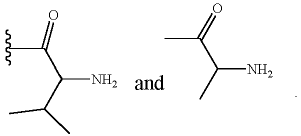

The term "C₃-C₈cycloalkyl" encompasses bridged, saturated or unsaturated cycloalkyl groups having 3 to 8 carbons. "Cycloalkyl" also includes non-aromatic rings as well as monocyclic, non-aromatic rings fused to a saturated cycloalkyl group. Examples of cycloalkyl include cyclopropyl, cyclobutyl, cyclopentyl, cyclohexyl, cycloheptyl, tetrahydronaphthyl, decahydronaphthyl, indanyl and the like.

The term "heterocycle" means mono- or bicyclic or bridged partially unsaturated and saturated rings containing at least one heteroatom selected from N, S and O, each of said ring having from 3 to 10 atoms in which the point of attachment may be carbon or nitrogen. Examples include azetidinyl, thiolanyl, thietanyl, oxetanyl, tetrahydropyranyl, tetrahydrofuranyl, pyrrolidinyl, piperidinyl, piperazinyl, dioxanyl, imidazolidinyl, 2,3-dihydrofuro(2,3-b)pyridyl, benzoxazinyl, benzoxazolinyl, 2-H-phthalazinyl, isoindolinyl, benzoxazepinyl, 5,6-dihydroimidazo[2,1-b]thiazolyl, tetrahydroquinolinyl, morpholinyl, tetrahydroisoquinolinyl, dihydroindolyl, tetrahydropyran, and the like. The term also includes partially unsaturated monocyclic rings that are not aromatic, such as 2- or 4-pyridones attached through the nitrogen or N-substituted-(1H, 3H)-pyrimidine-2,4-diones (N-substituted uracils). The term also includes bridged rings such as 5-azabicyclo[2.2.1]heptyl, 2,5-diazabicyclo[2.2.1]heptyl, 2-azabicyclo[2.2.1]heptyl, 7-azabicyclo[2.2.1]heptyl, 2,5-diazabicyclo[2.2.2]octyl, 2-azabicyclo[2.2.2]octyl, and 3-azabicyclo[3.2.2]nonyl, and azabicyclo[2.2.1]heptanyl.

The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of this invention which are generally prepared by reacting the free base with a suitable organic or inorganic acid. Representative salts of basic compounds of the present invention include, but are not limited to, the following: acetate, benzenesulfonate, benzoate, bicarbonate, bisulfate, bitartrate, borate, bromide, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, oxalate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate. Furthermore, where the compounds of the invention carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like.

Particularly preferred are the ammonium, calcium, magnesium, potassium, and sodium salts. Salts derived from pharmaceutically acceptable organic non-toxic bases include salts of primary, secondary, and tertiary amines, cyclic amines, and basic ion-exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidinyl, glucamine, glucosamine, histidine, hydrabamine, isopropylamine, lysine, methylglucamine, morpholine, piperazine, piperidinyl, polyamine resins, procaine, purines, theobromine, triethylamine, trimethylamine, tripropylamine, tromethamine, and the like.

The term "patient" refers to a mammalian patient, preferably a human patient, receiving or about to receive medical treatment.

The term *"rac"* means a mixture.

The compounds of the present invention may contain one or more asymmetric centers and can thus occur as racemates, racemic mixtures, single enantiomers, diastereomeric mixtures, and individual diastereomers. The present invention is meant to comprehend all such isomeric forms of these compounds.

Some of the compounds described herein contain olefinic double bonds, and unless specified otherwise, are meant to include both E and Z geometric isomers.

Some of the compounds described herein contain substituted cycloalkanes having cis-and trans-isomers, and unless specified otherwise, are meant to include both cis- and trans- geometric isomers.

The independent syntheses of these diastereomers or their chromatographic separations may be achieved as known in the art by appropriate modification of the methodology disclosed herein. Their absolute stereochemistry may be determined by the X-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base. The diasteromeric derivatives may then be converted to the pure enantiomers by cleavage of the added chiral residue. The racemic mixture of the compounds can also be separated directly by chromatographic methods utilizing chiral stationary phases, which methods are well known in the art.

Alternatively, any enantiomer of a compound may be obtained by stereoselective synthesis using optically pure starting materials or reagents of known configuration by methods well known in the art.

It will be understood that the present invention is meant to include the pharmaceutically acceptable salts, and also salts that are not pharmaceutically acceptable, of the compounds described herein, when they are used as precursors to the free compounds or their pharmaceutically acceptable salts or in other synthetic manipulations.

Solvates, and in particular, the hydrates of the compounds of the structural formulas described herein are included in the present invention as well.

Some of the compounds described herein may exist as tautomers, which have different points of attachment of hydrogen accompanied by one or more double bond shifts. For example, a ketone and its enol form are keto-enol tautomers. The individual tautomers as well as mixtures thereof are encompassed with compounds of the present invention.

The compounds of the present invention may also exist in open-chain or cyclized forms. In some cases one or more of the cyclized forms may result in loss of water. The specific composition of the open-chain and cyclized forms may be dependent on how the compound is isolated, stored or administered. For example, the compound may exist primarily in an open-chained form under acidic conditions but cyclize under neutral conditions. All forms are included in the invention.

In the compounds described herein, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of the formulas described herein. For example, different isotopic forms of hydrogen (H) include protium (¹H) and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds can be prepared without undue experimentation by conventional techniques well known to those skilled in the art or by processes analogous to those described in the Schemes and Examples herein using appropriate isotopically-enriched reagents and/or Intermediates.

### Methods of Treatment

Also encompassed by the present invention are methods of treating arginase-related diseases. The compounds described herein can be effective in preventing or treating various arginase-related diseases, such as gastrointestinal diseases, pulmonary inflammatory diseases, sexual arousal disorders, cardiovascular disorders, diseases caused by pathogenic microorganisms, immunological disorders, cancer, pre-term labor, Reynaud's disease, psoriasis, rheumatoid arthritis, and Peyronie's Disease, among others.

An increase in arginase activity has been associated with the pathophysiology of a number of conditions including impairment in non-adrenergic and non-cholinergic (NANC) nerve-mediated relaxation of gastrointestinal smooth muscle. An arginase inhibitor can be used to alleviate such impairment by administering the inhibitor to a mammal experiencing such impairment or a mammal which is anticipated to experience such impairment (e.g., a human afflicted with a gastrointestinal motility disorder).

Accordingly, the compounds of the invention may be useful in the treatment or prevention of gastrointestinal motility disorders, which is based on the observation that arginase is present in opossum internal anal sphincter muscle and the known arginase inhibitor, (S)-2-amino-6-boronohexanoic acid (ABH), has been shown to relax this muscle. See, e.g., Baggio et al., J. Pharm. Exp. Ther. 290, 1409-16 (1999).

The compounds of the invention may also be useful in the treatment or prevention of inflammatory bowel disease (IBD, e.g., Crohn's disease and ulcerative colitis). In fact, IBD has been shown to be characterized by increased arginase activity and endothelial dysfunction. See, e.g., Horowitz et al., Am. J. Physiol. Gastrointest. Liver Physiol. 292, G1323-36 (2007).

Likewise, the compounds of the invention may be useful in the treatment or prevention of gastric ulcers, because the bacterium that causes stomach ulcers, Helicobacter pylori, exhibits increased arginase activity upon colonization in order to evade the human immune response. See, e.g., Gobert et al., Proc. Natl. Acad. Sci. (USA) 98, 13844-49 (2001).

The compounds of the invention may be useful in the treatment or prevention of asthma based on the observation that arginase is upregulated in the asthmatic airway. See, e.g., Zimmermann and Rothenberg, Eur. J. Pharmacol. 533, 253-62 (2006). Furthermore, nebulizer treatment of guinea pigs with ABH in an allergic asthma model prevents airway hyperresponsiveness. See, e.g., Maarsingh, "Arginase: A Novel Key Enzyme in the Pathophysiology of Allergic Asthma," Ph. D. dissertation, Chapter 9, University of Groningen, Netherlands (2006); Maarsingh et al., Am. J. Respir. Crit. Care Med. 178, 565-73 (2008). The asthma phenotype is characterized by airway constriction, airway smooth muscle hyperplasia, and the chronic accumulation of fibrotic tissue; an arginase inhibitor can relax airway smooth muscle and attenuate cellular hyperplasia and fibrosis.

Additionally, the compounds of the invention may be useful in the treatment or prevention of chemically-induced lung fibrosis because arginase I and II are induced in bleomycin-induced lung fibrosis in order to provide more L-ornithine for collagen biosynthesis. See, e.g., Endo et al., Am. J. Physiol. Lung Cell Mol. Physiol. 285, L313-21 (2003).

The compounds of the invention may also be useful in the treatment or prevention of idiopathic pulmonary fibrosis, based on the observation that virus-induced upregulation of arginase I is observed in an animal model. See, e.g., Mora et al., Am. J. Respir. Cell Mol. Biol. 35, 466-73 (2006).

Furthermore, the compounds of the invention may be useful in the treatment or prevention of cystic fibrosis. Increased sputum arginase activity contributes to nitric oxide deficiency in cystic fibrosis lung disease; arginase activity also contributes to fibrosis. See, e.g., Graseman et al., Am. J. Respir. Crit. Care Med. 172, 1523-28 (2005).

Erectile dysfunction afflicts one-half of the male population over the age of forty. This malady often results from defects in the complex cascade of enzyme-catalyzed reactions governing blood flow into and out of the corpus cavernosum, a chamber of muscular, spongy tissue that becomes engorged with blood in the erect penis. Defects that compromise cavernosal blood flow often occur as secondary complications related to other health conditions, such as heart disease, hypertension, diabetes, use of certain medications, and the like.

In an important embodiment, the invention relates to use of an arginase inhibitor described herein for enhancing penile erectile function in a mammal (preferably a male human) or for alleviating erectile dysfunction in a mammal. Nitric oxide is an important regulator of erectile function and mediates NANC neurotransmission in penile corpus cavernosum smooth muscle, leading to rapid relaxation, which in turn leads to erection. Nitric oxide synthase, which catalyzes oxidation of L-arginine to form L-citrulline and nitric oxide, is for this reason a key enzyme in penile smooth muscle physiology. Arginase catalyzes hydrolysis of L-arginine to form L-ornithine and urea. Arginase regulates nitric oxide synthase activity by affecting the amount of L-arginine available for oxidation catalyzed by nitric oxide synthase activity. Thus, inhibition of arginase activity can enhance nitric oxide synthase activity, thereby enhancing nitric oxide-dependent smooth muscle relaxation in the corpus cavernosum and enhancing penile erection.

Arginase is present in rabbit and human penile corpus cavernosum and ABH enhances the nitric oxide-dependent relaxation of this tissue. See, e.g., Cox et al., Nature Struct. Biol. 6, 1043-47 (1999). The arginase inhibitor, ABH, enhances the erectile response in live male rabbits. See, e.g., Cama et al., Biochemistry 42, 8445-51 (2003). Arginase II is upregulated in the corpus cavernosum of the diabetic man, resulting in reduced nitric oxide biosynthesis which, in turn, leads to erectile dysfunction; administration of ABH in ex vivo experiments restores nitric oxide biosynthesis. See, e.g., Bivalacqua et al., Biochem. Biophys. Res. Commun. 283, 923-27 (2001). Arginase I is upregulated in the penis of aged mice and impairs erectile function. See, e.g., Bivalacqua et al., Am. J. Physiol. Heart Circ. Physiol. 292, H1340-51 (2007).

The compounds of the invention may also be useful in the treatment or prevention of female sexual arousal disorder. The arginase inhibitor, ABH, enhances the engorgement response in the genitalia of live female rabbits. See, e.g., Cama et al., Biochemistry 42, 8445-51 (2003).

The compounds of the invention may be useful in the treatment or prevention of endothelial vascular dysfunction in atherosclerosis, hypertension, hypercholesterolemia, and diabetes. Arginase modulates NOS activity by regulation of L-arginine availability, and the deleterious effects of arginase can be blocked by an arginase inhibitor. See, e.g., Berkowitz et al., Circulation 108, 2000-06 (2003); Yang and Ming, Clin. Med. Res. 4, 53-65 (2006). Increased arginase activity in diabetes contributes to vascular endothelial dysfunction by decreasing L-arginine availability to nitric oxide synthase. See, e.g., Romero et al., Circ. Res. 102, 95-102 (2008). Arginase inhibition attenuates hypertension in spontaneously hypertensive rats. See, e.g., Demougeot et al., J. Hypertens. 23, 971-78 (2005). Other relevant conditions include ischemia-reperfusion injury, peripheral vascular disease (PVD), peripheral arterial disease (PAD), and subarachnoid hemorrhage. Arginase has been identified as a new drug target for the treatment of atherosclerosis. See, e.g., Yang and Ming, Curr. Hypertension Rep. 8, 54-59 (2006).

The compounds of the invention may be useful in the treatment or prevention of pulmonary arterial hypertension. Elevated arginase activity contributes to vascular endothelial dysfunction by compromising L-arginine availability to nitric oxide synthase. See, e.g., Morris et al., Adv. Pulmonary Hypertension 5, 31-36 (2007).

The compounds of the invention may be useful in the treatment or prevention of African sleeping sickness, Chagas' disease, leishmaniasis, malaria, and other diseases caused by pathogenic microorganisms. Polyamine biosynthetic enzymes are essential for growth and survival of protozoa. See, e.g., Heby et al., Biochem. Soc. Trans. 31, 415-19 (2003). Arginase is essential for viability. See, e.g., Roberts et al., J. Biol. Chem. 279, 23668-78 (2004). Therefore, inhibitors of protozoan arginases can kill the protozoa.

The compounds of the invention may be useful in the treatment or prevention of multiple sclerosis, and possibly other autoimmune diseases, based upon the observation that arginase I is upregulated in an animal model of multiple sclerosis (experimental autoimmune encephalomyelitis) and administration of the arginase inhibitor ABH improves the disease score of animals. See, e.g., Xu et al., Immunology 110, 141-48 (2003).

Tumor-induced tolerance impairs the therapeutic efficacy of immunotherapy; one mechanism leading to T-cell tolerance is the generation of myeloid-derived suppressor cells (MDSCs), which produce arginase, thereby depleting the tumor microenvironment of L-arginine, which impairs T-cell signal transduction and function. Notably, arginase activity is a mechanism of immune system evasion that is also shared by certain bacteria, e.g., Helicobacter pylori. MDSCs are regarded as "cancer's bulwark against immune attack." See, e.g., Marx, Science 319, 154-56 (2008).

Accordingly, arginase is upregulated in the following types of cancers, which may be treated with an arginase inhibitor described herein: Renal cell carcinoma (see, e.g., Zea et al., Cancer Res. 65, 3044-48 (2005); Ochoa et al., Clin. Cancer Res. 13, 721s-26s (2007)); prostate cancer (see, e.g., Bronte et al., J. Exp. Med. 201, 1257-68 (2005) (arginase inhibition with N-hydroxy-L-arginine facilitates tumor immunotherapy); colorectal cancer (see, e.g., Leu and Wang, Cancer 70, 733-36 (1992); Bronte and Zanovello, Nature Rev. Immunol. 5, 641-54 (2005)); breast cancer (see, e.g., Singh et al., Cancer Res. 60, 3305-12 (2000); Bronte and Zanovello, Nature Rev. Immunol. 5, 641-54 (2005) (the arginase inhibitor, N-hydroxy-L-arginine, inhibits cell proliferation and induces apoptosis)); skin cancer (squamous cell and basal cell cancers) (see, e.g., Gokmen et al., J. Lab. Clin. Med. 137, 340-44 (2001); Bronte and Zanovello, Nature Rev. Immunol. 5, 641-54 (2005)); lung cancer (see, e.g., Rodriguez et al., J. Exp. Med. 202, 931-39 (2005); Bronte and Zanovello, Nature Rev. Immunol. 5, 641-54 (2005)); ovarian cancer (see, e.g., Melichar et al., J. Translational Med. 1, 1-5 (2003) (doi:10.11861479-5876-1-5)); and gastric cancer (see, e.g., Wu et al., Life Sci. 51, 1355-61 (1992)); among others.

Enhancement of uterine smooth muscle relaxation with an arginase inhibitor may be useful in the management of pre-term labor.

Reynaud's disease is a disease of the microvasculature. Because subcutaneous administration of the arginase inhibitor (S)-(2-Boronoethyl)-L-cysteine (BEC, which is an analogue of ABH) in humans is vasodilatory and enhances circulation, an arginase inhibitor may be useful in treating Reynaud's disease. See, e.g., Holowatz et al., J. Physiol. 574, 573-81 (2006).

Arginase I is highly overexpressed in the hyperproliferative psoriatic epidermis in human skin, and therefore arginase inhibitors may be useful in the treatment of psoriasis. See, e.g., Bruch-Gerharz et al., Am. J. Pathology 162, 203-11 (2003).

Arginase II is upregulated in synovial fluid from human patients, and therefore arginase inhibitors may be useful in the treatment of arthritis. See, e.g., Huang et al., Kaohsiung J. Med. Sci. 17, 358-63 (2001); Corraliza and Moncada, J. Rheumatol. 29, 2261-65 (2002).

The compounds of the invention may be useful in the treatment or prevention of Peyronie's disease. Arginase II is upregulated in the rat penis in an animal model for this disease. See, e.g., Bivalacqua et al., J. Andrology 22, 497-506 (2001). While this disorder can contribute to erectile dysfunction, it is principally an inflammatory condition in which fibrotic tissue builds up in the penis.

The composition of the invention can be used to treat a disorder in a mammal, wherein the disorder is associated with expression of an abnormally high level of arginase activity in a tissue of the mammal. Because nitric oxide synthase activity is regulated in a reciprocal fashion with respect to arginase activity in mammals, more particularly humans, the compounds and compositions of the invention can be used to treat a disorder in a mammal, wherein the disorder is associated with expression of an abnormally low level of nitric oxide synthase activity in a tissue of the mammal. Since the reciprocal interaction of arginase and nitric oxide synthase has implications for the function of smooth muscle, the use of the compounds described herein for the regulation of smooth muscle activity in an animal is also contemplated in the invention. Of course, a compound of the invention or a composition comprising the compound of the invention which comprises an arginase inhibitor described herein can also be used to inhibit arginase in a mammal having normal levels of arginase and nitric oxide synthase activity, particularly where the physiology which is desired to be effected is one which is affected by arginase or nitric oxide synthase activity, or where a disorder which is not caused by aberrant arginase or nitric oxide synthase activity levels can nonetheless be alleviated or inhibited by inhibiting arginase activity (e.g., certain forms of erectile dysfunction).

The invention also includes a method of enhancing smooth muscle relaxation comprising contacting the smooth muscle with an arginase inhibitor. The smooth muscle is preferably within the body of an animal. The type of smooth muscle to be relaxed includes, but is not limited to, gastrointestinal smooth muscle, anal sphincter smooth muscle, esophageal sphincter muscle, sphincter of Oddi, arterial smooth muscle, heart smooth muscle, pulmonary smooth muscle, kidney smooth muscle, uterine smooth muscle, vaginal smooth muscle, cervical smooth muscle, placental smooth muscle, and ocular smooth muscle. When the smooth muscle is gastrointestinal smooth muscle, the type of gastrointestinal smooth muscle includes, but is not limited to, the internal anal sphincter muscle.

When the smooth muscle is within the body of the animal, the invention includes a method of alleviating (e.g., reducing the incidence or severity) or inhibiting (e.g., reducing the likelihood of developing, or preventing) an arginase-related disorder in an animal. In a preferred embodiment, the animal is a human.

To alleviate an arginase-related disorder in a mammal, an arginase inhibitor described herein is administered to a mammal afflicted with the disorder. The inhibitor is preferably administered in combination with one or more pharmaceutically acceptable carriers, as described in further detail herein. The inhibitor (preferably in combination with a carrier) can also be administered to a mammal afflicted with a disorder characterized by aberrant nitric oxide synthase activity, or to one which exhibits normal (i.e. non-diseased) levels of arginase and nitric oxide synthase activities, but in which inhibition of arginase activity is desired. The invention also contemplates use of an arginase inhibitor in an in vitro arginase inhibition/smooth muscle relaxation functional assay, for the purpose of identifying compounds which affect smooth muscle function.

Accordingly, in certain embodiments, the invention is directed to methods of inhibiting arginase in a mammal, comprising the step of administering to said mammal an effective amount of a compound of the formulas described herein or a pharmaceutically acceptable salt thereof.

Accordingly, in certain embodiments, the invention is directed to methods of treating an arginase-related disorder in a mammal, comprising the step of administering to said mammal an effective amount of a compound of any of the formulas described herein or a pharmaceutically acceptable salt thereof. In certain preferred embodiments, the arginase-related disorder is a disorder associated with an abnormally low level of nitric oxide synthase activity in a tissue of the human, a disorder associated with an abnormally high level of arginase activity in a tissue of the human, or combinations thereof, including heart disease, systemic hypertension, pulmonary hypertension, erectile dysfunction, autoimmune encephalomyelitis, chronic renal failure, gastrointestinal motility disorders, gastric cancers, reduced hepatic blood flow, insufficient hepatic blood flow, cerebral vasospasm, or a combination thereof.

In still other certain embodiments, the invention is directed to methods of relaxing smooth muscle in a mammal, comprising the step of administering to said mammal an effective amount of a compound of the formulas described herein or a pharmaceutically acceptable salt thereof. In certain preferred embodiments, the smooth muscle which is relaxed according to this method is at least one selected from the group consisting of a gastrointestinal smooth muscle, anal sphincter smooth muscle, esophageal sphincter muscle, corpus cavemosum, sphincter of Oddi, arterial smooth muscle, heart smooth muscle, pulmonary smooth muscle, kidney smooth muscle, uterine smooth muscle, vaginal smooth muscle, cervical smooth muscle, placental smooth muscle, and ocular smooth muscle.

In certain embodiments, the invention is directed to methods of treating a disease or condition associated with upregulation of arginase in a mammal, comprising the step of administering to said mammal an effective amount of a compound of the formulas described herein or a pharmaceutically acceptable salt thereof; wherein said disease or condition is a gastrointestinal disease, a pulmonary inflammatory disease, a sexual arousal disorder, a cardiovascular disorder, a hemolytic disorder, an autoimmune disease, wound healing, a cancer, pre-term labor, psoriasis, or a combination thereof.

In certain embodiments, the invention is directed to methods of treating a disease or condition caused by parasitic protozoa, a disease caused by bacteria, or a combination thereof.

Inhibiting arginase impacts cancer in two ways. The first way is relief from immune-suppression that leads to tolerance of the tumor, and the second way is by restricting the production of ornithine and subsequent polyamines, which have a role in proliferation.

In certain preferred embodiments, the gastrointestinal disease is a gastrointestinal motility disorder, inflammatory bowel disease, Crohn's disease, ulcerative colitis, gastric ulcer, adenotonsilar disease or a combination thereof.

In certain preferred embodiments, the pulmonary inflammatory disease is asthma, chemically-induced lung fibrosis, idiopathic pulmonary fibrosis, cystic fibrosis, chronic obstructive pulmonary disease (COPD) or a combination thereof.

In certain preferred embodiments, the sexual arousal disorder is male erectile dysfunction, Peyronie's Disease, or a female sexual arousal disorder.

In certain preferred embodiments, the cardiovascular disorder is endothelial vascular dysfunction in atherosclerosis, hypertension, ischemia reperfusion injury, peripheral vascular disease, peripheral arterial disease, subarachnoid hemorrhage, hypercholesterolemia, diabetes, or a combination thereof, diabetic cardiovascular disease, pulmonary arterial hypertension, Reynaud's disease, or a combination thereof.

In certain preferred embodiments, the hemolytic disorder is paroxysmal nocturnal hemoglobinuria (PNH), sickle-cell disease, thalassemias, hereditary spherocytosis and stomatocytosis, microangiopathic hemolytic anemias, pyruvate kinase deficiency, ABO mismatch transfusion reaction, paroxysmal cold hemoglobinuria, severe idiopathic autoimmune hemolytic anemia, infection-induced anemia, malaria, cardiopulmonary bypass, mechanical heart valve-induced anemia, chemical induced anemia, or a combination thereof.

In certain preferred embodiments, the autoimmune disease is encephalomyelitis, multiple sclerosis, anti-phospholipid syndrome 1, autoimmune hemolytic anaemia, chronic inflammatory demyelinating polyradiculoneuropathy, dermatitis herpetiformis ("Celiac Disease"), dermatomyositis, myasthenia gravis, pemphigus, rheumatoid arthritis, stiff-person syndrome, type 1 diabetes, ankylosing spondylitis, or a combination thereof.

In certain preferred embodiments, the condition is wound healing.

In certain preferred embodiments, the disease caused by parasitic protozoa is African sleeping sickness, Chagas' disease, leishmaniasis, malaria, or a combination thereof.

In certain preferred embodiments, the cancer is renal cell carcinoma, prostate cancer, colorectal cancer, breast cancer, skin cancer, lung cancer, ovarian cancer, gastric cancer, or a combination thereof. In certain embodiments, the skin cancer is a squamous cell cancer, basal cell cancer, or a combination thereof.

In certain preferred embodiments, the condition is pre-term labor.

In certain preferred embodiments, the condition is Reynaud's disease.

In certain embodiments, the invention is directed to methods of providing relief from immune suppression in a mammal, comprising the step of administering to said mammal an effective amount of a compound of the formulas described herein or a pharmaceutically acceptable salt thereof, wherein said mammal is suffering from a disease or condition selected from the group consisting of a chronic infectious disease, a bacterial infection, a parasitic infection, trauma, leprosy, tuberculosis, liver transplantation, a cancer, and combinations thereof.

In certain embodiments, the invention is directed to methods of inhibiting the production of ornithine or other related metabolites (e.g. agmatine, putrescine, spermine, spermidine, citruline, proline, glutamate, etc.) in a mammal suffering from at least one tumor, comprising the step of administering to said mammal an effective amount of a compound of the formulas described herein or a pharmaceutically acceptable salt thereof.

### Pharmaceutical Compositions

Compounds described herein may be administered orally or parenterally. As formulated into a dosage form suitable for administration, the compounds described herein can be used as a pharmaceutical composition for the prevention, treatment, or remedy of the above diseases.

In clinical use of the compounds described herein, usually, the compound is formulated into various preparations together with pharmaceutically acceptable additives according to the dosage form, and may then be administered. By "pharmaceutically acceptable" it is meant the additive, carrier, diluent or excipient must be compatible with the other ingredients of the formulation and not deleterious to the recipient thereof. As such, various additives ordinarily used in the field of pharmaceutical preparations are usable. Specific examples thereof include gelatin, lactose, sucrose, titanium oxide, starch, crystalline cellulose, hydroxypropyl methylcellulose, carboxymethylcellulose, corn starch, microcrystalline wax, white petrolatum, magnesium metasilicate aluminate, anhydrous calcium phosphate, citric acid, trisodium citrate, hydroxypropylcellulose, sorbitol, sorbitan fatty acid ester, polysorbate, sucrose fatty acid ester, polyoxyethylene, hardened castor oil, polyvinylpyrrolidone, magnesium stearate, light silicic acid anhydride, talc, vegetable oil, benzyl alcohol, gum arabic, propylene glycol, polyalkylene glycol, cyclodextrin, hydroxypropyl cyclodextrin, and the like.

Preparations to be formed with those additives include, for example, solid preparations such as tablets, capsules, granules, powders, suppositories; and liquid preparations such as syrups, elixirs, injections. These may be formulated according to conventional methods known in the field of pharmaceutical preparations. The liquid preparations may also be in such a form that may be dissolved or suspended in water or in any other suitable medium in their use. Especially for injections, if desired, the preparations may be dissolved or suspended in physiological saline or glucose liquid, and a buffer or a preservative may be optionally added thereto.

The pharmaceutical compositions may contain the compound of the invention in an amount of from 1 to 99.9 % by weight, preferably from 1 to 60 % by weight of the composition. The compositions may further contain any other therapeutically-effective compounds.

In case where the compounds of the invention are used for prevention or treatment for the above-mentioned diseases, the dose and the dosing frequency may be varied, depending on the sex, the age, the body weight and the disease condition of the patient and on the type and the range of the intended remedial effect. In general, when orally administered, the dose may be from 0.001 to 50 mg/kg of body weight/day, and it may be administered at a time or in several times. In specific embodiments, the dose is from about 0.01 to about 25 mg/kg/day, in particular embodiments, from about 0.05 to about 10 mg/kg/day. For oral administration, the compositions are preferably provided in the form of tablets or capsules containing from 0.01 mg to 1,000 mg. In specific embodiments, the dose is 0.01, 0.05, 0.1, 0.2, 0.5, 1.0, 2.5, 5, 10, 15, 20, 25, 30, 40, 50, 75, 100, 125, 150, 175, 200, 225, 250, 500, 750, 850 or 1,000 milligrams of a compound described herein. This dosage regimen may be adjusted to provide the optimal therapeutic response.

### Combination Therapy

The compounds of the present invention are further useful in methods for the prevention or treatment of the aforementioned diseases, disorders and conditions in combination with other therapeutic agents.

The compounds of the present invention may be used in combination with one or more other drugs in the treatment, prevention, suppression or amelioration of diseases or conditions for which compounds described herein or the other drugs may have utility, where the combination of the drugs together are safer or more effective than either drug alone. Such other drug(s) may be administered in an amount commonly used therefore, contemporaneously or sequentially with a compound described herein or a pharmaceutically acceptable salt thereof. When a compound described herein is used contemporaneously with one or more other drugs, the pharmaceutical composition may in specific embodiments contain such other drugs and the compound described herein or its pharmaceutically acceptable salt in unit dosage form. However, the combination therapy may also include therapies in which the compound described herein or its pharmaceutically acceptable salt and one or more other drugs are administered on different overlapping schedules. It is also contemplated that when used in combination with one or more other active ingredients, the compounds of the present invention and the other active ingredients may be used in lower doses than when each is used singly. Accordingly, the pharmaceutical compositions of the present invention include those that contain one or more other active ingredients, in addition to a compound described herein or a pharmaceutically acceptable salt thereof.

Examples of other active ingredients that may be administered in combination with a compound of any of the Formulas described herein or a pharmaceutically acceptable salt thereof and either administered separately or in the same pharmaceutical composition, include, but are not limited to pain relieving agents, anti-angiogenic agents, anti-neoplastic agents, anti-diabetic agents, anti-infective agents, or gastrointestinal agents, or combinations thereof.

Suitable compounds that may be used in combination with a compound according to the present invention include without limitation sildenafil, vardenafil, tadalafil and alprostadil, epoprostenol, iloprost, bosentan, amlodipine, diltiazem, nifedipine, ambrisentan and warfarin, fluticasone, budesonide, mometasone, flunisolide, beclomethasone, montelukast, zafirlukast, zileuton, salmeterol, formoterol, theophylline, albuterol, levalbuterol, pirbuterol, ipratropium, prednisone, methylprednisolone, omalizumab, corticosteroid and cromolyn, atorvastatin, lovastatin, simvastatin, pravastatin, fluvastatin, rosuvastatin, gemfibrozil, fenofibrate, nicotinic acid and clopidogrel.

Additionally, a compound of any of the Formulas disclosed herein may be used in combination with one or more other active agents, including but not limited to, other anti-cancer agents that are used in the prevention, treatment, control, amelioration, or reduction of risk of a particular disease or condition (e.g., cell proliferation disorders). In one embodiment, a compound disclosed herein is combined with one or more other anti-cancer agents for use in the prevention, treatment, control amelioration, or reduction of risk of a particular disease or condition for which the compounds disclosed herein are useful. Such other active agents may be administered, by a route and in an amount commonly used therefor, contemporaneously or sequentially with a compound of the present invention.

In one embodiment, the other active agent is selected from the group consisting of vascular endothelial growth factor (VEGF) receptor inhibitors, topoisomerase II inhibitors, smoothen inhibitors, alkylating agents, anti-tumor antibiotics, anti-metabolites, retinoids, immunomodulatory agents including but not limited to anti-cancer vaccines, CTLA-4, LAG-3 and PD-1 antagonists.

Examples of vascular endothelial growth factor (VEGF) receptor inhibitors include, but are not limited to, bevacizumab (sold under the trademark AVASTIN by Genentech/Roche), axitinib, (N-methyl-2-({3-[(1E)-2-(pyridin-2-yl)ethenyl]-1H-indazol-6-yl}sulfanyl)benzamide, also known as AG013736, and described in PCT Publication No. WO 01 /002369), Brivanib Alaninate ((S)-((R)-1-(4-(4-Fluoro-2-methyl-lH-indol-5-yloxy)-5-methylpyrrolo[2,l-f][1,2,4]triazin-6-yloxy)propan-2-yl)2-aminopropanoate, also known as BMS-582664), motesanib (N-(3,3-dimethyl-1,2-dihydroindol-6-yl)-2-(pyridin-4-ylmethylamino)pyridine-3-carboxamide and described in PCT Publication No. WO 02/068470), pasireotide (also known as SO 230, and described in PCT Publication No. WO 02/010192), and sorafenib (sold under the tradename NEXAVAR).

Examples of topoisomerase II inhibitors include but are not limited to, etoposide (also known as VP-16 and Etoposide phosphate, sold under the tradenames TOPOSAR, VEPESID and ETOPOPHOS), and teniposide (also known as VM-26, sold under the tradename VUMON).

Examples of alkylating agents include but are not limited to, 5-azacytidine (sold under the trade name VIDAZA), decitabine (sold under the trade name of DECOGEN), temozolomide (sold under the trade names TEMODAR and TEMODAL by Schering-Plough/Merck), dactinomycin (also known as actinomycin-D and sold under the tradename COSMEGEN), melphalan (also known as L-PAM, L-sarcolysin, and phenylalanine mustard, sold under the tradename ALKERAN), altretamine (also known as hexamethylmelamine (HMM), sold under the tradename HEXALEN), carmustine (sold under the tradename BCNU), bendamustine (sold under the tradename TREANDA), busulfan (sold under the tradenames BUSULFEX and MYLERAN), carboplatin (sold under the tradename PARAPLATIN), lomustine (also known as CCNU, sold under the tradename CeeNU), cisplatin (also known as CDDP, sold under the tradenames PLATINOL and PLATINOL-AQ), chlorambucil (sold under the tradename LEUKERAN), cyclophosphamide (sold under the tradenames CYTOXAN and NEOSAR), dacarbazine (also known as DTIC, DIC and imidazole carboxamide, sold under the tradename DTIC-DOME), altretamine (also known as hexamethylmelamine (HMM) sold under the tradename HEXALEN), ifosfamide (sold under the tradename IFEX), procarbazine (sold under the tradename MATULANE), mechlorethamine (also known as nitrogen mustard, mustine and mechloroethamine hydrochloride, sold under the tradename MUSTARGEN), streptozocin (sold under the tradename ZANOSAR), thiotepa (also known as thiophosphoamide, TESPA and TSPA, and sold under the tradename THIOPLEX).

Examples of anti-tumor antibiotics include, but are not limited to, doxorubicin (sold under the tradenames ADRIAMYCIN and RUB EX), bleomycin (sold under the tradename LENOXANE), daunorubicin (also known as dauorubicin hydrochloride, daunomycin, and rubidomycin hydrochloride, (2-CdA) sold under the tradename CERUBIDINE), daunorubicin liposomal (daunorubicin citrate liposome, sold under the tradename DAUNOXOME), mitoxantrone (also known as DHAD, sold under the tradename NOVANTRONE), epirubicin (sold under the tradename ELLENCE), idarubicin (sold under the tradenames IDAMYCIN, IDAMYCIN PFS), and mitomycin C (sold under the tradename MUTAMYCIN).

Examples of anti-metabolites include, but are not limited to 5-fluorouracil (sold under the tradename ADRUCIL), 6-thioguanine (sold under the tradename PURINETHOL), pemetrexed (sold under the tradename ALIMTA), cytarabine (also known as arabinosylcytosine (Ara-C), sold under the tradename CYTOSAR-U), cytarabine liposomal (also known as Liposomal Ara-C, sold under the tradename DEPOCYT), decitabine (sold under the tradename DACOGEN), hydroxyurea (sold under the tradenames HYDREA, DROXIA and MYLOCEL), fludarabine (sold under the tradename FLUDARA), floxuridine (sold under the tradename FUDR), cladribine (also known as 2-chlorodeoxyadenosine (2-CdA) sold under the tradename LEUSTATIN), methotrexate (also known as amethopterin, methotrexate sodium (MTX), sold under the tradenames RHEUMATREX and TREXALL), and pentostatin (sold under the tradename NIPENT).

Examples of retinoids include, but are not limited to, alitretinoin (sold under the tradename PANRETIN), tretinoin (all-trans retinoic acid, also known as ATRA, sold under the tradename VESANOID), Isotretinoin (13-c/s-retinoic acid, sold under the tradenames ACCUTANE, AMNESTEEM, CLARAVIS, CLARUS, DECUTAN, ISOTANE, IZOTECH, ORATANE, ISOTRET, and SOTRET), and bexarotene (sold under the tradename TARGRETIN).

"PD-1 antagonist" means any chemical compound or biological molecule that blocks binding of PD-L1 expressed on a cancer cell to PD-1 expressed on an immune cell (T cell, B cell or NKT cell) and preferably also blocks binding of PD-L2 expressed on a cancer cell to the immune-cell expressed PD-1. Alternative names or synonyms for PD-1 and its ligands include: PDCD1, PD1, CD279 and SLEB2 for PD-1 ; PDCD1L1, PDL1, B7H1, B7-4, CD274 and B7-H for PD-Ll; and PDCD1L2, PDL2, B7-DC, Btdc and CD273 for PD-L2. In any of the treatment method, medicaments and uses of the present invention in which a human individual is being treated, the PD-1 antagonist blocks binding of human PD-Ll to human PD-1, and preferably blocks binding of both human PD-Ll and PD-L2 to human PD-1. Human PD-1 amino acid sequences can be found in NCBI Locus No.: NP 005009. Human PD-Ll and PD-L2 amino acid sequences can be found in NCBI Locus No.: NP_054862 and NP_079515, respectively.

PD-1 antagonists useful in any of the treatment method, medicaments and uses of the present invention include a monoclonal antibody (mAb), or antigen binding fragment thereof, which specifically binds to PD-1 or PD-Ll, and preferably specifically binds to human PD-1 or human PD-Ll. The mAb may be a human antibody, a humanized antibody or a chimeric antibody, and may include a human constant region. In some embodiments the human constant region is selected from the group consisting of IgGl, IgG2, IgG3 and IgG4 constant regions, and in preferred embodiments, the human constant region is an IgGl or IgG4 constant region. In some embodiments, the antigen binding fragment is selected from the group consisting of Fab, Fab'-SH, F(ab')2, scFv and Fv fragments. Examples of PD-1 antagonists include, but are not limited to, pembrolizumab (sold under the tradename KEYTRUDA) and nivolumab (sold under the tradename OPDIVO).

Examples of mAbs that bind to human PD-1, and useful in the treatment method, medicaments and uses of the present invention, are described in US7488802, US7521051, US8008449, US8354509, US8168757, WO2004/004771, WO2004/072286, WO2004/056875, and US2011/0271358.

Examples of mAbs that bind to human PD-Ll, and useful in the treatment method, medicaments and uses of the present invention, are described in WO2013/019906, WO2010/077634 Al and US8383796. Specific anti-human PD-Ll mAbs useful as the PD-1 antagonist in the treatment method, medicaments and uses of the present invention include MPDL3280A, BMS-936559, MEDI4736 and MSB0010718C of WO2013/019906.

Other PD-1 antagonists useful in any of the treatment method, medicaments and uses of the present invention include an immunoadhesin that specifically binds to PD-1 or PD- LI, and preferably specifically binds to human PD-1 or human PD-Ll, e.g., a fusion protein containing the extracellular or PD-1 binding portion of PD-Ll or PD-L2 fused to a constant region such as an Fc region of an immunoglobulin molecule. Examples of immunoadhesion molecules that specifically bind to PD-1 are described in WO2010/027827 and WO2011/066342. Specific fusion proteins useful as the PD-1 antagonist in the treatment method, medicaments and uses of the present invention include AMP-224 (also known as B7-DCIg), which is a PD-L2-FC fusion protein and binds to human PD-1.

Examples of other cytotoxic agents include, but are not limited to, arsenic trioxide (sold under the tradename TRISENOX), asparaginase (also known as L-asparaginase, and Erwinia L-asparaginase, sold under the tradenames ELSPAR and KIDROLASE)

When a compound of the present invention is used contemporaneously with one or more other drugs a specific embodiment hereof pertains to, a pharmaceutical composition containing such other drugs in addition to the compound of the present invention. Accordingly, the pharmaceutical compositions of the present invention include those that also contain one or more other active ingredients, in addition to a compound of the present invention.

The weight ratio of the compound of the present invention to the second active ingredient may be varied and will depend upon the effective dose of each ingredient. Generally, an effective dose of each will be used. Thus, for example, when a compound of the present invention is combined with another agent, the weight ratio of the compound of the present invention to the other agent will generally range from about 1000:1 to about 1:1000, in particular embodiments from about 200:1 to about 1:200. Combinations of a compound of the present invention and other active ingredients will generally also be within the aforementioned range, but in each case, an effective dose of each active ingredient should be used.

In such combinations the compound of the present invention and other active agents may be administered separately or in conjunction. In addition, the administration of one element may be prior to, concurrent to, or subsequent to the administration of other agent(s).

### EXAMPLES

In the following examples, those compounds which are covered by the appended claims are part of the presently claimed invention. Other examples are provided for reference.

The meanings of the abbreviations in Examples are shown below.
ACN = MeCN = CH₃CN = acetonitrile
AcOH=acetic acid
AIBN = Azobisisobutyronitrile
Ar=argon
BBr₃ = Boron tribromide
BF₃ Et₂O = Boron trifluoride etherate
Boc₂O= di-tert-butyl dicarbonate
Cbz = carboxybenzyl
CbzOSu = N-(Benzyloxycarbonyloxy)succinimide
CBr₄ = Tetrabromomethane
CCl₄= carbontetrachloride
CELITE = diatomaceous earth
Conc. = concentrated
Cs₂CO₃ = Cesium carbonate
DBU = 1,8-Diazabicyclo[5.4.0]undec-7-ene
DCE=dichloroethane
DCM = dichloromethane
DDQ = 2,3-Dichloro-5,6-dicyano-1,4-benzoquinone
DIBALH = diisobutylaluminum hydride
DIPEA = DIEA= N, N-Diisopropylethylamine, or Hünig's base
DMA = dimethylacetamide
DMAP = 4-dimethylaminopyridine
DMF = N,N-Dimethylformamide
DMP = Dess-Martin periodinane
DMSO = dimethyl sulfoxide
DPPE = 1,2-Bis(diphenylphosphino)ethane
DPPF = 1,1'-Bis(diphenylphosphino)ferrocene
EDCI = 1-ethyl-3-(3-dimethylaminopropyl)carbodiimide
Et₂O = diethyl ether
EtOAc = ethyl acetate
EtOH = ethanol
h = hours
H₂=hydrogen
HATU = 1-[Bis(dimethylamino)methylene]-1H-1,2,3-triazolo[4,5-b]pyridinium 3-oxid hexafluorophosphate
HCl= hydrochloric acid
HFBA= Heptafluorobutyric acid
HOAc = acetic acid
I₂=iodine
IPA= isopropyl alcohol
[Ir(cod)Cl]₂ = cyclooctadiene iridium chloride dimer
K₂CO₃= potassium carbonate
K₃PO₄ = Tripotassium phosphate
KF = Potassium fluoride
KHMDS = Potassium bis(trimethylsilyl)amide
KOTMS = Potassium trimethylsilanolate
LCMS=Liquid chromatography-mass spectrometry
LHMDS = LiHMDS= lithium bis(trimethylsilyl)amide
LiAlH₄=lithium aluminum hydride
LiOH = lithium hydroxide
min = minutes
Me = methyl
MeOH= methanol
MgSO₄ = Magnesium sulfate
MsCl = methanesulfonyl chloride
N₂=nitrogen
NaBH₄ = sodium borohydrate
NaH=sodium hydride
NaHCO₃ = Sodium hydrogencarbonate
NaIO₄= sodium periodate
NaOH=sodium hydroxide
Na₂CO₃=sodium carbonate
Na₂SO₃= sodium sulfite
Na₂SO₄= sodium sulfate
NH₄Cl= Ammonium chloride
NH₄OH= Ammonium hydroxide
NH₄OAc= Ammonium acetate
NaHMDS = sodium bis(trimethylsilyl)amide
Pd(OH)₂/C = Pearlman's Catalyst - Palladium hydroxide on carbon-C
Pd/C = Palladium on carbon-C
[Pd(C₃H₅)Cl₂]= Allylpalladium(II) chloride dimer
PdCl₂(dppf)-CH₂Cl₂ = [1,1'-Bis(diphenylphosphino)ferrocene]dichloropalladium(II)
PPh₃ = Triphenylphosphine
RuO₂.H₂O= ruthenium(IV)oxide hydrate
RP-HPLC = reverse phase high performance liquid chromatography
SFC = Supercritical Fluid Chromatography
TBDPS-Cl = TBSCl = tert-Butyl(chloro)diphenylsilane
TEA = Et₃N = triethylamine
TBAF = Tetra-n-butylammonium fluoride
TFA = trifluoroacetic acid
THF= tetrahydrofuran
TMS = Trimethylsilylb
TsOH = p-Toluenesulfonic acid
CDCl₃ = heavy chloroform
CD₃OD = heavy methanol

### 1 Standard atmosphere [atm] = 101325 pascal [Pa] = 14.6959488 psi

The meanings of the abbreviations in the nuclear magnetic resonance spectra are shown below:
s = singlet, d = doublet, dd = double doublet, dt = double triplet, ddd = double double doublet, Sept = septet, t = triplet, m = multiplet, br = broad, brs = broad singlet, q = quartet J = coupling constant and Hz = hertz.

Compounds of this invention can be prepared using the intermediates and processes outlined below. The various starting materials used are commercially available or are readily made by persons skilled in the art. An asterisk (*) may be used in a chemical structure drawing that indicates the location of a chiral center.

### Example 1: (3aS,4S,6aR)-3a-(3-boronopropyl)octahydropyrrolo[3,4-b]pyrrole-4-carboxylic acid

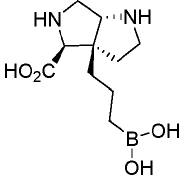

### Step 1: 1-(tert-butyl) 2-methyl (2S)-3-allyl-4-hydroxy-3-(2-hydroxyethyl)pyrrolidine-1,2-dicarboxylate

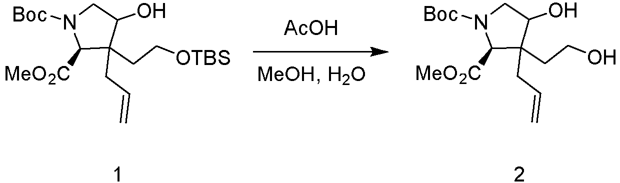

1-(tert-butyl) 2-methyl (2*S*)-3-allyl-3-(2-((*tert*-butyldimethylsilyl)oxy)ethyl)-4-hydroxypyrrolidine-1,2-dicarboxylate (23 g, 52 mmol) was taken up in a mixture of MeOH (58 mL), water (58 mL) and AcOH (58 mL). The reaction mixture was stirred at ambient temperature for 19 hours then diluted with water and ethyl acetate. The organic layer was separated, dried over Na₂SO₄, filtered and concentrated under reduced pressure. The resulting residue was purified by silica gel chromatography (MeOH in DCM) to afford 1-(*tert*-butyl) 2-methyl (2*S*)-3-allyl-4-hydroxy-3-(2-hydroxyethyl)pyrrolidine-1,2-dicarboxylate (mixture of diastereomers). LCMS (C₁₁H₂₀NO₄⁺) (ES, m/z): 230 [M-Boc+H]⁺.

### Step 2: 1-(tert-butyl) 2-methyl (2S)-3-allyl-4-oxo-3-(2-oxoethyl)pyrrolidine-1,2-dicarboxylate

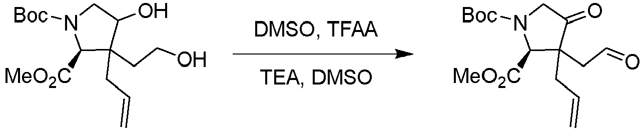

A solution of trifluoroacetic anhydride (13 mL, 91 mmol) in DCM (40 mL) was added to a solution of DMSO (8.6 mL, 120 mmol) in DCM (120 mL) at -78 °C. The resulting solution was held at -78 °C for 45 minutes. A solution of 1-(tert-butyl) 2-methyl (2*S*)-3-allyl-4-hydroxy-3-(2-hydroxyethyl)pyrrolidine-1,2-dicarboxylate (mixture of diastereomers, 10 g, 30 mmol) in DCM (40 mL) was added and the reaction mixture was held at -78 °C for 1 hour. Triethylamine (21 mL, 150 mmol) was added, and the reaction mixture was warmed to ambient temperature and held for 3 hours. The reaction mixture was concentrated under reduced pressure affording crude 1-(*tert*-butyl) 2-methyl (2*S*)-3-allyl-4-oxo-3-(2-oxoethyl)pyrrolidine-1,2-dicarboxylate (mixture of diastereomers) which was used in the next step without purification. LCMS (C₁₁H₁₆NO₄⁺) (ES, m/z): 226 [M-Boc+H]⁺.

### Step 3: 5-(tert-butyl) 4-methyl (4S)-3a-allyl-1-benzylhexahydropyrrolo[3,4-b]pyrrole-4,5(1H)-dicarboxylate

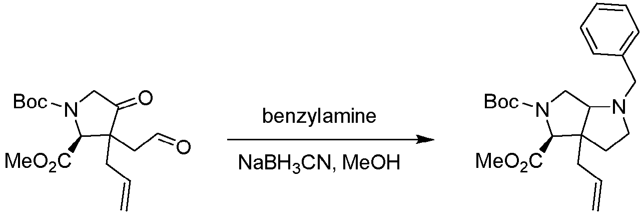

Benzylamine (3.5 mL, 32 mmol) was added to a solution of 1-(tert-butyl) 2-methyl (2*S*)-3-allyl-4-oxo-3-(2-oxoethyl)pyrrolidine-1,2-dicarboxylate (mixture of diastereomers) (9.9 g, 30 mmol) in MeOH (610 mL) at 0 °C followed by portion wise addition of sodium cyanoborohydride (7.6 g, 120 mmol). The reaction mixture was warmed to ambient temperature and held for 19 hours. The reaction was diluted with water and ethyl acetate. The organic layer was separated, dried over Na₂SO₄, filtered and concentrated under reduced pressure. The resulting oil was purified by silica gel chromatography (EtOAc in hexanes) to afford 5-(tert-butyl) 4-methyl (4*S*)-3a-allyl-1-benzylhexahydropyrrolo[3,4-b]pyrrole-4,5(1H)-dicarboxylate (mixture of diastereomers). LCMS (C₂₃H₃₃N₂O₄⁺) (ES, m/z): 401 [M+H]⁺.

### Step 4: 5-(tert-butyl) 4-methyl (3aS,4S,6aR)-1-benzyl-3a-(3-((3aS,4S,6S,7aR)-3a,5,5-trimethylhexahydro-4,6-methanobenzo[d][1,3,2]dioxaborol-2-yl)propyl)hexahydropyrrolo[3,4-b]pyrrole-4,5(1H)-dicarboxylate

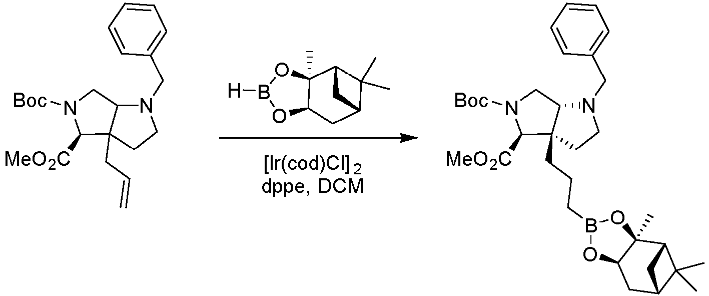

(3aS,4S,6S,7aR)-3a,5,5-trimethylhexahydro-4,6-methanobenzo[d][1,3,2]dioxaborole (6.2 g, 35 mmol), chloro(1,5-cyclooctadiene)Iridium(I) dimer (0.58 g, 0.86 mmol) and 1,2-bis(diphenylphosphino)ethane (0.69 g, 1.7 mmol) in anhydrous DCM (230 mL) were placed under an atmosphere of argon, and the resulting mixture was stirred at ambient temperature for 20 minutes, followed by addition of a solution of 5-(*tert*-butyl) 4-methyl (4*S*)-3a-allyl-1-benzylhexahydropyrrolo[3,4-b]pyrrole-4,5(1H)-dicarboxylate (mixture of diastereomers) (6.9 g, 17 mmol) in DCM (120 mL). The reaction mixture was stirred at ambient temperature for 1.5 hours under argon. The reaction was quenched by slow addition of methanol then diluted with water and DCM. The organic layer was separated, washed with brine, dried over Na₂SO₄, filtered and concentrated under reduced pressure. The resulting residue was purified by silica gel chromatography (EtOAc in hexanes) to afford 5-(tert-butyl) 4-methyl (4*S*)-1-benzyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (mixture of diastereomers). The product was further purifided by chiral SFC (Regis (R,R)-Whelk-O1 column, 20%/80% methanol + 0.1% NH₄OH/CO₂). Retention time for desired material was 3.2 min. 5-(tert-butyl) 4-methyl (3a*S*,4*S*,6a*R*)-1-benzyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate was isolated. LCMS (C₃₃H₅₀BN₂O₆⁺) (ES, m/z): 581 [M+H]⁺.

### Step 5: 5-(tert-butyl) 4-methyl (3aS,4S,6aR)-3a-(3-((3aS,4S,6S,7aR)-3a,5,5-trimethylhexahydro-4,6-methanobenzo[d][1,3,2]dioxaborol-2-yl)propyl)hexahydropyrrolo[3,4-b]pyrrole-4,5(1H)-dicarboxylate

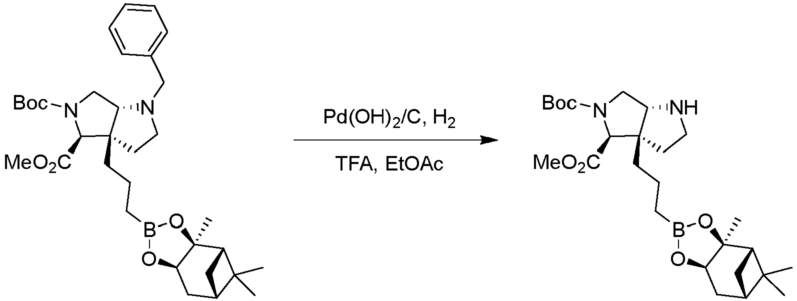

Pd(OH)₂/C (0.33 g, 0.47 mmol) was added to a solution of 5-(*tert*-butyl) 4-methyl (3a*S*,4*S*,6a*R*)-1-benzyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (5.5 g, 9.4 mmol) in EtOAc (100 mL). The reaction mixture was degassed and backfilled with H₂ three times then stirred under an atmosphere of H₂ for 40 hours. The mixture was filtered and concentrated under reduced pressure. The resulting residue was purified by silica gel chromatography (3:1 EtOAc/EtOH in hexanes) to afford 5-(*tert*-butyl) 4-methyl (3aS,4S,6aR)-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-b]pyrrole-4,5(1*H*)-dicarboxylate as the TFA salt. LCMS (C₂₆H₄₄BN₂O₆⁺) (ES, m/z): 491 [M+H]⁺.

### Step 6: (3aS,4S,6aR)-3a-(3-boronopropyl)octahydropyrrolo[3,4-b]pyrrole-4-carboxylic acid

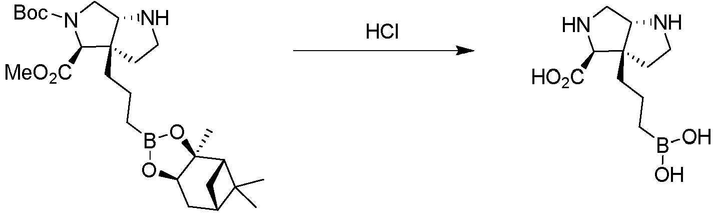

A mixture of 5-(*tert*-butyl) 4-methyl (3a*S*,4*S*,6a*R*)-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-b]pyrrole-4,5(1H)-dicarboxylate (1.1 g, 1.7 mmol) and 6 M HCl (10 mL, 60 mmol) was heated in a microwave reactor with stirring at 120 °C for 1.5 hours. The reaction mixture was washed with DCM and the resulting aqueous layer was concentrated to give (3a*S*,4*S*,6a*R*)-3a-(3-boronopropyl)octahydropyrrolo[3,4-*b*]pyrrole-4-carboxylic acid as an HCl salt. LCMS (C₁₀H₁₈BN₂O₃⁺) (ES, m/z): 225 [M-H₂O+H]⁺. ¹H NMR (499 MHz, D₂O) δ 4.27 (dd, 1H), 4.13 - 4.07 (m, 1H), 3.95 (dd, *J =* 14.0, 8.3 Hz, 1H), 3.61 - 3.47 (m, 3H), 2.37 - 2.27 (m, 1H), 2.24 - 2.13 (m, 1H), 1.64 - 1.49 (m, 2H), 1.47 - 1.27 (m, 2H), 0.77 - 0.69 (m, 2H).

### Step 7: (3aS,4S,6aR)-3a-(3-boronopropyl)octahydropyrrolo[3,4-b]pyrrole-4-carboxylic acid (free base)

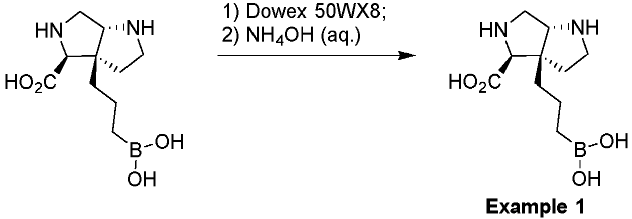

(3a*S*,4*S*,6a*R*)-3a-(3-boronopropyl)octahydropyrrolo[3,4-*b*]pyrrole-4-carboxylic acid (2HCl salt, 0.64 g, 2.0 mmol) was purified on 39 g of Dowex 50WX8 acidic resin (washed with water until pH neutral, then eluted with 2N aqueous ammonium hydroxide) to afford (3aS,4S,6aR)-3a-(3-boronopropyl)octahydropyrrolo[3,4-*b*]pyrrole-4-carboxylic acid as a free base. LCMS (C₁₀H₁₈BN₂O₃⁺) (ES, m/z): 225 [M-H₂O+H]⁺. ¹H NMR (499 MHz, D₂O) δ 3.91 - 3.81 (m, 1H), 3.61 (dd, *J* = 13.1, 8.0 Hz, 1H), 3.48 (s, 1H), 3.33 - 3.26 (m, 1H), 3.26 - 3.17 (m, 1H), 2.82 (dd, *J* = 13.2, 5.7 Hz, 1H), 2.12 - 2.04 (m, 1H), 1.97 - 1.88 (m, 1H), 1.52 - 1.26 (m, 4H), 0.74 - 0.67 (m, 2H).

### Example 2: (3aR,4S,6aR)-1-benzyl-3a-(3-boronopropyl)octahydropyrrolo[3,4-b]pyrrole-4-carboxylic acid

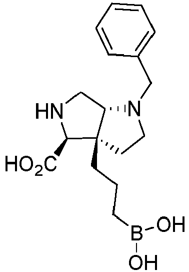

### Step 1: 5-(tert-butyl) 4-methyl (3aR,4S)-3a-allyl-6a-hydroxyhexahydro-5H-furo[2,3-c]pyrrole-4,5-dicarboxylate

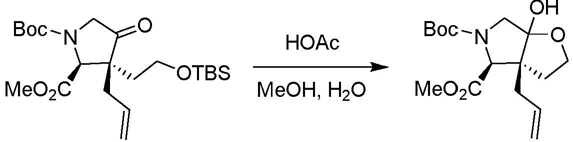

1-(*tert*-butyl) 2-methyl (2*S*,3*R*)-3-allyl-3-(2-((*tert*-butyldimethylsilyl)oxy)ethyl)-4-oxopyrrolidine-1,2-dicarboxylate (600 mg, 1.4 mmol) was taken up in a mixture of MeOH (1.5 mL), water (1.5 mL) and AcOH (1.5 mL). The reaction mixture was stirred at ambient temperature for 19 hours then diluted with water and ethyl acetate. The organic layer was separated, dried over Na₂SO₄, filtered and concentrated under reduced pressure. The resulting residue was purified by silica gel chromatography (EtOAc in hexanes) to afford 5-(tert-butyl) 4-methyl (3a*R*,4*S*)-3a-allyl-6a-hydroxyhexahydro-5*H*-furo[2,3-*c*]pyrrole-4,5-dicarboxylate. LCMS (C₁₁H₁₈NO₄⁺) (ES, m/z): 228 [M-Boc+H]⁺.

### Step 2: 1-(tert-butyl) 2-methyl (2S,3R)-3-allyl-4-hydroxy-3-(2-hydroxyethyl)pyrrolidine-1,2-dicarboxylate

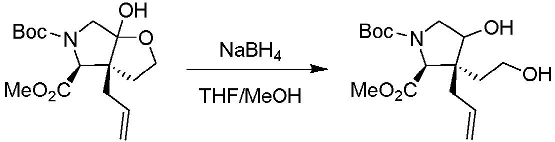

Sodium borohydride (110 mg, 2.8 mmol) was added to a solution of 5-(tert-butyl) 4-methyl (3a*R*,4*S*)-3a-allyl-6a-hydroxyhexahydro-5*H*-furo[2,3-*c*]pyrrole-4,5-dicarboxylate (230 mg, 0.71 mmol) in THF (2.3 mL) and MeOH (2.3 mL) at 0 °C. Reaction mixture was held at 0 °C for 1 hour then diluted with saturated aqueous ammonium chloride. The resulting mixture was made acidic by addition of 1N citiric acid and extracted with EtOAc. The organic layer was separated, dried over Na₂SO₄, filtered and concentrated under reduced pressure. The resulting oil was purified by silica gel chromatography (MeOH in DCM) to afford 1-(*tert*-butyl) 2-methyl (2S,3R)-3-allyl-4-hydroxy-3-(2-hydroxyethyl)pyrrolidine-1,2-dicarboxylate. LCMS (C₁₁H₂₀NO₄⁺) (ES, m/z): 230 [M-Boc+H]⁺.

### Step 3: 1-(tert-butyl) 2-methyl (2S,3R)-3-allyl-4-oxo-3-(2-oxoethyl)pyrrolidine-1,2-dicarboxylate

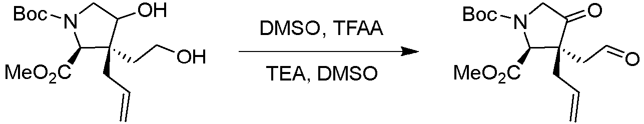

A solution of trifluoroacetic anhydride (0.26 mL, 1.8 mmol) in DCM (0.8 mL) was added to a solution of DMSO (0.17 mL, 2.4 mmol) in DCM (2.4 mL) at -78 °C. The resulting solution was held at -78 °C for 15 minutes. A solution of 1-(tert-butyl) 2-methyl (2*S*,3*R*)-3-allyl-4-hydroxy-3-(2-hydroxyethyl)pyrrolidine-1,2-dicarboxylate (200 mg, 0.6 mmol) in DCM (0.8 mL) was added and the reaction mixture was held at -78 °C for 45 minutes. Triethylamine (0.42 mL, 3.0 mmol) was added and the reaction mixture was warmed to ambient temperature and held for 1.5 hours. Reaction mixture was concentrated under reduced pressure affording crude 1-(*tert*-butyl) 2-methyl (2*S*,3*R*)-3-allyl-4-oxo-3-(2-oxoethyl)pyrrolidine-1,2-dicarboxylate which was used in the next step without purification. LCMS (C₁₁H₁₆NO₄⁺) (ES, m/z): 226 [M-Boc+H]⁺.

### Step 4: 5-(tert-butyl) 4-methyl (3aS,4S,6aR)-3a-allyl-1-benzylhexahydropyrrolo[3,4-b]pyrrole-4,5(1H)-dicarboxylate

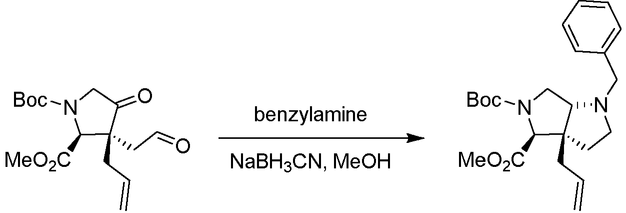

Benzylamine (0.066 mL, 0.6 mmol) was added to a solution of 1-(*tert*-butyl) 2-methyl (2*S*,3*R*)-3-allyl-4-oxo-3-(2-oxoethyl)pyrrolidine-1,2-dicarboxylate (0.2 g, 0.6 mmol) in MeOH (30 mL) at 0 °C followed by addition of sodium cyanoborohydride (0.15 g, 2.4 mmol). The reaction mixture was warmed to ambient temperature and held for 66 hours. The reaction concentrated under reduced pressure and the resulting residue was purified by silica gel chromatography (EtOAc in hexanes) to afford 5-(*tert*-butyl) 4-methyl (3a*S*,4*S*,6a*R*)-3a-allyl-1-benzylhexahydropyrrolo[3,4-b]pyrrole-4,5(1H)-dicarboxylate. LCMS (C₂₃H₃₃N₂O₄⁺) (ES, m/z): 401 [M+H]⁺.

### Step 5: 5-(tert-butyl) 4-methyl (3aS,4S,6aR)-1-benzyl-3a-(3-(4,4,5,5-tetramethyl-1, 3,2-dioxaborolan-2-yl)propyl)hexahydropyrrolo[3,4-b]pyrrole-4,5(1H)-dicarboxylate

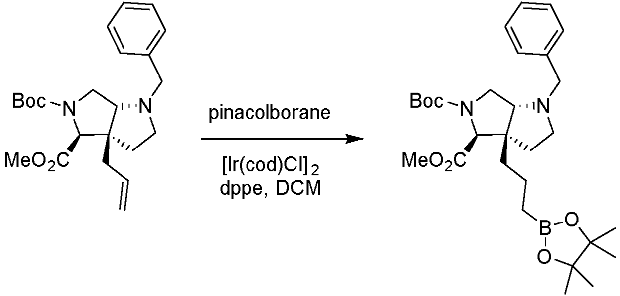

Pinacolborane (1M in THF) (0.67 mL, 0.67 mmol), chloro(1,5-cyclooctadiene)Iridium(I) dimer (0.009 g, 0.013 mmol) and 1,2-bis(diphenylphosphino)ethane (0.011 g, 0.027 mmol) in anhydrous DCM (3.5 mL) was placed under an atmosphere of argon and the resulting mixture was stirred at ambient temperature for 30 minutes, followed by addition of a solution of 5-(*tert-*butyl) 4-methyl (3a*S*,4*S*,6a*R*)-3a-allyl-1-benzylhexahydropyrrolo[3,4-*b*]pyrrole-4,5(1*H*)-dicarboxylate (0.11 g, 0.27 mmol) in DCM (1.8 mL). The reaction mixture was stirred at ambient temperature for 2 hours under argon. Reaction was quenched by slow addition of methanol then diluted with water and EtOAc. The organic layer was separated, washed with brine, dried over Na₂SO₄, filtered and concentrated under reduced pressure. The resulting residue was purified by silica gel chromatography (EtOAc in hexanes) to afford 5-(*tert*-butyl) 4-methyl (3a*S*,4*S*,6a*R*)-1-benzyl-3a-(3-(4,4,5,5-tetramethyl-1,3,2-dioxaborolan-2-yl)propyl)hexahydropyrrolo[3,4-b]pyrrole-4,5(1H)-dicarboxylate. LCMS (C₂₉H₄₆BN₂O₆⁺) (ES, m/z): 529 [M+H]⁺.

### Step 6: (3aR,4S,6aR)-1-benzyl-3a-(3-boronopropyl)octahydropyrrolo[3,4-b]pyrrole-4-carboxylic acid

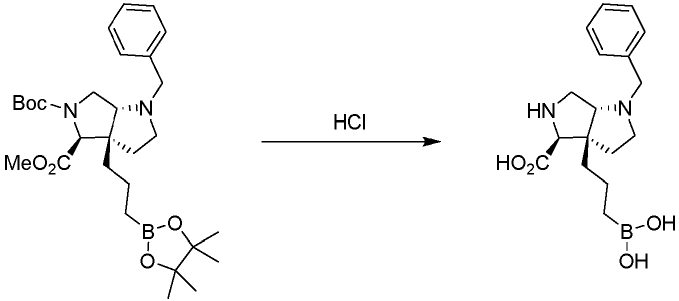

A mixture of 5-(*tert*-butyl) 4-methyl (3a*S*,4*S*,6a*R*)-1-benzyl-3a-(3-(4,4,5,5-tetramethyl-1,3,2-dioxaborolan-2-yl)propyl)hexahydropyrrolo[3,4-b]pyrrole-4,5(1H)-dicarboxylate (0.0059 g, 0.011 mmol) and 6 M HCl (0.5 mL, 3 mmol) was heated in a microwave reactor while stirring at 120 °C for 1 hour. The reaction mixture was concentrated to give (3aR,4S,6aR)-1-benzyl-3a-(3-boronopropyl)octahydropyrrolo[3,4-*b*]pyrrole-4-carboxylic acid as an HCl salt. LCMS (C₁₇H₂₄BN₂O₃⁺) (ES, m/z): 315 [M-H₂O+H]⁺. ¹H NMR (499 MHz, D₂O) δ 7.55 - 7.41 (m, 5H), 4.49 (d, *J =* 13.0 Hz, 1H), 4.38 (d, *J =* 13.0 Hz, 1H), 4.20 (dd, *J =* 8.0, 4.5 Hz, 1H), 4.10 (s, 1H), 3.88 - 3.75 (m, 1H), 3.63 - 3.48 (m, 2H), 3.19 - 3.08 (m, 1H), 2.38 - 2.25 (m, 2H), 1.62 - 1.47 (m, 2H), 1.47 - 1.31 (m, 2H), 0.81 - 0.67 (m, 2H).

### Example 3: (3aR,4S,6aR)-3a-(3-boronopropyl)-1-methyloctahydropyrrolo[3,4-b]pyrrole-4-carboxylic acid

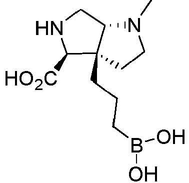

### Step 1: 5-(tert-butyl) 4-methyl (3aS,4S,6aR)-1-methyl-3a-(3-((3aS,4S,6S,7aR)-3a,5,5-trimethylhexahydro-4,6-methanobenzo[d][1,3,2]dioxaborol-2-yl)propyl)hexahydropyrrolo[3,4-b]pyrrole-4,5(1H)-dicarboxylate

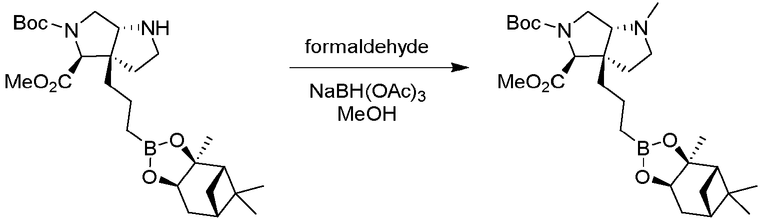

Formaldehyde (37 wt % in water) (16 µL, 0.22 mmol) followed by sodium triacetoxyborohydride (23 mg, 0.0.11 mmol) were added to a solution of 5-(*tert*-butyl) 4-methyl *(3aS,4S,6aR)-3a-(3-((3aS,4S,6S,7aR)-3a,5,5-trimethylhexahydro-4,6-*methanobenzo[*d*][1,3,2]dioxaborol-2-yl)propyl)hexahydropyrrolo[3,4-*b*]pyrrole-4,5(1*H*)-dicarboxylate (27 mg, 0.055 mmol) in MeOH (1 mL). The reaction mixture was stirred at ambient temperature for 3.5 hours. More formaldehyde (37 wt % in water) (16 µL, 0.22 mmol) and sodium triacetoxyborohydride (23 mg, 0.0.11 mmol) were added to the solution which was held for an additional 18 hours at ambient temperature. A final addition of formaldehyde (37 wt % in water) (16 µL, 0.22 mmol) and sodium triacetoxyborohydride (23 mg, 0.0.11 mmol) was added and the mixture was stirred for an additional 1.5 hours at ambient temperature. The reaction mixture was diluted with saturated aqueous NH₄Cl and ethyl acetate. The organic layer was separated, washed with brine, dried over Na₂SO₄, filtered and concentrated under reduced pressure. The resulting residue was purified by silica gel chromatography (EtOAc in hexanes) to afford 5-(*tert*-butyl) 4-methyl (3aS,4S,6aR)-1-methyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate. LCMS (C₂₇H₄₆BN₂O₆⁺) (ES, m/z): 505 [M+H]⁺.

### Step 2: (3aR,4S,6aR)-3a-(3-boronopropyl)-1-methyloctahydropyrrolo[3,4-b]pyrrole-4-carboxylic acid

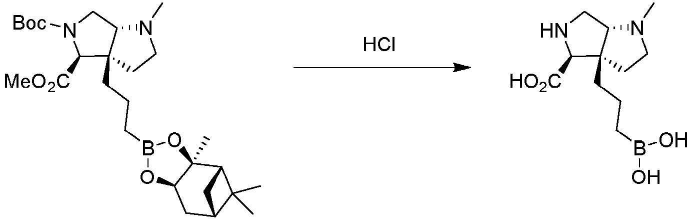

A mixture of 5-(*tert*-butyl) 4-methyl (3a*S*,4*S*,6a*R*)-1-methyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (15 mg, 0.029 mmol) and 6 M HCl (0.75 mL, 4.5 mmol) was heated in a microwave reactor with stirring at 120 °C for 1 hour. The reaction mixture was washed with DCM and the resulting aqueous layer was concentrated to give (3a*R*,4*S*,6a*R*)-3a-(3-boronopropyl)-1-methyloctahydropyrrolo[3,4-*b*]pyrrole-4-carboxylic acid as an HCl salt. LCMS (C₁₁H₂₀BN₂O₃⁺) (ES, m/z): 239 [M-H₂O+H]⁺. ¹H NMR (499 MHz, D₂O) δ 4.22 - 4.12 (m, 2H), 3.99 - 3.75 (m, 2H), 3.72 - 3.60 (m, 1H), 3.57 - 3.41 (m, 1H), 2.92 (s, 3H), 2.42 - 2.27 (m, 2H), 1.65 - 1.50 (m, 2H), 1.49 - 1.31 (m, 2H), 0.80 - 0.70 (m, 2H).

### Example 4: (3aR,4S,6aR)-1-(L-alanyl)-3a-(3-boronopropyl)octahydropyrrolo[3,4-b]pyrrole-4-carboxylic acid

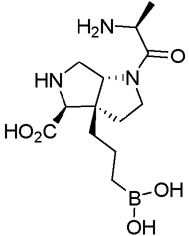

### Step 1: 5-(tert-butyl) 4-methyl (3aS,4S,6aR)-1-((tert-butoxycarbonyl)-L-alanyl)-3a-(3-((3aS,4S,6S,7aR)-3a,5, 5-trimethylhexahydro-4, 6-methanobenzo[d][1, 3,2]dioxaborol-2-yl)propyl)hexahydropyrrolo[3,4-b]pyrrole-4,5(1H)-dicarboxylate

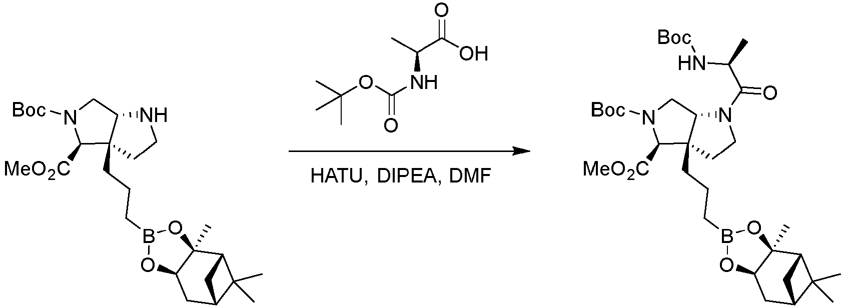

Boc-Ala-OH (39 mg, 0.21 mmol), DIPEA (140 µL, 0.82 mmol) and HATU (93 mg, 0.25 mmol) were added to a solution of 5-(*tert*-butyl) 4-methyl (3a*S*,4*S*,6a*R*)-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (TFA salt, 150 mg, 0.21 mmol) in DMF (1 mL). The reaction mixture was stirred at ambient temperature for 18 hours then diluted with aqueous NaHCO₃ and ethyl acetate. The organic layer was separated, dried over Na₂SO₄, filtered and concentrated under reduced pressure. The resulting oil was purified by silica gel chromatography (EtOAc in hexanes) to afford 5-(*tert*-butyl) 4-methyl (3a*S,*4*S*,6a*R*)-1-((*tert-*butoxycarbonyl)-*L*-alanyl)-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate. LCMS (C₂₉H₄₉BN₃O₇⁺) (ES, m/z): 562 [M-Boc+H]⁺.

### Step 2: (3aS,4S,6aR)-5-(tert-butoxycarbonyl)-1-((tert-butoxycarbonyl)-L-alanyl)-3a-(3-((3aS,4S,6S,7aR)-3a,5, 5-trimethylhexahydro-4,6-methanobenzo[d][1,3,2]dioxaborol-2-yl)propyl)octahydropyrrolo[3,4-b]pyrrole-4-carboxylic acid

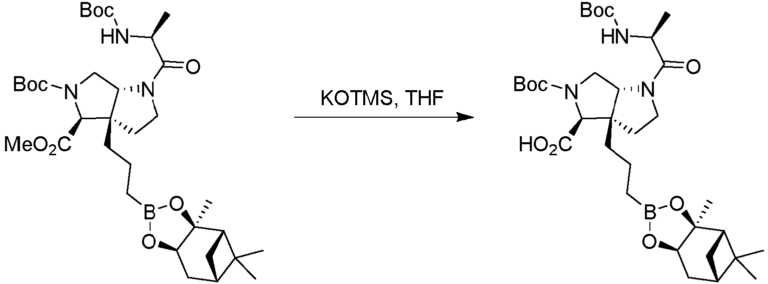

KOTMS (63 mg, 0.49 mmol) was added to a solution of 5-(tert-butyl) 4-methyl (3a*S*,4*S*,6a*R*)-1-((*tert*-butoxycarbonyl)-*L*-alanyl)-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (130 mg, 0.20 mmol) in THF (2 mL). The reaction mixture stirred at ambient temperature for 5 hours. More KOTMS (63 mg, 0.49 mmol) was added to the reaction which was held at ambient temperature for an additional 17 hours. Reaction mixture was concentrated under reduced pressure and the resulting oil was purified by silica gel chromatography ((3:1 EtOAc/EtOH) in hexanes) to afford (3a*S*,4*S*,6a*R*)-5-(*tert*-butoxycarbonyl)-1-((*tert*-butoxycarbonyl)-*L*-alanyl)-3a-(3-((3a*S*,4*S*,6*S*,7a*R*)-3a,5,5-trimethylhexahydro-4,6-methanobenzo[*d*][1,3,2]dioxaborol-2-yl)propyl)octahydropyrrolo[3,4-*b*]pyrrole-4-carboxylic acid. LCMS (C₂₈H₄₇BN₃O₇⁺) (ES, m/z): 548 [M-Boc+H]⁺.

### Step 3: (3aR,4S,6aR)-1-(L-alanyl)-3a-(3-boronopropyl)octahydropyrrolo[3,4-b]pyrrole-4-carboxylic acid

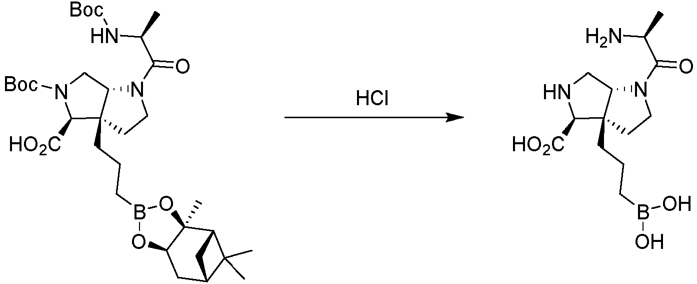

A mixture of (3a*S*,4*S*,6a*R*)-5-(*tert*-butoxycarbonyl)-1-((*tert*-butoxycarbonyl)-*L*-alanyl)-3a-(3-((3a*S*,4*S*,6*S*,7a*R*)-3a,5,5-trimethylhexahydro-4,6-methanobenzo[*d*][1,3,2]dioxaborol-2-yl)propyl)octahydropyrrolo[3,4-*b*]pyrrole-4-carboxylic acid (74 mg, 0.11 mmol) and 6 M HCl (1 mL, 6 mmol) was heated to 60 °C for 5 hours. The reaction mixture was washed with DCM and the resulting aqueous layer was concentrated to give (3a*R*,4*S*,6a*R*)-1-(*L*-alanyl)-3a-(3-boronopropyl)octahydropyrrolo[3,4-*b*]pyrrole-4-carboxylic acid as an HCl salt. LCMS (C₁₃H₂₃BN₃O₄⁺) (ES, m/z): 296 [M-H₂O+H]⁺. ¹H NMR (499 MHz, D₂O) δ 4.35 - 4.28 (m, 1H), 4.25 - 4.18 (m, 1H), 4.03 - 3.99 (m, 1H), 3.87 - 3.74 (m, 3H), 3.40 - 3.33 (m, 1H), 2.27 - 2.15 (m, 2H), 1.59 - 1.48 (m, 2H), 1.47 - 1.38 (m, 4H), 1.37 - 1.27 (m, 1H), 0.75 - 0.68 (m, 2H).

### Step 4: (3aR,4S,6aR)-1-(L-alanyl)-3a-(3-boronopropyl)octahydropyrrolo[3,4-b]pyrrole-4-carboxylic acid (free base)

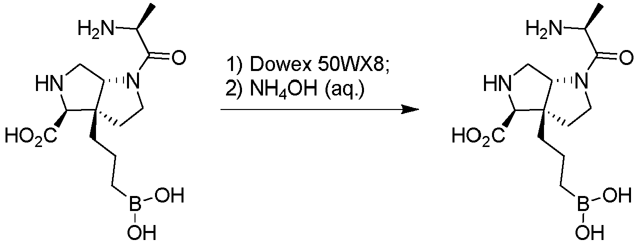

(3a*R*,4*S*,6a*R*)-1-(*L*-alanyl)-3a-(3-boronopropyl)octahydropyrrolo[3,4-*b*]pyrrole-4-carboxylic acid (2HCl salt, 25 mg, 0.065 mmol) was purified on 1.5 g of Dowex 50WX8 acidic resin (washed with water until pH neutral, then eluted with 2N aqueous ammonium hydroxide) to afford (3a*R*,4*S*,6a*R*)-1-(*L*-alanyl)-3a-(3-boronopropyl)octahydropyrrolo[3,4-*b*]pyrrole-4-carboxylic acid as a free base. LCMS (C₁₃H₂₃BN₃O₄⁺) (ES, m/z): 296 [M-H₂O+H]⁺. ¹H NMR (499 MHz, D₂O) δ 4.25 - 4.17 (m, 1H), 4.00 - 3.93 (m, 1H), 3.82 - 3.68 (m, 4H), 3.20 - 3.12 (m, 1H), 2.20 - 2.09 (m, 2H), 1.50 - 1.27 (m, 7H), 0.75 - 0.64 (m, 2H).

### Example 5: (3aR,4S,6aR)-1-(L-valyl)-3a-(3-boronopropyl)octahydropyrrolo[3,4-b]pyrrole-4-carboxylic acid

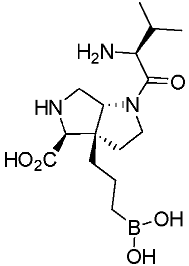

Example 5 was synthesized according to the procedure described in Example 4, using appropriate starting materials. LCMS (C₁₅H₂₇BN₃O₄⁺) (ES, m/z): 324 [M-H₂O+H]⁺. ¹H NMR (499 MHz, D₂O) δ 4.45 - 4.29 (m, 1H), 4.15 - 3.71 (m, 5H), 3.42 - 3.25 (m, 1H), 2.32 - 2.11 (m, 3H), 1.66 - 1.25 (m, 4H), 1.13 - 0.89 (m, 6H), 0.84 - 0.66 (m, 2H).

### Example 6: (3aR,4S,6aR)-3a-(3-boronopropyl)-1-ethyloctahydropyrrolo[3,4-b]pyrrole-4-carboxylic acid

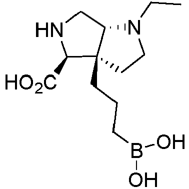

### Step 1: 5-(tert-butyl) 4-methyl (3aS,4S,6aR)-1-ethyl-3a-(3-((3aS,4S,6S,7aR)-3a,5,5-trimethylhexahydro-4,6-methanobenzo[d][1,3,2]dioxaborol-2-yl)propyl)hexahydropyrrolo[3,4-b]pyrrole-4,5(1H)-dicarboxylate

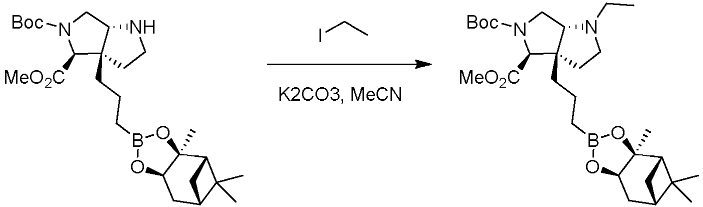

Potassium carbonate (69 mg, 0.5 mmol) and iodoethane (15 µL, 0.18 mmol) were added to a solution of 5-(*tert*-butyl) 4-methyl (3a*S*,4*S*,6a*R*)-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (TFA salt, 100 mg, 0.17 mmol) in MeCN (1.7 mL). The resulting mixture was stirred at ambient temperature for 18 hours then filtered. The filtrate was concentrated under reduced pressure and the resulting residue was purified by silica gel chromatography (EtOAc in hexanes) to afford 5-(*tert*-butyl) 4-methyl (3a*S*,4*S*,6a*R*)-1-ethyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate. LCMS (C₂₈H₄₈BN₂O₆⁺) (ES, m/z): 519 [M+H]⁺.

### Step 2: (3aR,4S,6aR)-3a-(3-boronopropyl)-1-ethyloctahydropyrrolo[3,4-b]pyrrole-4-carboxylic acid

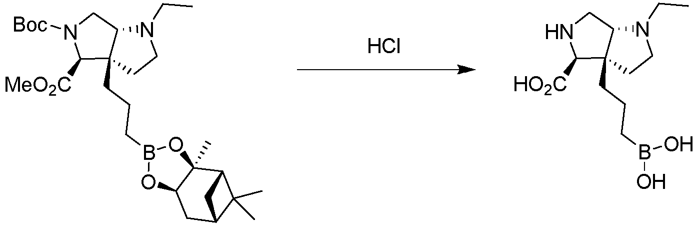

A mixture of 5-(*tert*-butyl) 4-methyl (3a*S*,4*S*,6a*R*)-1-ethyl-3a-(3-((3a*S*,4*S*,6*S*,7a*R*)-3a,5,5-trimethylhexahydro-4,6-methanobenzo[*d*][1,3,2]dioxaborol-2-yl)propyl)hexahydropyrrolo[3,4-*b*]pyrrole-4,5(1*H*)-dicarboxylate (28 mg, 0.054 mmol) and 6 M HCl (500 µL, 3.0 mmol) was heated in a microwave reactor with stirring at 120 °C for 1.5 hours. The reaction mixture was washed with DCM and the resulting aqueous layer was concentrated to give (3a*R*,4*S*,6a*R*)-3a-(3-boronopropyl)-1-ethyloctahydropyrrolo[3,4-*b*]pyrrole-4-carboxylic acid as an HCl salt. LCMS (C₁₂H₂₂BN₂O₃⁺) (ES, m/z): 253 [M-H₂O+H]⁺. ¹H NMR (499 MHz, D₂O) δ 4.28 - 4.05 (m, 2H), 4.03 - 3.79 (m, 2H), 3.65 (d, *J =* 14.2 Hz, 1H), 3.54 - 3.28 (m, 2H), 3.27 - 3.15 (m, 1H), 2.44 - 2.23 (m, 2H), 1.68 - 1.49 (m, 2H), 1.48 - 1.32 (m, 2H), 1.32 - 1.22 (m, 3H), 0.80 - 0.69 (m, 2H).

### Example 7: (3aR,4S,6aR)-1-(2-aminoethyl)-3a-(3-boronopropyl)octahydropyrrolo[3,4-b]pyrrole-4-carboxylic acid

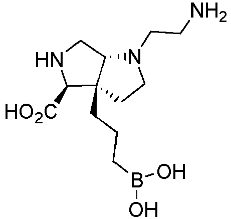

Example 7 was made according to the reaction described in Example 6, using appropriate starting materials. LCMS (C₁₂H₂₃BN₃O₃⁺) (ES, m/z): 268 [M-H₂O+H]⁺. ¹H NMR (499 MHz, D₂O) δ 4.22 (s, 1H), 4.10 - 4.02 (m, 1H), 3.88 (dd, *J =* 14.1, 7.3 Hz, 1H), 3.83 - 3.73 (m, 1H), 3.64 (dd, *J* = 14.1, 3.7 Hz, 1H), 3.55 - 3.42 (m, 1H), 3.39 - 3.26 (m, 4H), 2.39 - 2.22 (m, 2H), 1.61 - 1.47 (m, 2H), 1.47 - 1.33 (m, 2H), 0.77 - 0.71 (m, 2H).

### Example 8: (3aS,6aS)-3a-(3-boronopropyl)hexahydrocyclopenta[b]pyrrole-6a(1H)-carboxylic acid

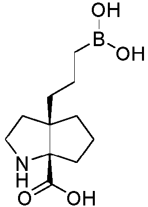

### Step 1: 1-(tert-butyl) 2-methyl (2S,3S)-3-allyl-3-(3-((tert-butyldimethylsilyl)oxy)propyl)-4-oxopyrrolidine-1,2-dicarboxylate

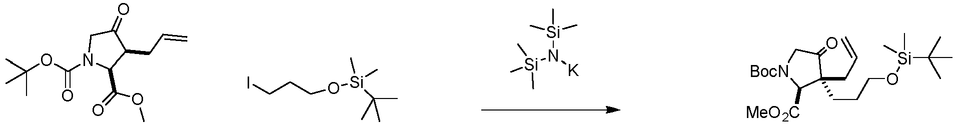

KHMDS (1.0 M in THF, 39 mL, 39 mmol) was added dropwise to a stirred solution of 1-(tert-butyl) 2-methyl (2S,3S)-3-allyl-4-oxopyrrolidine-1,2-dicarboxylate (10 g, 35 mmol) in THF (132 ml) and 1,3-dimethyl-3,4,5,6-tetrahydro-2(1H)-pyrimidinone (44 mL) at -78 °C. The resulting mixture was stirred at -78°C for 1 h, followed by dropwise addition of tert-butyl(3-iodopropoxy)dimethylsilane (21 g, 71 mmol) in THF (5.0 mL) at -78 °C. The reaction mixture was stirred at -78 °C for 1 hour then at room temperature for 12 hours. The reaction mixture was quenched with saturated aqueous NH₄Cl, and extracted with EtOAc. The combined organic phases were concentrated and the residue was purified by silica gel column chromatography (EtOAc in hexanes) to afford 1-(tert-butyl) 2-methyl (2S,3S)-3-allyl-3-(3-((tertbutyldimethylsilyl)oxy)propyl)-4-oxopyrrolidine-1,2-dicarboxylate. LCMS (C₁₈H₃₄NO₄Si⁺) (ES, m/z): 356 [M+H-Boc]⁺.

### Step 2: 1-(tert-butyl) 2-methyl (2S,3S)-3-allyl-3-(3-((tert-butyldimethylsilyl)oxy)propyl)-4-hydroxypyrrolidine-1,2-dicarboxylate

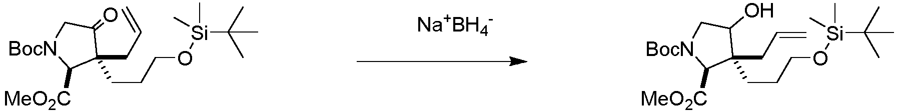

NaBH₄ (2.491 g, 65.8 mmol) was added in small portions to a stirred solution of 1-(tert-butyl) 2-methyl (2S,3S)-3-allyl-3-(3-((tert-butyldimethylsilyl)oxy)propyl)-4-oxopyrrolidine-1,2-dicarboxylate (10 g, 22 mmol) in methanol (200 mL) at 0°C, and the resulting mixture was stirred at 0°C for 30 min then at room temperature for 12 h. The reaction mixture was diluted with brine and water, and extracted with EtOAc. The combined organic layers were washed with brine, dried over anhydrous MgSO₄, filtered, and concentrated under reduced pressure. The residue was purified by silica gel column chromatography (EtOAc in hexanes) to give 1-(tert-butyl) 2-methyl (2S,3S)-3-allyl-3-(3-((tert-butyldimethylsilyl)oxy)propyl)-4-hydroxypyrrolidine-1,2-dicarboxylate. LCMS (C₁₈H₃₆NO₄Si⁺) (ES, m/z): 358 [M+H-Boc]⁺.

### Step 3: 1-(tert-butyl) 2-methyl (2S,3S)-3-allyl-3-(3-((tert-butyldimethylsilyl)oxy)propyl)-4-((4-methoxybenzyl)oxy)pyrrolidine-1,2-dicarboxylate

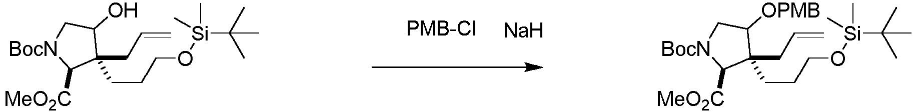

NaH (60 wt% in mineral oil, 262 mg, 6.6 mmol) was added to a stirred solution of 1-(tert-butyl) 2-methyl (2S,3S)-3-allyl-3-(3-((tert-butyldimethylsilyl)oxy)propyl)-4-hydroxypyrrolidine-1,2-dicarboxylate (2.0 g, 4.4 mmol) in DMF (10 mL) at 0°C, and the resulting mixture was stirred at 0 °C for 30 min, followed by dropwise addition of 4-methoxybenzyl chloride (0.65 mL, 4.8 mmol) at 0 °C. The reaction mixture was then stirred at room temperature for 12 hours, then diluted with water and extracted with EtOAc. The combined organic phase was washed with brine, dried over anhydrous MgSO₄, filtered, and concentrated under reduced pressure. The residue was purified by silica gel column chromatography (EtOAc in hexanes) to give 1-(tert-butyl) 2-methyl (2S,3S)-3-allyl-3-(3-((tert-butyldimethylsilyl)oxy)propyl)-4-((4-methoxybenzyl)oxy)pyrrolidine-1,2-dicarboxylate. LCMS (C₂₆H₄₄NO₅Si⁺) (ES, m/z): 478 [M+H-Boc]⁺.

### Step 4: 1-(tert-butyl) 2-methyl (2S,3S)-3-allyl-3-(3-hydroxypropyl)-4-((4-methoxybenzyl)oxy)pyrrolidine-1,2-dicarboxylate

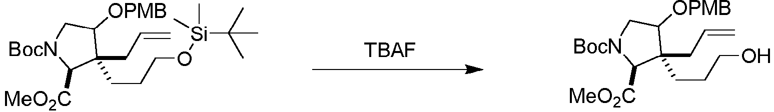

TBAF (1.0 M in THF, 19 mL, 19 mmol) was added to a stirred solution of 1-(tert-butyl) 2-methyl (2S,3S)-3-allyl-3-(3-((tert-butyldimethylsilyl)oxy)propyl)-4-((4-methoxybenzyl)oxy)pyrrolidine-1,2-dicarboxylate (2.2 g, 3.8 mmol) in THF (5.0 mL) and the resulting mixture was stirred at room temperature for 1 hour, then quenched with saturated aqueous NH₄Cl, and extracted with EtOAc. The combined organic phases were concentrated, and the residue was purified by silica gel column chromatography (EtOAc in hexanes) to give 1-(tert-butyl) 2-methyl (2S,3S)-3-allyl-3-(3-hydroxypropyl)-4-((4-methoxybenzyl)oxy)pyrrolidine-1,2-dicarboxylate. LCMS (C₂₀H₃₀NO₅⁺) (ES, m/z): 364 [M+H-Boc]⁺.

### Step 5: 1-(tert-butyl) 2-methyl (2S,3S)-3-allyl-3-(3-bromopropyl)-4-((4-methoxybenzyl)oxy)pyrrolidine-1,2-dicarboxylate

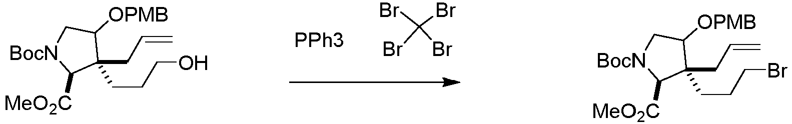

CBr₄ (973 mg, 2.93 mmol) was added to a stirred solution of 1-(tert-butyl) 2-methyl (2S,3S)-3-allyl-3-(3-hydroxypropyl)-4-((4-methoxybenzyl)oxy)pyrrolidine-1,2-dicarboxylate (1.4 g, 2.9 mmol) and PPh₃ (769 mg, 2.9 mmol) in DCM (15 mL) at 0 °C, and the resulting mixture was stirred for 90 min at room temperature. The reaction mixture was concentrated and the residue was purified by silica gel column chromatography (EtOAc in hexanes) to give (1-(tert-butyl) 2-methyl (2S,3S)-3-allyl-3-(3-bromopropyl)-4-((4-methoxybenzyl)oxy)pyrrolidine-1,2-dicarboxylate. LCMS (C₂₀H₂₉BrNO₄⁺) (ES, m/z): 426 [M+H-Boc]⁺.

### Step 6: 1-(tert-butyl) 6a-methyl (3aS,6aS)-3a-allyl-3-((4-methoxybenzyl)oxy)hexahydrocyclopenta[b]pyrrole-1, 6a-dicarboxylate

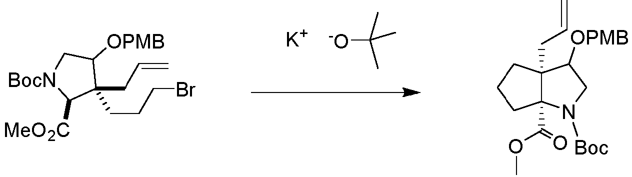

Potassium tert-butoxide (1.0 M in THF, 2.8 mL, 2.8 mmol) was added dropwise to a stirred solution of 1-(tert-butyl) 2-methyl (2S,3S)-3-allyl-3-(3-bromopropyl)-4-((4-methoxybenzyl)oxy)pyrrolidine-1,2-dicarboxylate (1.2 g, 2.3 mmol) in DCM (15 mL), and the resulting mixture was sirred at room temperature for 12 hours, then quenched with methanol and concentrated under reduced pressure. The residue was purified by silica gel column chromatography (EtOAc in hexanes) to give 1-(tert-butyl) 6a-methyl (3aS,6aS)-3a-allyl-3-((4-methoxybenzyl)oxy)hexahydrocyclopenta[b]pyrrole-1,6a-dicarboxylate. LCMS (C₂₀H₂₈NO₄⁺) (ES, m/z): 346 [M+H-Boc]⁺.

### Step 7: 1-(tert-butyl) 6a-methyl (3aR,6aS)-3-((4-methoxybenzyl)oxy)-3a-(3-(4,4,5,5-tetramethyl-1,3,2-dioxaborolan-2-yl)propyl)hexahydrocyclopenta[b]pyrrole-1,6a-dicarboxylate

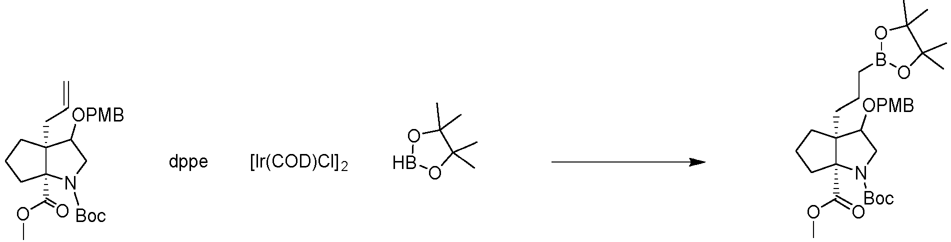

4,4,5,5-Tetramethyl-1,3,2-dioxaborolane (1.5 mL, 9.6 mmol) was added to a stirred mixture of chloro(1,5-cyclooctadiene)iridium(I) dimer (49 mg, 0.096 mmol), and DPPE (76 mg, 0.19 mmol) in DCM (9.6 mL) under N₂, and the resulting mixture was stirred for 20 min at 25°C, followed by addition of 1-(tert-butyl) 6a-methyl (3aS,6aS)-3a-allyl-3-((4-methoxybenzyl)oxy)hexahydrocyclopenta[b]pyrrole-1,6a-dicarboxylate (852 mg, 1.9 mmol) in DCM (9.6 mL) under N₂. The reaction mixture was stirred at 25 °C for 15 hours, and then concentrated. The residue was directly purified by silica gel column chromatography (EtOAc in hexanes) to give 1-(tert-butyl) 6a-methyl (3aR,6aS)-3-((4-methoxybenzyl)oxy)-3a-(3-(4,4,5,5-tetramethyl-1,3,2-dioxaborolan-2-yl)propyl)hexahydrocyclopenta[b]pyrrole-1,6a-dicarboxylate. LCMS (C₂₆H₄₁BNO₆⁺) (ES, m/z): 474 [M+H-Boc]⁺.

### Step 8: 1-(tert-butyl) 6a-methyl (3aR,6aS)-3-hydroxy-3a-(3-(4,4,5,5-tetramethyl-1,3,2-dioxaborolan-2-yl)propyl)hexahydrocyclopenta[b]pyrrole-1,6a-dicarboxylate

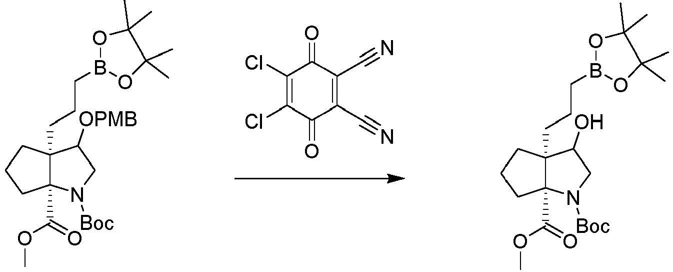

DDQ (387 mg, 1.7 mmol) was added to a stirred mixture of 1-(tert-butyl) 6a-methyl (3aR,6aS)-3-((4-methoxybenzyl)oxy)-3a-(3-(4,4,5,5-tetramethyl-1,3,2-dioxaborolan-2-yl)propyl)hexahydrocyclopenta[b]pyrrole-1,6a-dicarboxylate (978 mg, 1.7 mmol) in DCM (10 mL) and water (1.0 mL) at 0°C under N₂. The reaction mixture was stirred at room temperature under N₂ for 2 hours, then quenched with saturated aqueous NaHCO₃ and extracted with DCM. The combined organic phases were dried over anhydrous MgSO₄, filtered, and concentrated. The residue was purified by silica gel column chromatography (EtOAc in hexanes) to give 1-(tert-butyl) 6a-methyl (3aR,6aS)-3-hydroxy-3a-(3-(4,4,5,5-tetramethyl-1,3,2-dioxaborolan-2-yl)propyl)hexahydrocyclopenta[b]pyrrole-1,6a-dicarboxylate. LCMS (C₁₈H₃₃BNO₅⁺) (ES, m/z): 354 [M+H-Boc]⁺.

### Step 9: 1-(tert-butyl) 6a-methyl (3aR,6aS)-3a-(3-(4,4,5,5-tetramethyl-1,3,2-dioxaborolan-2-yl)propyl)-3-(((p-tolyloxy)carbonothioyl)oxy)hexahydrocyclopenta[b]pyrrole-1,6a-dicarboxylate

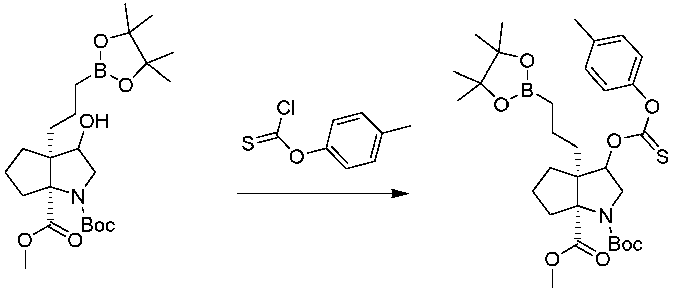

p-Tolyl chlorothionoformate (0.27 mL, 1.8 mmol) was added dropwise to a stirred mixture of 1-(tert-butyl) 6a-methyl (3aR,6aS)-3-hydroxy-3a-(3-(4,4,5,5-tetramethyl-1,3,2-dioxaborolan-2-yl)propyl)hexahydrocyclopenta[b]pyrrole-1,6a-dicarboxylate (160 mg, 0.35 mmol), DMAP (17 mg, 0.14 mmol), and pyridine (0.14 mL, 1.8 mmol) in DCM (5.0 mL) at 0°C, and the resulting mixture was stirred at room temperature for 15 hours. Additional DMAP (17 mg, 0.14 mmol), pyridine (0.14 mL, 1.8 mmol) and p-Tolyl chlorothionoformate (0.27 mL, 1.8 mmol) were added, and the reaction mixture was stirred at 40 °C for 2 hours. The reaction was then quenched by saturated aqueous NaHCO₃, and extracted with DCM. The combined organic phases were dried over anhydrous Na₂SO₄, filtered, and concentrated. The residue was purified by silica gel column chromatography (EtOAc in hexanes) to give 1-(tert-butyl) 6a-methyl (3aR,6aS)-3a-(3-(4,4,5,5-tetramethyl-1,3,2-dioxaborolan-2-yl)propyl)-3-(((p-tolyloxy)carbonothioyl)oxy)hexahydrocyclopenta[b]pyrrole-1,6a-dicarboxylate. LCMS (C₂₆H₃₉BNO₆S⁺) (ES, m/z): 504 [M+H-Boc]⁺.

### Step 10: 1-(tert-butyl) 6a-methyl (3aS,6aS)-3a-(3-(4,4,5,5-tetramethyl-1,3,2-dioxaborolan-2-yl)propyl)hexahydrocyclopenta[b]pyrrole-1,6a-dicarboxylate

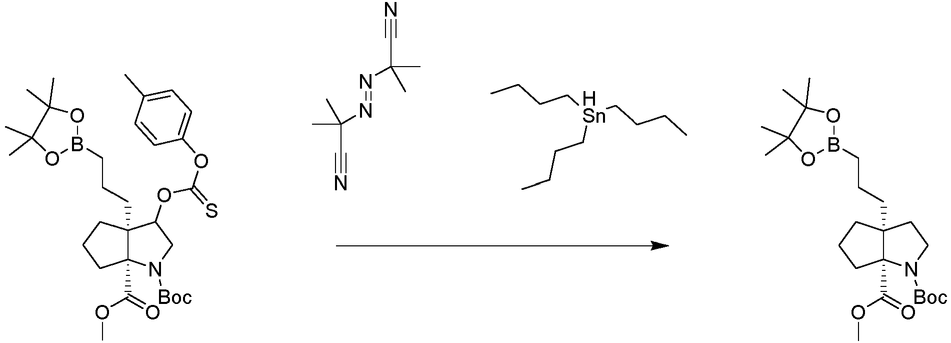

2,2'-Azobis(2-methylpropionitrile) (5.9 mg, 0.036 mmol) and tri-n-butyltin hydride (0.21 mL, 0.80 mmol) were added to a stirred solution of 1-(tert-butyl) 6a-methyl (3aR,6aS)-3a-(3-(4,4,5,5-tetramethyl-1,3,2-dioxaborolan-2-yl)propyl)-3-(((p-tolyloxy)carbonothioyl)oxy)hexahydrocyclopenta[b]pyrrole-1,6a-dicarboxylate (218 mg, 0.36 mmol) in toluene (5.0 mL) in one portion at room temperature under N₂, and the resulting mixture was stirred at 110 °C for 1 hour, then concentrated under reduced pressure. The residue was purified by silica gel column chromatography (EtOAc in hexanes) to give 1-(tert-butyl) 6a-methyl (3aS,6aS)-3a-(3-(4,4,5,5-tetramethyl-1,3,2-dioxaborolan-2-yl)propyl)hexahydrocyclopenta[b]pyrrole-1,6a-dicarboxylate. LCMS (C₁₈H₃₃BNO₄⁺) (ES, m/z): 338 [M+H]⁺.

### Step 11: (3aS,6aS)-3a-(3-boronopropyl)hexahydrocyclopenta[b]pyrrole-6a(1H)-carboxylic acid

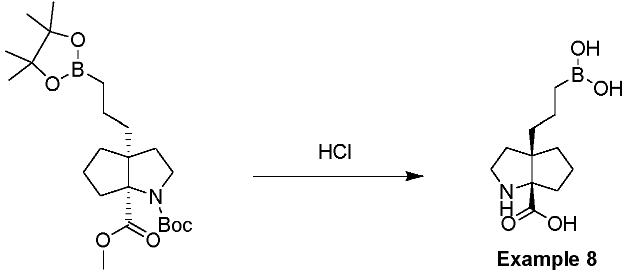

A mixture of 1-(tert-butyl) 6a-methyl (3aS,6aS)-3a-(3-(4,4,5,5-tetramethyl-1,3,2-dioxaborolan-2-yl)propyl)hexahydrocyclopenta[b]pyrrole-1,6a-dicarboxylate (95 mg, 0.22 mmol) in 8 N HCl in water (3.0 mL, 24 mmol) was stirred at 130 °C for 90 min in a microwave reactor. The reaction mixture was diluted with water, washed with DCM, and the aqueous phase was concentrated in vacuo. The crude mixture was purified by RP-HPLC [C18 column, water (20 mM HFBA and 0.1% TFA)-CH₃CN]. The fractions containing desired product mass were combined and lyophilized, and the residue was dissolved in 1 N HCl (2.0 mL, 2.0 mmol) and lyophilized to give (3aS,6aS)-3a-(3-boronopropyl)hexahydrocyclopenta[b]pyrrole-6a(1H)-carboxylic acid as an HCl salt. LCMS (C₁₁H₁₉BNO₃⁺) (ES, m/z): 224 [M-H₂O+H]⁺. ¹H NMR (D₂O) δ: 3.44 (ddd, J = 12.5, 7.1, 5.6 Hz, 1H), 3.37 (ddd, J = 11.8, 8.5, 7.0 Hz, 1H), 2.57 - 2.47 (m, 1H), 2.03 (dh, J = 14.0, 7.5 Hz, 3H), 1.95 - 1.86 (m, 1H), 1.80 (s, 2H), 1.84 - 1.74 (m, 1H), 1.57 - 1.38 (m, 3H), 1.32 (td, J = 11.9, 4.2 Hz, 1H), 0.79 (t, J = 7.6 Hz, 2H)

### Example 9: (3aS,6aS)-3a-(3-((3aS,4S,6S,7aR)-3a,5,5-trimethylhexahydro-4,6-methanobenzo[d][1,3,2]dioxaborol-2-yl)propyl)hexahydrocyclopenta[b]pyrrole-6a(1H)-carboxylic Acid

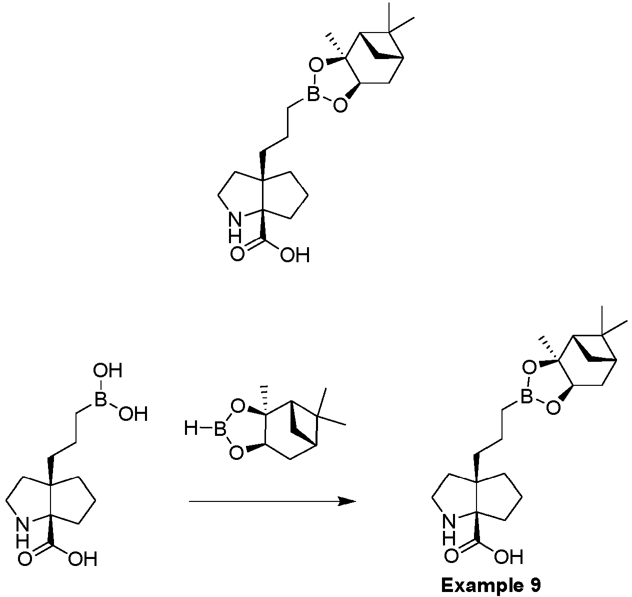

A mixture of (3aS,6aS)-3a-(3-boronopropyl)hexahydrocyclopenta[b]pyrrole-6a(1H)-carboxylic acid (20 mg, 0.083 mmol) and (1R,2R,3S,5R)-(-)-2,3-pinanediol (28 mg, 0.17 mmol) in MeCN (3.0 mL) was stirred at 85 °C for 2 hours under N₂. The reaction mixture was concentrated, and the residue was purified by RP-HPLC [C18 column, water (0.1% TFA)-CH₃CN] to give (3aS,6aS)-3a-(3-((3aS,4S,6S,7aR)-3a,5,5-trimethylhexahydro-4,6-methanobenzo[d][1,3,2]dioxaborol-2-yl)propyl)hexahydrocyclopenta[b]pyrrole-6a(1H)-carboxylic acid as a TFA salt. LCMS (C₂₁H₃₅BNO₄⁺) (ES, m/z): 376 [M+H]⁺. ¹H NMR (DMSO-*d*₆) δ: 4.27 (dd, J = 8.8, 1.9 Hz, 1H), 3.28 - 3.13 (m, 2H), 2.37 - 2.24 (m, 2H), 2.17 (dtd, J = 10.8, 6.2, 2.1 Hz, 1H), 1.97 - 1.87 (m, 2H), 1.85 (tt, J = 5.7, 2.8 Hz, 1H), 1.83 - 1.79 (m, 1H), 1.79 - 1.67 (m, 3H), 1.68 - 1.64 (m, 1H), 1.64 - 1.57 (m, 1H), 1.50 - 1.32 (m, 2H), 1.30 (s, 3H), 1.33 - 1.17 (m, 1H), 1.24 (s, 3H), 0.98 (d, J = 10.7 Hz, 1H), 0.81 (s, 3H), 0.71 (t, J = 7.4 Hz, 2H)

### Example 10: (3aR,6aS)-3a-(3-boronopropyl)-3-hydroxyhexahydrocyclopenta[b]pyrrole-6a(1H)-carboxylic acid

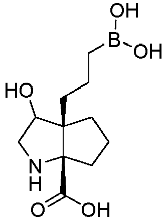

### Step 1: 1-(tert-butyl) 6a-methyl (3aR,6aS)-3-(((chloromethyl)sulfonyl)oxy)-3a-(3-(4,4,5,5-tetramethyl-1, 3, 2-dioxaborolan-2-yl)propyl)hexahydrocyclopenta[b]pyrrole-1,6a-dicarboxylate

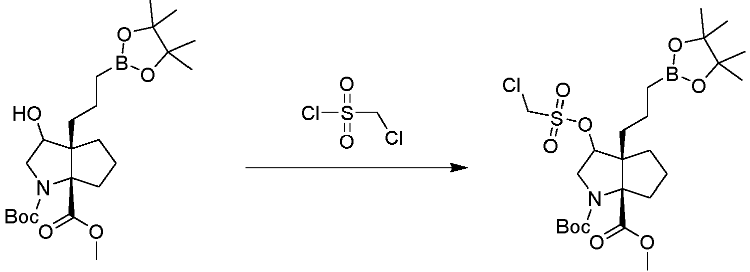

Chloromethansulfonyl chloride (0.15 mL, 1.5 mmol) was added dropwise to a stirred solution of 1-(tert-butyl) 6a-methyl (3aR,6aS)-3-hydroxy-3a-(3-(4,4,5,5-tetramethyl-1,3,2-dioxaborolan-2-yl)propyl)hexahydrocyclopenta[b]pyrrole-1,6a-dicarboxylate (230 mg, 0.51 mmol) and 2,6-lutidine (0.59 mL, 5.1 mmol) in DCM (1.7 mL) at 0 °C, and the resulting mixture was stirred at 0 °C for 20 min, then at room temperature for 30 min. The reaction mixture was concentrated, then diluted with H₂O, and extracted with EtOAc. The combined organic phase was dried over anhydrous Na₂SO₄, filtered, and concentrated under reduced pressure. The residue was purified by silica gel column chromatography (EtOAc in hexanes) to give 1-(tert-butyl) 6a-methyl (3aR,6aS)-3-(((chloromethyl)sulfonyl)oxy)-3a-(3-(4,4,5,5-tetramethyl-1,3,2-dioxaborolan-2-yl)propyl)hexahydrocyclopenta[b]pyrrole-1,6a-dicarboxylate. LCMS (C₁₉H₃₄BclNO₇S⁺) (ES, m/z): 466 [M+H-Boc]⁺.

### Step 2: 1-(tert-butyl) 6a-methyl (3aR,6aS)-3-acetoxy-3a-(3-(4,4,5,5-tetramethyl-1,3,2-dioxaborolan-2-yl)propyl)hexahydrocyclopenta[b]pyrrole-1,6a-dicarboxylate

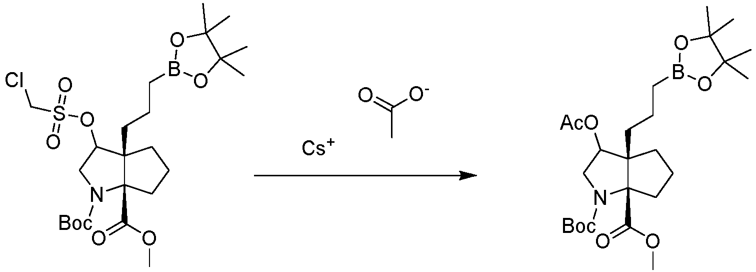

Cesium acetate (63 mg, 0.33 mmol) and 18-crown-6 (14 mg, 0.055 mmol) were added to a stirred solution of 1-(tert-butyl) 6a-methyl (3aR,6aS)-3-(((chloromethyl)sulfonyl)oxy)-3a-(3-(4,4,5,5-tetramethyl-1,3,2-dioxaborolan-2-yl)propyl)hexahydrocyclopenta[b]pyrrole-1,6a-dicarboxylate (62 mg, 0.11 mmol) in toluene (1.0 mL), and the resulting mixture was stirred at 80 °C for 2 hours. The reaction mixture was diluted with saturated aqueous NaHCO₃, and extracted with EtOAc. The combined organic layer was washed with brine, dried over anhydrous MgSO₄, filtered, and concentrated. The residue was purified by silica gel column chromatography (EtOAc in hexanes) to give 1-(tert-butyl) 6a-methyl (3aF,6aS)-3-acetoxy-3a-(3-(4,4,5,5-tetramethyl-1,3,2-dioxaborolan-2-yl)propyl)hexahydrocyclopenta[b]pyrrole-1,6a-dicarboxylate. LCMS (C₂₀H₃₅BNO₆⁺) (ES, m/z): 396 [M+H]⁺.

### Step 3: (3aR,6aS)-3a-(3-boronopropyl)-3-hydroxyhexahydrocyclopenta[b]pyrrole-6a(1H)-carboxylic acid

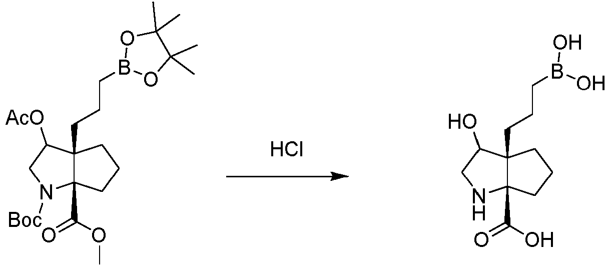

A mixture of 1-(tert-butyl) 6a-methyl (3aR,6aS)-3-acetoxy-3a-(3-(4,4,5,5-tetramethyl-1,3,2-dioxaborolan-2-yl)propyl)hexahydrocyclopenta[b]pyrrole-1,6a-dicarboxylate (25 mg, 0.050 mmol) in 8 N HCl in water (3.0 mL, 24 mmol) was stirred at 130 °C for 90 minutes in a microwave reactor. The reaction mixture was diluted with water, washed with DCM, and the aqueous layer was concentrated in vacuo. The crude mixture was purified by RP-HPLC [C18 column, water (20 mM HFBA and 0.1% TFA)-CH₃CN]. The fractions containing desired product mass were combined and lyophilized, and the residue was dissolved in 1 N HCl (2.0 mL, 2.0 mmol) and lyophilized to give (3aR,6aS)-3a-(3-boronopropyl)-3-hydroxyhexahydrocyclopenta[b]pyrrole-6a(1H)-carboxylic acid as an HCl salt. LCMS (C₁₁H₁₉BNO₄⁺) (ES, m/z): 240 [M-H₂O+H]⁺. ¹H NMR (D₂O) δ: 4.33 (broad s, 1H), 3.49 (broad s, 2H), 2.53 (dt, J = 15.3, 7.9 Hz, 1H), 2.11 - 2.03 (m, 1H), 1.99 (dt, J = 13.7, 7.2 Hz, 1H), 1.92 (dt, J = 13.2, 6.6 Hz, 1H),, 1.73 - 1.70 (m, 1H), 1.60 (dt, J = 13.4, 6.7 Hz, 1H), 1.52 - 1.44 (m, 4H), 0.82 (s, 2H)

### Example 11: (1R)-7-(3-boronopropyl)-2-azabicyclo[2.2.1]heptane-1-carboxylic acid

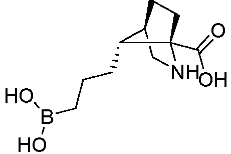

### Step 1: 1-(tert-butyl) 2-methyl (2R,3R,Z)-3-allyl-4-(2-(benzyloxy)-2-oxoethylidene)pyrrolidine-1,2-dicarboxylate

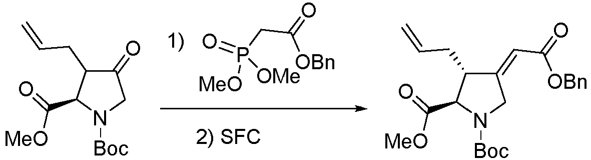

Benzyl 2-(dimethoxyphosphoryl)acetate (17 g, 66 mmol) was added to a mixture of sodium hydride (60 wt% in mineral oil, 2.3 g, 58 mmol) in THF (194 mL) at 0°C. The resulting mixture was stirred at room temperature for 30 minutes, followed by addition of a solution of 1-(tert-butyl) 2-methyl (2R)-3-allyl-4-oxopyrrolidine-1,2-dicarboxylate (11 g, 39 mmol) in THF (4.0 mL) at 0 °C. The reaction mixture was stirred at 0 °C for 30 minutes, then quenched by saturated aqueous NH₄Cl, and extracted with EtOAc. The combined organic phase was washed with brine, dried over anhydrous sodium sulfate, filtered, and concentrated under vacuum. The crude mixture was purified by silica gel chromatography (EtOAc in Hexanes), then further resolved by SFC [Column: Lux-4 (250 mm*21 mm), Mobile phase: A: CO₂, B: MeOH w/ 0.1% NH₄OH, Gradient: 5 % of B, Flow Rate (mL/min) 70] to give 1-(tert-butyl) 2-methyl (2R,3R,Z)-3-allyl-4-(2-(benzyloxy)-2-oxoethylidene)pyrrolidine-1,2-dicarboxylate as the second eluting peak (Rt = 7.4 min). LCMS (C₁₈H₂₂NO₄⁺) (ES, m/z): 316 [M-C₅H₈O₂+H]⁺.

### Step 2: 1-(tert-butyl) 2-methyl (2R,3R,Z)-4-(2-(benzyloxy)-2-oxoethylidene)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate

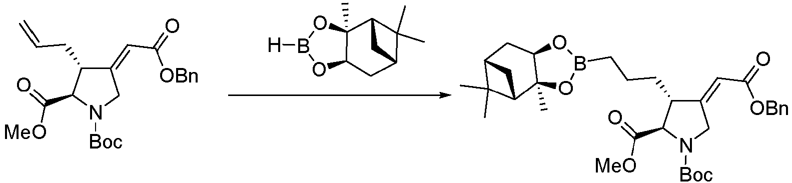

(3aS,4S,6S,7aR)-3a,5,5-trimethylhexahydro-4,6-methanobenzo[d][1,3,2]dioxaborole (16 g, 87 mmol) was added to a mixture of 1,2-bis(diphenylphosphino)ethane (0.60 g, 1.5 mmol), and chloro(1,5-cyclooctadiene)iridium(I) dimer (0.56 g, 1.1 mmol) in DCM (347 mL) under N₂, and the resulting mixture was stirred at 25 °C for 20 minutes, followed by addition of 1-(tert-butyl) 2-methyl (2R,3R,Z)-3-allyl-4-(2-(benzyloxy)-2-oxoethylidene)pyrrolidine-1,2-dicarboxylate (9.0 g, 22 mmol) in DCM (87 mL) under N₂. The reaction mixture was stirred at room temperature for 3 h, then quenched with MeOH, and concentrated under reduced pressure. The residue was purified by silica gel chromatography (ethyl acetate in hexanes) to give 1-(tert-butyl) 2-methyl (2R,3R,Z)-4-(2-(benzyloxy)-2-oxoethylidene)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate. LCMS (C₂₈H₃₉BNO₆⁺) (ES, m/z): 496 [M-C₅H₈O₂+H]⁺.

### Step 3: 2-((4R,5R)-1-(tert-butoxycarbonyl)-5-(methoxycarbonyl)-4-(3-((3aS,4S,6S,7aR)-3a,5,5-trimethylhexahydro-4, 6-methanobenzo[d][1,3,2]dioxaborol-2-yl)propyl)pyrrolidin-3-yl)acetic acid

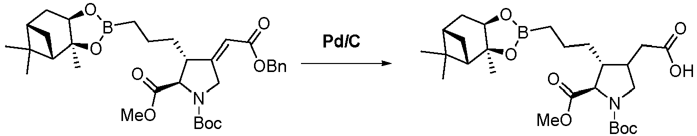

Pd/C (10% wt, 143 mg, 0.134 mmol) was added to a solution of 1-(tert-butyl) 2-methyl (2R,3R,Z)-4-(2-(benzyloxy)-2-oxoethylidene)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate (400 mg, 0.67 mmol) in MeOH (6.7 mL) under N₂. The resulting mixture was evacuated under vacuum and refilled with hydrogen (3x). The reaction mixture was stirred under H₂ (balloon) at room temperature for 40 h, then filtered and concentrated under reduced pressure to give crude 2-((4R,5R)-1-(tert-butoxycarbonyl)-5-(methoxycarbonyl)-4-(3-((3aS,4S,6S,7aR)-3a,5,5-trimethylhexahydro-4,6-methanobenzo[d][1,3,2]dioxaborol-2-yl)propyl)pyrrolidin-3-yl)acetic acid, which was directly used in the next step without further purification. LCMS (C₂₁H₃₅BNO₆⁺) (ES, m/z): 408 [M-C₅H₈O₂+H]⁺.

### Step 4: 1-(tert-butyl) 2-methyl (2R,3R)-4-(2-hydroxyethyl)-3-(3-((3aS,4S,6S,7aR)-3a,5, 5-trimethylhexahydro-4,6-methanobenzo[d][1,3,2]dioxaborol-2-yl)propyl)pyrrolidine-1,2-dicarboxylate

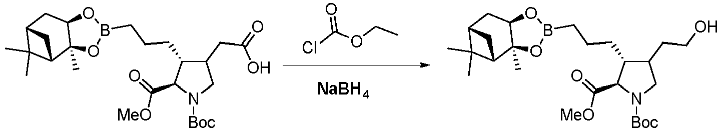

Triethylamine (1.0 mL, 7.0 mmol) and ethyl carbonochloridate (0.68 mL, 7.0 mmol) were added to a solution of 2-((4R,5R)-1-(tert-butoxycarbonyl)-5-(methoxycarbonyl)-4-(3-((3aS,4S,6S,7aR)-3a,5,5-trimethylhexahydro-4,6-methanobenzo[d][1,3,2]dioxaborol-2-yl)propyl)pyrrolidin-3-yl)acetic acid (2.8 g, 5.4 mmol) in THF (54 mL) at -10 °C, and the resulting mixture was stirred at -10 °C for 30 minutes. The reaction mixture was diluted with Et₂O, filtered, and concentrated under reduced pressure. The crude mixture was dissolved in MeOH (54 mL) and sodium tetrahydroborate (410 mg, 11 mmol) was added in small portions at -10 °C, and the reaction mixture was stirred at 0 °C for 1 h, then quenched with saturated aqueous NH₄Cl, and extracted with DCM. The combined organic layers were dried over anhydrous Na₂SO₄, filtered, and concentrated in vacuo. The residue was purified by silica gel chromatography (EtOAc in Hexanes) to provide 1-(tert-butyl) 2-methyl (2R,3R)-4-(2-hydroxyethyl)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate. LCMS (C₂₁H₃₇BNO₅⁺) (ES, m/z): 394 [M-C₅H₈O₂+H]⁺.

### Step 5: 1-(tert-butyl) 2-methyl (2R,3R)-4-(2-bromoethyl)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate

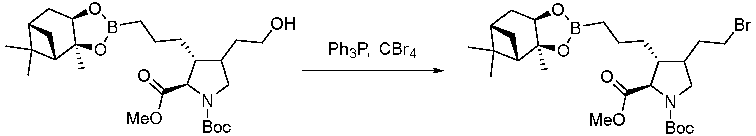

Perbromomethane (1.1 g, 3.5 mmol) was added to a solution of 1-(tert-butyl) 2-methyl (2R,3R)-4-(2-hydroxyethyl)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate (1.2 g, 2.3 mmol) and triphenylphosphane (917 mg, 3.5 mmol) in DCM (47 mL) at 0 °C, and the resulting mixture was stirred at room temperature for 90 minutes. The reaction mixture was concentrated under reduced pressure, and the residue was purified by silica gel chromatography (EtOAc in hexanes) to give 1-(tert-butyl) 2-methyl (2R,3R)-4-(2-bromoethyl)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate. LCMS (C₂₁H₃₆BBrNO₄⁺) (ES, m/z): 456 [M-C₅H₈O₂+H]⁺.

### Step 6: 2-(tert-butyl) 1-methyl (1R)-7-(3-((3aS,4S,6S,7aR)-3a,5,5-trimethylhexahydro-4,6-methanobenzo[d][1,3,2]dioxaborol-2-yl)propyl)-2-azabicyclo[2.2.1]heptane-1,2-dicarboxylate

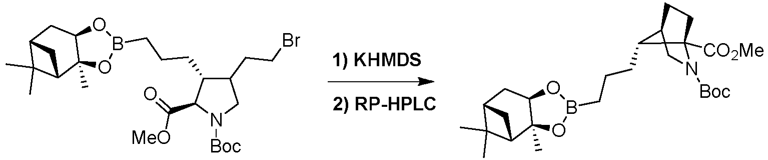

Potassium bis(trimethylsilyl)amid (1.0 M in THF, 1.7 mL, 1.7 mmol) was added to a solution of 1-(tert-butyl) 2-methyl (2R,3R)-4-(2-bromoethyl)-3-(3-((3aS,4S,6S,7aR)-3a,5,5-trimethylhexahydro-4,6-methanobenzo[d][1,3,2]dioxaborol-2-yl)propyl)pyrrolidine-1,2-dicarboxylate (800 mg, 1.4 mmol) in THF (58 mL) at - 78 °C, and the resulting mixture was stirred at - 78 °C for 30 minutes, then at -20°C for 10 hours. The reaction mixture was quenched with saturated aqueous NH₄Cl and brine, and extracted with EtOAc. The combined organic layer was washed with brine, dried over anhydrous Na₂SO₄, filtered, and concentrated under reduced pressure. The residue was purified by silica gel chromatography (EtOAc in hexanes) and further purified by reverse-phase HPLC (C18 column, MeCN / water with 0.1% TFA modifier) to afford 2-(tert-butyl) 1-methyl (1R)-7-(3-((3aS,4S,6S,7aR)-3a,5,5-trimethylhexahydro-4,6-methanobenzo[d][1,3,2]dioxaborol-2-yl)propyl)-2-azabicyclo[2.2.1]heptane-1,2-dicarboxylate. LCMS (C₂₁H₃₅BNO₄⁺) (ES, m/z): 376 [M-C₅H₈O₂+H]⁺.

### Step 7: methyl (1R)-7-(3-((3aS,4S,6S,7aR)-3a,5,5-trimethylhexahydro-4, 6-methanobenzo[d][1,3,2]dioxaborol-2-yl)propyl)-2-azabicyclo[2.2.1]heptane-1-carboxylate

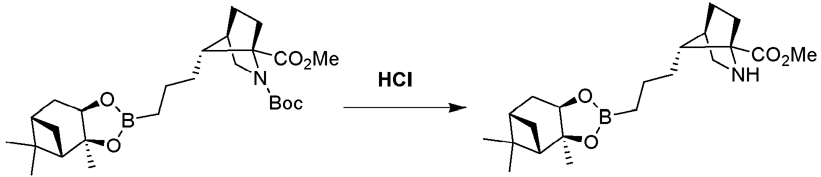

HCl (4.0 M in dixoane, 1.0 mL, 4.0 mmol) was added to a mixture of 2-(tert-butyl) 1-methyl (1R)-7-(3-((3aS,4S,6S,7aR)-3a,5,5-trimethylhexahydro-4,6-methanobenzo[d][1,3,2]dioxaborol-2-yl)propyl)-2-azabicyclo[2.2.1]heptane-1,2-dicarboxylate (105 mg, 0.22 mmol) in EtOAc (5.0 mL), and the reaction mixture was stirred at rom temperature for 16 hours, then concentrated under reduced pressure. The residue was purified by reverse-phase HPLC [C18 column, water (0.1% TFA)-CH₃CN] to afford methyl (1R)-7-(3-((3aS,4S,6S,7aR)-3a,5,5-trimethylhexahydro-4,6-methanobenzo[d][1,3,2]dioxaborol-2-yl)propyl)-2-azabicyclo[2.2.1]heptane-1-carboxylate. LCMS (C₂₁H₃₅BNO₄⁺) (ES, m/z): 376 [M+H]⁺.

### Step 8: (1R)-7-(3-boronopropyl)-2-azabicyclo[2.2.1]heptane-1-carboxylic acid

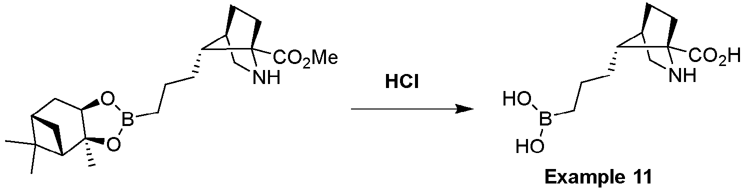

A mixture of methyl (1R)-7-(3-((3aS,4S,6S,7aR)-3a,5,5-trimethylhexahydro-4,6-methanobenzo[d][1,3,2]dioxaborol-2-yl)propyl)-2-azabicyclo[2.2.1]heptane-1-carboxylate; (TFA salt, 105 mg, 0.22 mmol) in 12 N HCl in water (1.0 mL, 12 mmol), acetic acid (0.50 mL), and water (0.50 mL) was stirred at 130 °C for 75 minutes in a microwave reactor. The reaction mixture was diluted with water, and washed with DCM. The aqueous layer was concentrated under reduced pressure, and the residue was dissolved in 2N NH₄OH in water (5.0 mL). The resulting solution was lyophilized overnight, and the crude product was dissolved in water, followed by addition of DOWEX 50WX8 resin (4.0 g). The mixture was stirred for 2 h, and the resin was washed with 2 N NH₄OH in water (10 mL X 4). The combined NH₄OH solution was lyophilized to give (1R)-7-(3-boronopropyl)-2-azabicyclo[2.2.1]heptane-1-carboxylic acid as a free base. LCMS (C₁₀H₁₉BNO₄⁺) (ES, m/z): 228 [M+H]⁺. ¹H NMR (499 MHz, D₂O) δ 3.47 (dt, *J =* 11.4, 3.3 Hz, 1H), 3.19 (d, *J* = 11.4 Hz, 1H), 2.69 (q, *J =* 3.0 Hz, 1H), 2.42 (dd, *J* = 9.5, 5.1 Hz, 1H), 2.38 - 2.28 (m, 1H), 2.12 - 1.96 (m, 2H), 1.67 (tq, *J =* 9.1, 4.0 Hz, 1H), 1.59 - 1.26 (m, 4H), 0.89 - 0.74 (m, 2H).

### Example 12: (5R)-8-(3-boronopropyl)-2,6-diazabicyclo[3.2.1]octane-5-carboxylic acid

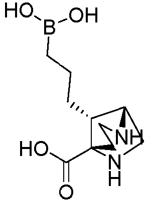

### Step 1: 1-(tert-butyl) 2-methyl (2S,3R,4R)-3-allyl-4-(benzylamino)pyrrolidine-1,2-dicarboxylate

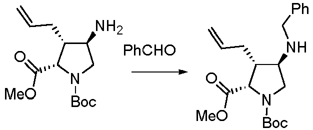

Benzaldehyde (33 uL, 0.32 mmol) and acetic acid (17 uL, 0.29 mmol) were added to a solution of 1-(*tert*-butyl) 2-methyl (2S,3R,4R)-3-allyl-4-aminopyrrolidine-1,2-dicarboxylate (83 mg, 0.29 mmol) in MeOH (2.0 mL), and the resulting mixture was stirred at room temperature for 1 hour, followed by addition of sodium cyanoborohydride (22 mg, 0.35 mmol) at 0 °C. The reaction mixture was stirred at room temperature for 18 hours, followed by addition of triethylamine (81 uL, 0.58 mmol), and concentrated under reduced pressure. The residue was purified by silica gel chromatography (EtOAc in hexanes) to afford 1-(tert-butyl) 2-methyl (2S,3R,4R)-3-allyl-4-(benzylamino)pyrrolidine-1,2-dicarboxylate. LCMS (C₂₁H₃₁N₂O₄⁺) (ES, m/z): 375 [M+H]⁺.

### Step 2: 1-(tert-butyl) 2-methyl (2S,3R,4R)-3-allyl-4-(benzyl(2-bromoethyl)amino)pyrrolidine-1,2-dicarboxylate

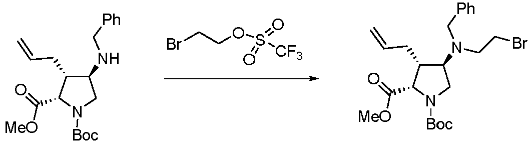

2-Bromoethyl trifluoromethanesulfonate (3.1 g, 12 mmol) was added to a solution of 1-(tert-butyl) 2-methyl (2S,3R,4R)-3-allyl-4-(benzylamino)pyrrolidine-1,2-dicarboxylate (1.5 g, 4.0 mmol) in toluene (20 mL) at 0 °C, and the resulting mixture was stirred at room temperature for 30 hours. The reaction mixture was diluted with EtOAc and quenched with saturated aqueous NaHCO₃. The organic layer was washed with brine, dried over Na₂SO₄, filtered, and concentrated under reduced pressure. The residue was purified by silica gel chromatography (EtOAc in hexanes) to give 1-(tert-butyl) 2-methyl (2S,3R,4R)-3-allyl-4-(benzyl(2-bromoethyl)amino)pyrrolidine-1,2-dicarboxylate. LCMS (C₂₃H₃₄BrN₂O₄⁺) (ES, m/z): 481 [M+H]⁺.

### Step 3: 6-(tert-butyl) 5-methyl 8-allyl-2-benzyl-2,6-diazabicyclo[3.2.1]octane-5,6-dicarboxylate

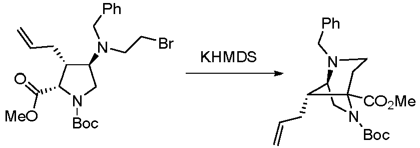

Potassium bis(trimethylsilyl)amide (1.0 M in THF, 4.5 mL, 4.5 mmol) was added dropwise to a solution of (2S,3R,4R)-1-tert-butyl 2-methyl 3-allyl-4-(benzyl(2-bromoethyl)amino)pyrrolidine-1,2-dicarboxylate (1.9 g, 4.0 mmol) in THF (79 mL) at - 78°C, and the resulting mixture was stirred at - 78 °C for 30 minutes, then at -20 °C for 10 h. The reaction mixture was quenched with saturated aqueous NH₄Cl and saturated aqueous NaHCO₃, then diluted with EtOAc. The organic layer was washed with brine, dried over anhydrous Na₂SO₄, filtered, and concentrated under reduced pressure. The residue was purified by silica gel chromatography (EtOAc in hexanes) to give 6-(tert-butyl) 5-methyl 8-allyl-2-benzyl-2,6-diazabicyclo[3.2.1]octane-5,6-dicarboxylate. LCMS (C₂₃H₃₃N₂O₄⁺) (ES, m/z): 401 [M+H]⁺.

### Step 4: 6-(tert-butyl) 5-methyl 2-benzyl-8-(3-(4,4,5,5-tetramethyl-1, 3,2-dioxaborolan-2-yl)propyl)-2,6-diazabicyclo[3.2.1]octane-5,6-dicarboxylate

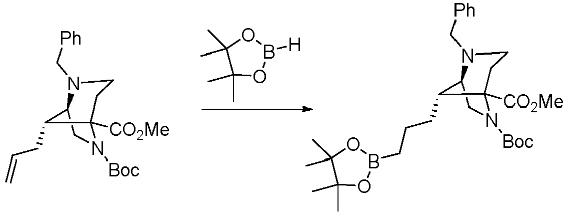

4,4,5,5-Tetramethyl-1,3,2-dioxaborolane (0.24 mL, 1.5 mmol) was added to a mixture of chloro(1,5-cyclooctadiene)iridium(I) dimer (19 mg, 0.037 mmol), DPPE (30 mg, 0.075 mmol) in DCM (2.5 mL) under N₂, and the resulting mixture was stirred at 25 °C for 20 minutes, followed by addition of 6-tert-butyl 5-methyl 8-allyl-2-benzyl-2,6-diazabicyclo[3.2.1]octane-5,6-dicarboxylate (100 mg, 0.25 mmol) in DCM (2.5 mL) under N₂. The reaction mixture was stirred at 25 °C for 3 h, then quenched with MeOH and water, and concentrated under reduced pressure. The residue was purified by silica gel chromatography (EtOAc in hexanes) to give 6-(tert-butyl) 5-methyl 2-benzyl-8-(3-(4,4,5,5-tetramethyl-1,3,2-dioxaborolan-2-yl)propyl)-2,6-diazabicyclo[3.2.1]octane-5,6-dicarboxylate. LCMS (C₂₉H₄₆BN₂O₆⁺) (ES, m/z): 529 [M+H]⁺.

### Step 5: 6-(tert-butyl) 5-methyl 8-(3-(4,4,5,5-tetramethyl-1,3,2-dioxaborolan-2-yl)propyl)-2,6-diazabicyclo[3.2.1]octane-5,6-dicarboxylate

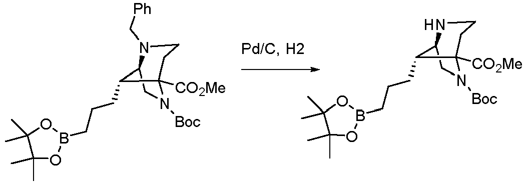

Pd/C (10% wt, 35 mg, 0.033 mmol) was added to a solution of 6-tert-butyl 5-methyl 2-benzyl-8-(3-(4,4,5,5-tetramethyl-1,3,2-dioxaborolan-2-yl)propyl)-2,6-diazabicyclo[3.2.1]octane-5,6-dicarboxylate (88 mg, 0.17 mmol) in EtOAc (5.0 mL). The resulting mixture was evacuated under vacuum and refilled with H₂ (3X), then stirred under H₂ (balloon) at room temperature for 15 hours. The reaction mixture was filtered, and concentrated under reduced pressure. The residue was purified by silica gel chromatography (MeOH in DCM) to afford 6-(tert-butyl) 5-methyl 8-(3-(4,4,5,5-tetramethyl-1,3,2-dioxaborolan-2-yl)propyl)-2,6-diazabicyclo[3.2.1]octane-5,6-dicarboxylate. LCMS (C₂₂H₄₀BN₂O₆⁺) (ES, m/z): 439 [M+H]⁺.

### Step 6: (5R)-8-(3-boronopropyl)-2,6-diazabicyclo[3.2.1]octane-5-carboxylic acid

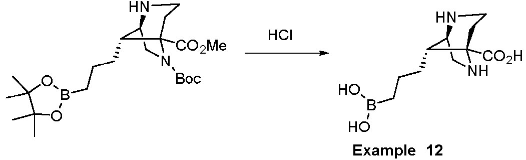

A mixture of 6-tert-butyl 5-methyl 8-(3-(4,4,5,5-tetramethyl-1,3,2-dioxaborolan-2-yl)propyl)-2,6-diazabicyclo[3.2.1]octane-5,6-dicarboxylate (55 mg, 0.125 mmol) in 12 N HCl in water (2.0 mL, 24 mmol), acetic acid (1.0 mL), and water (1.0 mL) was stirred at 130 °C for 1 h in a microwave reactor. The reaction mixture was diluted with water, washed with DCM, and the aqueous layer was concentrated under reduced pressure to give (5R)-8-(3-boronopropyl)-2,6-diazabicyclo[3.2.1]octane-5-carboxylic acid as an HCl salt. LCMS (C₁₀H₁₈BN₂O₃⁺) (ES, m/z): 225 [M-H₂O+H]⁺. ¹H NMR (499 MHz, D₂O) δ 4.36 (d, J = 4.7 Hz, 1H), 3.84 (dd, J = 14.8, 4.9 Hz, 1H), 3.76 (d, J = 14.8 Hz, 1H), 3.61 (dd, J = 14.6, 6.7 Hz, 1H), 3.24 (td, J = 13.9, 5.5 Hz, 1H), 2.63 - 2.43 (m, 2H), 2.29 (dd, J = 15.3, 5.3 Hz, 1H), 1.53 - 1.24 (m, 3H), 1.23 - 1.08 (m, 1H), 0.85 - 0.59 (m, 2H).

### Example 13: (5R)-2-(L-alanyl)-8-(3-boronopropyl)-2,6-diazabicyclo[3.2.1]octane-5-carboxylic acid

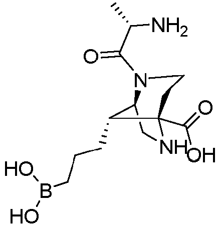

### Step 1: 6-(tert-butyl) 5-methyl (5R)-2-((tert-butoxycarbonyl)-L-alanyl)-8-(3-((3aS,4S,6S,7aR)-3a,5,5-trimethylhexahydro-4, 6-methanobenzo[d][1, 3,2]dioxaborol-2-yl)propyl)-2, 6-diazabicyclo[3.2.1]octane-5,6-dicarboxylate

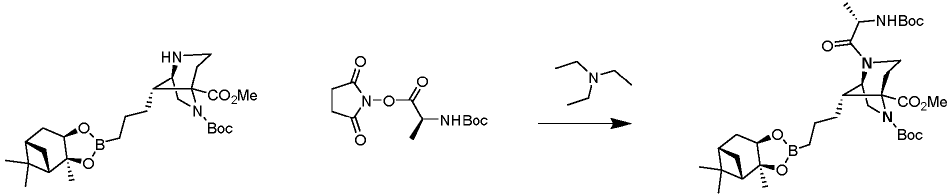

Triethylamine (0.029 mL, 0.20 mmol) and 2,5-dioxopyrrolidin-1-yl (tert-butoxycarbonyl)-L-alaninate (34 mg, 0.12 mmol) were added to a stirred solution of 6-(tert-butyl) 5-methyl 8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5,6-dicarboxylate (50 mg, 0.10 mmol)) in DCM (1.0 mL), and the resulting mixture was stirred at room temperature for 2 hours. The reaction mixture was quenched with saturated aqueous NH₄Cl, diluted with water, and extracted with DCM. The combined organic phase was dried over anhydrous Na₂SO₄, filtered, and concentrated. The residue was purified by silica gel column chromatography (EtOAc in DCM) to give 6-(tert-butyl) 5-methyl (5R)-2-((tert-butoxycarbonyl)-L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5,6-dicarboxylate. LCMS (C₃₄H₅₇BN₃O₉⁺) (ES, m/z): 662 [M+H]⁺.

### Step 2: methyl (5R)-2-(L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5-carboxylate

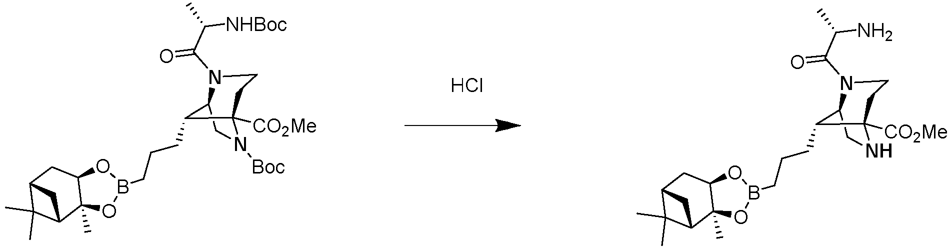

4 M HCl in dioxane (0.094 mL, 0.38 mmol) was added to a stirred solution of 6-(tert-butyl) 5-methyl (5R)-2-((tert-butoxycarbonyl)-L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5,6-dicarboxylate (25 mg, 0.038 mmol) in DCM (0.50 mL) at room temperature. The reaction mixture was stirred at room temperature for 2 hours, then concentrated in vacuo to give crude methyl (5R)-2-(L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5-carboxylate, which was used directly in the next step without further purification. LCMS (C₂₄H₄₁BN₃O₅⁺) (ES, m/z): 462 [M+H]⁺.

### Step 3: (5R)-2-(L-alanyl)-8-(3-((3aS,4S,6S,7aR)-3a,5, 5-trimethylhexahydro-4, 6-methanobenzo[d][1,3,2]dioxaborol-2-yl)propyl)-2,6-diazabicyclo[3.2.1]octane-5-carboxylic acid

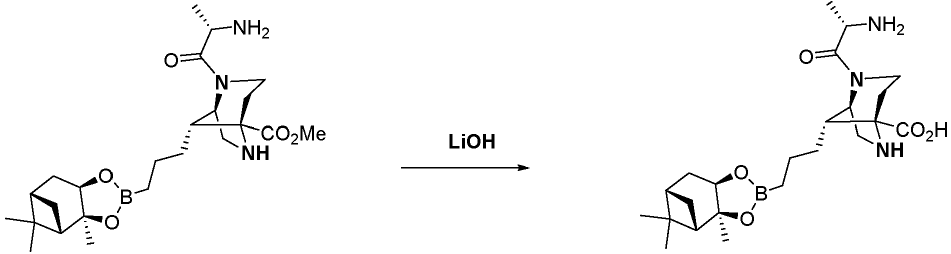

Lithium hydroxide (1.0 M in water, 0.22 mL, 0.22 mmol) was added to a stirred solution of methyl (5R)-2-(L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5-carboxylate dihydrochloride (20 mg, 0.043 mmol) in THF (0.45 mL) and MeOH (0.22 mL), and the resulting mixture was stirred at room temperature for 12 hours, then at 50 °C for 15 hours. The reaction mixture was concentrated, diluted with water and washed with EtOAc. The aqueous phase was concentrated to give crude (5R)-2-(L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5-carboxylic acid, which was used directly in the next step without further purification. LCMS (C₂₃H₃₉BN₃O₅⁺) (ES, m/z): 448 [M+H]⁺.

### Step 4: (5R)-2-(L-alanyl)-8-(3-boronopropyl)-2,6-diazabicyclo[3.2.1]octane-5-carboxylic acid

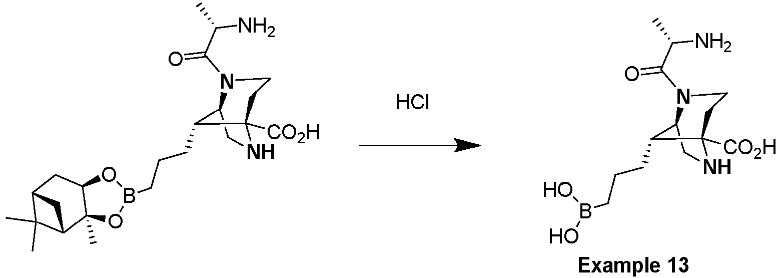

12 N HCl in water (0.14 mL, 1.7 mmol) was added dropwise to a mixture of (5R)-2-(L-alanyl)-8-(3-((3aS,4S,6S,7aR)-3a,5,5-trimethylhexahydro-4,6-methanobenzo[d][1,3,2]dioxaborol-2-yl)propyl)-2,6-diazabicyclo[3.2.1]octane-5-carboxylic acid (19 mg, 0.042 mmol) in water (1.0 mL) at 0 °C, and the resulting mixture was stirred at room temperature for 70 minutes. The reaction mixture was concentrated, and the residue was purified by RP-HPLC [C18 column, water (20 mM HFBA and 0.1% TFA)-CH₃CN] to give ((5R)-2-(L-alanyl)-8-(3-boronopropyl)-2,6-diazabicyclo[3.2.1]octane-5-carboxylic acid as an HFBA salt. LCMS (C₁₃H₂₃BN₃O₄⁺) (ES, m/z): 296 [M-H₂O+H]⁺. ¹H NMR (D₂O) δ: 5.34 - 5.21 (m, 0.7H), 4.69 - 4.53 (m, 0.6H), 4.51 - 4.36 (m, 0.7H), 4.03 (dd, J = 15.0, 6.8 Hz, 0.7H), 3.89 (dd, J = 13.6, 5.4 Hz, 0.3H), 3.86 - 3.71 (m, 1H), 3.69 - 3.52 (m, 1H), 3.68 - 3.48 (m, 0.7H), 3.19 - 3.01 (m, 0.3H), 2.55 - 2.41 (m, 0.7H), 2.43 - 2.32 (m, 0.3H), 2.26 - 2.17 (m, 2H), 1.73 (s, 0.3H), 1.65 - 1.55 (m, 0.7H), 1.53 (d, J *=* 7.0 Hz, 2.1H), 1.48 (d, J = 7.1 Hz, 0.9H), 1.47 - 1.31 (m, 1.3H), 1.36 - 1.14 (m, 1.7H), 0.95 - 0.68 (m, 2H).

### Example 14: (5R)-2-(L-valyl)-8-(3-boronopropyl)-2,6-diazabicyclo[3.2.1]octane-5-carboxylic acid

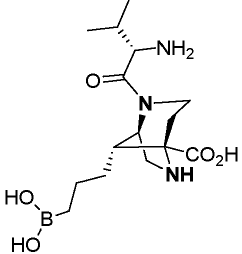

Example 14 was made following the synthisis described in Example 13 and using appropriate starting materials. Mass [M-H2O+H]+ 324.

### Example 15: (1R,3S,4R,5R,6S)-4-(3-boronopropyl)-6-hydroxy-4-methyl-2-azabicyclo[3.2.0]heptane-3-carboxylic acid

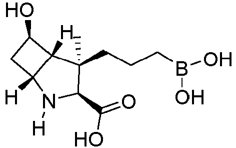

### Step 1: (2S,3S)-methyl 3-allyl-3-methyl-4-oxopyrrolidine-2-carboxylate

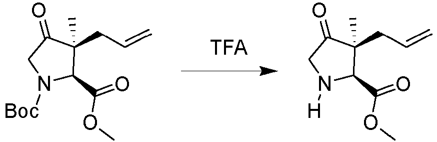

A mixture of (*2S*,*3S*)-1-*tert*-butyl 2-methyl 3-allyl-3-methyl-4-oxopyrrolidine-1,2-dicarboxylate (10 g, 34 mmol) and TFA (13 mL, 169 mmol) in DCM (200 mL) was stirred at 25 °C for 14 hours. The reaction mixture was concentrated to give crude (*2S*,*3S*)-methyl 3-allyl-3-methyl-4-oxopyrrolidine-2-carboxylate, which was used in the next step directly without further purification. LCMS (C₁₀H₁₆NO₃⁺) (ES, m/z): 198 [M+H]⁺.

### Step 2: (2S,3S)-1-benzyl 2-methyl 3-allyl-3-methyl-4-oxopyrrolidine-1,2-dicarboxylate

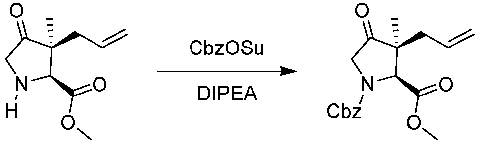

A mixture of (*2S*,*3S*)-methyl 3-allyl-3-methyl-4-oxopyrrolidine-2-carboxylate (7.0 g, 34 mmol), CbzOSu (13 g, 53 mmol), DIPEA (12 mL, 71 mmol) and DMAP (0.43 g, 3.6 mmol) in DCM (120 mL) was stirred at 25 °C for 20 h. The reaction mixture was diluted with water and extracted with DCM, and the combined organic phases were washed with brine, dried over anhydrous Na₂SO₄, filtered, and concentrated under reduced pressure. The residue was purified by silica gel column chromatography (EtOAc in hexanes) to give (*2S*,*3S*)-1-benzyl 2-methyl 3-allyl-3-methyl-4-oxopyrrolidine-1,2-dicarboxylate, which was resolved by chiral-SFC [Column: OD (250 mm*50 mm,10 um), Mobile phase: A: CO₂, B: EtOH (0.1% NH₃·H₂O), Gradient: 25% of B in 3.5 minutes, and hold 25% of B for 1 min, Flow Rate (mL/min) 200, Column temperature: 40 °C] to give (*2S*,*3S*)-1-benzyl 2-methyl 3-allyl-3-methyl-4-oxopyrrolidine-1,2-dicarboxylate (Peak 2, *t*_{R} = 1.192 min) as the second eluting peak. The stereochemistry was assigned by 2D NMR. Peak 2: LCMS (C₁₈H₂₂NO₅⁺) (ES, m/z): 332 [M+H]⁺. ¹H NMR (500 MHz, CDCl₃) *δ* 7.30 - 7.19 (m, 5H), 5.89 - 5.52 (m, 1H), 5.33 - 4.72 (m, 4H), 4.52 - 4.23 (m, 1H), 4.10 - 3.83 (m, 2H), 3.65 - 3.45 (m, 3H), 2.46 - 2.16 (m, 1H), 2.09 - 1.76 (m, 1H), 1.12 (s, 3H).

### Step 3: (2S,3S)-1-benzyl 2-methyl 3-allyl-4-hydroxy-3-methylpyrrolidine-1,2-dicarboxylate

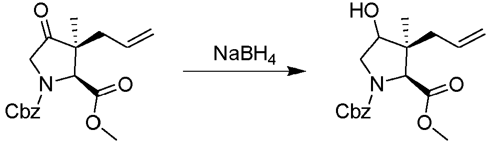

A mixture of (*2S*,*3S*)-1-benzyl 2-methyl 3-allyl-3-methyl-4-oxopyrrolidine-1,2-dicarboxylate (2.3 g, 6.9 mmol) and sodium borohydride (0.26 g, 6.9 mmol) in MeOH (30 mL) was stirred at 0 °C for 1h. The reaction mixture was quenched with saturated aqueous NH₄Cl and extracted with EtOAc, and the combined organic phases were washed with brine, dried over anhydrous Na₂SO₄, filtered, and concentrated under reduced pressure to give (*2S*,*3S*)-1-benzyl 2-methyl 3-allyl-4-hydroxy-3-methylpyrrolidine-1,2-dicarboxylate, which was used in the next step without further purification. LCMS (C₁₈H₂₄NO₅) (ESI, m/z): 334 [M+H]⁺.

### Step 4: (2S,3S)-1-benzyl 2-methyl 3-allyl-4-(((chloromethyl)sulfonyl)oxy)-3-methylpyrrolidine-1,2-dicarboxylate

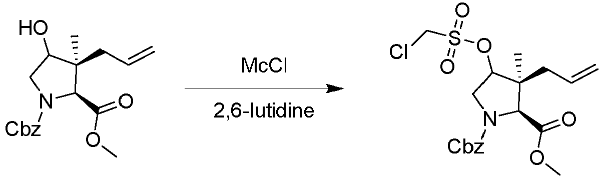

Chloromethanesulfonyl chloride (2.0 g, 13 mmol) was added dropwise to a solution of (*2S*,*3S*)-1-benzyl 2-methyl 3-allyl-4-hydroxy-3-methylpyrrolidine-1,2-dicarboxylate (2.2 g, 6.6 mmol) and 2,6-dimethylpyridine (4.2 g, 40 mmol) in DCM (30 mL) at 0 °C. The resulting mixture was slowly warmed to 25 °C and stirred for additional 4 h, then diluted with water and extracted with DCM. The combined organic phases were dried over Na₂SO₄, filtered, and concentrated *in vacuo* to give the crude (*2S*,*3S*)-1-benzyl 2-methyl 3-allyl-4-(((chloromethyl)sulfonyl)oxy)-3-methylpyrrolidine-1,2-dicarboxylate, which was used in the next step without further purification. LCMS (C₁₉H₂₅ClNO₇S⁺) (ES, m/z): 446 [M+H]⁺.

### Step 5: (2S, 3R)-1-benzyl 2-methyl 3-allyl-3-methyl-2,3-dihydro-1H-pyrrole-1,2-dicarboxylate

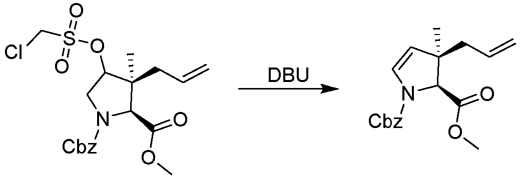

A mixture of (*2S*,*3S*)-1-benzyl 2-methyl 3-allyl-4-(((chloromethyl)sulfonyl)oxy)-3-methylpyrrolidine-1,2-dicarboxylate (2.9 g, 6.6 mmol) and DBU (2.0 mL, 13 mmol) in CH₃CN (30 mL) was stirred at 80 °C for 48 hours. The reaction mixture was quenched with 0.5 N HCl in water and extracted with EtOAc. The combined organic layers were dried over anhydrous Na₂SO₄, filtered, and concentrated *in vacuo.* The residue was purified by silica gel column chromatography (EtOAc in hexanes) to give (2S,3R)-1-benzyl 2-methyl 3-allyl-3-methyl-2,3-dihydro-1H-pyrrole-1,2-dicarboxylate. LCMS (C₁₈H₂₂NO₄⁺) (ES, m/z): 316 [M+H]⁺.

### Step 6: (3S, 4R)-2-benzyl 3-methyl 4-allyl-7,7-dichloro-4-methyl-6-oxo-2-azabicyclo[3.2.0]heptane-2,3-dicarboxylate

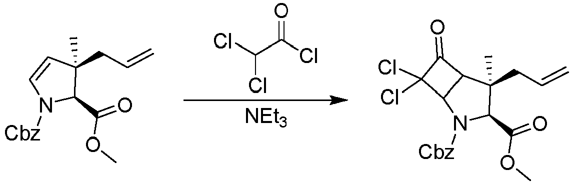

2,2-Dichloroacetyl chloride (0.32 mL, 3.3 mmol) in cyclohexane (3.0 mL) was added to a mixture of (*2S,3R*)-1-benzyl 2-methyl 3-allyl-3-methyl-2,3-dihydro-1*H*-pyrrole-1,2-dicarboxylate (400 mg, 1.3 mmol) and TEA (0.71 mL, 5.1 mmol) in cyclohexane (7.0 mL) at 45 °C. The resulting mixture was stirred at 45 °C for 24 h, followed by addition of 2,2-dichloroacetyl chloride (0.16 mL, 1.6 mmol) and triethylamine (0.35 mL, 2.5 mmol). The reaction mixture was stirred for additional 16 hours at 45 °C, then quenched with 1 N HCl in water and extracted with EtOAc. The combined organic phases were washed with brine, dried over anhydrous Na₂SO₄, filtered, and concentrated under reduced pressure. The residue was purified by RP-HPLC [C18 column, water (0.1% TFA)-CH₃CN] to give (*3S,4R*)-2-benzyl 3-methyl 4-allyl-7,7-dichloro-4-methyl-6-oxo-2-azabicyclo[3.2.0]heptane-2,3-dicarboxylate. LCMS (C₂₀H₂₂Cl₂NO₅⁺) (ESI, m/z): 426 [M+H]⁺.

### Step 7: (1R,3S, 4R, 5R)-2-benzyl 3-methyl 4-allyl-4-methyl-6-oxo-2-azabicyclo[3.2.0]heptane-2,3-dicarboxylate

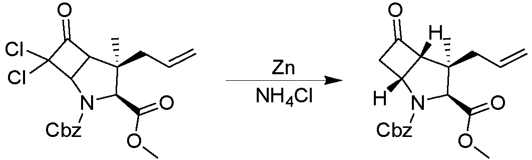

Zinc powder (391 mg, 6.0 mmol) was added to a mixture of (*3S,4R*)-2-benzyl 3-methyl 4-allyl-7,7-dichloro-4-methyl-6-oxo-2-azabicyclo[3.2.0]heptane-2,3-dicarboxylate (232 mg, 0.54 mmol) in MeOH (10 mL) and saturated aqueous NH₄Cl (5.0 mL) at 28 °C, and the resulting mixture was stirred for 2 hours at 28 °C. The reaction mixture was diluted with MeOH, filtered, and concentrated under reduced pressure. The residue was purified by RP-HPLC [C18 column, water (0.1% TFA)-CH₃CN] to give (*1R*,*3S*,*4R*,*5R*)-2-benzyl 3-methyl 4-allyl-4-methyl-6-oxo-2-azabicyclo[3.2.0]heptane-2,3-dicarboxylate. The stereochemistry was assigned by 2D NMR. LCMS (C₂₀H₂₄NO₅⁺) (ESI, m/z): 358 [M+H]⁺. ¹H NMR (500 MHz, CDCl₃) *δ* 7.34 - 7.20 (m, 5H), 5.82 - 5.66 (m, 1H), 5.20 - 4.94 (m, 4H), 4.42 - 4.32 (m, 1H), 4.30 (s, 0.5H), 4.21 (s, 0.5H), 3.72 (s, 1.6H), 3.61 - 3.55 (m, 1.4H), 3.27 - 3.08 (m, 1H), 2.92 - 2.80 (m, 1H), 2.93 - 2.77 (m, 1H), 2.28 (m, 1H), 2.12 - 1.96 (m, 1H), 1.26 - 1.14 (m, 3H).

### Step 8: 1: (1R,3S,4R,5R,6R)-2-benzyl 3-methyl 4-allyl-6-hydroxy-4-methyl-2-azabicyclo[3.2.0]heptane-2,3-dicarboxylate and 2: (1R,3S,4R,5R,6S)-2-benzyl 3-methyl 4-allyl-6-hydroxy-4-methyl-2-azabicyclo[3.2.0]heptane-2,3-dicarboxylate

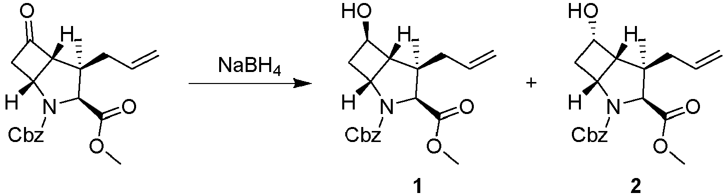

A mixture of *(1R,3S,4R*,*5R*)-2-benzyl 3-methyl 4-allyl-4-methyl-6-oxo-2-azabicyclo[3.2.0]heptane-2,3-dicarboxylate (60 mg, 0.17 mmol) and sodium borohydride (6.4 mg, 0.17 mmol) in MeOH (5.0 mL) was stirred at 0 °C for 2 h, then at 26 °C for 8 h. The reaction mixture was quenched with acetone and concentrated under reduced pressure, and the residue was purified by RP-HPLC [C18 column, water (0.1% TFA)-CH₃CN] to give (*1R,3S,4R,5R,6R*)-2-benzyl 3-methyl 4-allyl-6-hydroxy-4-methyl-2-azabicyclo[3.2.0]heptane-2,3-dicarboxylate as the first eluting peak (**Peak-1**) and (*1R*,*3S*,*4R*,*5R*,*6S*)-2-benzyl 3-methyl 4-allyl-6-hydroxy-4-methyl-2-azabicyclo[3.2.0]heptane-2,3-dicarboxylate as the second eluting peak (**Peak-2**). The stereochemistry was assigned by 2D NMR. **Peak-1:** LCMS (C₂₀H₂₆NO₅⁺) (ESI, m/z): 360 [M+H]⁺. ¹H NMR (400 MHz, CDCl₃) *δ* 7.40 - 7.27 (m, 5H), 5.85 - 5.64 (m, 1H), 5.23 - 4.93 (m, 4H), 4.30 - 4.17 (m, 2H), 4.14 (s, 0.6H), 4.10 (s, 0.4H), 3.76 (s, 1.6H), 3.48 (s, 1.4H), 2.69 (m, 1H), 2.47 - 2.18 (m, 1H), 2.12 - 1.91 (m, 2H), 1.84 - 1.71 (m, 1H), 1.27 - 1.19 (m, 3H). **Peak-2:** LCMS (C₂₀H₂₆NO₅⁺) (ESI, m/z): 360 [M+H]⁺. ¹H NMR (400 MHz, CDCl₃) *δ* 7.38 - 7.26 (m, 5H), 5.85 - 5.67 (m, 1H), 5.24 - 5.16 (m, 1H), 5.12 - 4.97 (m, 3H), 4.68 (s, 0.5H), 4.64 (s, 0.5H), 4.58 - 4.44 (m, 1H), 4.29 - 4.15 (m, 1H), 3.76 (s, 1.6H), 3.49 (s, 1.4H), 2.85 (m, 1H), 2.63 - 2.48 (m, 1H), 2.10 - 1.98 (m, 1H), 1.94 - 1.71 (m, 2H), 1.43 (s, 1.6H), 1.40 (s, 1.4H)

### Step 9: (3-((1R,3S,4R,5R, 6R)-2-((benzyloxy)carbonyl)-6-hydroxy-3-(methoxycarbonyl)-4-methyl-2-azabicyclo[3.2.0]heptan-4-yl)propyl)boronic acid

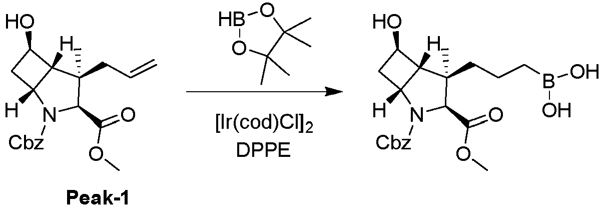

4,4,5,5-Tetramethyl-1,3,2-dioxaborolane (0.046 mL, 0.32 mmol) in DCM (1.0 mL) was added to a mixture of dppe (2.0 mg, 5.1 µmol) and [Ir(cod)Cl]₂ (2.6 mg, 3.8 µmol) in DCM (1.0 mL) under N₂ at 0 °C. The resulting mixture was stirred for 20 minutes at 0 °C, followed by addition of (*1R,3S,4R,5R,6R*)-2-benzyl 3-methyl 4-allyl-6-hydroxy-4-methyl-2-azabicyclo[3.2.0]heptane-2,3-dicarboxylate (23 mg, 0.064 mmol) in DCM (1.0 mL). The reaction mixture was stirred at 26 °C for 16 hours, then quenched with MeOH and concentrated under reduced pressure. The residue was purified by RP-HPLC [C18 column, water (0.1% TFA)-CH₃CN] to give (3-((*1R,3S,4R,5R,6R*)-2-((benzyloxy)carbonyl)-6-hydroxy-3-(methoxycarbonyl)-4-methyl-2-azabicyclo[3.2.0]heptan-4-yl)propyl)boronic acid. LCMS (C₂₀H₂₉BNO₇⁺) (ESI, m/z): 406 [M+H]⁺.

### Step 10: (1R,3S,4R,5R,6R)-4-(3-boronopropyl)-6-hydroxy-4-methyl-2-azabicyclo[3.2.0]heptane-3-carboxylic acid

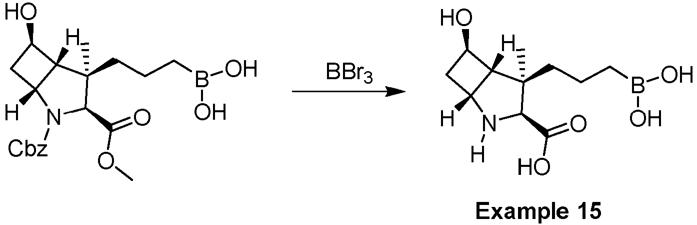

### Example 16: (1R,3S,4R,5R,6S)-4-(3-boronopropyl)-6-hydroxy-4-methyl-2-azabicyclo[3.2.0]heptane-3-carboxylic acid

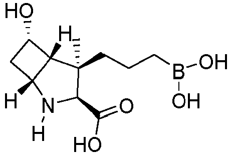

BBr₃ (500 µL, 5.3 mmol) was added to a solution of (3-((*1R,3S,4R,5R,6R*)-2-((benzyloxy)carbonyl)-6-hydroxy-3-(methoxycarbonyl)-4-methyl-2-azabicyclo[3.2.0]heptan-4-yl)propyl)boronic acid (15 mg, 0.037 mmol) in DCM (1.0 mL) at 0 °C, and the resulting mixture was stirred for 40 hours. The solvent was removed by a stream of N₂, and the residue was purified by RP-HPLC [C18 column, water (20 mMHFBA and 0.1% TFA)-CH₃CN] to give (*1R*,*3S*,*4R*,*5R*,*6R*)-4-(3-boronopropyl)-6-hydroxy-4-methyl-2-azabicyclo[3.2.0]heptane-3-carboxylic acid as an HFBA salt. LCMS (C₁₁H₁₉BNO₄⁺) (ESI, m/z): 240 [M+H-H₂O]⁺. ¹H NMR (500 MHz, D₂O) *δ* 4.44 - 4.35 (m, 1H), 4.25 - 4.19 (m, 1H), 4.17 (s, 1H), 2.93 (t, *J =* 5.8 Hz, 1H), 2.46 - 2.31 (m, 2H), 1.42 - 1.25 (m, 2H), 1.23 (s, 3H), 1.17 - 1.02 (m, 2H), 0.76 - 0.63 (m, 2H).

### Step 1: (3-((1R,3S,4R,5R,6S)-2-((benzyloxy)carbonyl)-6-hydroxy-3-(methoxycarbonyl)-4-methyl-2-azabicyclo[3.2.0]heptan-4-yl)propyl)boronic acid

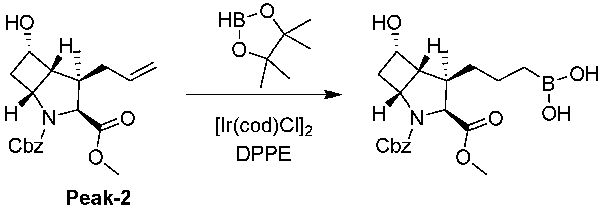

4,4,5,5-Tetramethyl-1,3,2-dioxaborolane (0.046 mL, 0.32 mmol) in DCM (1.0 mL) was added to a mixture of dppe (2.0 mg, 5.1 µmol) and [Ir(cod)Cl]₂ (2.6 mg, 3.8 µmol) in DCM (1.0 mL) under N₂ at 0 °C, and the resulting mixture was stirred for 20 minutes at 0 °C, followed by addition of (*1R*,*3S*,*4R*,*5R*,*6S*)-2-benzyl 3-methyl 4-allyl-6-hydroxy-4-methyl-2-azabicyclo[3.2.0]heptane-2,3-dicarboxylate (23 mg, 0.064 mmol) in DCM (1.0 mL). The reaction mixture was stirred at 26 °C for 16 h, then quenched with MeOH and concentrated under reduced pressure. The residue was purified by RP-HPLC [C18 column, water (0.1% TFA)-CH₃CN] to give (3-((*1R,3S,4R,5R,6S*)-2-((benzyloxy)carbonyl)-6-hydroxy-3-(methoxycarbonyl)-4-methyl-2-azabicyclo[3.2.0]heptan-4-yl)propyl)boronic acid. LCMS (C₂₀H₂₉BNO₇⁺) (ESI, m/z): 406 [M+H]⁺.

### Step 2: (1R,3S,4R,5R,6S)-4-(3-boronopropyl)-6-hydroxy-4-methyl-2-azabicyclo[3.2.0]heptane-3-carboxylic acid

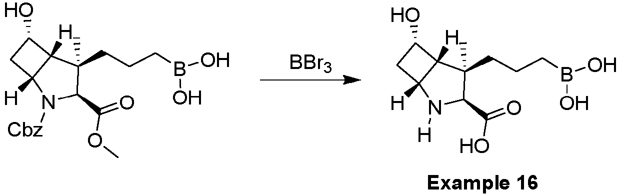

BBr₃ (500 µL, 5.3 mmol) was added to a solution of (3-((*1R,3S,4R,5R,6S*)-2-((benzyloxy)carbonyl)-6-hydroxy-3-(methoxycarbonyl)-4-methyl-2-azabicyclo[3.2.0]heptan-4-yl)propyl)boronic acid (7.0 mg, 0.017 mmol) in DCM (1.0 mL) at 0 °C, and the resulting mixture was stirred for 40 h. The solvent was removed by a stream of N₂, and the residue was purified by Pre-HPLC RP-HPLC [C18 column, water (20 mM HFBA and 0.1% TFA)-CH₃CN] to give (*1R*,*3S*,*4R*,*5R*,*6S*)-4-(3-boronopropyl)-6-hydroxy-4-methyl-2-azabicyclo[3.2.0]heptane-3-carboxylic acid as an HFBA salt. LCMS (C₁₁H₁₉BNO₄⁺) (ESI, m/z): 240 [M+H-H₂O]⁺. ¹H NMR (500 MHz, D₂O) *δ* 4.55 (s, 1H), 4.53 - 4.48 (m, 1H), 4.05 - 3.98 (m, 1H), 3.22 - 3.12 (m, 1H), 3.00 - 2.85 (m, 1H), 1.95 - 1.85 (m, 1H), 1.41 - 1.32 (m, 4H), 1.32 - 1.25 (m, 1H), 1.07 - 0.95 (m, 2H), 0.74 - 0.62 (m, 2H).

### Example 17: (1R,3S,4R,5R)-4-(3-boronopropyl)-4-methyl-2-azabicyclo[3.2.0]heptane-3-carboxylic acid

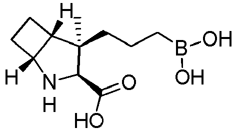

### Step 1: (1R,3S,4R,5R)-2-benzyl 3-methyl 4-allyl-6-hydroxy-4-methyl-2-azabicyclo[3.2.0]heptane-2,3-dicarboxylate

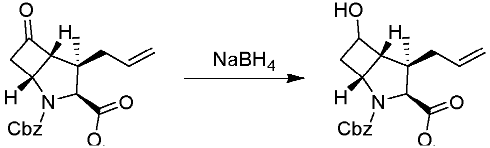

A mixture of (1*R*,3*S*,4*R*,5*R*)-2-benzyl 3-methyl 4-allyl-4-methyl-6-oxo-2-azabicyclo[3.2.0]heptane-2,3-dicarboxylate (500 mg, 1.4 mmol) and sodium borohydride (27 mg, 0.70 mmol) in MeOH (10 mL) was stirred at 24 °C for 70 minutes to give a colorless solution. The reaction mixture was quenched with saturated aqueous NH₄Cl and extracted with EtOAc, and the combined organic phases were washed with brine, dried over anhydrous Na₂SO₄, filtered, and concentrated under reduced pressure to give (1*R*,3*S*,4*R*,5*R*)-2-benzyl 3-methyl 4-allyl-6-hydroxy-4-methyl-2-azabicyclo[3.2.0]heptane-2,3-dicarboxylate, which was used in the next steps without further purification. LCMS (C₂₀H₂₆NO₅⁺) (ES, m/z): 360 [M+H]⁺.

### Step 2: (1R,3S,4R,5R)-2-benzyl 3-methyl 6-((1H-imidazole-1-carbonothioyl)oxy)-4-allyl-4-methyl-2-azabicyclo[3.2.0]heptane-2, 3-dicarboxylate

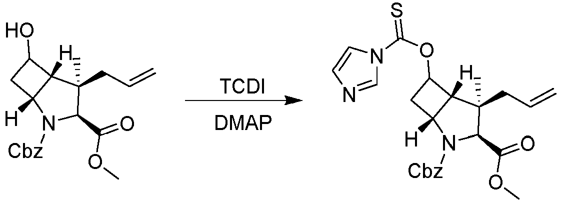

A mixture of (1*R*,3*S*,4*R*,5*R*)-2-benzyl 3-methyl 4-allyl-6-hydroxy-4-methyl-2-azabicyclo[3.2.0]heptane-2,3-dicarboxylate (480 mg, 1.3 mmol), *N,N*-dimethylpyridin-4-amine (245 mg, 2.0 mmol) and di(1*H*-imidazol-1-yl)methanethione (476 mg, 2.7 mmol) in THF (15 mL) was stirred for 50 h at 75 °C. The reaction mixture was diluted with EtOAc, filtered and the filtrate was concentrated under reduced pressure. The residue was purified by silica gel column chromatography (EtOAc in hexanes) to give (1*R*,3*S*,4*R*,5*R*)-2-benzyl 3-methyl 6-((1H-imidazole-1-carbonothioyl)oxy)-4-allyl-4-methyl-2-azabicyclo[3.2.0]heptane-2,3-dicarboxylate. LCMS (C₂₄H₂₈N₃O₅S⁺) (ES, m/z): 470 [M+H]⁺.

### Step 3: (1R,3S, 4R, 5R)-2-benzyl 3-methyl 4-allyl-4-methyl-2-azabicyclo[3.2.0]heptane-2,3-dicarboxylate

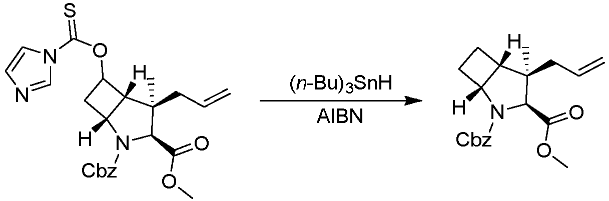

AIBN (53 mg, 0.32 mmol) was added to a mixture of (1*R*,3*S*,4*R*,5*R*)-2-benzyl 3-methyl 6-((1*H*-imidazole-1-carbonothioyl)oxy)-4-allyl-4-methyl-2-azabicyclo[3.2.0]heptane-2,3-dicarboxylate (300 mg, 0.64 mmol) and (*n*-Bu)₃SnH (1.0 mL, 3.7 mmol) in toluene (15 mL) at 25 °C under N₂. The reaction mixture was stirred at 120 °C under N₂ for 14 hours, and was then quenched with saturated aqueous KF and extracted with EtOAc. The combined organic phases were washed with brine, dried over anhydrous Na₂SO₄, filtered and concentrated under reduced pressure. The resulting residue was purified by RP-HPLC [C18 column, water (0.1% TFA)-CH₃CN] to give (1*R*,3*S*,4*R*,5*R*)-2-benzyl 3-methyl 4-allyl-4-methyl-2-azabicyclo[3.2.0]heptane-2,3-dicarboxylate. LCMS (C₂₀H₂₆NO₄⁺) (ES, m/z): 344 [M+H]⁺.

### Step 4: (1R,3S,4R,5R)-2-benzyl 3-methyl 4-methyl-4-(3-(4, 4, 5, 5-tetramethyl-1,3,2-dioxaborolan-2-yl)propyl)-2-azabicyclo[3.2.0]heptane-2,3-dicarboxylate

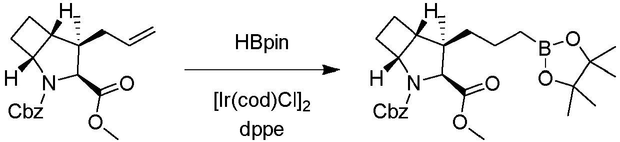

A mixture of [Ir(cod)Cl]₂ (4.9 mg, 7.3 µmol) and 1,2-bis(diphenylphosphino)ethane (5.8 mg, 0.015 mmol) in DCM (2.0 mL) was stirred at 0 °C under N₂ for 5 minutes, followed by addition of 4,4,5,5-tetramethyl-1,3,2-dioxaborolane (0.13 mL, 0.73 mmol), and a solution of (1*R,*3*S,*4*R,*5*R*)-2-benzyl 3-methyl 4-allyl-4-methyl-2-azabicyclo[3.2.0]heptane-2,3-dicarboxylate (50 mg, 0.15 mmol) in DCM (0.50 mL). The reaction mixture was stirred at 20 °C under N₂ for 15 hours, and was then concentrated under reduced pressure. The resulting residue was purified by RP-HPLC [C18 column, water (0.1% TFA)-CH₃CN] to give (1*R*,3*S*,4*R*,5*R*)-2-benzyl 3-methyl 4-methyl-4-(3-(4,4,5,5-tetramethyl-1,3,2-dioxaborolan-2-yl)propyl)-2-azabicyclo[3.2.0]heptane-2,3-dicarboxylate. LCMS (C₂₆H₃₉BNO₆⁺) (ES, m/z): 472 [M+H]⁺.

### Step 5: (1R,3S,4R,5R)-4-(3-boronopropyl)-4-methyl-2-azabicyclo[3.2.0]heptane-3-carboxylic acid

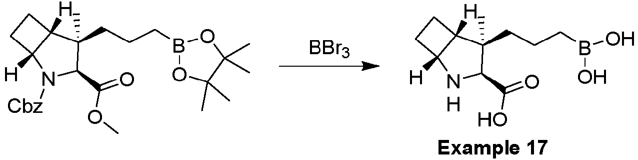

BBr₃ (500 µL, 5.3 mmol) was added to a solution of (1*R*,3*S*,4*R*,5*R*)-2-benzyl 3-methyl 4-methyl-4-(3-(4,4,5,5-tetramethyl-1,3,2-dioxaborolan-2-yl)propyl)-2-azabicyclo[3.2.0]heptane-2,3-dicarboxylate (11 mg, 0.023 mmol) in DCM (1.0 mL) at 0 °C, and the resulting mixture was stirred for 40 h. The solvent was removed by a stream of N₂, and the residue was purified by RP-HPLC [C18 column, water (20 mM HFBA and 0.1% TFA)-CH₃CN] to give (1*R*,3*S*,4*R*,5*R)-*4-(3-boronopropyl)-4-methyl-2-azabicyclo[3.2.0]heptane-3-carboxylic acid as an HFBA salt. LCMS (C₁₁H₁₉BNO₃⁺) (ES, m/z): 224 [M+H-H₂O]⁺. ¹H NMR (500 MHz, D₂O) *δ* 4.51 (s, 1H), 4.25-4.23 (m, 1H), 3.22-3.14 (m, 1H), 2.59-2.45 (m, 1H), 2.18-1.99 (m, 2H), 1.98-1.89 (m, 1H), 1.43-1.26 (m, 2H), 1.19 (s, 3H), 1.18-1.04 (m, 2H), 0.80-0.65 (m, 2H).

### Example 18: (2S,3S,4R)-2-amino-3-(3-boronopropyl)-7-azabicyclo[2.2.1]heptane-2-carboxylic acid

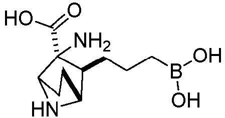

### Step 1: tert-butyl 2-allyl-3-oxo-7-azabicyclo[2.2.1]heptane-7-carboxylate

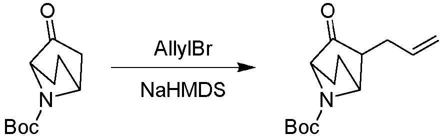

NaHMDS (10 mL, 10 mmol, 1.0 M in THF) was added to a mixture of *tert*-butyl 2-oxo-7-azabicyclo[2.2.1]heptane-7-carboxylate (2.0 g, 9.5 mmol) in THF (30 mL) at -40 °C under N₂. The resulting mixture was stirred at -40 °C for 0.5 h, followed by dropwise addition of pyrrolidine (0.34 g, 4.7 mmol) in THF (1.0 mL) at -40 °C, and subsequent dropwise addition of 3-bromoprop-1-ene (0.86 mL, 9.9 mmol) in THF (1.0 mL) at -40 °C under N₂. The reaction mixture was stirred at -40 °C for 0.5 h, then warmed to 25 °C and stirred for additional 14 h. The reaction mixture was quenched with water, and extracted with EtOAc. The combined organic layers were washed with brine, dried over anhydrous Na₂SO₄, filtered and concentrated in vacuo to give a crude mixture of *tert*-butyl 2,2-diallyl-3-oxo-7-azabicyclo[2.2.1]heptane-7-carboxylate, *tert*-butyl 2-allyl-3-oxo-7-azabicyclo[2.2.1]heptane-7-carboxylate, and *tert*-butyl 2-oxo-7-azabicyclo[2.2.1]heptane-7-carboxylate, which was used in the next steps without further purification. LCMS (C₁₀H₁₄NO₃⁺ (ES, m/z): 196 [M+H-C₄H₈]⁺.

### Step 2: 3-allyl-7-azabicyclo[2.2.1]heptan-2-one

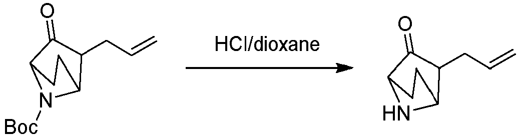

4N HCl in dioxane (20 mL, 80 mmol) was added to a mixture of *tert*-butyl 2,2-diallyl-3-oxo-7-azabicyclo[2.2.1]heptane-7-carboxylate, *tert*-butyl 2-allyl-3-oxo-7-azabicyclo[2.2.1]heptane-7-carboxylate, and *tert*-butyl 2-oxo-7-azabicyclo[2.2.1]heptane-7-carboxylate (6.3 g, 8.4 mmol) in DCM (4.0 mL) at 25 °C, and the mixture was stirred at 25 °C for 2 h. The reaction mixture was concentrated in vacuo to give a crude mixture of 3,3-diallyl-7-azabicyclo[2.2.1]heptan-2-one, 3-allyl-7-azabicyclo[2.2.1]heptan-2-one, and 7-azabicyclo[2.2.1]heptan-2-one, which was used in the next step without further purification. LCMS (C₉H₁₄NO⁺) (ES, m/z): 152 [M+H]⁺.

### Step 3: (1R,2S)-benzyl 2-allyl-3-oxo-7-azabicyclo[2.2.1]heptane-7-carboxylate

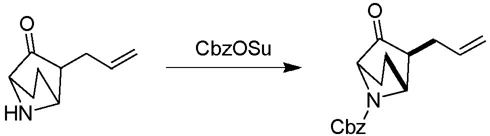

Benzyl (2,5-dioxopyrrolidin-1-yl) carbonate (7.0 g, 28 mmol) was added to a mixture of 3,3-diallyl-7-azabicyclo[2.2.1]heptan-2-one, 3-allyl-7-azabicyclo[2.2.1]heptan-2-one, and 7-azabicyclo[2.2.1]heptan-2-one (6.3 g, 14 mmol) and triethylamine (10 mL, 72 mmol) in DCM (80 mL) at 25 °C, and the mixture was stirred at 25 °C for 3 h. The reaction mixture was quenched with saturated aqueous NaHCO₃ and extracted with DCM. The combined organic layers were washed with brine, dried over anhydrous Na₂SO₄, filtered, and concentrated, and the residue was purified by silica gel column chromatography (EtOAc in hexanes) to give a mixture of benzyl 2-allyl-3-oxo-7-azabicyclo[2.2.1]heptane-7-carboxylate compound with benzyl 2,2-diallyl-3-oxo-7-azabicyclo[2.2.1]heptane-7-carboxylate and benzyl 2-allyl-3-oxo-7-azabicyclo[2.2.1]heptane-7-carboxylate, which was further purified by RP-HPLC [C18 column, water (0.1% TFA)-CH₃CN] to give (*1R,2S*)-benzyl 2-allyl-3-oxo-7-azabicyclo[2.2.1]heptane-7-carboxylate, and the stereochemistry was assigned by 2D NMR. LCMS (C₁₇H₂₀NO₃⁺) (ES, m/z): 286 [M+H]⁺. ¹H NMR (400MHz, MeOD) *δ* 7.41-7.27 (m, 5H), 5.88-5.75 (m, 1H), 5.14 (s, 2H), 5.11-5.01 (m, 2H), 4.55-4.50 (m, 1H), 4.32-4.27 (m, 1H), 2.62-2.53 (m, 1H), 2.52-2.42 (m, 1H), 2.09-2.02 (m, 1H), 1.99-1.90 (m, 1H), 1.89-1.71 (m, 2H), 1.57-1.47 (m, 1H).

### Step 4: (3S,4R)-benzyl 2-acetamido-3-allyl-2-(tert-butylcarbamoyl)-7-azabicyclo[2.2.1]heptane-7-carboxylate

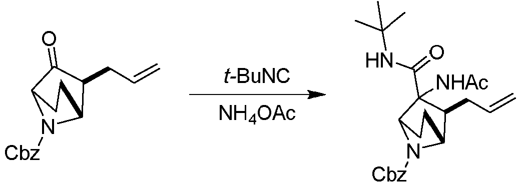

*Tert*-butyl isocyanide (0.60 mL, 5.3 mmol) was added to a mixture of (*1R,2S*)-benzyl 2-allyl-3-oxo-7-azabicyclo[2.2.1]heptane-7-carboxylate (500 mg, 1.8 mmol) and ammonium acetate (540 mg, 7.0 mmol) in 2,2,2-trifluoroethyl alcohol (5.0 mL) under N₂. The reaction mixture was allowed to stir at 40 °C for 13 h, and then quenched with water and extracted with EtOAc. The combined organic layers were washed with brine, dried over anhydrous Na₂SO₄, filtered, and concentrated in vacuo. The residue was purified by silica gel column chromatography (EtOAc in hexanes) to give *(3S,4R)-benzyl* 2-acetamido-3-allyl-2-(*tert*-butylcarbamoyl)-7-azabicyclo[2.2.1]heptane-7-carboxylate as a mixture of isomers. LCMS (C₂₄H₃₄N₃O₄⁺) (ES, m/z): 428 [M+H]⁺.

### Step 5: (2S, 3S, 4R)-benzyl 2-acetamido-3-allyl-2-(tert-butylcarbamoyl)-7-azabicyclo[2.2.1]heptane-7-carboxylate

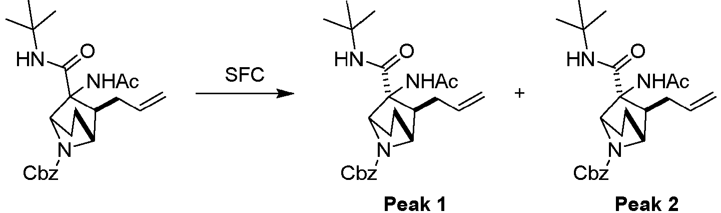

The (*3S,4R*)-benzyl 2-acetamido-3-allyl-2-(*tert*-butylcarbamoyl)-7-azabicyclo[2.2.1]heptane-7-carboxylate as a mixture of isomers (660 mg, 1.5 mmol) was resolved by chiral SFC [Column: DAICEL CHIRALPAK IC (250 mm * 30 mm, 5 um), Mobile phase: A: CO₂, B: IPA (0.1% NH₃·H₂O), Gradient: 35% of B in 5.5 minutes, and hold 35% of B for 1 min, Flow Rate (mL/min) 60, Column temperature: 40 °C] to give (*2S,3S,4R*)-benzyl 2-acetamido-3-allyl-2-(*tert-*butylcarbamoyl)-7-azabicyclo[2.2.1]heptane-7-carboxylate (**Peak 1,** tᵣ = 4.50 min) as the first eluting peak, and (*2S,3S,4R)*-benzyl 2-acetamido-3-allyl-2-(*tert*-butylcarbamoyl)-7-azabicyclo[2.2.1]heptane-7-carboxylate (**Peak 2**, tᵣ = 4.96 min) as the second eluting peak. The stereochemistry was assigned by 2D NMR. LCMS (C₂₄H₃₄N₃O₄⁺) (ES, m/z): 428 [M+H]⁺. **Peak 1:** ¹H NMR (400MHz, CDCl₃) *δ* 7.39-7.27 (m, 5H), 6.81 (br s, 1H), 5.94 (s, 1H), 5.86-5.73 (m, 1H), 5.24-5.05 (m, 5H), 4.23 (t, *J* = 3.9 Hz, 1H), 2.77-2.66 (m, 1H), 2.27-2.06 (m, 2H), 2.00 (s, 3H), 1.69-1.59 (m, 4H), 1.23 (s, 9H).

### Step 6: (2S,3S,4R)-benzyl 2-acetamido-2-(tert-butylcarbamoyl)-3-(3-(4,4,5,5-tetramethyl-1, 3,2-dioxaborolan-2-yl)propyl)-7-azabicyclo[2.2.1]heptane-7-carboxylate

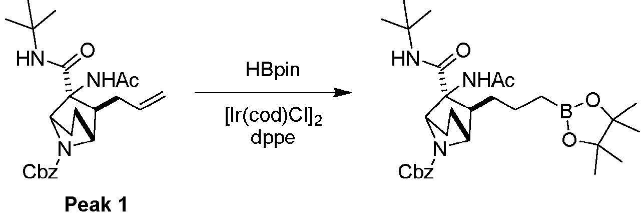

[Ir(cod)Cl]₂ (19 mg, 0.028 mmol) and dppe (22 mg, 0.056 mmol) were added to a stirred mixture of 4,4,5,5-tetramethyl-1,3,2-dioxaborolane (0.24 mL, 1.7 mmol) in anhydrous DCM (5.0 mL) at 25 °C under N₂ for 20 minutes, and the resulting mixture was treated with (*2S,3S,4R*)-benzyl 2-acetamido-3-allyl-2-(*tert*-butylcarbamoyl)-7-azabicyclo[2.2.1]heptane-7-carboxylate (240 mg, 0.56 mmol) and stirred at 25 °C for 12 h under N₂. The reaction mixture was concentrated in vacuo and the residue was purified by silica gel column chromatography (EtOAc in hexanes) to give (*2S,3S,4R*)-benzyl 2-acetamido-2-(*tert*-butylcarbamoyl)-3-(3-(4,4,5,5-tetramethyl-1,3,2-dioxaborolan-2-yl)propyl)-7-azabicyclo[2.2.1]heptane-7-carboxylate LCMS (C₃₀H₄₇BN₃O₆⁺) (ES, m/z): 556 [M+H]⁺.

### Step 7: (2S, 3S, 4R)-2-amino-3-(3-boronopropyl)-7-azabicyclo[2.2.1]heptane-2-carboxylic acid

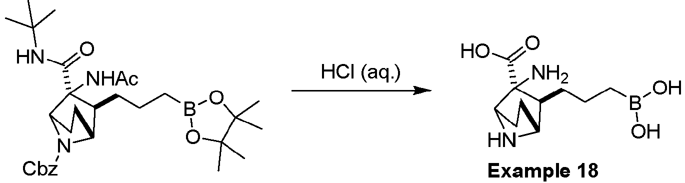

A mixture of (*2S,3S,4R*)-benzyl 2-acetamido-2-(*tert*-butylcarbamoyl)-3-(3-(4,4,5,5-tetramethyl-1,3,2-dioxaborolan-2-yl)propyl)-7-azabicyclo[2.2.1]heptane-7-carboxylate (290 mg, 0.52 mmol) and 12N HCl in water (5.0 mL, 60 mmol) was stirred at 105 °C for 13 h. The reaction mixture was washed with DCM, and the aqueous layer was concentrated in vacuum. The residue was dissolved in saturated aqueous Na₂CO₃ and washed with DCM, and the aqueous layer was concentrated in vacuum. The crude product was purified by RP-HPLC [C18 column, water (20 mM HFBA and 0.1% TFA)-CH₃CN] to give (*2S,3S,4R*)-2-amino-3-(3-boronopropyl)-7-azabicyclo[2.2.1]heptane-2-carboxylic acid as an HFBA salt. LCMS (C₁₀H₁₈BN₂O₃⁺) (ES, m/z): 225 [M+H-H₂O]⁺. ¹H NMR (400MHz, D₂O) *δ* 4.52-4.39 (m, 1H), 4.36-4.17 (m, 1H), 2.79-2.29 (m, 1H), 2.07-1.72 (m, 5H), 1.56-0.91 (m, 3H), 0.84-0.36 (m, 2H)

### Example 19: (4aS,5S,7aR)-4a-(3-boronopropyl)octahydro-1H-pyrrolo[3,4-b]pyridine-5-carboxylic acid

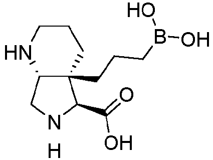

### Step 1: 1-(tert-butyl) 2-methyl (2S,3S)-3-allyl-3-(3-((tert-butyldimethylsilyl)oxy)propyl)-4-oxopyrrolidine-1,2-dicarboxylate

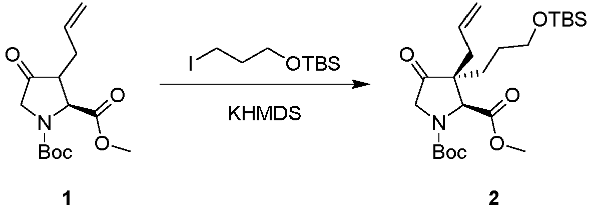

Potassium bis(trimethylsilyl)amide (4.2 mL, 2.1 mmol, 0.5 M in toluene) was added dropwise to a stirred solution of (*2S*)-1-*tert*-butyl 2-methyl 3-allyl-4-oxopyrrolidine-1,2-dicarboxylate (500 mg, 1.8 mmol) in THF (6.0 mL) and DMPU (2.0 mL) at -78 °C under N₂, and the resulting mixture was stirred at -78 °C for 1 h, followed by addition of tert-butyl(3-iodopropoxy)dimethylsilane (636 mg, 2.1 mmol) in one portion. The reaction mixture was stirred at -78 °C for 1 h, then warmed to 25 °C and stirred at 25 °C for additional 16 h. The reaction mixture was quenched by saturated aqueous NH₄Cl, then diluted with water, and extracted with EtOAc. The combined organic layers were washed with brine, dried over anhydrous Na₂SO₄, filtered, and concentrated in vacuo. The residue was purified by flash silica gel chromatography (EtOAc in hexanes) to give (*2S,3S*)-1-*tert*-butyl 2-methyl 3-allyl-3-(3-((*tert*-butyldimethylsilyl)oxy)propyl)-4-oxopyrrolidine-1,2-dicarboxylate. LCMS (C₁₈H₃₄NO₄Si⁺) (ES, m/z): 356 [M+H-Boc]⁺.

### Step 2: 1-(tert-butyl) 2-methyl (2S, 3S, 4S)-3-allyl-3-(3-((tert-butyldimethylsilyl)oxy)propyl)-4-hydroxypyrrolidine-1,2-dicarboxylate

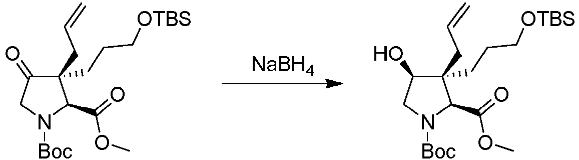

NaBH₄ (264 mg, 3.5 mmol) was added to a stirred solution of (*2S,3S*)-1-*tert*-butyl 2-methyl 3-allyl-3-(3-((*tert*-butyldimethylsilyl)oxy)propyl)-4-oxopyrrolidine-1,2-dicarboxylate (530 mg, 1.2 mmol) in MeOH (10 mL) at 0 °C under N₂, and the reaction mixture was stirred at 0 °C for 1 h, then at 25 °C for additional 12 h. The reaction mixture was quenched by addition of saturated aqueous NH₄Cl, then diluted with water, and extracted with EtOAc. The combined organic layers were washed with brine, dried over anhydrous Na₂SO₄, filtered, and concentrated in vacuo. The residue was purified by flash silica gel chromatography (EtOAc in hexanes) to give *(2S,3S,4S)-1-tert-butyl* 2-methyl 3-allyl-3-(3-((*tert*-butyldimethylsilyl)oxy)propyl)-4-hydroxypyrrolidine-1,2-dicarboxylate. LCMS (C₁₈H₃₆NO₄Si⁺) (ES, m/z): 358 [M+H-Boc]⁺.

### Step 3: 1-(tert-butyl) 2-methyl (2S, 3S, 4S)-3-allyl-4-hydroxy-3-(3-hydroxypropyl)pyrrolidine-1, 2-dicarboxylate

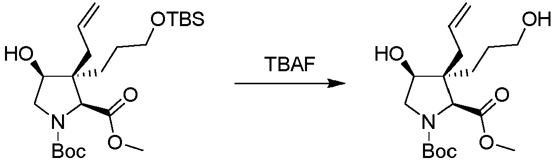

A mixture of TBAF (1.0 M in THF, 1.1 mL, 1.1 mmol) and (*2S,3S,4S*)-1-*tert*-butyl 2-methyl 3-allyl-3-(3-((*tert*-butyldimethylsilyl)oxy)propyl)-4-hydroxypyrrolidine-1,2-dicarboxylate (350 mg, 0.77 mmol) in THF (5.0 mL) was stirred at 0 °C for 1 h, and the reaction mixture was quenched by saturated aqueous NH₄Cl, then diluted with water, and extracted with EtOAc. The combined organic layers were washed with brine, dried over anhydrous Na₂SO₄, filtered, and concentrated in vacuo. The residue was purified by flash silica gel chromatography (EtOAc in hexanes) to give (*2S,3S,4S*)-1-*tert*-butyl 2-methyl 3-allyl-4-hydroxy-3-(3-hydroxypropyl)pyrrolidine-1,2-dicarboxylate. LCMS (C₁₇H₂₉NO₆Na⁺) (ES, m/z): 366 [M+Na]⁺.

### Step 4: 1-(tert-butyl) 2-methyl (2S,3S,4S)-3-allyl-4-(((chloromethyl)sulfonyl)oxy)-3-(3-chloropropyl)pyrrolidine-1, 2-dicarboxylate

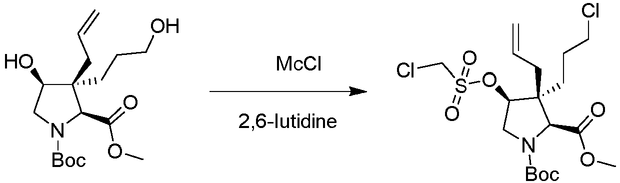

Chloromethanesulfonyl chloride (39 mg, 0.26 mmol) was added to a solution of 2,6-dimethylpyridine (187 mg, 1.7 mmol) and (*2S,3S,4S*)-1-*tert*-butyl 2-methyl 3-allyl-4-hydroxy-3-(3-hydroxypropyl)pyrrolidine-1,2-dicarboxylate (30 mg, 0.087 mmol) in DCM (1.0 mL) at 0 °C, and the resulting mixture was stirred at 0 °C for 2 h, then at 28 °C for additional 12 h. The reaction mixture was quenched by addition of saturated aqueous NH₄Cl, then diluted with water, and extracted with DCM. The combined organic layers were washed with brine, dried over anhydrous Na₂SO₄, filtered, and concentrated in vacuo to give crude (*2S,3S,4S*)-1-*tert*-butyl 2-methyl 3-allyl-4-(((chloromethyl)sulfonyl)oxy)-3-(3-chloropropyl)pyrrolidine-1,2-dicarboxylate, which was used in the next step without further purification. LCMS (C₁₈H₂₉Cl₂NO₇SNa⁺) (ES, m/z): 496 [M+Na]⁺.

### Step 5: 6-(tert-butyl) 5-methyl (4aR,5S,7aR)-4a-allyl-1-benzyloctahydro-6H-pyrrolo[3,4-b]pyridine-5,6-dicarboxylate

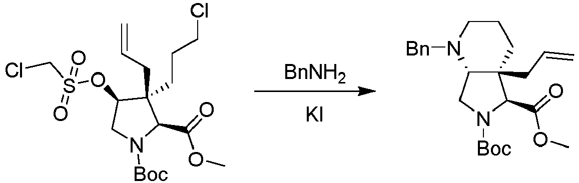

Benzylamine (1.7 g, 16 mmol) was added to a mixture of (*2S,3S,4S*)-1-*tert*-butyl 2-methyl 3-allyl-4-(((chloromethyl)sulfonyl)oxy)-3-(3-chloropropyl)pyrrolidine-1,2-dicarboxylate (150 mg, 0.32 mmol) and KI (5.3 mg, 0.032 mmol) in DMF (5.0 mL) under N₂. The resulting mixture was stirred at 70 °C for 2 h, then at 50 °C for additional 12 h. The reaction mixture was filtered and concentrated in vacuo, and the residue was purified by flash silica gel chromatography (EtOAc in hexanes) to give ((*4aR,5S,7aR)*-6-*tert*-butyl 5-methyl 4a-allyl-1-benzylhexahydro-1*H*-pyrrolo[3,4-*b*]pyridine-5,6(2H)-dicarboxylate. The stereochemistry was assigned by 2D NMR. LCMS (C₂₄H₃₅N₂O₄⁺) (ES, m/z): 415 [M+H]⁺. ¹H NMR (400 MHz, CDCl₃) *δ* 7.30 - 7.22 (m, 5H), 6.02 - 5.56 (m, 1H), 5.27 - 4.95 (m, 2H), 4.13 - 3.88 (m, 1H), 3.70 - 3.64 (m, 3H), 3.63 - 3.41 (m, 3H), 3.36 - 3.13 (m, 1H), 2.78 (m, 1H), 2.57 - 2.33 (m, 2H), 1.94 - 1.77 (m, 1H), 1.68 (m, 1H), 1.47 - 1.46 (m, 4H), 1.44 - 1.34 (m, 7H), 1.29 - 1.18 (m, 1H), 0.95 - 0.72 (m, 1H).

### Step 6: 6-(tert-butyl) 5-methyl (4aS,5S,7aR)-1-benzyl-4a-(3-(4,4,5,5-tetramethyl-1,3,2-dioxaborolan-2-yl)propyl)octahydro-6H-pyrrolo[3,4-b]pyridine-5, 6-dicarboxylate

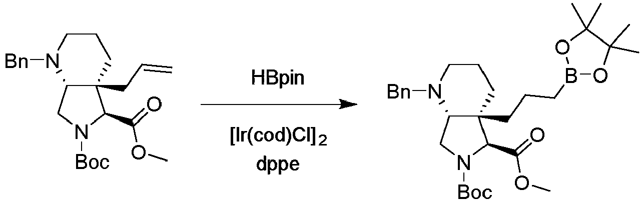

(*4aR,5S,7aR*)-6-*tert*-butyl 5-methyl 4a-allyl-1-benzylhexahydro-1*H*-pyrrolo[3,4-*b*]pyridine-5,6(2*H*)-dicarboxylate (80 mg, 0.19 mmol) in DCM (1.0 mL) was added to a stirred solution of 1,2-bis(diphenylphosphino)ethane (6.2 mg, 0.015 mmol), [Ir(cod)Cl]₂ (6.5 mg, 9.7 µmol) and 4,4,5,5-tetramethyl-1,3,2-dioxaborolane (0.14 mL, 0.97 mmol) in anhydrous DCM (2.0 mL) under N₂, and the resulting mixture was stirred at 25 °C for 15 h under N₂. The reaction mixture was concentrated, and the residue was purified by flash silica gel chromatography (EtOAc in hexanes) to give (*4aS,5S,7aR)-6-tert-butyl* 5-methyl 1-benzyl-4a-(3-(4,4,5,5-tetramethyl-1,3,2-dioxaborolan-2-yl)propyl)hexahydro-1*H*-pyrrolo[3,4-*b*]pyridine-5,6(2*H*)-dicarboxylate. LCMS (C₃₀H₄₈BN₂O₆⁺) (ES, m/z): 543 [M+H]⁺.

### Step 7: 6-(tert-butyl) 5-methyl (4aS,5S,7aR)-4a-(3-(4,4,5,5-tetramethyl-1, 3,2-dioxaborolan-2-yl)propyl)octahydro-6H-pyrrolo[3,4-b]pyridine-5,6-dicarboxylate

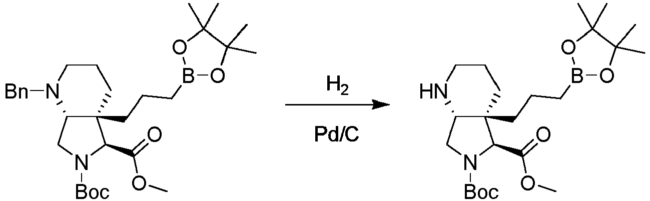

10% Pd-C (12 mg, 0.011 mmol) was added to a solution of (*4aS,5S,7aR)-6-tert-butyl* 5-methyl 1-benzyl-4a-(3-(4,4,5,5-tetramethyl-1,3,2-dioxaborolan-2-yl)propyl)hexahydro-1H-pyrrolo[3,4-*b*]pyridine-5,6(2H)-dicarboxylate (60 mg, 0.11 mmol) in MeOH (2.0 mL) under N₂. The resulting mixture was degassed and backfilled with H₂, and then stirred under H₂ (Pressure: 103 (15 psi)) at 25 °C for 2 h. The reaction mixture was filtered, and then concentrated under reduced pressure to give a crude mixture of 6-(*tert*-butyl) 5-methyl (*4aS*,*5S*,*7aR*)-4a-(3-(4,4,5,5-tetramethyl-1,3,2-dioxaborolan-2-yl)propyl)octahydro-6*H*-pyrrolo[3,4-*b*]pyridine-5,6-dicarboxylate which contained corresponding boronic acid. The crude mixture was used in next step directly without further purification. LCMS (C₁₇H₃₂BN₂O₆⁺) (ES, m/z): 371 [M-C₆H₁₀+H]⁺.

### Step 8: (4aS,5S,7aR)-4a-(3-boronopropyl)octahydro-1H-pyrrolo[3,4-b]pyridine-5-carboxylic acid

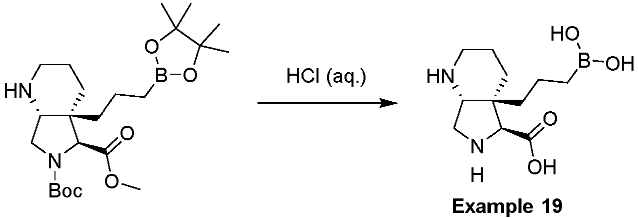

A mixture of (*4aS,5S,7aR*)-6-*tert-*butyl 5-methyl 4a-(3-(4,4,5,5-tetramethyl-1,3,2-dioxaborolan-2-yl)propyl)hexahydro-1*H*-pyrrolo[3,4-*b*]pyridine-5,6(2*H*)-dicarboxylate (50 mg, 0.111 mmol) and 12 N HCl in water (5.0 mL, 60 mmol) was stirred at 110 °C for 12 h under N₂, and the resulting mixture was concentrated in vacuo. The residue was purified by RP-HPLC [C18 column, water (20 mM HFBA and 0.1% TFA)-CH₃CN] to give (*4aS,3S,7aR*)-4a-(3-boronopropyl)octahydro-1*H*-pyrrolo[3,4-*b*]pyridine-5-carboxylic acid as an HFBA salt. LCMS (C₁₁H₂₀BN₂O₃⁺) (ES, m/z): 239 [M+H-H₂O]⁺. ¹H NMR (500 MHz, D₂O) *δ* 4.27 (br s, 1H), 3.95-3.93 (m, 2H), 3.54 - 3.51 (m, 1H), 3.36 - 3.33 (m, 1H), 2.99 - 2.94 (m, 1H), 2.04 - 2.01 (m, 1H), 1.90 - 1.74 (m, 3H), 1.42 - 1.41 (m, 2H), 1.35 - 1.24 (m, 2H), 0.74 (br s, 2H).

### Example 20: (4aR,5S,7aR)-1-(L-alanyl)-4a-(3-boronopropyl)octahydro-1H-pyrrolo[3,4-b]pyridine-5-carboxylic acid

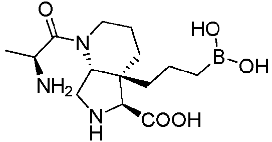

### Step 1: 6-(tert-butyl) 5-methyl (4aS,5S, 7aR)-1-((tert-butoxycarbonyl)-L-alanyl)-4a-(3-(4,4,5,5-tetramethyl-1,3,2-dioxaborolan-2-yl)propyl)octahydro-6H-pyrrolo[3,4-b]pyridine-5,6-dicarboxylate

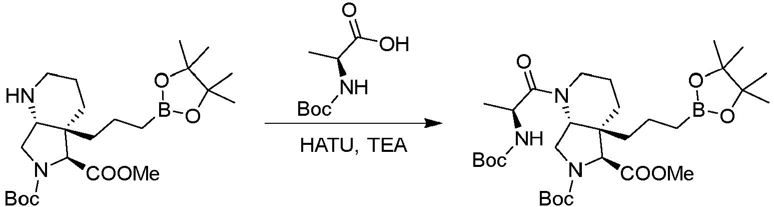

HATU (109 mg, 0.29 mmol) was added to a mixture of (4aS,5S,7aR)-6-*tert*-butyl 5-methyl 4a-(3-(4,4,5,5-tetramethyl-1,3,2-dioxaborolan-2-yl)propyl)hexahydro-1*H*-pyrrolo[3,4-*b]*pyridine-5,6(2*H*)-dicarboxylate (100 mg, 0.22 mmol), (*S*)-2-((*tert*-butoxycarbonyl)amino)propanoic acid (50 mg, 0.27 mmol), and Et₃N (0.092 mL, 0.66 mmol) in DMF (2.0 mL) at 20 °C, and the resulting mixture was stirred at 20 °C for 1 h. The reaction mixture was quenched by water and extracted with EtOAc, and the combined organic phases were dried over Na₂SO₄, filtered, and concentrated in vacuo. The residue was purified by flash silica gel chromatography (EtOAc in hexanes) to give (*4aS,5S,7aR)-6-tert-butyl* 5-methyl 1-((S)-2-((tert-butoxycarbonyl)amino)propanoyl)-4a-(3-(4,4,5,5-tetramethyl-1,3,2-dioxaborolan-2-yl)propyl)hexahydro-1H-pyrrolo[3,4-b]pyridine-5,6(2H)-dicarboxylate. LCMS (C₃₁H₅₅BN₃O₉⁺) (ES, m/z): 624 [M+H]⁺.

### Step 2: (4aR,5S,7aR)-1-(L-alanyl)-4a-(3-boronopropyl)octahydro-1H-pyrrolo[3,4-b]pyridine-5-carboxylic acid

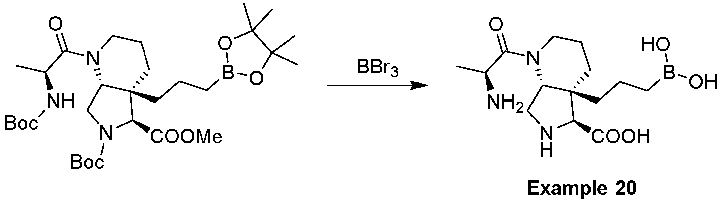

BBr₃ (0.70 mL, 0.11 mmol) was added to a solution of (*4aS,5S,7aR*)-6-*tert*-butyl 5-methyl 1-((*S*)-2-((*tert*-butoxycarbonyl)amino)propanoyl)-4a-(3-(4,4,5,5-tetramethyl-1,3,2-dioxaborolan-2-yl)propyl)hexahydro-1*H*-pyrrolo[3,4-*b*]pyridine-5,6(2H)-dicarboxylate (70 mg, 0.11 mmol) in DCM (1.0 mL) at 0 °C, and the resulting mixture was stirred for 16 h at 15 °C. The reaction mixture was concentrated in vacuo, and the residue was purified by RP-HPLC [C18 column, water (20 mM HFBA and 0.1% TFA)-CH₃CN] to give the (*4aR,5S,7aR*)*-*1-((*S*)-2-aminopropanoyl)-4a-(3-boronopropyl)octahydro-1*H*-pyrrolo[3,4-*b*]pyridine-5-carboxylic acid as an HFBA salt. LCMS (C₁₄H₂₅BN₃O₄⁺) (ES, m/z): 310 [M+H-H₂O]⁺. ¹H NMR (400 MHz, D₂O) *δ* 4.76-4.71 (m, 1H), 4.36-4.31 (m, 1H), 4.02 (s, 1H), 3.60-3.52 (m, 2H), 3.36-3.30 (m, 1H), 3.17-3.10 (m, 1H), 1.87-1.83 (m, 1H), 1.62-1.46 (m, 3H), 1.41-1.26 (m, 6H), 1.08-1.01 (m, 1H), 0.64-0.53 (m, 2H).

### Example 21: (4aR,5S,7aR)-1-(L-valyl)-4a-(3-boronopropyl)octahydro-1H-pyrrolo[3,4-b]pyridine-5-carboxylic acid

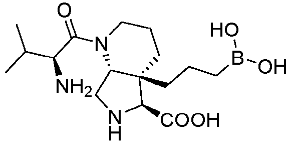

### Step 1: 6-(tert-butyl) 5-methyl (4aS,5S,7aR)-1-((tert-butoxycarbonyl)-L-valyl)-4a-(3-(4,4,5,5-tetramethyl-1,3,2-dioxaborolan-2-yl)propyl)octahydro-6H-pyrrolo[3,4-b]pyridine-5,6-dicarboxylate

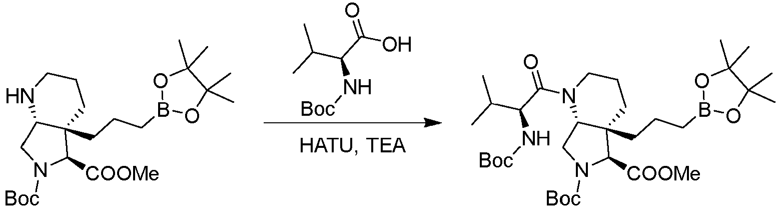

HATU (109 mg, 0.29 mmol) was added to a mixture of (*4aS,5S,7aR*)-6-*tert-*butyl 5-methyl 4a-(3-(4,4,5,5-tetramethyl-1,3,2-dioxaborolan-2-yl)propyl)hexahydro-1*H*-pyrrolo[3,4-*b*]pyridine-5,6(2*H*)-dicarboxylate (100 mg, 0.22 mmol), (*S*)-2-((*tert*-butoxycarbonyl)amino)-3-methylbutanoic acid (58 mg, 0.27 mmol) and Et₃N (0.092 mL, 0.66 mmol) in DMF (2.0 mL) at 20 °C, and the resulting mixture was stirred at 20 °C for 1 h. The reaction mixture was quenched by water and extracted with EtOAc, and the combined organic phases were dried over Na₂SO₄, filtered, and concentrated in vacuo. The residue was purified by flash silica gel chromatography (EtOAc in hexanes) to give (*4aS,5S,7aR*)-6-*tert*-butyl 5-methyl 1-((*S*)-2-((*tert*-butoxycarbonyl)amino)-3-methylbutanoyl)-4a-(3-(4,4,5,5-tetramethyl-1,3,2-dioxaborolan-2-yl)propyl)hexahydro-1H-pyrrolo[3,4-*b*]pyridine-5,6(2*H*)-dicarboxylate. LCMS (C₃₃H₅₉BN₃O₉⁺) (ES, m/z): 652 [M+H]⁺.

### Step 2: (4aR,5S,7aR)-1-(L-valyl)-4a-(3-boronopropyl)octahydro-1H-pyrrolo[3,4-b]pyridine-5-carboxylic acid

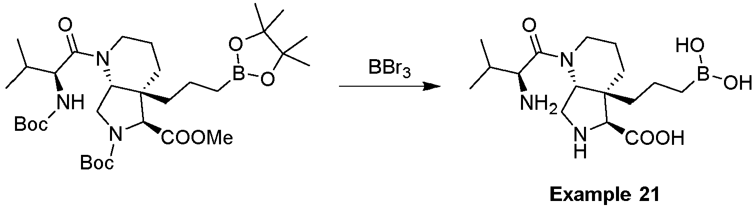

BBr₃ (0.50 mL, 0.14 mmol) was added to a solution of (*4aS,5S,7aR*)-6-*tert*-butyl 5-methyl 1-((*S*)-2-((*tert*-butoxycarbonyl)amino)-3-methylbutanoyl)-4a-(3-(4,4,5,5-tetramethyl-1,3,2-dioxaborolan-2-yl)propyl)hexahydro-1*H*-pyrrolo[3,4-*b*]pyridine-5,6(2*H*)-dicarboxylate (90 mg, 0.14 mmol) in DCM (1.0 mL) at 0 °C, and the resulting mixture was stirred for 16 h. The reaction mixture was concentrated in vacuo, and the residue was purified by RP-HPLC [C18 column, water (20 mM HFBA and 0.1% TFA)-CH₃CN]) to give the (*4aR,5S,7aR*)-1-((*S*)-2-amino-3-methylbutanoyl)-4a-(3-boronopropyl)octahydro-1*H*-pyrrolo[3,4-*b*]pyridine-5-carboxylic acid as an HFBA salt. LCMS (C₁₆H₂₉BN₃O₄⁺) (ES, m/z): 338 [M+H-H₂O]⁺. ¹H NMR (500 MHz, D₂O) *δ* 4.81-4.77 (m, 1H), 4.32-4.31 (m, 1H), 4.10 (s, 1H), 3.75-3.70 (m, 1H), 3.64-3.59 (m, 1H), 3.40-3.36 (m, 1H), 3.21-3.15 (m, 1H), 2.17-2.16 (m, 1H), 1.92-1.90 (m, 1H), 1.65-1.55 (m, 3H), 1.44-1.29 (m, 3H), 1.14-1.08 (m, 1H), 1.01 (d, *J =* 6.9 Hz, 3H), 0.88 (d, *J* = 6.9 Hz, 3H), 0.74-0.56 (m, 2H). **Example 22:** *(1S,2R,4R)-2-(3-boronopropyl)-7-azabicyclo[2.2.1]heptane-1-carboxylic acid*

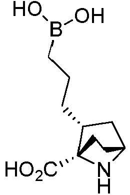

### Step 1: methyl (1S,2S,4R)-7-benzoyl-2-((E)-3-(4,4,5,5-tetramethyl-1,3,2-dioxaborolan-2-yl)prop-1-en-1-yl)-7-azabicyclo[2.2.1]heptane-1-carboxylate

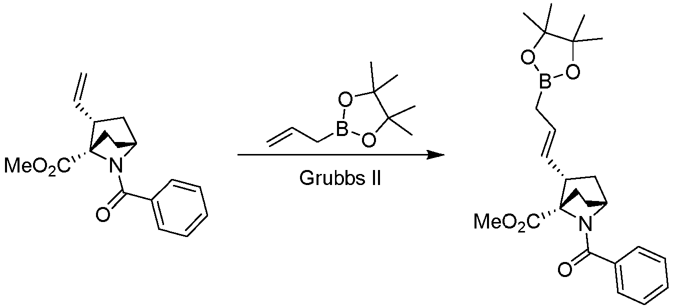

Grubbs' catalyst G₂ (74 mg, 0.088 mmol) was added to a mixture of (*1S,2S,4R*)-methyl 7-benzoyl-2-vinyl-7-azabicyclo[2.2.1]heptane-1-carboxylate (500 mg, 1.8 mmol) and 2-allyl-4,4,5,5-tetramethyl-1,3,2-dioxaborolane (883 mg, 5.3 mmol) in DCM (13 mL) under argon at ambient temperature, and the resulting mixture was stirred at 35 °C for 48 h. The reaction mixture was concentrated in vacuo, and the residue was purified by flash silica gel chromatography (EtOAc in hexanes) to give (*1S,2S,4R*)-methyl 7-benzoyl-2-((*E*)-3-(4,4,5,5-tetramethyl-1,3,2-dioxaborolan-2-yl)prop-1-en-1-yl)-7-azabicyclo[2.2.1]heptane-1-carboxylate. LCMS (C₂₄H₃₃BNO₅⁺) (ES, m/z): 426 [M+H]⁺.

### Step 2: methyl (1S,2R,4R)-7-benzoyl-2-(3-(4,4,5,5-tetramethyl-1,3,2-dioxaborolan-2-yl)propyl)-7-azabicyclo[2.2.1]heptane-1-carboxylate

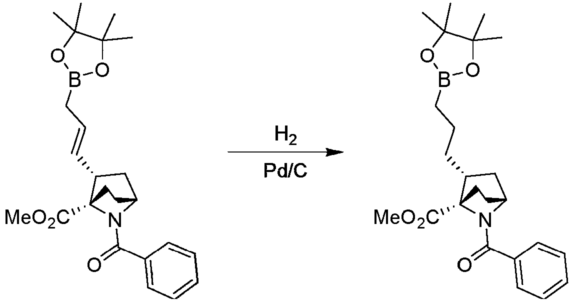

10% Pd-C (78 mg, 0.073 mmol) was added to a solution of (*1S,2S,4R*)-methyl 7-benzoyl-2-((*E*)-3-(4,4,5,5-tetramethyl-1,3,2-dioxaborolan-2-yl)prop-1-en-1-yl)-7-azabicyclo[2.2.1]heptane-1-carboxylate (310 mg, 0.73 mmol) in MeOH (4.0 mL) under N₂. The resulting mixture was degassed and backfilled with H₂, then stirred under H₂ (Pressure: 103 kPa (15 psi)) at 15 °C for 1 h. The reaction mixture was filtered and concentrated under reduced pressure to give crude (*1S,2R,4R*)-methyl 7-benzoyl-2-(3-(4,4,5,5-tetramethyl-1,3,2-dioxaborolan-2-yl)propyl)-7-azabicyclo[2.2.1]heptane-1-carboxylate, which was used in next step directly without further purification. LCMS (C₂₄H₃₅BNO₅⁺) (ES, m/z): 428 [M+H]⁺.

### Step 3: (1S,2R,4R)-2-(3-boronopropyl)-7-azabicyclo[2.2.1]heptane-1-carboxylic acid

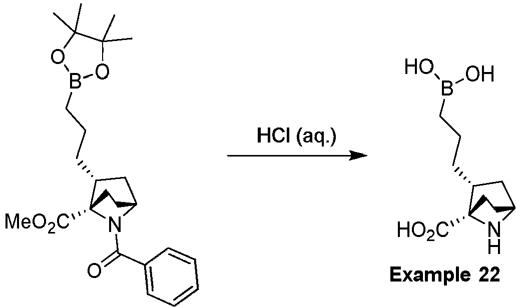

A mixture of methyl (*1S,2R,4R*)-7-benzoyl-2-(3-(4,4,5,5-tetramethyl-1,3,2-dioxaborolan-2-yl)propyl)-7-azabicyclo[2.2.1]heptane-1-carboxylate (120 mg, 0.28 mmol) and 12 N HCl in water (4.0 mL, 48 mmol) was stirred at 100 °C for 12 h, and the reaction mixture was concentrated in vacuo. The residue was purified by RP-HPLC [C18 column, water (20 mM HFBA and 0.1% TFA)-CH₃CN] to give (*1S*,*2R*,*4R*)-2-(3-boronopropyl)-7-azabicyclo[2.2.1]heptane-1-carboxylic acid as an HFBA salt. LCMS (C₁₀H₁₇BNO₃⁺) (ES, m/z): 210 [M+H-H₂O]⁺. ¹H NMR (500 MHz, D₂O) *δ* 4.05 - 4.03 (m, 1H), 2.24 - 2.14 (m, 1H), 2.09 - 1.91 (m, 4H), 1.80 - 1.69 (m, 1H), 1.68 - 1.53 (m, 1H), 1.39 - 1.25 (m, 2H), 1.23 - 0.96 (m, 2H), 0.80 - 0.44 (m, 2H).

### Example 23: (3aR,4S,6aR)-3a-(3-boronopropyl)-5-(tert-butoxycarbonyl)hexahydro-2H-thieno[2,3-c]pyrrole-4-carboxylic acid

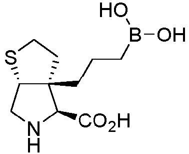

### Step 1: 1-(tert-butyl) 2-methyl (2S,3R)-3-allyl-3-(2-((tert-butyldimethylsilyl)oxy)ethyl)-4-oxopyrrolidine-1,2-dicarboxylate

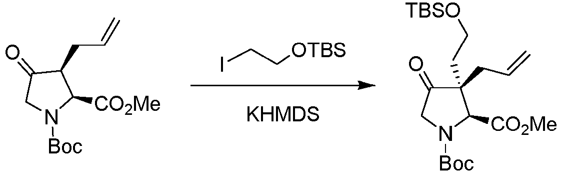

KHMDS (25 mL, 13 mmol, 0.5 M in toluene) was added dropwise to a stirred solution of (25)-1-*tert*-butyl 2-methyl 3-allyl-4-oxopyrrolidine-1,2-dicarboxylate (3.0 g, 11 mmol) in THF (36 mL) and DMPU (12 mL) at -78 °C under N₂. Upon completion of addition, the reaction mixture was stirred at -78 °C for 1 h, followed by addition of *tert*-butyl(2-iodoethoxy)dimethylsilane (2.6 mL, 12 mmol) in one portion and the resulting mixture was allowed to warm to 14 °C and stirred for additional 16 h. The reaction mixture was quenched by addition of saturated aqueous NH₄Cl, then diluted with water, and extracted with EtOAc. The combined organic layers were washed with brine, dried over anhydrous Na₂SO₄, filtered, and concentrated in vacuo. The residue was purified by flash silica gel chromatography (EtOAc in hexanes) to give (*2S,3R*)-1-*tert*-butyl 2-methyl 3-allyl-3-(2-((*tert*-butyldimethylsilyl)oxy)ethyl)-4-oxopyrrolidine-1,2-dicarboxylate, which was further resolved by Chiral-SFC [Column: DAICEL CHIRALPAK IC (250 mm*30 mm, 5 um), Mobile phase: A: CO₂, B: EtOH (0.1% NH₃·H₂O), Gradient: 7% of B in 3.5 minutes, and hold 7% of B for 1 min, Flow Rate (mL/min) 50, Column temperature: 40 °C] to give (*2S,3R*)-1-*tert*-butyl 2-methyl 3-allyl-3-(2-((tert-butyldimethylsilyl)oxy)ethyl)-4-oxopyrrolidine-1,2-dicarboxylate (tᵣ = 1.565 min). LCMS (C₁₇H₃₂NO₄Si⁺) (ES, m/z): 342 [M+H-Boc]⁺. ¹H NMR (500 MHz, CDCl₃) *δ* 5.94 - 5.64 (m, 1H), 5.22 - 4.90 (m, 2H), 4.63 - 4.62 (m, 1H), 4.07 - 4.00 (m, 1H), 3.93 - 3.82 (m, 1H), 3.75 - 3.59 (m, 5H), 2.43 - 2.39 (m, 1H), 2.15 - 1.95 (m, 2H), 1.87 - 1.73 (m, 1H), 1.46 - 1.44 (m, 9H), 0.86 - 0.85 (m, 9H), 0.09 - 0.06 (m, 6H).

### Step 2: 1-(tert-butyl) 2-methyl (2S,3R,4S)-3-allyl-3-(2-((tert-butyldimethylsilyl)oxy)ethyl)-4-hydroxypyrrolidine-1,2-dicarboxylate

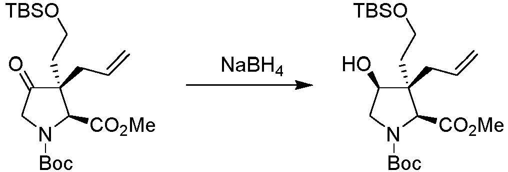

NaBH₄ (86 mg, 2.3 mmol) was added to a solution of (*2S,3R*)-1-*tert*-butyl 2-methyl 3-allyl-3-(2-((te*r*t-butyldimethylsilyl)oxy)ethyl)-4-oxopyrrolidine-1,2-dicarboxylate (500 mg, 1.1 mmol) in MeOH (10 mL) at 0 °C under N₂, and the resulting mixture was stirred for 60 minutes at 0 °C under N₂. The reaction mixture was quenched by addition of saturated aqueous NaHCO₃, then diluted with water, and extracted with EtOAc. The combined organic layers were washed with brine, dried over anhydrous Na₂SO₄, filtered, and concentrated in vacuo. The residue was purified by flash silica gel chromatography (EtOAc in hexanes) to give (*2S,3R,4R*)-1-*tert*-butyl 2-methyl 3-allyl-3-(2-((*tert*butyldimethylsilyl)oxy)ethyl)-4-hydroxypyrrolidine-1,2-dicarboxylate. The stereochemistry was assigned by 2D NMR. LCMS (C₁₇H₃₄NO₄Si⁺) (ES, m/z): 344 [M+H-Boc]⁺. ¹H NMR (500 MHz, CDCl₃) *δ* 5.88 - 5.61 (m, 1H), 5.22 - 5.05 (m, 2H), 3.96 - 3.78 (m, 2H), 3.78 - 3.72 (m, 5H), 3.71 - 3.66 (m, 1H), 3.66 - 3.56 (m, 1H), 2.43 - 2.10 (m, 2H), 1.62 - 1.51 (m, 2H), 1.47 - 1.36 (m, 9H), 0.92 - 0.86 (m, 9H), 0.11 - 0.01 (m, 6H).

### Step 3: 1-(tert-butyl) 2-methyl (2S,3R,4S)-3-allyl-4-hydroxy-3-(2-hydroxyethyl)pyrrolidine-1,2-dicarboxylate

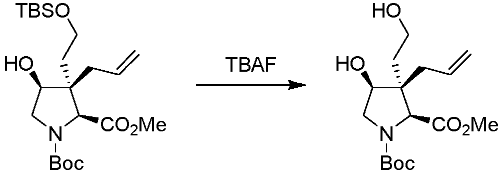

A mixture of 1M TBAF in THF (1.3 mL, 1.3 mmol) and (*2S,3R,4R*)-1-*tert*-butyl 2-methyl 3-allyl-3-(2-((*tert*-butyldimethylsilyl)oxy)ethyl)-4-hydroxypyrrolidine-1,2-dicarboxylate (380 mg, 0.86 mmol) in THF (5.0 mL) was stirred at 0 °C for 1 h, and the reaction mixture was quenched by addition of saturated aqueous NH₄Cl, then diluted with water, and extracted with EtOAc. The combined organic layers were washed with brine, dried over anhydrous Na₂SO₄, filtered, and concentrated in vacuo. The residue was purified by flash silica gel chromatography (EtOAc in hexanes) to give (*2S,3R,4R*)-1-*tert*-butyl 2-methyl 3-allyl-4-hydroxy-3-(2-hydroxyethyl)pyrrolidine-1,2-dicarboxylate. LCMS (C₁₁H₂₀NO₄⁺) (ES, m/z): 230 [M+H-Boc]⁺.

### Step 4: 1-(tert-butyl) 2-methyl (2S,3R,4S)-3-allyl-4-((methylsulfonyl)oxy)-3-(2-((methylsulfonyl)oxy)ethyl)pyrrolidine-1,2-dicarboxylate

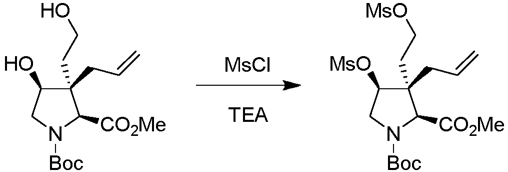

Methanesulfonyl chloride (522 mg, 4.6 mmol) was added dropwise to a solution of *(2S,3R, 4R)-1-tert-butyl* 2-methyl 3-allyl-4-hydroxy-3-(2-hydroxyethyl)pyrrolidine-1,2-dicarboxylate (250 mg, 0.76 mmol) and triethylamine (1.1 mL, 7.6 mmol) in DCM (6.0 mL) at 0 °C, and the resulting mixture was stirred at 0-10 °C for 2 h. The reaction mixture was quenched by addition of acetic acid to pH ~ 7, then diluted with water and extracted with DCM. The combined organic layers were washed with brine, dried over anhydrous Na₂SO₄, filtered, and concentrated in vacuo. The residue was purified by flash silica gel chromatography (EtOAc in hexanes) to give (*2S,3R,4R*)-1-tert-butyl 2-methyl 3-allyl-4-((methylsulfonyl)oxy)-3-(2-((methylsulfonyl)oxy)ethyl)pyrrolidine-1,2-dicarboxylate. LCMS (C₁₃H₂₄NO₈S₂⁺) (ES, m/z): 386 [M+H-Boc]⁺.

### Step 5: 5-(tert-butyl) 4-methyl (3aR,4S,6aR)-3a-allylhexahydro-5H-thieno[2,3-c]pyrrole-4,5-dicarboxylate

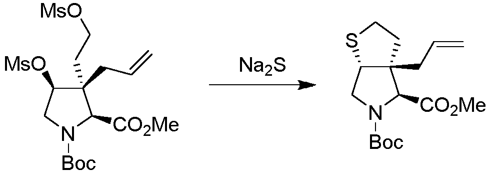

Sodium sulfide (24 mg, 0.31 mmol) and triethylamine (0.13 mL, 0.93 mmol) were added to a stirred solution of (*2S,3R,4R*)-1-*tert*-butyl 2-methyl 3-allyl-4-((methylsulfonyl)oxy)-3-(2-((methylsulfonyl)oxy)ethyl)pyrrolidine-1,2-dicarboxylate (150 mg, 0.31 mmol) dissolved in DMF (3.0 mL), and the resulting mixture was stirred at 40 °C for 12 h. The reaction mixture was diluted with water and extracted with EtOAc, and the combined organic layers were washed with brine, dried over anhydrous Na₂SO₄, filtered, and concentrated in vacuo. The residue was purified by flash silica gel chromatography (EtOAc in hexanes) to give (*3aR,4S,6aR*)-5-*tert-*butyl 4-methyl 3a-allyltetrahydro-2*H*-thieno[2,3-c]pyrrole-4,5(3*H*)-dicarboxylate. LCMS (C₁₆H₂₅NO₄SNa⁺) (ES, m/z): 350 [M+Na]⁺.

### Step 6: 5-(tert-butyl) 4-methyl (3aR,4S,6aR)-3a-(3-(4,4,5,5-tetramethyl-1,3,2-dioxaborolan-2-yl)propyl)hexahydro-5H-thieno[2,3-c]pyrrole-4,5-dicarboxylate

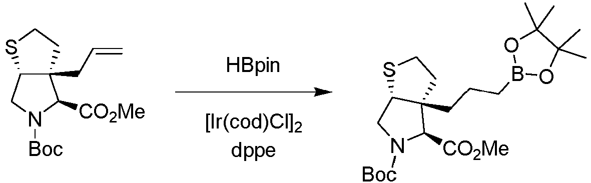

4,4,5,5-Tetramethyl-1,3,2-dioxaborolane (0.21 mL, 1.5 mmol) and (*3aR,4S,6aR*)-5-*tert*-butyl 4-methyl 3a-allyltetrahydro-2*H*-thieno[2,3-*c*]pyrrole-4,5(3*H*)-dicarboxylate (95 mg, 0.29 mmol) were added to a mixture of dppe (14 mg, 0.035 mmol) and [Ir(cod)Cl]₂ (16 mg, 0.023 mmol) in DCM (2.0 mL) at 20 °C under N₂, and the resulting mixture was stirred at 20 °C under N₂ for 14 h. The reaction mixture was concentrated under reduced pressure and the residue was purified directly by flash silica gel chromatography (EtOAc in hexanes) to give (*3aR,4S,6aR*)-5-*tert*-butyl 4-methyl 3a-(3-(4,4,5,5-tetramethyl-1,3,2-dioxaborolan-2-yl)propyl)tetrahydro-2H-thieno[2,3-c]pyrrole-4,5(3*H*)-dicarboxylate. LCMS (C₂₂H₃₈BNO₆SNa⁺) (ES, m/z): 478 [M+Na]⁺.

### Step 7: (3aR, 4S, 6aR)-3a-(3-boronopropyl)-5-(tert-butoxycarbonyl)hexahydro-2H-thieno[2,3-c]pyrrole-4-carboxylic acid

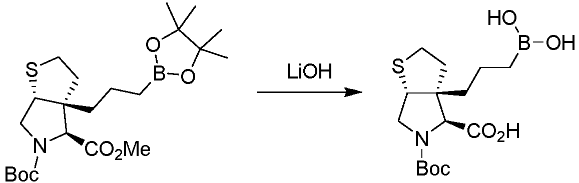

A mixture of (*3aR,4S,6aR*)-5*-tert-*butyl 4-methyl 3a-(3-(4,4,5,5-tetramethyl-1,3,2-dioxaborolan-2-yl)propyl)tetrahydro-2*H*-thieno[2,3-*c*]pyrrole-4,5(3*H*)-dicarboxylate (45 mg, 0.099 mmol) and lithium hydroxide monohydrate (25 mg, 0.59 mmol) in MeOH (1.0 mL) and water (0.50 mL) was stirred at 45 °C under N₂ for 40 h. The reaction mixture was concentrated under reduced pressure to give crude (*3aR*,*4S*,*6aR*)-3a-(3-boronopropyl)-5-(*tert*-butoxycarbonyl)hexahydro-2*H-*thieno[2,3-*c*]pyrrole-4-carboxylic acid, which was used in the next step directly without further purification. LCMS (C₁₀H₁₇BNO₃S⁺) (ES, m/z): 242 [M+H-Boc-H₂O]⁺.

### Step 8: (3aR,4S,6aR)-3a-(3-boronopropyl)hexahydro-2H-thieno[2,3-c]pyrrole-4-carboxylic acid

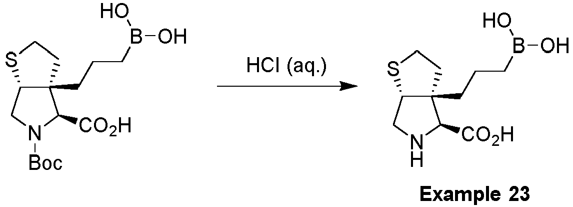

A mixture of (*3aR, 4S,6aR*)-3a-(3-boronopropyl)-5-(*tert*-butoxycarbonyl)hexahydro-2*H-*thieno[2,3-*c*]pyrrole-4-carboxylic acid (56 mg, 0.099 mmol) and 4 N HCl in water (0.50 mL, 2.0 mmol) in MeOH (1.0 mL) was stirred at 20 °C under N₂ for 60 minutes. The reaction mixture was purified directly by RP-HPLC [C18 column, water (20 mMHFBA and 0.1% TFA)-CH₃CN] to give [(*3aR,4S,6aR*)-3a-(3-boronopropyl)hexahydro-2*H*-thieno[2,3-c]pyrrole-4-carboxylic acid as an HFBA salt. LCMS (C₁₀H₁₇BNO₃S⁺) (ES, m/z): 242 [M+H-H₂O]⁺. ¹H NMR (500 MHz, D₂O) *δ* 4.10 (s, 1H), 3.84 - 2.82 (m, 1H), 3.72 - 3.69 (m, 1H), 3.33 - 3.32 (m, 1H), 3.04 (m, 1H), 2.96 - 2.80 (m, 1H), 2.34 -2.31 (m, 1H), 2.13 (m, 1H), 1.58 - 1.20 (m, 4H), 0.67 (t, *J =* 7.2 Hz, 2H).

### Example 24: (1S,3S,4R,5S)-4-(3-boronopropyl)-4-methyl-2-azabicyclo[3.1.0]hexane-3-carboxylic acid

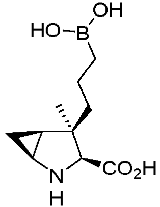

### Step 1: 1-benzyl 2-methyl (2S,3S)-3-allyl-3-methyl-4-oxopyrrolidine-1,2-dicarboxylate

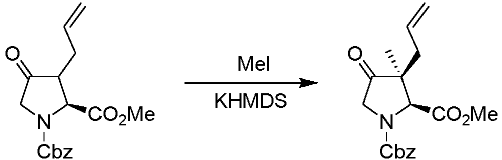

KHMDS (15 mL, 7.6 mmol, 0.5 M in toluene) was added dropwise to a stirred solution of (2*S*)-1-benzyl 2-methyl 3-allyl-4-oxopyrrolidine-1,2-dicarboxylate (2.0 g, 6.3 mmol) in THF (40 mL) at -78 °C under N₂ over 10 minutes, and the resulting mixture was stirred at -78 °C for 1 h, followed by addition of iodomethane (3.5 mL, 54 mmol) in one portion. The reaction mixture was stirred at 15 °C for 10 h, then quenched with saturated aqueous NH₄Cl, and extracted with EtOAc. The combined organic phases were washed with brine, dried over anhydrous Na₂SO₄, filtered, and concentrated under reduced pressure. The residue was purified by flash silica gel chromatography (EtOAc in hexanes) to give 1-benzyl 2-methyl (2*S*)-3-allyl-3-methyl-4-oxopyrrolidine-1,2-dicarboxylate as a mixture of diastereomers, which was resolved by Chiral-SFC [Column: DAICEL CHIRALPAK AD-H (250 mm*30 mm, 5 um), Mobile phase: A: CO₂, B: EtOH (0.1% NH₃·H₂O), Gradient: 25% of B in 4.5 minutes, and hold 25% of B for 1 min, Flow Rate (mL/min) 50, Column temperature: 40 °C] to give (*2S,3S,4S*)-1-benzyl 2-methyl 3-allyl-3-methyl-4-oxopyrrolidine-1,2-dicarboxylate (tᵣ = 3.085 min) as the second eluting peak. LCMS (C₁₈H₂₂NO₅⁺) (ES, m/z): 332 [M+H]⁺. ¹H NMR (500 MHz, CDCl₃) *δ* 7.32 - 7.18 (m, 5H), 5.83 - 5.53 (m, 1H), 5.17 - 4.97 (m, 4H), 4.41 (s, 0.5H), 4.34 (s, 0.5H), 4.06 - 3.87 (m, 2H), 3.63 (s, 1.5H), 3.48 (s, 1.5H), 2.33 - 2.37 (m, 1H), 1.99 - 1.92 (m, 1H), 1.12 (s, 3H).

### Step 2: 1-benzyl 2-methyl (2S,3S)-3-allyl-3-methyl-4-(((trifluoromethyl)sulfonyl)oxy)-2,3-dihydro-1H-pyrrole-1,2-dicarboxylate

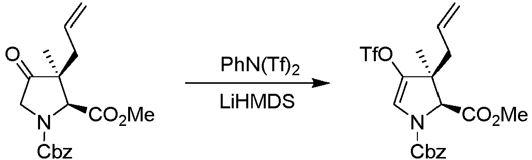

LiHMDS (3.02 mL, 3.02 mmol, 1.0 M in THF) was added dropwise to a stirred solution of (*2S,3S,4S*)-1-benzyl 2-methyl 3-allyl-3-methyl-4-oxopyrrolidine-1,2-dicarboxylate (0.50 g, 1.5 mmol) in THF (7.0 mL) at -78 °C under argon, and the resulting mixture was stirred at -78 °C for 40 min. A cold (-78 °C) solution of 1,1,1-trifluoro-N-phenyl-N-((trifluoromethyl)sulfonyl)methanesulfonamide (0.70 g, 2.0 mmol) in THF (7.0 mL) and DMPU (0.50 mL) was added dropwise and the reaction mixture was allowed to warm to 10 °C, and stirred for additional 16 h. The reaction mixture was quenched by water, and extracted with EtOAc. The combined organic phases were washed with saturated aqueous NaHCO₃, water, and brine, then dried over anhydrous Na₂SO₄, filtered, and concentrated in vacuo. The residue was purified by flash silica gel chromatography (EtOAc in hexanes) to give (*2S,3S,4S*)-1-benzyl 2-methyl 3-allyl-3-methyl-4-(((trifluoromethyl)sulfonyl)oxy)-2,3-dihydro-1*H*-pyrrole-1,2-dicarboxylate. LCMS (C₁₉H₂₁F₃NO₇S⁺) (ES, m/z): 464 [M+H]⁺.

### Step 3: 1-benzyl 2-methyl (2S, 3R)-3-allyl-3-methyl-2,3-dihydro-1H-pyrrole-1,2-dicarboxylate

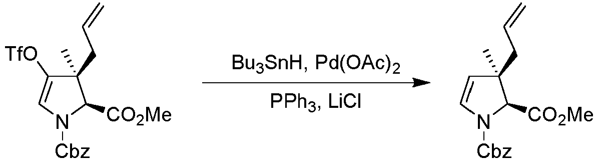

Lithium chloride (80 mg, 1.9 mmol) and triphenylphosphine (49 mg, 0.19 mmol) were added to a suspension of palladium(II) acetate (14 mg, 0.063 mmol) in THF (5.0 mL) under N₂, followed by addition of a solution of (*2S,3S,4S*)-1-benzyl 2-methyl 3-allyl-3-methyl-4-(((trifluoromethyl)sulfonyl)oxy)-2,3-dihydro-1*H*-pyrrole-1,2-dicarboxylate (290 mg, 0.626 mmol) in THF (2.0 mL), and subsequently tributylstannane (1.2 mL, 4.3 mmol). The resulting mixture was stirred at 10 °C for 1 h, then quenched with saturated aqueous KF and extracted with EtOAc. The combined organic phases were washed with brine, dried over anhydrous Na₂SO₄, filtered, and concentrated in vacuo. The residue was purified by flash silica gel chromatography (EtOAc in hexanes) to give (2*S*,3*R*)-1-benzyl 2-methyl 3-allyl-3-methyl-2,3-dihydro-1*H*-pyrrole-1,2-dicarboxylate, which was further resolved by Chiral-SFC [Column: Phenomenex-Amylose-1 (250 mm*30 mm, 5 um), Mobile phase: A: CO₂, B: EtOH (0.1% NH₃·H₂O), Gradient: 35% of B in 5.5 min, and hold 35% of B for 1 min, Flow Rate (mL/min) 50, Column temperature: 40 °C] to give (2*S*,3*R*)-1-benzyl 2-methyl 3-allyl-3-methyl-2,3-dihydro-1*H*-pyrrole-1,2-dicarboxylate. The stereochemistry was assigned by 2D NMR. LCMS (C₁₈H₂₂NO₄⁺) (ES, m/z): 316 [M+H]⁺. ¹H NMR (500 MHz, CDCl₃) *δ* 7.32 - 7.11 (m, 5H), 6.55 - 6.44 (m, 1H), 5.68 - 5.58 (m, 1H), 5.15 - 4.82 (m, 5H), 4.22 - 4.17 (m, 1H), 3.65 (s, 1.5H), 3.43 (s, 1.5H), 2.06 - 2.04 (m, 2H), 1.15 - 1.10 (m, 3H).

### Step 5: 1-benzyl 2-methyl (2S,3R)-3-methyl-3-(3-((3aS,4S,6S)-3a,5,5-trimethylhexahydro-4,6-methanobenzo[d][1,3,2]dioxaborol- 2-yl)propyl)-2,3-dihydro-1H-pyrrole-1,2-dicarboxylate

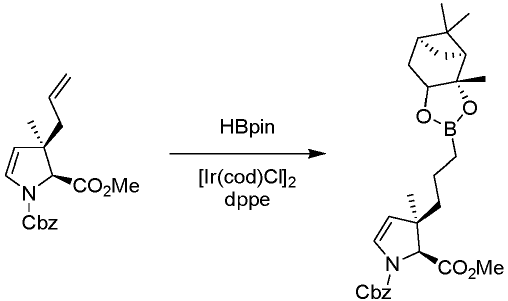

(*3aS,4S,6S*)-3a,5,5-Trimethylhexahydro-4,6-methanobenzo[*d*][1,3,2]dioxaborole (285 mg, 1.6 mmol) and (2*S*,3*R*)-1-benzyl 2-methyl 3-allyl-3-methyl-2,3-dihydro-1*H*-pyrrole-1,2-dicarboxylate (100 mg, 0.32 mmol) were added to a mixture of 1,2-bis(diphenylphosphino)ethane (10 mg, 0.025 mmol) and [Ir(cod)Cl]₂ (11 mg, 0.016 mmol) in DCM (4.0 mL) at 20 °C under N₂. The reaction mixture was stirred at 20 °C under N₂ for 14 h, then purified directly by flash silica gel chromatography (EtOAc in hexanes) to give 1-benzyl 2-methyl (*2S,3R*)-3-methyl-3-(3-((*3aS,4S,6S*)-3a,5,5-trimethylhexahydro-4,6-methanobenzo[*d*][1,3,2]dioxaborol-2-yl)propyl)-2,3-dihydro-1*H-*pyrrole-1,2-dicarboxylate. LCMS (C₂₈H₃₉BNO₆⁺) (ES, m/z): 496 [M+H]⁺.

### Step 6: 2-benzyl 3-methyl (3S,4R)-4-methyl-4-(3-((3aS,4S,6S)-3a,5,5-trimethylhexahydro-4,6-methanobenzo[d][1,3,2]dioxaborol-2-yl)propyl)-2-azabicyclo[3.1.0]hexane-2,3-dicarboxylate

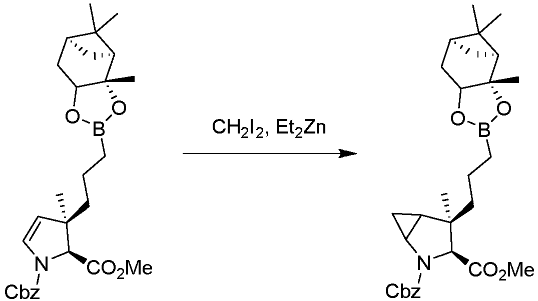

Diethylzinc (0.76 mL, 0.76 mmol, 1 M in toluene) was added dropwise to a solution of diiodomethane (0.098 mL, 1.2 mmol) in DCM (0.50 mL) over 1 min at -40 °C under N₂, and the resulting mixture was stirred at -40 °C for 0.5 h, followed by dropwise addition of 1-benzyl 2-methyl (*2S,3R*)-3-methyl-3-(3-((*3aS,4S,*6S)-3a,5,5-trimethylhexahydro-4,6-methanobenzo[*d*][1,3,2]dioxaborol-2-yl)propyl)-2,3-dihydro-1*H*-pyrrole-1,2-dicarboxylate (75 mg, 0.15 mmol) and TFA (0.035 mL, 0.45 mmol) in DCM (1.0 mL) over 1 min. The reaction mixture was stirred at -40 °C for 0.3 h and then at 20 °C for additional 12 h before concentrated in vacuo. The crude mixture was diluted with saturated aqueous NaHCO₃ and extracted with DCM. The combined organic phase was dried over anhydrous Na₂SO₄, filtered, and concentrated in vacuo. The residue was purified by flash silica gel chromatography (EtOAc in hexanes) to give 2-benzyl 3-methyl (*3S,4R*)-4-methyl-4-(3-((*3aS,4S,6S*)-3a,5,5-trimethylhexahydro-4,6-methanobenzo[*d*][1,3,2]dioxaborol-2-yl)propyl)-2-azabicyclo[3.1.0]hexane-2,3-dicarboxylate. LCMS (C₂₉H₄₁BNO₆⁺) (ES, m/z): 510 [M+H]⁺.

### Step 7: (3S, 4R)-2-((benzyloxy)carbonyl)-4-methyl-4-(3-((3aS, 4S, 6S)-3a, 5, 5-trimethylhexahydro-4, 6-methanobenzo[d][1,3,2]dioxaborol-2-yl)propyl)-2-azabicyclo[3.1.0]hexane-3-carboxylic acid

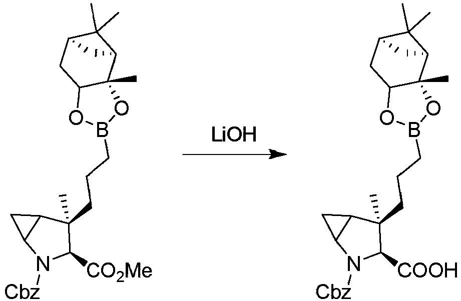

A mixture of 2-benzyl 3-methyl *(3S,4R)-4-methyl-4-(3-((3aS, 4S, 6,S)-3a,5,5-*trimethylhexahydro-4,6-methanobenzo[*d*][1,3,2]dioxaborol-2-yl)propyl)-2-azabicyclo[3.1.0]hexane-2,3-dicarboxylate (40 mg, 0.079 mmol) and lithium hydroxide monohydrate (20 mg, 0.47 mmol) in MeOH (1.0 mL) and water (0.50 mL) was stirred at 20 °C under N₂ for 12 h, then at 60 °C for 36 h. The reaction mixture was concentrated under reduced pressure, diluted with MeOH (5.0 mL), then filtered, and concentrated in vacuo to give crude (*3S*,*4R*)-2-((benzyloxy)carbonyl)-4-methyl-4-(3-*((3aS, 4S,6S*)-3a, 5, 5-trimethylhexahydro-4,6-methanobenzo[*d*][1,3,2]dioxaborol-2-yl)propyl)-2-azabicyclo[3.1.0]hexane-3-carboxylic acid, which was used in the next step directly without further purification. LCMS (C₂₇H₃₉BNO₄⁺) (ES, m/z): 452 [M+H-CO₂]⁺.

### Step 8: (3S, 4R)-4-methyl-4-(3-((3aS, 4S,6S)-3a,5,5-trimethylhexahydro-4, 6-methanobenzo[d][1,3,2]dioxaborol-2-yl)propyl)-2-azabicyclo[3.1.0]hexane-3-carboxylic acid

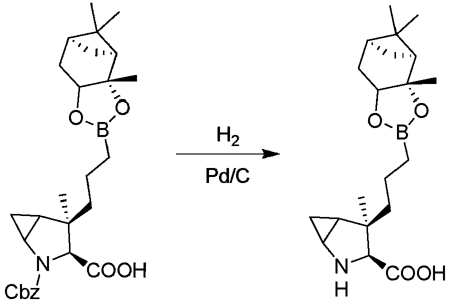

10% Pd-C (1.5 mg, 0.014 mmol) was added to a solution of (*3S,4R*)-2-((benzyloxy)carbonyl)-4-methyl-4-(3-((*3aS,*4*S*,*6S*)-3a,5,5-trimethylhexahydro-4,6-methanobenzo[*d*][1,3,2]dioxaborol-2-yl)propyl)-2-azabicyclo[3.1.0]hexane-3-carboxylic acid (60 mg, 0.068 mmol) in MeOH (1.0 mL) under N₂. The resulting mixture was degassed and backfilled with H₂ (three times), and stirred under H₂ (Pressure: 103 kPa (15 psi)) at 20 °C for 1 h. The reaction mixture was filtered and concentrated under reduced pressure to give crude (*3S,4R*)-4-methyl-4-(3-*((3aS, 4S,6S*)-3a,5,5-trimethylhexahydro-4,6-methanobenzo[*d*][1,3,2]dioxaborol-2-yl)propyl)-2-azabicyclo[3.1.0]hexane-3-carboxylic acid, which was used in the next step directly without further purification. LCMS (C₂₀H₃₃BNO₄⁺) (ES, m/z): 362 [M+H]⁺.

### Step 9: (1S,3S,4R,5S)-4-(3-boronopropyl)-4-methyl-2-azabicyclo[3.1.0]hexane-3-carboxylic acid

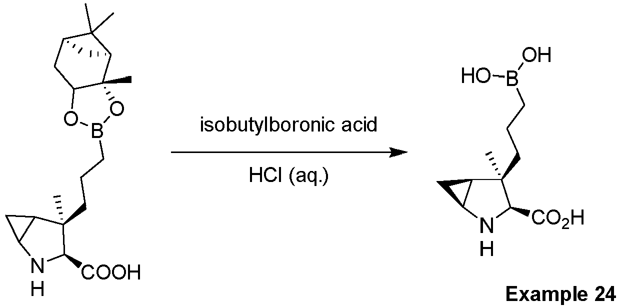

*(3S, 4R)-4-methyl-4-(3-((3aS, 4S,6S*)-3a,5,5-trimethylhexahydro-4,6-methanobenzo[*d*][1,3,2]dioxaborol-2-yl)propyl)-2-azabicyclo[3.1.0]hexane-3-carboxylic acid (20 mg, 0.028 mmol) and isobutylboronic acid (4.8 mg, 0.047 mmol) were dissolved in methanol (0.20 mL) and hexane (0.20 mL), followed by addition of 3 M HCl in water (0.16 mL, 0.47 mmol) at 20 °C. The reaction mixture was stirred at 20 °C for 12 h, then concentrated in vacuo. The residue was purified by RP-HPLC [C18 column, water (20 mM HFBA and 0.1% TFA)-CH₃CN] to give (*1S,3S, 4R,5S*)-4-(3-boronopropyl)-4-methyl-2-azabicyclo[3.1.0]hexane-3-carboxylic acid as an HFBA salt. The stereochemistry was assigned by 2D-NMR. LCMS (C₁₀H₁₇BNO₃⁺) (ES, m/z): 210 [M+H-H₂O]⁺. ¹H NMR (500 MHz, D₂O) *δ* 3.83 (s, 1H), 3.37 - 3.21 (m, 1H), 1.64 - 1.57 (m, 1H), 1.54 - 1.44 (m, 2H), 1.35 - 1.29 (m, 1H), 1.25 (s, 3H), 1.20 - 1.14 (m, 1H), 1.04 - 0.93 (m, 1H), 0.77-0.65 (m, 2H).

### Example 25: 7-(boronomethyl)-2-azaspiro[4.5]decane-1-carboxylic acid

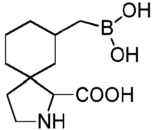

### Step 1: methyl 3-((tert-butyldiphenylsilyl)oxy)cyclohexanecarboxylate

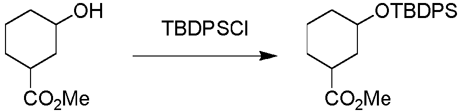

Imidazole (17 g, 253 mmol) and TBDPS-Cl (39 mL, 152 mmol) were added to a solution of methyl 3-hydroxycyclohexanecarboxylate (20 g, 126 mmol) in DCM (250 mL) at 10 °C, and the resulting mixture was stirred at 10 °C for 16 h, then diluted with water, and extracted with DCM. The combined organic phases were washed with brine, dried over anhydrous Na₂SO₄, filtered, and concentrated under reduced pressure. The residue was purified by silica gel column chromatography (EtOAc in hexanes) to give methyl 3-((*tert*-butyldiphenylsilyl)oxy)cyclohexanecarboxylate. LCMS (C₂₄H₃₃O₃Si⁺) (ES, m/z): 397 [M+H]⁺.

### Step 2: methyl 3-((tert-butyldiphenylsilyl)oxy)-1-(cyanomethyl)cyclohexanecarboxylate

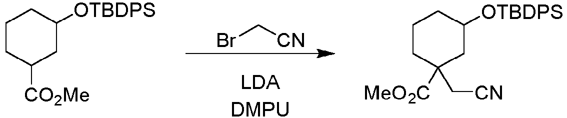

*n*-BuLi (39 mL, 97 mmol, 2.5M in hexanes) was added to a solution of diisopropylamine (9.8 g, 97 mmol) in THF (200 mL) at -78 °C, and the resulting mixture was stirred for 1 h at -78 °C, followed by addition of methyl 3-((*tert*-butyldiphenylsilyl)oxy)cyclohexanecarboxylate (35 g, 88 mmol) at -78 °C. The resulting mixture was stirred for additional 2 h, followed by addition of a solution of DMPU (5.3 mL, 44 mmol) and 2-bromoacetonitrile (7.4 mL, 106 mmol) in THF (50 mL) at -78 °C. The reaction mixture was stirred at 10 °C for additional 3 h, then quenched with saturated aqueous NH₄Cl and extracted with EtOAc. The combined organic phases were washed with brine, dried over anhydrous Na₂SO₄, filtered, and concentrated under reduced pressure. The residue was purified by silica gel column chromatography (EtOAc in hexanes) to give methyl 3-((*tert-*butyldiphenylsilyl)oxy)-1-(cyanomethyl)cyclohexanecarboxylate. LCMS (C₂₆H₃₃NO₃SiNa⁺) (ES, m/z): 458 [M+Na]⁺.

### Step 3: 7-((tert-butyldiphenylsilyl)oxy)-2-azaspiro[4.5]decan-1-one

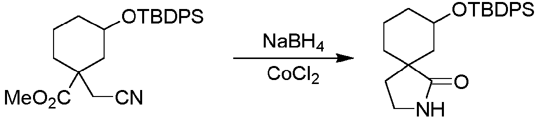

NaBH₄ (4.8 g, 126 mmol) was added to a solution of methyl 3-((*tert-*butyldiphenylsilyl)oxy)-1-(cyanomethyl)cyclohexanecarboxylate (11 g, 25 mmol) and cobalt(II) chloride hexahydrate (3.0 g, 13 mmol) in THF (100 mL) and water (50 mL) at 0 °C, and the resulting mixture was stirred at 25 °C for 16 h. The reaction mixture was quenched with H₂O and extracted with EtOAc. The combined organic phases were washed with brine, dried over anhydrous Na₂SO₄, filtered, and concentrated under reduced pressure. The residue was purified by silica gel column chromatography (EtOAc in hexanes) to give 7-((*tert*-butyldiphenylsilyl)oxy)-2-azaspiro[4.5]decan-1-one. LCMS (C₂₅H₃₄NO₂Si⁻) (ES, m/z): 408 [M+H]⁺.

### Step 4: 2-benzyl-7-((tert-butyldiphenylsilyl)oxy)-2-azaspiro[4.5]decan-1-one

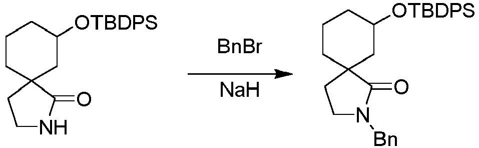

NaH (0.95 g, 24 mmol, 60 wt% in mineral oil) was added to a solution of 7-((*tert-*butyldiphenylsilyl)oxy)-2-azaspiro[4.5]decan-1-one (8.8 g, 22 mmol) in THF (100 mL) at 0 °C under N₂. The resulting mixture was stirred for 1 h at 0 °C, followed by addition of (bromomethyl)benzene (2.8 mL, 24 mmol) at 0 °C. The reaction mixture was stirred at 0 °C for 16 h, then quenched with saturated aqueous NH₄Cl and extracted with EtOAc. The combined organic phases were washed with brine, dried over anhydrous Na₂SO₄, filtered, and concentrated under reduced pressure. The residue was purified by flash silica gel column chromatography (EtOAc in hexanes) to give 2-benzyl-7-((*tert*-butyldiphenylsilyl)oxy)-2-azaspiro[4.5]decan-1-one. LCMS (C₃₂H₄₀NO₂Si⁺) (ES, m/z): 498 [M+H]⁺.

### Step 5: 2-benzyl-7-((tert-butyldiphenylsilyl)oxy)-2-azaspiro[4.5]decane-1-carbonitrile

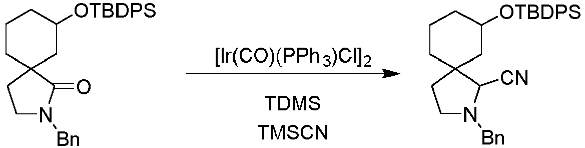

Carbonylchlorobis(triphenylphosphine)iridium(I) (0.47 g, 0.60 mmol) was added to a solution of 2-benzyl-7-((*tert*-butyldiphenylsilyl)oxy)-2-azaspiro[4.5]decan-1-one (4.0 g, 8.0 mmol) in toluene (40 mL) at 15 °C, and the resulting mixture was stirred for 5 minutes, followed by addition of 1,1,3,3-tetramethyldisiloxane (5.7 mL, 32 mmol), then stirred for another 15 minutes. Trimethylsilanecarbonitrile (4.0 mL, 32 mmol) was added, and the reaction mixture was stirred at 15 °C for 15 h, then washed with 1 M aqueous NaOH, and extracted with EtOAc. The combined organic phases were dried over Na₂SO₄, filtered, and concentrated under reduced pressure. The residue was purified by silica gel column chromatography (EtOAc in hexanes) to give 2-benzyl-7-((*tert*-butyldiphenylsilyl)oxy)-2-azaspiro[4.5]decane-1-carbonitrile. LCMS (C₃₃H₄₁N₂OSi⁺) (ES, m/z): 509 [M+H]⁺.

### Step 6: 2-benzyl-7-((tert-butyldiphenylsilyl)oxy)-2-azaspiro[4.5]decane-1-carboxamide

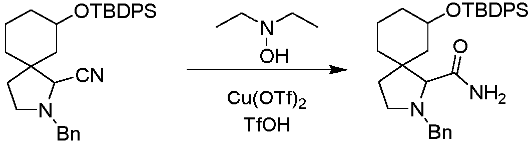

Trifluoromethanesulfonic acid (0.026 mL, 0.30 mmol) was added to a solution of 2-benzyl-7-((*tert*-butyldiphenylsilyl)oxy)-2-azaspiro[4.5]decane-1-carbonitrile (1.5 g, 3.0 mmol) in DCE (15 mL) at 30 °C under N₂, followed by addition of N,N-diethylhydroxylamine (0.91 mL, 8.8 mmol) and bis(((trifluoromethyl)sulfonyl)oxy)copper (107 mg, 0.30 mmol). The reaction mixture was stirred at 50 °C for 16 h, then quenched with water and extracted with DCM. The combined organic phases were washed with brine, dried over anhydrous Na₂SO₄, filtered, and concentrated under reduced pressure. The residue was purified by silica gel column chromatography (EtOAc in hexanes) to give 2-benzyl-7-((*tert*-butyldiphenylsilyl)oxy)-2-azaspiro[4.5]decane-1-carboxamide. LCMS (C₃₃H₄₃N₂O₂Si⁺) (ES, m/z): 527 [M+H]⁺.

### Step 7: methyl 2-benzyl-7-((tert-butyldiphenylsilyl)oxy)-2-azaspiro[4.5]decane-1-carboxylate

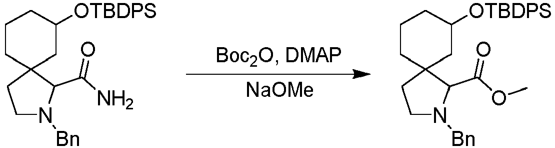

Boc₂O (0.62 mL, 2.7 mmol) was added to a solution of 2-benzyl-7-((*tert-*butyldiphenylsilyl)oxy)-2-azaspiro[4.5]decane-1-carboxamide (350 mg, 0.66 mmol) and DMAP (81 mg, 0.66 mmol) in DCE (3.0 mL) at 25 °C, and the resulting mixture was stirred at 60~80 °C for additional 13 h, then concentrated. Sodium methoxide (72 mg, 1.3 mmol) and MeOH (1.5 mL) were added to the crude mixture and the reaction mixture was stirred at 80 °C for 12 h, then concentrated, quenched by addition of saturated aqueous NH₄Cl and extracted with EtOAc. The combined organic phases were dried over anhydrous Na₂SO₄, filtered, and concentrated under reduced pressure. The residue was purified by silica gel column chromatography (EtOAc in hexanes) to give methyl 2-benzyl-7-((*tert*-butyldiphenylsilyl)oxy)-2-azaspiro[4.5]decane-1-carboxylate. LCMS (C₃₄H₄₄NO₃Si⁺) (ES, m/z): 542 [M+H]⁺.

### Step 8: methyl 2-benzyl-7-hydroxy-2-azaspiro[4.5]decane-1-carboxylate

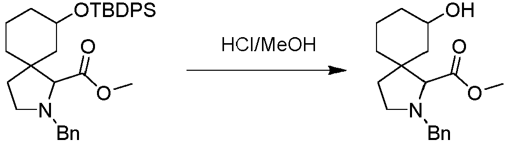

A mixture of 4 M HCl in MeOH (5.0 mL, 20 mmol) and methyl 2-benzyl-7-((*tert-*butyldiphenylsilyl)oxy)-2-azaspiro[4.5]decane-1-carboxylate (400 mg, 0.74 mmol) was stirred at 60 °C for 16 h. The reaction mixture was concentrated, then quenched by addition of saturated aqueous NaHCO₃ and extracted with EtOAc. The combined organic phases were dried over anhydrous Na₂SO₄, filtered, and concentrated under reduced pressure. The residue was purified by silica gel column chromatography (EtOAc in hexanes) to give methyl 2-benzyl-7-hydroxy-2-azaspiro[4.5]decane-1-carboxylate. LCMS (C₁₈H₂₆NO₃⁺) (ES, m/z): 304 [M+H]⁺.

### Step 9: methyl 2-benzyl-7-oxo-2-azaspiro[4.5]decane-1-carboxylate

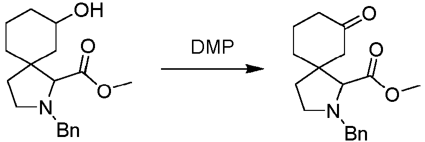

Dess-Martin Periodinane (559 mg, 1.3 mmol) was added to a mixture of methyl 2-benzyl-7-hydroxy-2-azaspiro[4.5]decane-1-carboxylate (200 mg, 0.66 mmol) and DCM (2.0 mL) and the resulting mixture was stirred at 26 °C for 12 h, then quenched by addition of saturated aqueous NaHCO₃ and extracted with EtOAc. The combined organic phases were washed with brine, dried over anhydrous Na₂SO₄, filtered, and concentrated under reduced pressure. The residue was purified by silica gel column chromatography (EtOAc in hexanes) to give methyl 2-benzyl-7-oxo-2-azaspiro[4.5]decane-1-carboxylate. LCMS (C₁₈H₂₄NO₃⁺) (ES, m/z): 302 [M+H]⁺.

### Step 10: methyl 2-benzyl-7-methylene-2-azaspiro[4.5]decane-1-carboxylate

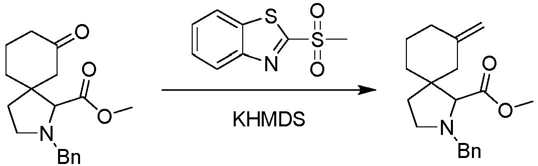

LiHMDS (0.64 mL, 0.64 mmol, 1.0 M in THF) was added to a mixture of methyl 2-benzyl-7-oxo-2-azaspiro[4.5]decane-1-carboxylate (80 mg, 0.27 mmol) and 2-(methylsulfonyl)benzo[*d*]thiazole (113 mg, 0.53 mmol) in THF (2.0 mL) at -78 °C under N₂, and the resulting mixture was stirred at -78 °C for 0.5 h, then at 26 °C for 12 h under N₂. The reaction mixture was quenched with saturated aqueous NH₄Cl and extracted with DCM. The combined organic layers were washed with brine, dried over anhydrous Na₂SO₄, filtered, and concentrated. The residue was purified by silica gel column chromatography (EtOAc in hexanes) to give methyl 2-benzyl-7-methylene-2-azaspiro[4.5]decane-1-carboxylate. LCMS (C₁₉H₂₆NO₂⁺) (ES, m/z): 300 [M+H] ⁺.

### Step 11: methyl 2-benzyl-7-((4, 4, 5, 5-tetramethyl-1, 3, 2-dioxaborolan-2-yl)methyl)-2-azaspiro[4.5]decane-1-carboxylate

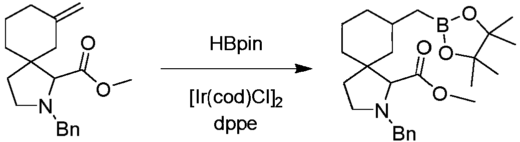

4,4,5,5-Tetramethyl-1,3,2-dioxaborolane (0.044 mL, 0.30 mmol) was added to a solution of [Ir(cod)Cl]₂ (4.7 mg, 7.0 µmol) and dppe (6.0 mg, 0.015 mmol) in DCM (2.0 mL) under N₂, followed by addition of methyl 2-benzyl-7-methylene-2-azaspiro[4.5]decane-1-carboxylate (30 mg, 0.10 mmol), and the resulting mixture was stirred at 30 °C for 12 h. The reaction mixture was concentrated, and the residue was purified by RP-HPLC [C18 column, water (0.1% TFA)-CH₃CN] to give methyl 2-benzyl-7-((4,4,5,5-tetramethyl-1,3,2-dioxaborolan-2-yl)methyl)-2-azaspiro[4.5]decane-1-carboxylate. LCMS (C₂₅H₃₉BNO₄⁺) (ES, m/z): 428 [M+H]⁺.

### Step 12: methyl 7-((4,4,5,5-tetramethyl-1,3,2-dioxaborolan-2-yl)methyl)-2-azaspiro[4.5]decane-1-carboxylate

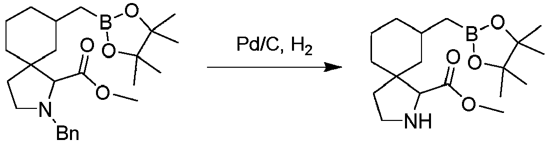

10% Pd-C (50 mg, 0.047 mmol) and 10% Pd(OH)₂ (33 mg, 0.047 mmol) were added to a solution of methyl 2-benzyl-7-((4,4,5,5-tetramethyl-1,3,2-dioxaborolan-2-yl)methyl)-2-azaspiro[4.5]decane-1-carboxylate (20 mg, 0.047 mmol) in MeOH (5.0 mL) under N₂. The resulting mixture was degassed and backfilled with H₂ (three times), then stirred under H₂ (345 kPa (50 at 35 °C for 16 h. The reaction mixture was filtered, and concentrated under reduced pressure to give a mixture of methyl 7-((4,4,5,5-tetramethyl-1,3,2-dioxaborolan-2-yl)methyl)-2-azaspiro[4.5]decane-1-carboxylate and its corresponding boronic acid, which was used in next step directly without further purification. LCMS (C₁₈H₃₃BNO₄⁺) (ES, m/z): 338 [M+H]⁺.

### Step 13: 7-(boronomethyl)-2-azaspiro[4.5]decane-1-carboxylic acid

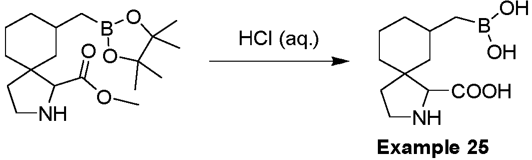

A mixture of 12 N HCl in water (2.0 mL, 24 mmol) and methyl 7-((4,4,5,5-tetramethyl-1,3,2-dioxaborolan-2-yl)methyl)-2-azaspiro[4.5]decane-1-carboxylate (15 mg, 0.044 mmol) was stirred at 100 °C for 12 h. The reaction mixture was concentrated, and the residue was purified by RP-HPLC [C18 column, water (20 mM HFBA and 0.1% TFA)-CH₃CN] to give 7-(boronomethyl)-2-azaspiro[4.5]decane-1-carboxylic acid as an HFBA salt. LCMS (C₁₁H₂₁BNO₄⁺) (ES, m/z): 242 [M+H]⁺. ¹H NMR (500 MHz, D₂O) *δ* 3.74 (s, 1H), 3.45 - 3.28 (m, 2H), 2.21-2.16 (m, 1H), 1.88 - 1.78 (m, 1H), 1.63-1.60 (m, 2H), 1.55 - 1.47 (m, 2H), 1.45 - 1.28 (m, 3H), 1.12 - 1.01 (m, 1H), 0.89 - 0.73 (m, 1H), 0.66 (d, *J* = 7.0 Hz, 2H).

### Example 26: (1S,2S,5R)-1-(3-boronopropyl)-6-oxa-3-azabicyclo[3.2.0]heptane-2-carboxylic acid

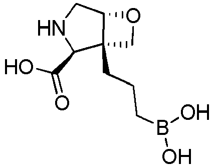

### Step 1: (2S,3S)-1-benzyl 2-methyl 3-allyl-3-(hydroxymethyl)-4-oxopyrrolidine-1,2-dicarboxylate

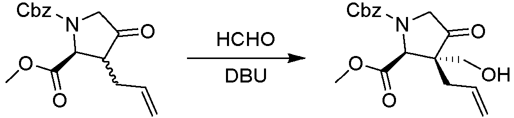

DBU (0.14 mL, 0.95 mmol) in THF (0.30 mL) was added to a mixture of (*2S*)-1-benzyl 2-methyl 3-allyl-4-oxopyrrolidine-1,2-dicarboxylate (3.0 g, 9.5 mmol) and 37 wt% formaldehyde in H₂O (0.70 mL, 9.5 mmol) in THF (40 mL) at 0 °C. The resulting mixture was stirred at 0 °C for 1 h, then at 20 °C for 14 h. The reaction mixture was concentrated and the residue was purified by flash silica gel chromatography (EtOAc in hexanes) to give (*2S,3S*)-1-benzyl 2-methyl 3-allyl-3-(hydroxymethyl)-4-oxopyrrolidine-1,2-dicarboxylate. LCMS (C₁₈H₂₂NO₆⁺) (ES, m/z): 348 [M+H]⁺.

### Step 2: (2S,3S)-1-benzyl 2-methyl 3-allyl-3-(((tert-butyldimethylsilyl)oxy)methyl)-4-oxopyrrolidine-1,2-dicarboxylate

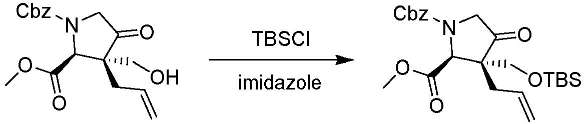

TBSCl (2.1 g, 14 mmol) was added to a mixture of (*2S,3S*)-1-benzyl 2-methyl 3-allyl-3-(hydroxymethyl)-4-oxopyrrolidine-1,2-dicarboxylate (2.4 g, 6.9 mmol) and imidazole (1.2 g, 17 mmol) in DCM (30 mL) at 0 °C. The resulting mixture was stirred at 0 °C for 1 h, then at 20 °C for 14 h. The reaction mixture was quenched with saturated aqueous NH₄Cl and extracted with DCM. The combined organic phases were washed with brine, dried over anhydrous Na₂SO₄, filtered, and concentrated under reduced pressure. The residue was purified by flash silica gel chromatography (EtOAc in hexanes) to give (*2S,3S*)-1-benzyl 2-methyl 3-allyl-3-(((tertbutyldimethylsilyl)oxy)methyl)-4-oxopyrrolidine-1,2-dicarboxylate. LCMS (C₂₄H₃₆NO₆Si⁺) (ES, m/z): 462 [M+H]⁺.

### Step 3: (2S, 3S, 4R)-1-benzyl 2-methyl 3-allyl-3-(((tert-butyldimethylsilyl)oxy)methyl)-4-hydroxypyrrolidine-1,2-dicarboxylate

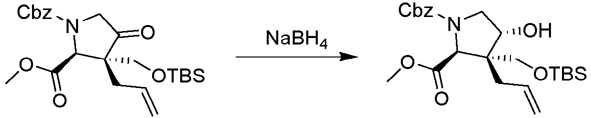

NaBH₄ (0.25 g, 6.5 mmol) was added to a mixture of (*2S,3S*)-1-benzyl 2-methyl 3-allyl-3-(((*tert*-butyldimethylsilyl)oxy)methyl)-4-oxopyrrolidine-1,2-dicarboxylate (3.0 g, 6.5 mmol) in MeOH (40 mL), and the resulting mixture was stirred at 25 °C for 0.5 h, then quenched with acetone. The reaction mixture was concentrated, and the residue was purified by flash silica gel chromatography (EtOAc in hexanes) to give (*2S,3S,4R*)-1-benzyl 2-methyl 3-allyl-3-(((tertbutyldimethylsilyl)oxy)methyl)-4-hydroxypyrrolidine-1,2-dicarboxylate. The stereochemistry was assigned by 2D NMR. LCMS (C₂₄H₃₈NO₆Si⁺) (ES, m/z): 464 [M+H]⁺. ¹H NMR (400 MHz, CDCl₃) *δ* 7.38-7.26 (m, 5H), 5.89-5.73 (m, 1H), 5.22-4.99 (m, 4H), 4.42-4.32 (m, 2H), 3.94-3.76 (m, 3H), 3.76-3.51 (m, 4H), 3.49-3.40 (m, 1H), 2.32-2.17 (m, 1H), 2.15-2.05 (m, 1H), 0.91-0.87 (m, 9H), 0.12--0.02 (m, 6H).

### Step 4: (2S, 3S, 4R)-1-benzyl 2-methyl 3-allyl-4-hydroxy-3-(hydroxymethyl)pyrrolidine-1, 2-dicarboxylate

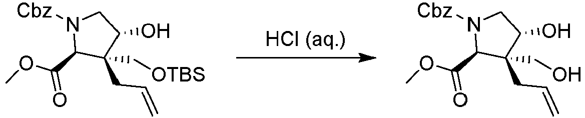

2 N HCl in water (7.0 mL, 14 mmol) was added to a mixture of (*2S,3S,4R*)-1-benzyl 2-methyl 3-allyl-3-(((*tert*-butyldimethylsilyl)oxy)methyl)-4-hydroxypyrrolidine-1,2-dicarboxylate (400 mg, 0.86 mmol) in THF (6.0 mL), and the resulting mixture was stirred at 35 °C for 2 h, then quenched with NaHCO₃ to pH 7~8 at 0 °C and extracted with EtOAc. The combined organic phases were washed with brine, dried over anhydrous Na₂SO₄, filtered, and concentrated under reduced pressure. The residue was purified by flash silica gel chromatography (EtOAc in hexanes) to give (*2S,3S,4R*)-1-benzyl 2-methyl 3-allyl-4-hydroxy-3-(hydroxymethyl)pyrrolidine-1,2-dicarboxylate. LCMS (C₁₈H₂₄NO₆⁺) (ES, m/z): 350 [M+H]⁺.

### Step 5: (2S,3S,4R)-1-benzyl 2-methyl 3-allyl-4-hydroxy-3-((((4-(trifluoromethyl)phenyl)sulfonyl)oxy)methyl)pyrrolidine-1,2-dicarboxylate

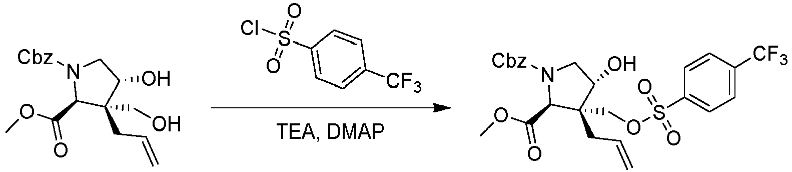

N,N-dimethylpyridin-4-amine (3.9 mg, 0.031 mmol) was added to a mixture of (*2S,3S,4R*)-1-benzyl 2-methyl 3-allyl-4-hydroxy-3-(hydroxymethyl)pyrrolidine-1,2-dicarboxylate (110 mg, 0.32 mmol), 4-(trifluoromethyl)benzene-1-sulfonyl chloride (81 mg, 0.33 mmol) and triethylamine (0.22 mL, 1.6 mmol) in DCM (8.0 mL) at 0 °C. The resulting mixture was stirred at 0 °C for 2.5 h, followed by addition of 4-(trifluoromethyl)benzene-1-sulfonyl chloride (77 mg, 0.32 mmol). The reaction mixture was stirred at 0 °C for 1 h, then at 25 °C for 13 h, before quenched with saturated aqueous NH₄Cl and extracted with DCM. The combined organic layers were washed with brine, dried over anhydrous Na₂SO₄, filtered, and concentrated. The residue was purified by flash silica gel chromatography (EtOAc in hexanes) to give (*2S,3S,4R*)-1-benzyl 2-methyl 3-allyl-4-hydroxy-3-((((4-(trifluoromethyl)phenyl)sulfonyl)oxy)methyl)pyrrolidine-1,2-dicarboxylate. LCMS (C₂₄H₂₇F₃NO₆S ⁺) (ES, m/z): 514 [M+H-CO₂]⁺.

### Step 6: (1S,2S, 5R)-3-benzyl 2-methyl 1-allyl-6-oxa-3-azabicyclo[3.2.0]heptane-2,3-dicarboxylate

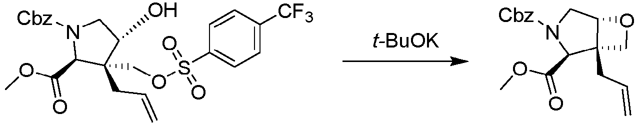

Potassium 2-methylpropan-2-olate (0.63 mL, 0.63 mmol, 1.0 M in THF) was added to a mixture of (*2S,3S,*4R)-1-benzyl 2-methyl 3-allyl-4-hydroxy-3-((((4-(trifluoromethyl)phenyl)sulfonyl)oxy)methyl)pyrrolidine-1,2-dicarboxylate (320 mg, 0.57 mmol) in THF (7.0 mL) at 0 °C under N₂, and the resulting mixture was stirred at 0 °C for 1 h, then quenched with saturated aqueous NH₄Cl and extracted with DCM. The combined organic layers were washed with brine, dried over anhydrous Na₂SO₄, filtered, and concentrated. The residue was purified by flash silica gel chromatography (EtOAc in hexanes) to give (*1S,2S,5R*)-3-benzyl 2-methyl 1-allyl-6-oxa-3-azabicyclo[3.2.0]heptane-2,3-dicarboxylate. LCMS (C₁₈H₂₂NO₅⁺) (ES, m/z): 332 [M+H]⁺.

### Step 7: (1S,2S, 5R)-3-benzyl 2-methyl 1-(3-(4, 4, 5, 5-tetramethyl-1, 3, 2-dioxaborolan-2-yl)propyl)-6-oxa-3-azabicyclo[3.2.0]heptane-2,3-dicarboxylate

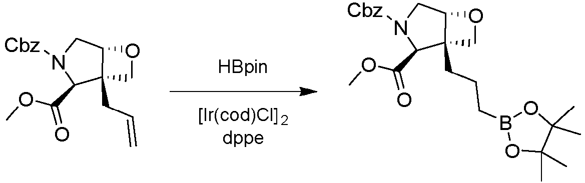

A mixture of 1,2-bis(diphenylphosphino)ethane (6.7 mg, 0.017 mmol), [Ir(cod)Cl]₂ (8.1 mg, 0.012 mmol) and 4,4,5,5-tetramethyl-1,3,2-dioxaborolane (0.18 mL, 1.2 mmol) in DCM (8.0 mL) was stirred at 25 °C for 15 minutes under N₂, followed by addition of (IS,2S,SR)-3-benzyl 2-methyl 1-allyl-6-oxa-3-azabicyclo[3.2.0]heptane-2,3-dicarboxylate (80 mg, 0.24 mmol) in DCM (1.5 mL). The reaction mixture was stirred at 25 °C for 15 h under N₂, then concentrated and the residue was purified by flash silica gel chromatography (EtOAc in hexanes) to give (IS,2S,SR)-3-benzyl 2-methyl 1-(3-(4,4,5,5-tetramethyl-1,3,2-dioxaborolan-2-yl)propyl)-6-oxa-3-azabicyclo[3.2.0]heptane-2,3-dicarboxylate. LCMS (C₂₄H₃₅BNO₇⁺) (ES, m/z): 460 [M+H]⁺.

### Step 8: (1S,2S, 5R)-methyl 1-(3-(4, 4, 5, 5-tetramethyl-1, 3, 2-dioxaborolan-2-yl)propyl)-6-oxa-3-azabicyclo[3.2.0]heptane-2-carboxylate

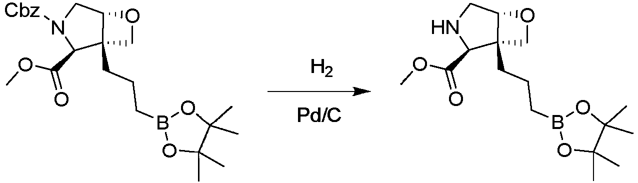

10%Pd-C (139 mg, 0.13 mmol) was added to a mixture of (*1S,2S,5R*)-3-benzyl 2-methyl 1-(3-(4,4,5,5-tetramethyl-1,3,2-dioxaborolan-2-yl)propyl)-6-oxa-3-azabicyclo[3.2.0]heptane-2,3-dicarboxylate (60 mg, 0.13 mmol) in MeOH (8.0 mL) under N₂. The resulting mixture was degassed and backfilled with H₂ (three times), then stirred under H₂ (103 kPa (15 psi)) at 25 °C for 1 h. The reaction mixture was filtered and concentrated under reduced pressure to give crude (IS,2S,SR)-methyl 1-(3-(4,4,5,5-tetramethyl-1,3,2-dioxaborolan-2-yl)propyl)-6-oxa-3-azabicyclo[3.2.0]heptane-2-carboxylate with corresponding boronic acid, which was used in next step directly without further purification. LCMS (C₁₆H₂₉BNO₅⁺) (ES, m/z): 326 [M+H]⁺.

### Step 9: (1S,2S, 5R)-1-(3-boronopropyl)-6-oxa-3-azabicyclo[3.2.0]heptane-2-carboxylic acid

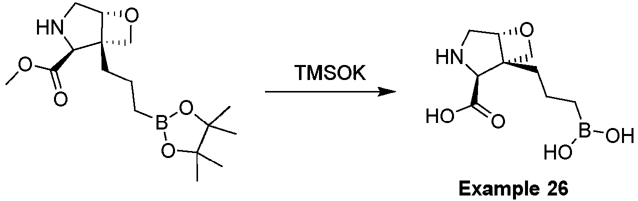

Potassium trimethylsilanolate (160 mg, 1.2 mmol) was added to a mixture of (*1S,2S,5R*)-methyl 1-(3-(4,4,5,5-tetramethyl-1,3,2-dioxaborolan-2-yl)propyl)-6-oxa-3-azabicyclo[3.2.0]heptane-2-carboxylate (80 mg, 0.14 mmol) and THF (6.0 mL) at 25 °C, and the resulting mixture was stirred at 25 °C for 12 h, followed by addition of 30 wt% NH₃·H₂O (0.50 mL) and H₂O (6.0 mL). The reaction mixture was stirred at 25 °C for 24 h, then concentrated under reduced pressure. The residue was purified by RP-HPLC [C18 column, water (10 mM NH₄HCO₃)-CH₃CN] to give (*1S,2S,5R*)-1-(3-boronopropyl)-6-oxa-3-azabicyclo[3.2.0]heptane-2-carboxylic acid. LCMS (C₉H₁₅BNO₄⁺) (ES, m/z): 212 [M+H-H₂O]⁺. ¹H NMR (400 MHz, D₂O) *δ* 5.18 (d, *J=* 3.1 Hz, 1H), 4.71-4.65 (m, 1H), 4.39 (d, *J= 7.0* Hz, 1H), 4.17 (s, 1H), 3.66-3.58 (m, 1H), 3.50-3.42 (m, 1H), 1.80-1.68 (m, 1H), 1.68-1.51 (m, 2H), 1.50-1.37 (m, 1H), 0.82-0.74 (m, 2H).

### Example 27: (3aS,4S,6aR)-3a-(3-boronopropyl)hexahydro-1H-furo[3,4-c]pyrrole-4-carboxylic acid

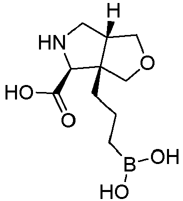

### Step 1: (2S, 3S,Z)-1-benzyl 2-methyl 3-allyl-3-(((tert-butyldimethylsilyl)oxy)methyl)-4-(methoxymethylene)pyrrolidine-1,2-dicarboxylate

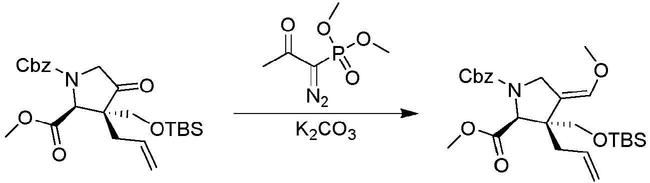

Dimethyl (1-diazo-2-oxopropyl)phosphonate (1.0 g, 5.3 mmol) was added to a solution of (*2S,3S*)-1-benzyl 2-methyl 3-allyl-3-(((*tert*-butyldimethylsilyl)oxy)methyl)-4-oxopyrrolidine-1,2-dicarboxylate (1.6 g, 3.5 mmol) and potassium carbonate (0.97 g, 7.0 mmol) in MeOH (30 mL) at 30 °C, and the resulting mixture was stirred for 12 h. The reaction mixture was quenched with saturated aqueous NH₄Cl and extracted with EtOAc. The combined organic phases were washed with brine, dried over anhydrous Na₂SO₄, filtered, and concentrated under reduced pressure. The residue was purified by silica gel column chromatography (EtOAc in hexanes) to give (*2S,3S*)-1-benzyl 2-methyl 3-allyl-3-(((*tert*-butyldimethylsilyl)oxy)methyl)-4-(methoxymethylene)pyrrolidine-1,2-dicarboxylate. LCMS (C₂₆H₄₀NO₆Si⁺) (ESI, m/z): 490 [M+H]⁺.

### Step 2: (3aS, 4S)-5-benzyl 4-methyl 3a-allyl-1-hydroxytetrahydro-1H-furo[3,4-c]pyrrole-4,5(3H)-dicarboxylate

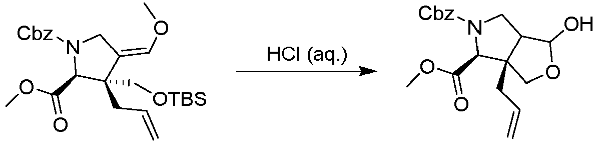

A mixture of (*2S,3S*)-1-benzyl 2-methyl 3-allyl-3-(((*tert*-butyldimethylsilyl)oxy)methyl)-4-(methoxymethylene)pyrrolidine-1,2-dicarboxylate (618 mg, 1.3 mmol) and 2 N HCl in water (30 mL, 60 mmol) in THF (16 mL) was stirred at 50 °C for 30 h. The reaction mixture was diluted with brine and extracted with EtOAc. The combined organic phases were washed with brine, dried over anhydrous Na₂SO₄, filtered, and concentrated under reduced pressure. The residue was purified by silica gel column chromatography (EtOAc in hexanes) to give (*3aS,4S*)-5-benzyl 4-methyl 3a-allyl-1-hydroxytetrahydro-1*H*-furo[3,4-*c*]pyrrole-4,5(3*H*)-dicarboxylate. LCMS (C₁₉H₂₄NO₆⁺) (ESI, m/z): 362 [M+H]⁺.

### Step 3: (3aS, 4S, 6aR)-5-benzyl 4-methyl 3a-allyltetrahydro-1H-furo[3,4-c]pyrrole-4,5(3H)-dicarboxylate

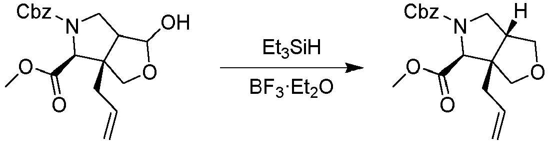

BF₃·Et₂O (0.13 mL, 1.1 mmol) was added to a solution of (*3aS,*4,*S*)-5-benzyl 4-methyl 3a-allyl-1-hydroxytetrahydro-1*H*-furo[3,4-*c*]pyrrole-4,5(3*H*)-dicarboxylate (190 mg, 0.53 mmol) and triethylsilane (0.14 mL, 0.89 mmol) in DCM (6.0 mL) at 0 °C, and the resulting mixture was stirred for 1.5 h at 0 °C. The reaction mixture was quenched with methanol and concentrated under reduced pressure. The residue was purified by silica gel column chromatography (EtOAc in hexanes) to give (*3aS,4S,6aR)-5-benzyl* 4-methyl 3a-allyltetrahydro-1*H*-furo[3,4-*c*]pyrrole-4,5(3*H*)-dicarboxylate. LCMS (C₁₉H₂₄NO₅⁺) (ESI, m/z): 346 [M+H]⁺.

### Step 4: (3aS,4S)-5-benzyl 4-methyl 3a-(3-(4,4,5,5-tetramethyl-1,3,2-dioxaborolan-2-yl)propyl)tetrahydro-1H-furo[3,4-c]pyrrole-4,5(3H)-dicarboxylate

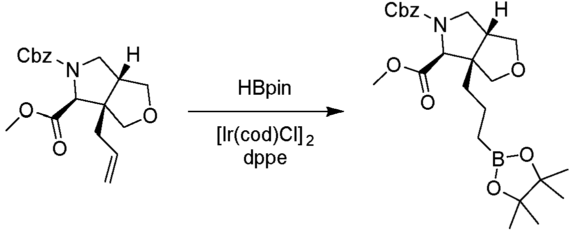

4,4,5,5-Tetramethyl-1,3,2-dioxaborolane (0.29 mL, 2.0 mmol) was added to a mixture of dppe (13 mg, 0.032 mmol) and [Ir(cod)Cl]₂ (16 mg, 0.024 mmol) in DCM (2.0 mL) under N₂ at 0 °C, followed by addition of (*3aS,4S,6aR*)-5-benzyl 4-methyl 3a-allyltetrahydro-1*H*-furo[3,4-c]pyrrole-4,5(3*H*)-dicarboxylate (140 mg, 0.41 mmol) in DCM (2.0 mL), and the resulting mixture was stirred at 28 °C for 16 h. The reaction mixture was quenched with MeOH and concentrated under reduced pressure. The residue was purified by RP-HPLC [C18 column, water (0.1% TFA)-CH₃CN] to give a mixture of *(3aS,4S,6aR)-5-benzyl* 4-methyl 3a-(3-(4,4,5,5-tetramethyl-1,3,2-*dioxaborolan-2-yl)propyl)tetrahydro-1H-furo[3,4-c]pyrrole-4,5(3H)-dicarboxylate* and its corresponding boronic acid. LCMS (C₂₅H₃₇BNO₇⁺) (ESI, m/z): 474 [M+H]⁺.

### Step 5: (3aS,4S,6aR)-3a-(3-boronopropyl)hexahydro-1H-furo[3,4-c]pyrrole-4-carboxylic acid

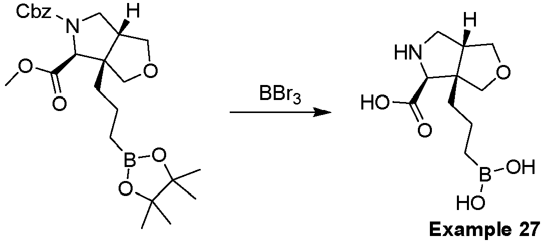

A mixture of *(3aS,4S,6aR)-5-benzyl* 4-methyl 3a-(3-(4,4,5,5-tetramethyl-1,3,2-dioxaborolan-2-yl)propyl)tetrahydro-1*H*-furo[3,4-c]pyrrole-4,5(3H)-dicarboxylate (90 mg, 0.19 mmol) and BBr₃ (1.8 mL, 19 mmol) in DCM (10 mL) was stirred at 28 °C for 40 h, and the solvent was removed by a stream of N₂. The residue was purified by RP-HPLC [C18 column, water (20 mM HFBA and 0.1% TFA)-CH₃CN] to give (*3aS,4S,6aR*)-3a-(3-boronopropyl)hexahydro-1*H*-furo[3,4-*c*]pyrrole-4-carboxylic acid as an HFBA salt. LCMS (C₁₀H₁₇BNO₄⁺) (ESI, m/z): 226 [M+H-H₂O]⁺. ¹H NMR (500 MHz, D₂O) *δ* 4.01 (s, 1H), 3.96 (d, *J=* 9.9 Hz, 1H), 3.81-3.62 (m, 3H), 3.52 (d, *J=* 9.9 Hz, 1H), 3.02-2.93 (m, 1H), 2.92-2.83 (m, 1H), 1.56-1.43 (m, 2H), 1.41-1.22 (m, 2H), 0.66 (t, *J= 7.5* Hz, 2H).

### Example 28: (1S,2S,5R)-1-(3-boronopropyl)-6-thia-3-azabicyclo[3.2.0]heptane-2-carboxylic acid hydrochloride

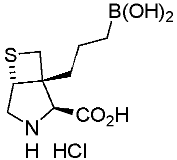

### Step 1: 1-(tert-butyl) 2-methyl (2S, 3S,4S)-3-allyl-4-((chloromethyl)sulfonyl)-3-(((chloromethyl)sulfonyl)methyl)pyrrolidine-1,2-dicarboxylate

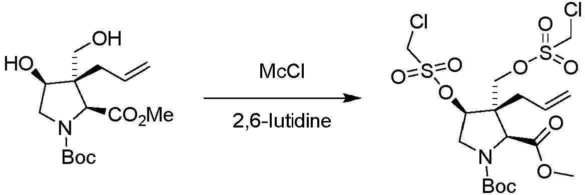

Chloromethanesulfonyl chloride (36 mL, 400 mmol) was added dropwise into a mixture of (*2S,3S,4S*)-1-*tert*-butyl 2-methyl 3-allyl-4-hydroxy-3-(hydroxymethyl)pyrrolidine-1,2-dicarboxylate (42 g, 133 mmol), 2,6-dimethylpyridine (124 mL, 1065 mmol) in anhydrous DCM (600 mL) at 0 °C, and the resulting mixture was stirred at 0 °C for 0.5 h, then at 25 °C for 1 h. The reaction mixture was diluted with H₂O and extracted with DCM. The combined organic phases were washed with 1 N HCl in water, saturated aqueous NaHCO₃ and brine, dried over Na₂SO₄, filtered, and concentrated. The residue was purified by flash silica gel chromatography (EtOAc in hexanes) to give (*2S,3S,4S*)-1-*tert*-butyl 2-methyl 3-allyl-4-(((chloromethyl)sulfonyl)oxy)-3-((((chloromethyl)sulfonyl)oxy)methyl)pyrrolidine-1,2-dicarboxylate. LCMS (C₁₂H₂₀Cl₂NO₈S₂⁺) (ES, m/z): 440 [M+H-Boc]⁺.

### Step 2: 1-(tert-butyl) 2-methyl (2S,3S,4S)-4-((chloromethyl)sulfonyl)-3-(((chloromethyl)sulfonyl)methyl)-3-(3-(4,4,5,5-tetramethyl-1,3,2-dioxaborolan-2-yl)propyl)pyrrolidine-1,2-dicarboxylate

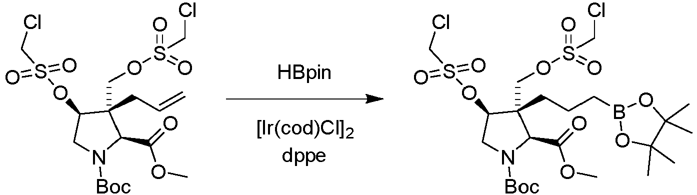

4,4,5,5-Tetramethyl-1,3,2-dioxaborolane (21 mL, 148 mmol) was added dropwise to a mixture of dppe (1.2 g, 3.0 mmol) and [Ir(cod)Cl]₂ (0.99 g, 1.5 mmol) in DCM (350 mL) at 0 °C under N₂ over 10 minutes, followed by dropwise addition of a solution of (*2S,3S,4S*)-1-*tert*-butyl *2-*methyl 3-allyl-4-(((chloromethyl)sulfonyl)oxy)-3-((((chloromethyl)sulfonyl)oxy)methyl)pyrrolidine-1,2-dicarboxylate (16 g, 30 mmol) in DCM (300 mL) over 1 h at 0 °C under N₂. The reaction mixture was stirred at 25 °C for 12 h, and concentrated in vacuo. The residue was directly purified by silica gel chromatography (EtOAc in hexanes) to give (*2S,3S,4S*)-1-*tert*-butyl 2-methyl 4-(((chloromethyl)sulfonyl)oxy)-3-((((chloromethyl)sulfonyl)oxy)methyl)-3-(3-(4,4,5,5-tetramethyl-1,3,2-dioxaborolan-2-yl)propyl)pyrrolidine-1,2-dicarboxylate. LCMS (C₁₈H₃₃BCl₂NO₁₀S₂⁺) (ES, m/z): 568 [M+H-Boc]⁺.

### Step 3: 1-(tert-butyl) 2-methyl (2S,3S,4S)-3-((acetylthio)methyl)-4-((chloromethyl)sulfonyl)-3-(3-(4,4,5,5-tetramethyl-1,3,2-dioxaborolan-2-yl)propyl)pyrrolidine-1,2-dicarboxylate

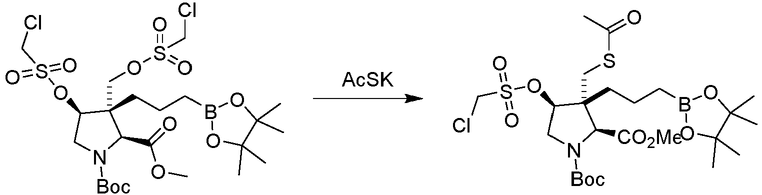

A mixture of potassium thioacetate (4.3 g, 38 mmol) in DMF (45 mL) was added dropwise to a mixture of *((2S,3S,4S*)-1-*tert*-butyl 2-methyl 4-(((chloromethyl)sulfonyl)oxy)-3-((((chloromethyl)sulfonyl)oxy)methyl)-3-(3-(4,4,5,5-tetramethyl-1,3,2-dioxaborolan-2-yl)propyl)pyrrolidine-1,2-dicarboxylate (19 g, 29 mmol) in DMF (57 mL) over 2 h at 35 °C under N₂, and the resulting mixture was stirred at 40 °C for 28 h under N₂. The reaction mixture was diluted with EtOAc and washed with water. The organic layer was separated, dried over anhydrous Na₂SO₄, filtered, and concentrated in vacuo. The residue was purified by silica gel chromatography (EtOAc in hexanes) to give (*2S,3R,4S*)-1-*tert*-butyl 2-methyl 3-((acetylthio)methyl)-4-(((chloromethyl)sulfonyl)oxy)-3-(3-(4,4,5,5-tetramethyl-1,3,2-dioxaborolan-2-yl)propyl)pyrrolidine-1,2-dicarboxylate. LCMS (C₁₉H₃₄BClNO₈S₂⁺) (ES, m/z): 514 [M+H-Boc]⁺.

### Step 4: 3-(tert-butyl) 2-methyl (1S,2S,5R)-1-(3-(4,4, 5, 5-tetramethyl-1, 3, 2-dioxaborolan-2-yl)propyl)-6-thia-3-azabicyclo[3.2.0]heptane-2,3-dicarboxylate

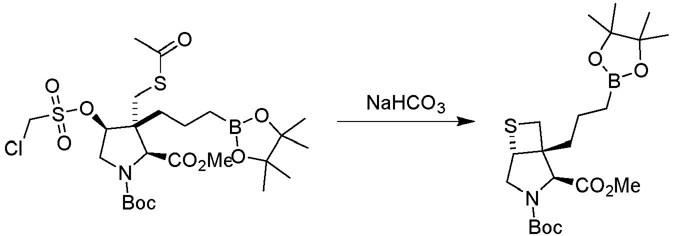

NaHCO₃ (8.8 g, 104 mmol) was added to a mixture of (*2S,3R,4S*)-1-*tert-*butyl 2-methyl 3-((acetylthio)methyl)-4-(((chloromethyl)sulfonyl)oxy)-3-(3-(4,4,5,5-tetramethyl-1,3,2-dioxaborolan-2-yl)propyl)pyrrolidine-1,2-dicarboxylate (16 g, 26 mmol) in EtOH (410 mL) and water (21 mL) under N₂, and the resulting mixture was stirred at 90 °C for 8 h. The reaction mixture was concentrated, and the crude mixture was diluted with DCM, filtered, and concentrated in vacuo. The residue was purified by SFC [Column: DAICEL CHIRALPAK AD (250 mm*50 mm, 10 um), Mobile phase: A: CO₂, B: Heptane-EtOH (0.1% NH₃·H₂O), Gradient: 10% of B in 3.5 minutes, and hold 10% of B for 1 min, Flow Rate (mL/min) 180, Column temperature: 40 °C] to give (*1S,2S,5R*)-3-*tert*-butyl 2-methyl 1-(3-(4,4,5,5-tetramethyl-1,3,2-dioxaborolan-2-yl)propyl)-6-thia-3-azabicyclo[3.2.0]heptane-2,3-dicarboxylate. LCMS (C₁₆H₂₉BNO₄S⁺) (ES, m/z): 342 [M+H-Boc]⁺.

### Step 5: (1S,2S,5R)-1-(3-boronopropyl)-6-thia-3-azabicyclo[3.2.0]heptane-2-carboxylic acid hydrochloride

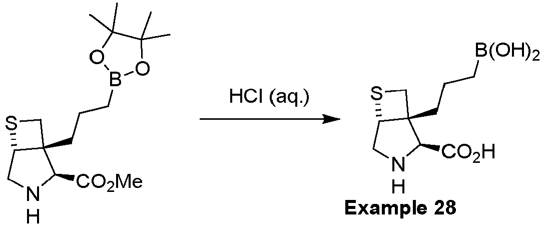

A mixture of (*1S,2S,5R*)-3-*tert*-butyl 2-methyl 1-(3-(4,4,5,5-tetramethyl-1,3,2-dioxaborolan-2-yl)propyl)-6-thia-3-azabicyclo[3.2.0]heptane-2,3-dicarboxylate (14 g, 32 mmol) and 12 N HCl in water (20 mL, 240 mmol) was stirred at 80 °C for 12 h under N₂. The reaction mixture was concentrated in vacuo, and the residue was diluted in water, washed with EtOAc and DCM. The aqueous phase was concentrated in vacuo to give (*1S,2S,5R*)-1-(3-boronopropyl)-6-thia-3-azabicyclo[3.2.0]heptane-2-carboxylic acid as an HCl salt. LCMS (C₉H₁₅BNO₃S⁺) (ES, m/z): 228 [M+H-H₂O]⁺. ¹H NMR (500 MHz, D₂O) *δ* 4.74 (s, 1H), 4.01 (d, *J =* 6.0 Hz, 1H), 3.76-3.72 (m, 1H), 3.55-3.49 (m, 1H), 3.35-3.33 (m, 1H), 2.91-2.89 (m, 1H), 1.59 - 1.41 (m, 4H), 0.76 - 0.67 (m, 2H).

### Example 29: 1-(2-boronoethyl)-5-azaspiro[2.4]heptane-4-carboxylic acid

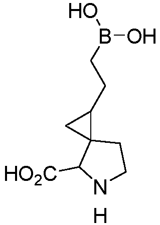

### Step 1: 1-tert-butyl 2-ethyl 3-(2-methoxy-2-oxoethylidene)pyrrolidine-1,2-dicarboxylate

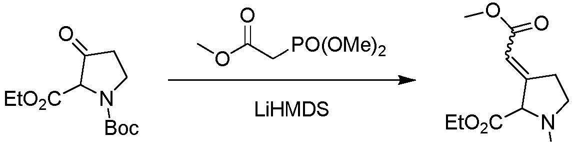

LiHMDS (12 mL, 12 mmol, 1.0 M in THF) was added to a mixture of methyl 2-(dimethoxyphosphoryl)acetate (2.3 g, 12 mmol) in THF (13 mL) at 20 °C under N₂, and the resulting mixture was stirred for 0.5 h at 20 °C, followed by addition of a solution of *1-tert-butyl* 2-ethyl 3-oxopyrrolidine-1,2-dicarboxylate (2.8 g, 11 mmol) in THF (12 mL) at 0 °C. The reaction mixture was stirred for 15 minutes at 0 °C, then for 1 h at 20 °C under N₂, before quenched with saturated aqueous NH₄Cl and extracted with EtOAc. The combined organic phases were washed with brine, dried over anhydrous Na₂SO₄, filtered, and concentrated under reduced pressure. The residue was purified by flash silica gel chromatography (EtOAc in hexanes) to give 1-*tert*-butyl 2-ethyl 3-(2-methoxy-2-oxoethylidene)pyrrolidine-1,2-dicarboxylate. LCMS (C₁₀H₁₆NO₄⁺) (ES, m/z): 214 [M+H-Boc]⁺.

### Step 2 1-tert-butyl 2-ethyl 3-(2-hydroxyethylidene)pyrrolidine-1,2-dicarboxylate

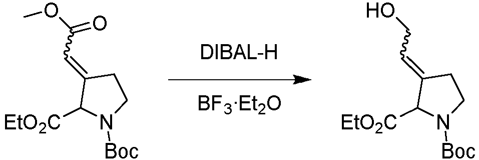

BF₃·OEt₂ (0.28 mL, 2.2 mmol) was added to a solution of 1-*tert*-butyl 2-ethyl 3-(2-methoxy-2-oxoethylidene)pyrrolidine-1,2-dicarboxylate (350 mg, 1.1 mmol) in DCM (32 mL) at -78 °C under N₂, and the resulting mixture was stirred at -78 °C for 15 minutes, followed by dropwise addition of DIBAL-H (6.7 mL, 6.7 mmol, 1 M in toluene) at -78 °C. The reaction mixture was stirred at -78 °C for 1 h under N₂, then quenched with saturated aqueous potassium sodium tartrate and extracted with DCM. The combined organic phases were washed with brine, dried over anhydrous Na₂SO₄, filtered, and concentrated under reduced pressure. The residue was purified by flash silica gel chromatography (EtOAc in hexanes) to give 1-*tert*-butyl 2-ethyl 3-(2-hydroxyethylidene)pyrrolidine-1,2-dicarboxylate. LCMS (C₁₀H₁₆NO₅⁺) (ES, m/z): 230 [M+H-C₄H₈]⁺.

### Step 3: 5-tert-butyl 4-ethyl 1-(hydroxymethyl)-5-azaspiro[2.4]heptane-4,5-dicarboxylate

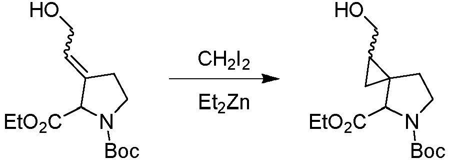

Diethylzinc (0.88 mL, 0.88 mmol, 1 M in toluene) was added dropwise over 5 minutes to a solution of 1-*tert*-butyl 2-ethyl 3-(2-hydroxyethylidene)pyrrolidine-1,2-dicarboxylate (100 mg, 0.35 mmol) in DCM (3.0 mL) at -78 °C under N₂. The resulting mixture was stirred for 5 minutes at -78 °C, followed by dropwise addition of a solution of diiodomethane (0.057 mL, 0.70 mmol) in DCM (2.0 mL) over 5 minutes at -78 °C under N₂. The reaction mixture was stirred at 25 °C for 15 h, then quenched with saturated aqueous NH₄Cl and extracted with DCM. The combined organic phases were washed with brine, dried over Na₂SO₄, filtered, and concentrated in vacuo. The residue was purified by RP-HPLC [C18 column, water (0.05%HCl)-CH₃CN] to give 5-*tert*-butyl 4-ethyl 1-(hydroxymethyl)-5-azaspiro[2.4]heptane-4,5-dicarboxylate. LCMS (C₁₀H₁₈NO₃⁺) (ES, m/z): 200 [M+H-Boc]⁺.

### Step 4: 5-tert-butyl 4-ethyl 1-formyl-5-azaspiro[2.4]heptane-4,5-dicarboxylate

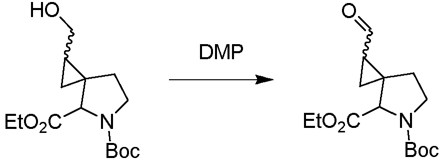

Dess-Martin Periodinane (179 mg, 0.42 mmol) was added to a solution of *5-tert-butyl* 4-ethyl 1-(hydroxymethyl)-5-azaspiro[2.4]heptane-4,5-dicarboxylate (70 mg, 0.23 mmol) in DCM (3.0 mL) at 25 °C, and the resulting mixture was stirred for 16 h at 25 °C, then quenched with saturated aqueous NaHCO₃ and extracted with DCM. The combined organic phases were washed with brine, dried over anhydrous Na₂SO₄, filtered, and concentrated under reduced pressure. The residue was purified by flash silica gel chromatography (EtOAc in hexanes) to give 5-tert-butyl 4-ethyl 1-formyl-5-azaspiro[2.4]heptane-4,5-dicarboxylate. LCMS (C₁₀H₁₅NO₃⁺) (ES, m/z): 198 [M+H-Boc]⁺.

### Step 5: 5-tert-butyl 4-ethyl 1-vinyl-5-azaspiro[2.4]heptane-4,5-dicarboxylate (PELN: 1007783-037)

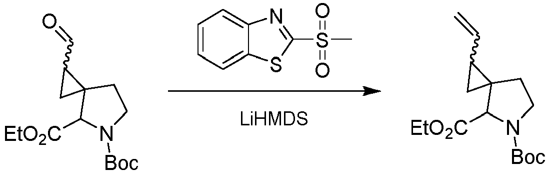

LiHMDS (0.65 mL, 0.65 mmol, 1.0 M in THF) was added to a mixture of 5-*tert*-butyl 4-ethyl 1-formyl-5-azaspiro[2.4]heptane-4,5-dicarboxylate (80 mg, 0.27 mmol) and 2-(methylsulfonyl)benzo[*d*]thiazole (75 mg, 0.35 mmol) in THF (3.0 mL) at -78 °C under N₂, and the resulting mixture was stirred at -78 °C for 0.5 h, then for 3 h at 25 °C under N₂. The reaction mixture was quenched with saturated aqueous NH₄Cl and extracted with EtOAc. The combined organic phases were washed with brine, dried over anhydrous Na₂SO₄, filtered, and concentrated under reduced pressure. The residue was purified by flash silica gel chromatography (EtOAc in hexanes) to give 5-*tert*-butyl 4-ethyl 1-vinyl-5-azaspiro[2.4]heptane-4,5-dicarboxylate. LCMS (C₁₆H₂₆NO₄⁺) (ES, m/z): 296 [M+H]⁺.

### Step 6: 5-tert-butyl 4-ethyl 1-(2-(4,4,5,5-tetramethyl-1,3,2-dioxaborolan-2-yl)ethyl)-5-azaspiro[2.4]heptane-4,5-dicarboxylate

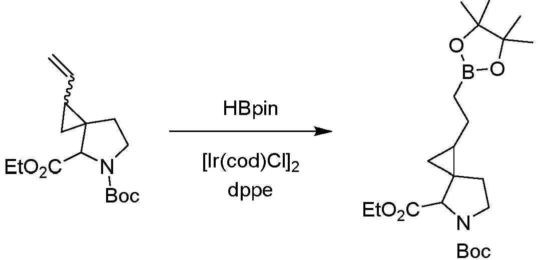

4,4,5,5-Tetramethyl-1,3,2-dioxaborolane (43 mg, 0.34 mmol) was added to a mixture of [Ir(cod)Cl]₂ (2.3 mg, 3.4 µmol) and dppe (1.9 mg, 4.7 µmol) in DCM (1.0 mL) at 25 °C under N₂, and the resulting mixture was stirred for 10 minutes, followed by addition of a solution of 5*-tert-*butyl 4-ethyl 1-vinyl-5-azaspiro[2.4]heptane-4,5-dicarboxylate (20 mg, 0.068 mmol) in DCM (0.50 mL) under N₂. The mixture was stirred for 15 h at 25 °C under N₂. The reaction mixture was diluted with water and extracted with EtOAc. The combined organic phases were washed with brine, dried over anhydrous Na₂SO₄, filtered, and concentrated under reduced pressure. The residue was purified by RP-HPLC [C18 column, water (0.1%TFA)-CH₃CN] to give a mixture of 5-*tert*-butyl 4-ethyl 1-(2-(4,4,5,5-tetramethyl-1,3,2-dioxaborolan-2-yl)ethyl)-5-azaspiro[2.4]heptane-4,5-dicarboxylate and its corresponding boronic acid. LCMS (C₁₇H₃₁BNO₄⁺) (ES, m/z): 324 [M+H-Boc]⁺.

### Step 7 1-(2-boronoethyl)-5-azaspiro[2.4]heptane-4-carboxylic acid

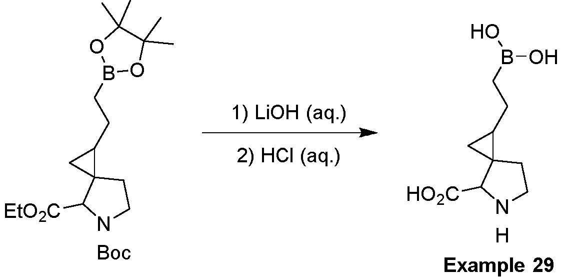

A mixture of 5*-tert-*butyl 4-ethyl 1-(2-(4,4,5,5-tetramethyl-1,3,2-dioxaborolan-2-yl)ethyl)-5-azaspiro[2.4]heptane-4,5-dicarboxylate (12 mg, 0.028 mmol) and 3 M LiOH in water (0.094 mL, 0.28 mmol) in THF (1.0 mL) was stirred for 20 h at 25 °C, followed by addition of more 3 M LiOH in water (0.047 mL, 0.14 mmol), and the resulting mixture was stirred at 50 °C for 53 h. Then a third portion of 3 M LiOH in water (0.094 mL, 0.28 mmol) was added, and the reaction mixture was stirred at 50 °C for 20 h, followed by addition of 3N HCl in water (0.50 mL, 1.5 mmol). The reaction mixture was stirred at 25 °C for 17 h. The reaction mixture was concentrated, and the residue was purified by RP-HPLC [C18 column, water (20 mM HFBA and 0.1% TFA)-CH₃CN] 1-(2-boronoethyl)-5-azaspiro[2.4]heptane-4-carboxylic acid as an HFBA salt. LCMS (C₉H₁₇BNO₄⁺) (ES, m/z): 214 [M+H]⁺. ¹H NMR (400 MHz, D₂O) *δ* 3.69 (s, 1H), 3.59 - 3.48 (m, 1H), 3.45 - 3.31 (m, 1H), 2.17 - 2.03 (m, 1H), 1.81 - 1.70 (m, 1H), 1.36-1.33 (m, 2H), 1.24 - 1.15 (m, 1H), 0.87-0.85 (m, 1H), 0.83-0.80 (m, 2H), 0.43-0.40 (m, 1H).

### Example 30: (3aS,6aR)-5-(boronomethyl)octahydrocyclopenta[c]pyrrole-1-carboxylic acid

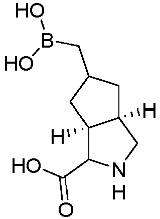

### Step 1: (3aR, 6aS)-tert-butyl tetrahydro-1H-spiro[cyclopenta[c]pyrrole-5,2'-[1,3]dioxolane]-2(3H)-carboxylate

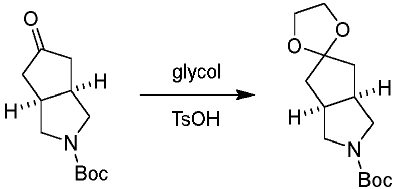

A mixture of (*3aR,6aS*)-*tert*-butyl 5-oxohexahydrocyclopenta[c]pyrrole-2(1H)-carboxylate (2.0 g, 8.9 mmol), ethane-1,2-diol (2.2 g, 36 mmol) and TsOH·H₂O (400 mg, 2.1 mmol) in toluene (40 mL) was stirred at 120 °C under N₂ for 96 h. The reaction mixture was quenched with saturated aqueous NaHCO₃ to pH ~ 7 and extracted with DCM. The combined organic phase was washed with brine, dried over anhydrous magnesium sulfate, filtered, and concentrated under reduced pressure. The residue was purified by flash silica gel chromatography (EtOAc in hexanes) to give (*3aR,6aS*)-*tert*-butyl tetrahydro-1*H*-spiro[cyclopenta[c]pyrrole-5,2'-[1,3]dioxolane]-2(3H)-carboxylate. LCMS (C₁₄H₂₄NO₄⁺) (ES, m/z): 270 [M+H]⁺.

### Step 2: (3aR, 6aS)-2-tert-butyl 1-methyl tetrahydro-1H-spiro[cyclopenta[c]pyrrole-5,2'-[1,3]dioxolane]-1,2(3H)-dicarboxylate

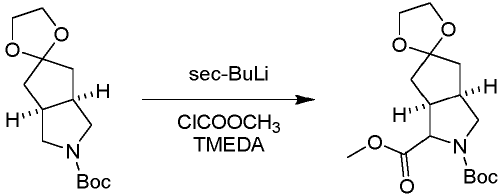

Sec-butyllithium (1.3 mL, 1.7 mmol, 1.3 M in n-hexane) was added to a mixture of N1,N1,N2,N2-tetramethylethane-1,2-diamine (0.13 g, 1.1 mmol) and (*3aR,6aS*)*-tert-*butyl tetrahydro-1H-spiro[cyclopenta[c]pyrrole-5,2'-[1,3]dioxolane]-2(3*H*)-carboxylate (0.30 g, 1.1 mmol) in THF (7.0 mL) at -78 °C under N₂ The resulting mixture was stirred at -78 °C for 1.5 h under N₂, followed by addtion of methyl carbonochloridate (0.10 mL, 1.3 mmol). The reaction mixture was stirred at -78 °C for 1 h, then at 15 °C for 13 h under N₂, before being quenched with saturated aqueous NH₄Cl and extracted with DCM. The combined organic layers were washed with brine, dried over anhydrous Na₂SO₄, filtered, and concentrated. The residue was purified by flash silica gel chromatography (EtOAc in hexanes) to give (*3aR,6aS*)-2*-tert-*butyl 1-methyl tetrahydro-1H-spiro[cyclopenta[c]pyrrole-5,2'-[1,3]dioxolane]-1,2(3*H*)-dicarboxylate. LCMS (C₁₆H₂₆NO₆⁺) (ES, m/z): 328 [M+H]⁺.

### Step 3: (3aS,6aR)-2-tert-butyl 1-methyl 5-oxohexahydrocyclopenta[c]pyrrole-1,2(1H)-dicarboxylate

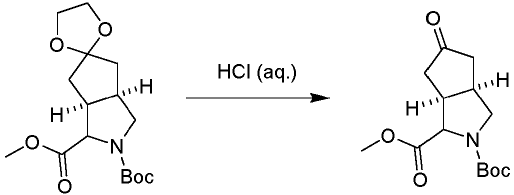

0.2 N HCl in water (15 mL, 3.0 mmol) was added to a mixture of (*3aS,6aR*)-2*-tert-*butyl 1-methyl tetrahydro-1*H*-spiro[cyclopenta[c]pyrrole-5,2'-[1,3]dioxolane]-1,2(3H)-dicarboxylate (0.98 g, 3.0 mmol) in THF (7.0 mL), and the resulting mixture was stirred at 0 °C for 4 h. The reaction mixture was quenched with anhydrous NaHCO₃ to pH 7~8 at 0 °C, and extracted with EtOAc. The combined organic layers were washed with brine, dried over anhydrous Na₂SO₄, filtered, and concentrated to give crude (*3aS,6aR*)*-*2*-tert-*butyl 1-methyl 5-oxohexahydrocyclopenta[c]pyrrole-1,2(1*H*)-dicarboxylate, which was used in the next step directly without further purification. LCMS (C₉H₁₃NO₃⁺) (ES, m/z): 184 [M+H-Boc]⁺.

### Step 4: (3aS, 6aR)-2-tert-butyl 1-methyl 5-methylenehexahydrocyclopenta[c]pyrrole-1,2(1H)-dicarboxylate

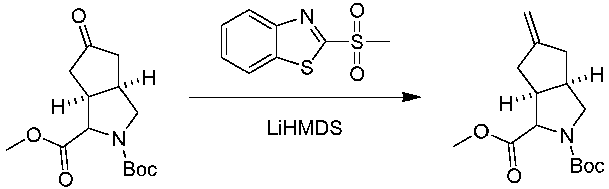

LiHMDS (5.1 mL, 5.1 mmol, 1.0 M in THF) was added to a mixture of (*3aS,6aR*)*-*2*-tert-*butyl 1-methyl 5-oxohexahydrocyclopenta[*c*]pyrrole-1,2(1H)-dicarboxylate (600 mg, 2.1 mmol) and 2-(methylsulfonyl)benzo[*d*]thiazole (587 mg, 2.8 mmol) in THF (10 mL) at -78 °C under N₂. The resulting mixture was stirred at -78 °C for 0.5 h, then at 20 °C for 12 h under N₂. The reaction mixture was quenched with saturated aqueous NH₄Cl and extracted with DCM. The combined organic layers were washed with brine, dried over anhydrous Na₂SO₄, filtered, and concentrated. The residue was purified by flash silica gel chromatography (EtOAc in hexanes) to give (*3aS,6aR*)-2-*tert*-butyl 1-methyl 5-methylenehexahydrocyclopenta[c]pyrrole-1,2(1*H*)-dicarboxylate. LCMS (C₁₅H₂₄NO₄⁺) (ES, m/z): 282 [M+H]⁺.

### Step 5: (3aS, 6aR)-2-tert-butyl 1-methyl 5-((4,4,5,5-tetramethyl-1,3,2-dioxaborolan-2-yl)methyl)hexahydrocyclopenta[c]pyrrole-1,2(1H)-dicarboxylate

A mixture of bis(diphenylphosphino)methane (14 mg, 0.037 mmol), [Ir(cod)Cl]₂ (18 mg, 0.027 mmol) and 4,4,5,5-tetramethyl-1,3,2-dioxaborolane (0.39 mL, 2.7 mmol) in DCM (8.0 mL) was stirred at 25 °C for 15 minutes under N₂, followed by addition of *(3aS, 6aR)-2-tert-butyl 1-*methyl 5-methylenehexahydrocyclopenta[c]pyrrole-1,2(1*H*)-dicarboxylate (150 mg, 0.53 mmol) in DCM (0.50 mL). The resulting mixture was stirred at 25 °C for 15 h under N₂, then concentrated. The residue was purified by flash silica gel chromatography (EtOAc in hexanes) to give (*3aS,6aR*)-2-*tert*-butyl 1-methyl 5-((4,4,5,5-tetramethyl-1,3,2-dioxaborolan-2-yl)methyl)hexahydrocyclopenta[*c]*pyrrole-1,2(1*H*)-dicarboxylate. LCMS (C₂₁H₃₇BNO₆⁺) (ES, m/z): 410 [M+H]⁺.

### Step 6: (3aS,6aR)-5-(boronomethyl)octahydrocyclopenta[c]pyrrole-1-carboxylic acid

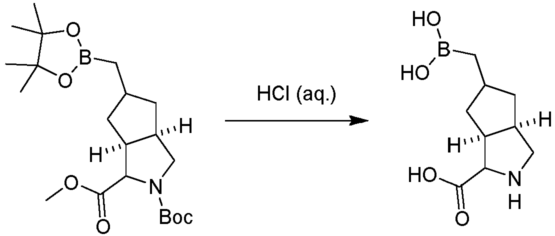

A mixture of (*3aS,6aR*)*-*2*-tert-*butyl 1-methyl 5-((4,4,5,5-tetramethyl-1,3,2-dioxaborolan-2-yl)methyl)hexahydrocyclopenta[c]pyrrole-1,2(1*H*)-dicarboxylate (70 mg, 0.17 mmol) and 12 N HCl in water (4.0 mL, 48 mmol) was stirred at 105 °C for 13 h. The reaction mixture was washed with DCM, and the aqueous layer was concentrated to give (*3aS, 6aR*)-5-(boronomethyl)octahydrocyclopenta[c]pyrrole-1-carboxylic acid as an HCl salt. LCMS (C₉H₁₇BNO₄⁺) (ES, m/z): 214 [M+H]⁺. ¹H NMR (400 MHz, D₂O) *δ* 4.42-3.84 (m, 1H), 3.65-2.98 (m, 3H), 2.97-2.77 (m, 1H), 2.30-1.81 (m, 2H), 1.69-1.23 (m, 2H), 1.16-0.88 (m, 1H), 0.87-0.69 (m, 2H).

### Example 31: 3-[3-(dihydroxyboranyl)propyl]octahydro-1H-pyrrolo[3,2-b]pyridine-2-carboxylic acid

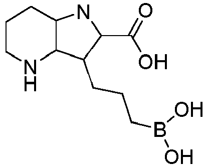

### Step 1: 1-tert-butyl 2-ethyl 3-bromo-1H-pyrrolo[3,2-b]pyridine-1,2-dicarboxylate

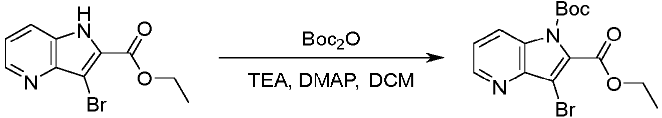

Boc₂O (2.1 g, 9.7 mmol), DMAP (454 mg, 3.7 mmol) and TEA (1.5 g, 15 mmol) were added to a mixture of ethyl 3-bromo-1*H*-pyrrolo[3,2-b]pyridine-2-carboxylate (2.0 g, 7.4 mmol) in DCM (15 mL), and the reaction mixture was stirred at 25 °C for 1 h. The mixture was concentrated and the residue was purified by flash silica gel chromatography (EtOAc in hexanes) to give *1-tert-*butyl 2-ethyl 3-bromo-1*H*-pyrrolo[3,2-*b*]pyridine-1,2-dicarboxylate. LCMS (C₁₀H₁₀BrN₂O₂⁺) (ES, m/z): 269 [M-CO₂C₄H₈+H]⁺.

### Step 2: 1-tert-butyl 2-ethyl 3-allyl-1H-pyrrolo[3,2-b]pyridine-1,2-dicarboxylate

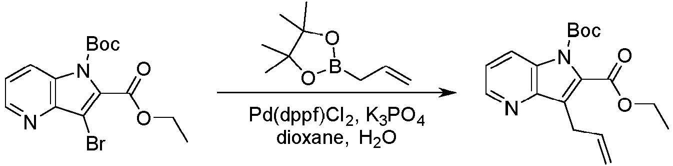

A mixture of 2-allyl-4,4,5,5-tetramethyl-1,3,2-dioxaborolane (2.3 g, 14 mmol), 1*-tert-*butyl 2-ethyl 3-bromo-1*H*-pyrrolo[3,2-*b*]pyridine-1,2-dicarboxylate (2.5 g, 6.8 mmol), Pd(dppf)Cl₂ (495 mg, 0.68 mmol) and K₃PO₄ (4.3 g, 20 mmol) in 1,4-dioxane (60 mL) and water (15 mL) was degassed and backfilled with N₂ (three times), and the reaction mixture was heated to 90 °C for 15 h. The mixture was filtered at room temperature and the filtrate was concentrated under reduced pressure. To the residue was added Boc₂O (3.0 g, 14 mmol), DMAP (413 mg, 3.4 mmol), TEA (1.4 g, 14 mmol) and DCM (50 mL), and the resulting mixture was stirred at 25 °C for 1 h. The solvent was removed and the residue was purified by flash silica gel chromatography (EtOAc in hexanes) to give 1-*tert*-butyl 2-ethyl 3-allyl-1*H*-pyrrolo[3,2-*b*]pyridine-1,2-dicarboxylate. LCMS (C₁₃H₁₅N₂O₂⁺) (ES, m/z): 231 [M-CO₂C₄H₈+H]⁺.

### Step 3: 1-tert-butyl 2-ethyl 3-(3-(4,4,5,5-tetramethyl-1,3,2-dioxaborolan-2-yl)propyl)-1H-pyrrolo[3,2-b]pyridine-1,2-dicarboxylate

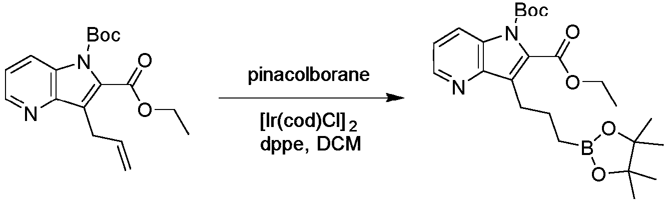

4,4,5,5-Tetramethyl-1,3,2-dioxaborolane (4.4 g, 34 mmol) was added to a mixture of DPPE (271 mg, 0.68 mmol) and [Ir(cod)Cl]₂ (229 mg, 0.34 mmol) in DCM (100 mL), and the resulting mixture was stirred at 25 °C for 30 minutes, followed by addition of 1-*tert*-butyl 2-ethyl 3-allyl-1*H-*pyrrolo[3,2-*b*]pyridine-1,2-dicarboxylate (2.3 g, 6.8 mmol) in DCM (20 mL). The reaction mixture was stirred at 25 °C for 15 h, and concentrated. The residue was purified by flash silica gel chromatography (EtOAc in hexanes) to give 1-*tert*-butyl 2-ethyl 3-(3-(4,4,5,5-tetramethyl-1,3,2-dioxaborolan-2-yl)propyl)-1*H*-pyrrolo[3,2-*b*]pyridine-1,2-dicarboxylate. LCMS (C₁₉H₂₈BN₂O₄⁺) (ES, m/z): 359 [M-CO₂C₄H₈+H]⁺.

### Step 4: 1-tert-butyl 2-ethyl 3-(3-(4,4,5,5-tetramethyl-1,3,2-dioxaborolan-2-yl)propyl)octahydro-1H-pyrrolo[3, 2-b]pyridine-1, 2-dicarboxylate

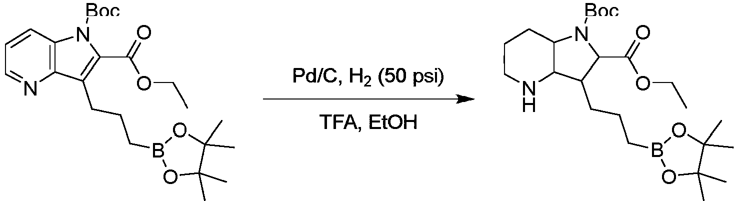

TFA (32 mg, 0.33 mmol) and 10% Pd/C (10 mg) were added to a mixture of 1-*tert*-butyl 2-ethyl 3-(3-(4,4,5,5-tetramethyl-1,3,2-dioxaborolan-2-yl)propyl)-1*H*-pyrrolo[3,2-*b*]pyridine-1,2-dicarboxylate (100 mg, 0.22 mmol) in EtOH (10 mL) under Ar, and the resulting mixture was degassed and backfilled with H₂ (three times). The reaction mixture was stirred under H₂ (345 kPa (50 psi)) at 50 °C for 20 h, then the mixture was filtered, and the filtrate was concentrated under reduced pressure. The residue was purified by RP-HPLC [C18 column, water (0.1%TFA)-CH₃CN] to give 1-*tert*-butyl 2-ethyl 3-(3-(4,4,5,5-tetramethyl-1,3,2-dioxaborolan-2-yl)propyl)octahydro-1*H-*pyrrolo[3,2-*b*]pyridine-1,2-dicarboxylate. LCMS (C₁₈H₃₄BN₂O₆⁺) (ES, m/z): 385 [M-C₆H₁₀+H]⁺. *Step 5: 3-[3-(dihydroxyboranyl)propyl]octahydro-1H-pyrrolo[3,2-b]pyridine-2-carboxylic acid*

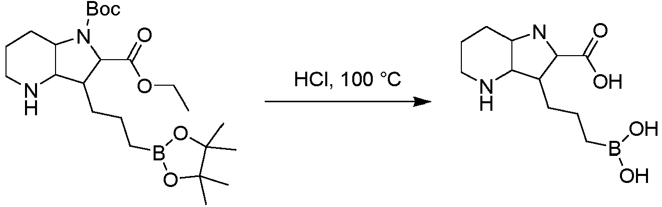

A mixture of *1-tert-butyl* 2-ethyl 3-(3-(4,4,5,5-tetramethyl-1,3,2-dioxaborolan-2-yl)propyl)octahydro-1*H*-pyrrolo[3,2-*b*]pyridine-1,2-dicarboxylate (35 mg, 0.075 mmol) and 12 M HCl in water (2.0 mL, 24 mmol) was stirred at 100 °C for 16 h, and the mixture was concentrated under reduced pressure to give 3-[3-(dihydroxyboranyl)propyl]octahydro-1H-pyrrolo[3,2-b]pyridine-2-carboxylic acid as a HCl salt. ¹H NMR (400 MHz, CD₃OD) *δ* 4.88 - 4.70 (m, 2H), 4.36 - 3.95 (m, 2H), 3.58 (br s, 1H), 3.26 - 2.89 (m, 2H), 2.34 - 1.91 (m, 3H), 1.59 (br s, 4H), 0.85 (br s, 2H).

### Example 32: (3aR, 4S,6aR)-3a-(3-boronopropyl)hexahydro-2H-furo[2,3-c]pyrrole-4-carboxylic acid

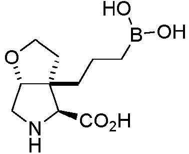

### Step 1: 1-(tert-butyl) 2-methyl (2S,3R)-3-allyl-3-(2-((tert-butyldimethylsilyl)oxy)ethyl)-4-oxopyrrolidine-1,2-dicarboxylate

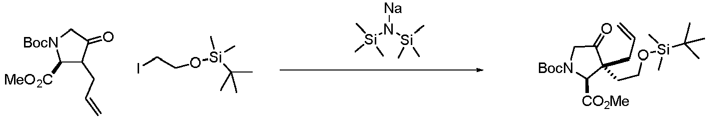

Sodium bis(trimethylsilyl)amide (1.0 M in THF, 2.3 mL, 2.3 mmol) was added dropwise to a stirred solution of (2S)-1-*tert*-butyl 2-methyl 3-allyl-4-oxopyrrolidine-1,2-dicarboxylate (582 mg, 2.1 mmol) in THF (7.7 mL) and 1,3-dimethyl-3,4,5,6-tetrahydro-2(1H)-pyrimidinone(DMPU) (2.6 mL) at -78 °C. The resulting mixture was stirred at -78 °C for 1 h. Then t*ert*-butyl(2-iodoethoxy)dimethylsilane (0.9 mL, 4.1 mmol) in THF (0.5 mL) was added dropwise at -78 °C. The reaction mixture was stirred at -78 °C for 2 h and warmed to room temperature overnight. The reaction mixture was quenched with saturated aqueous NH₄Cl and extracted with EtOAc. The combined organic phases were concentrated and purified by silica gel chromatography (EtOAc in hexanes) to give to afford 1-(*tert*-butyl) 2-methyl (2S,3R)-3-allyl-3-(2-((*tert*butyldimethylsilyl)oxy)ethyl)-4-oxopyrrolidine-1,2-dicarboxylate. LCMS (C₁₇H₃₂NO₄Sᵢ⁺) (ES, m/z): 342 [M+H-Boc]⁺.

### Step 2: 1-(tert-butyl) 2-methyl (2S,3R)-3-allyl-3-(2-((tert-butyldimethylsilyl)oxy)ethyl)-4-hydroxypyrrolidine-1,2-dicarboxylate

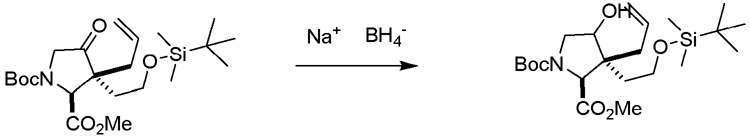

Sodium borohydride (39 mg, 1.0 mmol) was added to a stirred solution of 1*-*(*tert-*butyl) 2-methyl (2S,3R)-3-allyl-3-(2-((*tert*-butyldimethylsilyl)oxy)ethyl)-4-oxopyrrolidine-1,2-dicarboxylate (300 mg, 0.68 mmol) in MeOH (2.3 mL) at 0 °C. The reaction mixture was allowed to warm to room temperature for 2h. The reaction mixture was quenched with saturated aqeous NH₄Cl, and extracted with EtOAc. The combined organic phases were concentrated and purified by silica gel chromatography (EtOAc in hexanes) to give to afford 1-(*tert-*butyl) 2-methyl (2S,3R)-3-allyl-3-(2-((*tert*-butyldimethylsilyl)oxy)ethyl)-4-hydroxypyrrolidine-1,2-dicarboxylate. LCMS (C₂₂H₄₁NO₆SiNa⁺) (ES, m/z): 466 [M+Na]⁺.

### Step 3: 1-(tert-butyl) 2-methyl (2S,3R,4S)-3-allyl-3-(2-((tert-butyldimethylsilyl)oxy)ethyl)-4-((methylsulfonyl)oxy)pyrrolidine-1,2-dicarboxylate

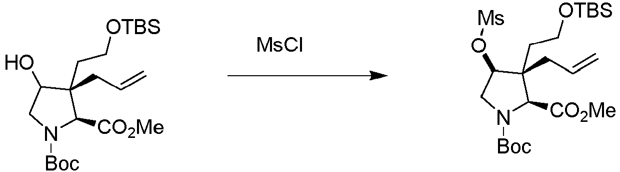

Et₃N (0.28 mL, 2.0 mmol) was added to a stirred solution of *1-(tert-butyl)* 2-methyl (2S,3R)-3-allyl-3-(2-((tert-butyldimethylsilyl)oxy)ethyl)-4-hydroxypyrrolidine-1,2-dicarboxylate (300 mg, 0.68 mmol) in THF (3.4mL) at 0 °C, followed by dropwise addition of methanesulfonyl chloride (0.11 mL, 1.4 mmol). The resulting mixture was then stirred at room temperature for 1h, quenched with saturated aqueous NaHCO₃, and extracted with EtOAc twice. The combined organic phases were washed with brine, dried over anhydrous Na₂SO₄, filtered, and concentrated under reduced pressure to give 1-(*tert*-butyl) 2-methyl (2S,3R,4S)-3-allyl-3-(2-((*tert*butyldimethylsilyl)oxy)ethyl)-4-((methylsulfonyl)oxy)pyrrolidine-1,2-dicarboxylate, which was used for next step without further purification. LCMS (C₂₃H₄₃NO₈SSiNa⁺) (ES, m/z): 544 [M+Na]⁺.

### Step 4: 1-(tert-butyl) 2-methyl (2S,3R,4S)-3-allyl-3-(2-hydroxyethyl)-4-((methylsulfonyl)oxy)pyrrolidine-1,2-dicarboxylate

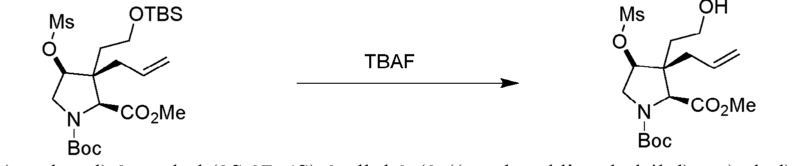

*1-(tert-butyl)* 2-methyl (2S,3R,4S)-3-allyl-3-(2-((*tert*-butyldimethylsilyl)oxy)ethyl)-4-((methylsulfonyl)oxy)pyrrolidine-1,2-dicarboxylate (340 mg, 0.67 mmol) was treated with TBAF (1.0 M in THF, 3.3 mL, 3.26 mmol) and stirred at room temperature for 2h. The reaction mixture was quenched with saturated aqueous NH4Cl, and extracted with EtOAC. The combined organic phases were washed with brine, dried over anhydrous Na₂SO₄, filtered, and concentrated under reduced pressure to give *1-(tert-butyl)* 2-methyl (2S,3R,4S)-3-allyl-3-(2-hydroxyethyl)-4-((methylsulfonyl)oxy)pyrrolidine-1,2-dicarboxylate, which was used for next step without further purification. LCMS (C₁₇H₂₉NO₈SNa⁺) (ES, m/z): 430 [M+Na]⁺.

### Step 5: 5-(tert-butyl) 4-methyl (3aR,4S,6aR)-3a-allylhexahydro-5H-furo[2,3-c]pyrrole-4,5-dicarboxylate

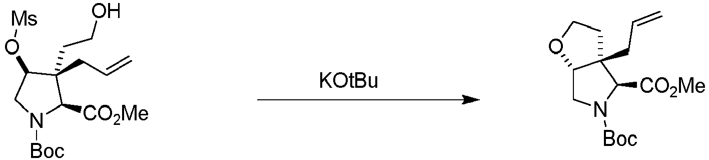

1-(*tert*-butyl) 2-methyl (2S,3R,4S)-3-allyl-3-(2-hydroxyethyl)-4-((methylsulfonyl)oxy)pyrrolidine-1,2-dicarboxylate (271 mg, 0.67 mmol) in DCM (6.5 mL) was treated with potassium tert-butoxide (1.0 M in THF, 1.0 mL, 1.0 mmol) and stirred at room temperature for 1 h. The reaction mixture was quenched with saturated aqueous NH₄Cl, and extracted with EtOAc. The combined organic phases were washed with brine, dried over anhydrous Na₂SO₄, filtered, and concentrated under reduced pressure. The residue was purified by flash silica gel chromatography (EtOAc in hexanes) to give 5-(tert-butyl) 4-methyl (3aR,4S,6aR)-3a-allylhexahydro-5H-furo[2,3-c]pyrrole-4,5-dicarboxylate. LCMS (C₁₆H₂₅NO₅Na⁺) (ES, m/z): 340 [M+Na]⁺.

### Step 6: 5-(tert-butyl) 4-methyl (3aR,4S,6aR)-3a-(3-(4,4,5,5-tetramethyl-1,3,2-dioxaborolan-2-yl)propyl)hexahydro-5H-furo[2,3-c]pyrrole-4,5-dicarboxylate

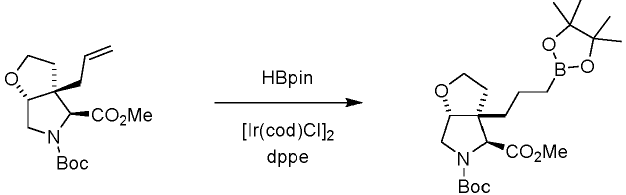

4,4,5,5-Tetramethyl-1,3,2-dioxaborolane (210 µl, 1.445 mmol) was added dropwise to a mixture of dppe (23.03 mg, 0.058 mmol) and [Ir(cod)Cl]₂ (19.42 mg, 0.029 mmol) in DCM (0.6mL) at 0 °C under N₂, followed by dropwise addition of a solution of (3aR,4S,6aR)-5-*tert*-butyl 4-methyl 3a-allyltetrahydro-2H-furo[2,3-c]pyrrole-4,5(3H)-dicarboxylate (90 mg, 0.289 mmol) in DCM (0.6 mL) under N₂. The reaction mixture was stirred at 25 °C for 12 h, and concentrated in vacuo. The residue was directly purified by silica gel chromatography (EtOAc in hexanes) to give 5-(tert-butyl) 4-methyl (3aR,4S,6aR)-3a-(3-(4,4,5,5-tetramethyl-1,3,2-dioxaborolan-2-yl)propyl)hexahydro-5H-furo[2,3-c]pyrrole-4,5-dicarboxylate. LCMS (C₂₂H₃₉BNO₇⁺) (ES, m/z): 340 [M+H-Boc]⁺.

### Step 7: (3aR, 4S,6aR)-3a-(3-boronopropyl)hexahydro-2H-furo[2,3-c]pyrrole-4-carboxylic acid

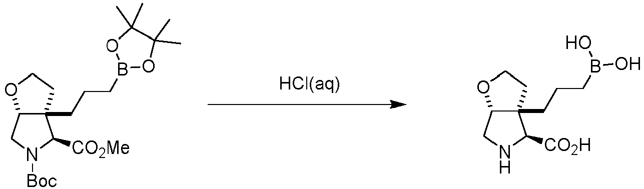

(3aR,4S,6aR)-5-*tert*-butyl 4-methyl 3a-(3-(4,4,5,5-tetramethyl-1,3,2-dioxaborolan-2-yl)propyl)tetrahydro-2H-furo[2,3-c]pyrrole-4,5(3H)-dicarboxylate (55 mg, 0.125 mmol) was treated with 1 mL of 6M HCl and stirred at 90 °C for 4h. The reaction mixture was diluted in water, washed with DCM. The aqueous phase was concentrated in vacuo to give (1S,2S,5R)-1-(3-boronopropyl)-3,6-diazabicyclo[3.2.0]heptane-2-carboxylic acid as an HCl salt. LCMS (C₁₀H₁₇BNO₃⁺) (ES, m/z): 226 [M+H-H₂O]⁺. ¹H NMR (500 MHz, D₂O) δ 4.39 (d, *J =* 3.5 Hz, 1H), 4.13 (s, 1H), 4.07 (q, *J =* 7.7 Hz, 1H), 3.96 (td, *J =* 9.0, 4.5 Hz, 1H), 3.63 (dd, *J =* 13.5, 4.8 Hz, 1H), 3.37 (dd, *J =* 13.5, 1.2 Hz, 1H), 2.23 (ddd, *J =* 12.0, 7.3, 4.6 Hz, 1H), 2.15 (dt, *J =* 13.4, 8.4 Hz, 1H), 1.60 - 1.50 (m, 1H), 1.46 - 1.37 (m, 2H), 1.33 (dtd, *J* = 16.3, 12.4, 10.4, 5.9 Hz, 1H), 0.71 (t,*J* = 7.3 Hz, 2H).

### Example 33: 5-(3-boronopropyl)-2-azabicyclo[3.2.0]heptane-1-carboxylic acid

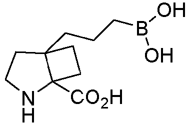

### Step 1: 2-benzyl 1-methyl 5-(3-(4,4,5,5-tetramethyl-1,3,2-dioxaborolan-2-yl)propyl)-2-azabicyclo[3.2.0]heptane-1,2-dicarboxylate

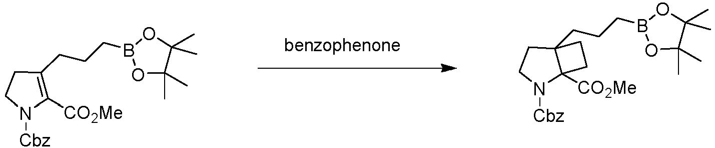

A solution of 1-benzyl 2-methyl 3-(3-(4,4,5,5-tetramethyl-1,3,2-dioxaborolan-2-yl)propyl)-4,5-dihydro-1H-pyrrole-1,2-dicarboxylate (290 mg, 0.68 mmol) and benzophenone (25 mg, 0.14 mmol) in toluene (14 mL) was bubbled with ethene gas for 10 min and irraidated with Na/Hg lump at rt for 3h. The reaction mixture was concentrated and purified by flash silica gel chromatography (MeOH in DCM) to give 2-benzyl 1-methyl 5-(3-(4,4,5,5-tetramethyl-1,3,2-dioxaborolan-2-yl)propyl)-2-azabicyclo[3.2.0]heptane-1,2-dicarbxylate. LCMS (C₂₅H₃₇BNO₆⁺) (ES, m/z): 458 [M+H]⁺.

### Step2: 5-(3-boronopropyl)-2-azabicyclo[3.2.0]heptane-1-carboxylic acid

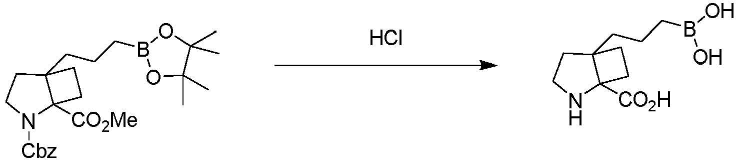

2-benzyl 1-methyl 5-(3-(4,4,5,5-tetramethyl-1,3,2-dioxaborolan-2-yl)propyl)-2-azabicyclo[3.2.0]heptane-1,2-dicarboxylate(44 mg, 0.096 mmol) was treated with 6 M HCl in water (1.0 mL, 6.0 mmol), and stirred at 90 ° C overnight, and the reaction mixture was diluted in water, washed with DCM. The aqueous phase was concentrated in vacuo to give 5-(3-boronopropyl)-2-azabicyclo[3.2.0]heptane-1-carboxylic acid as an HCl salt. LCMS (C₁₀H₁₇BNO₃⁺) (ES, m/z): 210 [M+H-H₂O]⁺. ¹H NMR (500 MHz, D₂O) δ 3.61 (dh, *J =* 24.4, 6.9, 6.2 Hz, 2H), 2.69 (ddd, *J =* 15.4, 11.5, 6.5 Hz, 1H), 2.08 - 1.60 (m, 5H), 1.47 - 1.09 (m, 4H), 0.71 (dt, *J =* 14.6, 7.3 Hz, 2H).

### Example 34: (1S,2S, 5R)-1-(3-boronopropyl)-3, 6-diazabicyclo[3.2.0]heptane-2-carboxylic acid

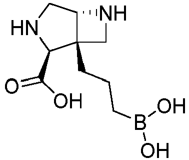

### Step 1: 1-(tert-butyl) 2-methyl (2S,3S,4S)-3-allyl-4-((chloromethyl)sulfonyl)-3-(((chloromethyl)sulfonyl)methyl)pyrrolidine-1,2-dicarboxylate

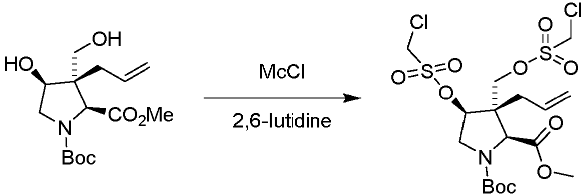

Chloromethanesulfonyl chloride (36 mL, 0.40 mol) was added dropwise into a mixture of (*2S,3S,4S*)-1-*tert*-butyl 2-methyl 3-allyl-4-hydroxy-3-(hydroxymethyl)pyrrolidine-1,2-dicarboxylate (42 g, 133 mmol), 2,6-dimethylpyridine (124 mL, 1.1 mol) in DCM (600 mL) at 0 °C. The resulting mixture was stirred at 0 °C for 0.5 h, then at 25 °C for 1 h. The reaction mixture was diluted with H₂O and extracted with DCM. The combined organic phases were washed with 1 N HCl in water, saturated aqueous NaHCO₃ and brine, dried over Na₂SO₄, filtered, and concentrated. The residue was purified by flash silica gel chromatography (EtOAc in hexanes) to give (*2S,3S,4S*)-1*-tert-*butyl 2-methyl 3-allyl-4-(((chloromethyl)sulfonyl)oxy)-3-((((chloromethyl)sulfonyl)oxy)methyl)pyrrolidine-1,2-dicarboxylate. LCMS (C₁₂H₂₀Cl₂NO₈S₂⁺) (ES, m/z): 440 [M+H-Boc]⁺.

### Step 2: 3-(tert-butyl) 2-methyl (1S,2S,5R)-1-allyl-6-benzyl-3,6-diazabicyclo[3.2.0]heptane-2,3-dicarboxylate

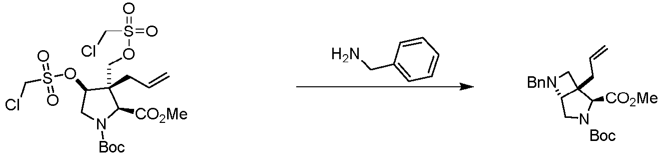

Benzylamine (1.8 mL, 16 mmol) was added to 1-(tert-butyl) 2-methyl (2S,3S,4S)-3-allyl-4-(((chloromethyl)sulfonyl)oxy)-3-((((chloromethyl)sulfonyl)oxy)methyl)pyrrolidine-1,2-dicarboxylate(435 mg, 0.81 mmol) in 1,4-dioxane (2.7 mL). The resulting mixture was heated up to 90°C and stirred for 12h. The reaction mixture was concentrated in vacuo, and the residue was purified by flash silica gel chromatography (EtOAc in hexanes) to afford 3-(tert-butyl) 2-methyl (1S,2S,5R)-6-benzyl-1-(3-(4,4,5,5-tetramethyl-1,3,2-dioxaborolan-2-yl)propyl)-3,6-diazabicyclo[3.2.0]heptane-2,3-dicarboxylate. LCMS (C₂₂H₃₁N₂O₄⁺) (ES, m/z): 387 [M+H]⁺.

### Step 3: 3-(tert-butyl) 2-methyl (1S,2S,5R)-6-benzyl-1-(3-(4,4,5,5-tetramethyl-1,3,2-dioxaborolan-2-yl)propyl)-3, 6-diazabicyclo[3.2.0]heptane-2,3-dicarboxylate

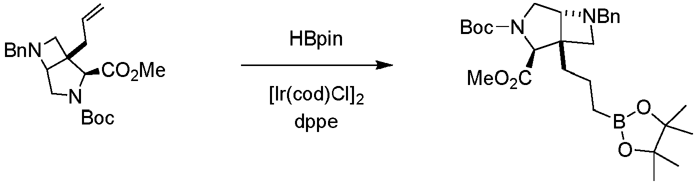

4,4,5,5-Tetramethyl-1,3,2-dioxaborolane (98 µL, 0.67 mmol) was added dropwise to a mixture of dppe (13 mg, 0.034 mmol) and [Ir(cod)Cl]₂ (11 mg, 0.017 mmol) in DCM (0.30 mL) at 0 °C under N₂, followed by dropwise addition of a solution of 3-(tert-butyl) 2-methyl (1S,2S)-1-allyl-6-benzyl-3,6-diazabicyclo[3.2.0]heptane-2,3-dicarboxylate (65 mg, 0.17 mmol) in DCM (0.40 mL) under N₂. The reaction mixture was stirred at 25 °C for 12 h, and concentrated in vacuo. The residue was directly purified by silica gel chromatography (EtOAc in hexanes) to give *3-(tert-butyl)* 2-methyl (1S,2S,5R)-6-benzyl-1-(3-(4,4,5,5-tetramethyl-1,3,2-dioxaborolan-2-yl)propyl)-3,6-diazabicyclo[3.2.0]heptane-2,3-dicarboxylate. LCMS (C₂₈H₄₄BN₂O₆⁺) (ES, m/z): 515 [M+H]⁺.

### Step 4: 3-(tert-butyl) 2-methyl (1S,2S,5R)-1-(3-(4,4, 5, 5-tetramethyl-1, 3,2-dioxaborolan-2-yl)propyl)-3, 6-diazabicyclo[3.2.0]heptane-2,3-dicarboxylate

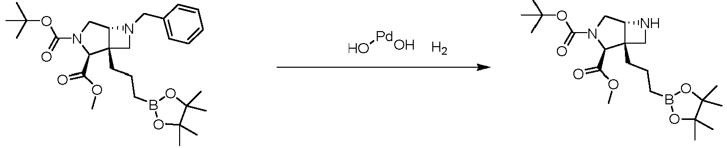

Palladium hydroxide on carbon (59 mg, 0.084 mmol) was added to a solution of *3-(tert-*butyl) 2-methyl (1S,2S,5R)-6-benzyl-1-(3-(4,4,5,5-tetramethyl-1,3,2-dioxaborolan-2-yl)propyl)-3,6-diazabicyclo[3.2.0]heptane-2,3-dicarboxylate (86 mg, 0.17 mmol) in THF (3.5 mL). The resulting mixture was sealed and degassed under H₂ balloon multiple times and stirred at room temperature for 3h. The reaction mixture was filtered and conconcentrated to afford 3-(tert-butyl) 2-methyl (1S,2S,5R)-1-(3-(4,4,5,5-tetramethyl-1,3,2-dioxaborolan-2-yl)propyl)-3,6-diazabicyclo[3.2.0]heptane-2,3-dicarboxylate. LCMS (C₂₁H₃₈BN₂O₆⁺) (ES, m/z): 425 [M+H]⁺.

### Step 5: (1S,2S,5R)-1-(3-boronopropyl)-3,6-diazabicyclo[3.2.0]heptane-2-carboxylic acid

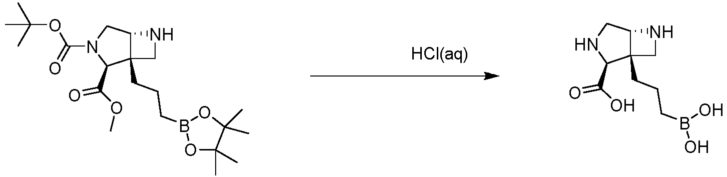

3-(*tert*-butyl) 2-methyl (1S,2S,5R)-1-(3-(4,4,5,5-tetramethyl-1,3,2-dioxaborolan-2-yl)propyl)-3,6-diazabicyclo[3.2.0]heptane-2,3-dicarboxylate (17 mg, 0.040 mmol) was treated with 6 M HCl in water (0.06 mL, 3.6 mmol) and stirred at 90°C for 3h, and the reaction mixture was diluted in water, and washed with DCM. The aqueous phase was concentrated in vacuo to give (1S,2S,5R)-1-(3-boronopropyl)-3,6-diazabicyclo[3.2.0]heptane-2-carboxylic acid as an HCl salt. LCMS (C₉H₁₆BN₂O₃⁺) (ES, m/z): 211 [M+H-H₂O]⁺. ¹H NMR (500 MHz, D₂O) δ 4.86 - 4.77 (m, 1H), 4.36 (d, *J* = 10.7 Hz, 1H), 4.12 (d, *J =* 11.9 Hz, 1H), 4.06 (dd, *J =* 14.5, 6.9 Hz, 1H), 4.00 (d, *J* = 12.2 Hz, 1H), 3.93 (t, *J=* 12.9 Hz, 1H), 1.84 - 1.75 (m, 1H), 1.74 - 1.61 (m, 1H), 1.58 - 1.50 (m, 1H), 1.49 - 1.20 (m, 1H), 0.89 - 0.73 (m, 2H).

### Assay

### Arginase Thioornithine Generating Assay (TOGA)

Compounds were serially diluted in ten 3-fold steps in DMSO starting from 10 mM DMSO stocks. Compound dilutions or DMSO alone were then dispensed from the dilution plate into a Greiner black 384-well assay plate (catalog #781086) using an Echo 555 acoustic liquid handler (Labcyte).

Arginase protein was recombinantly expressed in Escherichia coli. Purified protein was then diluted in assay buffer (50 mM Tris pH 7.5, 50 mM NaCl, 1mM manganese chloride, 0.05% bovine serum albumin to obtain a final Arginase concentration of 1.88 nM. Arginase solution (20 µL) or buffer alone (20 µL) were dispensed to wells of the assay plate using a BioRAPTR liquid dispenser (Beckman Coulter). Assay plates containing compound and arginase enzyme were incubated at room temperature for 30 minutes. Afterwards, 5µL of 2.5 mM thioarginine (Cayman Chemicals) in assay buffer were added to each well of the assay plate using a BioRAPTR liquid dispenser. Plates were incubated at room temperature for 60 minutes and reactions were quenched by addition of 15 µL of 200 uM 7-Diethylamine-3-(4-maleimidophenyl)-4-methylcoumarin (Sigma Chemical) in 70% ethanol. Plates were briefly shaken to mix and the fluorescence was measured in an Spectramax plate reader (Molecular Devices) with a 410 nm excitation wavelength and an 490 nm emission wavelength.

The fluorescence intensity of each well was corrected for the background observed in wells that did not receive arginase and was expressed as a fraction of the intensity observed in wells that received arginase enzyme and DMSO only. Potencies were calculated by linear least squares fit to the four parameter logistic IC50 equation.

| **Example** | **TOGA IC50 (nM)** |
|---|---|
| **1** | 1.611 |
| **2** | 1524 |
| **3** | 27.55 |
| **4** | 11.49 |
| **5** | 216.7 |
| **6** | 162.4 |
| **7** | 1.738 |
| **8** | 377.1 |
| **9** | 653.5 |
| **10** | 5824 |
| **11** | 3.937 |
| **12** | 2.288 |
| **13** | 2.128 |
| **14** | 8.783 |
| **15** | 14.22 |
| **16** | 19.64 |
| **17** | 66.49 |
| **18** | 1172 |
| **19** | 3.273 |
| **20** | 892.1 |
| **21** | 13610 |
| **22** | 181.3 |
| **23** | 114.4 |
| **24** | 5742 |
| **25** | 10000 |
| **26** | 444.2 |
| **27** | 5.083 |
| **28** | 25.88 |
| **29** | 1222 |
| **30** | 1860 |
| **31 (racemic)** | 5724 |
| **32** | 51.63 |
| **33 (racemic)** | 370 |
| **34** | 104.5 |

## Claims

1. A compound of Formula I: 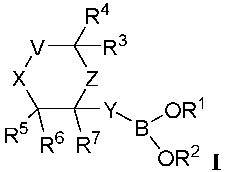 or a pharmaceutically acceptable salt thereof, wherein:
Y is a straight or branched (C₁-C₅)alkylene or cycloalkyl(C₁-C₅)alkylene, wherein one or more -CH₂- groups in Y are optionally and independently replaced with a moiety selected from the group consisting of O, S and NH;
Z is a bond, or CH₂;
V is O, NR⁸ or CR⁹R¹⁰, wherein X and V cannot be simultaneously O or S and O or NR⁸, respectively;
X is O, S, NR¹¹, CH₂, or CR¹²R¹³, wherein V and X cannot be simultaneously O and S, O or NR¹¹, respectively;
R¹ is hydrogen, C₃-C₆cycloalkyl or C₁-C₆alkyl or, taken with R² forms a C₃-C₈cycloalkyl, wherein the C₃-C₈cycloalkyl is unsubstituted or substituted with one to four substituents selected from the group consisting of halogen, C₁-C₆alkyl or OH;
R² is hydrogen, C₃-C₆cycloalkyl or C₁-C₆alkyl or, taken with R¹ forms a C₃-C₈cycloalkyl, wherein the C₃-C₈cycloalkyl is unsubstituted or substituted with one to four substituents selected from the group consisting of halogen, C₁-C₆alkyl or OH;
R³ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH, NH₂ or COOC₁-C₆alkyl;
R⁴ is taken with R⁷to form a heterocycle, wherein the heterocycle is pyrrolidine, azetidine, thiolane, thietane, oxetane, tetrahyrdrofurane or piperidine;
R⁵ is COOH;
R⁶ is hydrogen, halogen, C₁-C₆alkyl, haloC₁-C₆alkyl, OH, C₁-C₆alkylOH, C₁-C₆alkylOC₁-C₆alkyl, COOH, C₁-C₆alkoxy, COOH or COOC₁-C₆alkyl;
R⁸ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl, C₁-C₆alkylOH or COC₁-C₆alkyl;
R⁹ is hydrogen, halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl;
R¹⁰ is hydrogen, halogen, C₁-C₆alkyl, haloC₁-C₆alkyl, OH, C₁-C₆alkylOH, C₁-C₆alkylOC₁-C₆alkyl, COOH, C₁-C₆alkoxy, COOH or COOC₁-C₆alkyl or is taken with R¹² forms a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl , or taken with R⁶ forms an (C₁-C₅)alkylene bridge, wherein one or more -CH₂- groups are optionally and independently replaced with a moiety selected from the group consisting of O, S and NH; R¹¹ is hydrogen, C₃-C₆cycloalkyl, C₁-C₆alkyl, C₁-C₆alkylaryl, C₁-C₆alkylNH₂, COC₁-C₆alkylNH₂, COC₁-C₆alkylNH(C₁-C₆alkyl), COC₁-C₆alkylN(C₁-C₆alkyl)₂, C₁-C₆haloalkyl,
C₁-C₆alkylOH or COC₁-C₆alkyl;
R¹² is halogen, C₁-C₆alkyl, haloC₁-C₆alkyl, OH, C₁-C₆alkylOH, C₁-C₆alkylOC₁-C₆alkyl, COOH, C₁-C₆alkoxy, COOH or COOC₁-C₆alkyl or is taken with R⁶ or R¹⁰ to form a C₃-C₆cycloalkyl or heterocycle, wherein the C₃-C₆cycloalkyl or heterocycle is unsubstituted or substituted with 1 to 3 substituents selected from the group consisting of halogen, OH, C₁-C₆alkylOH, oxo, C₁-C₆alkyl, C₁-C₆haloalkyl, COC₁-C₆alkyl, C₁-C₆alkoxy, COOH, NH₂, C₁-C₆alkylNH₂, C₁-C₆alkylphenyl, CO(C₁-C₆alkyl)NH₂ and COOC₁-C₆alkyl; and
R¹³ is halogen, C₁-C₆alkyl, haloC₁-C₆alkyl, OH, C₁-C₆alkylOH, C₁-C₆alkylOC₁-C₆alkyl, C₁-C₆alkoxy, COOH or COOC₁-C₆alkyl.

2. The compound of Claim 1, or a pharmaceutically acceptable salt thereof, wherein R¹ and R² are both hydrogen.

3. The compound of Claim 1 or 2, or a pharmaceutically acceptable salt thereof, wherein Y is propylenyl.

4. The compound of Claim 1 or 2, or a pharmaceutically acceptable salt thereof, wherein Y is

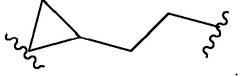

5. The compound of any one of Claims 1-3, or a pharmaceutically acceptable salt thereof, wherein R³ is hydrogen or COOH.

6. The compound of any one of Claims 1-5, or a pharmaceutically acceptable salt thereof, wherein Z is a bond.

7. The compound of any one of Claims 1-5, or a pharmaceutically acceptable salt thereof, wherein X is CR¹²R¹³ or NR¹¹.

8. A compound of claim 1 which is: 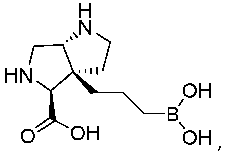 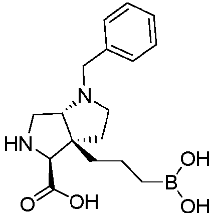 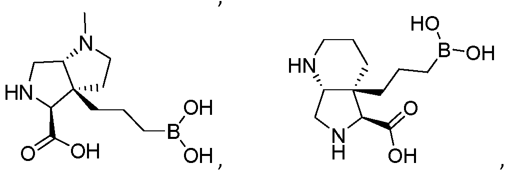 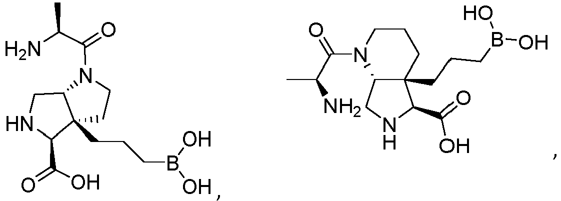 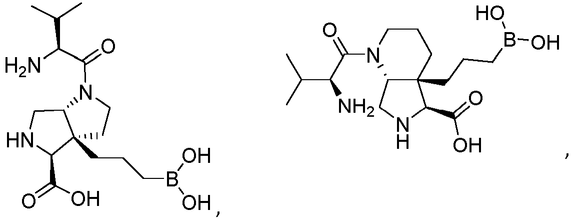 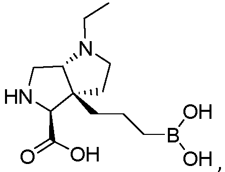 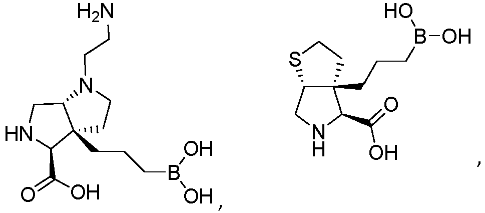 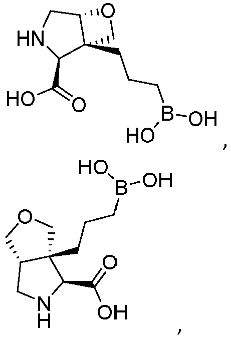 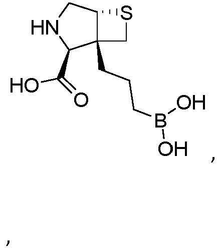 and 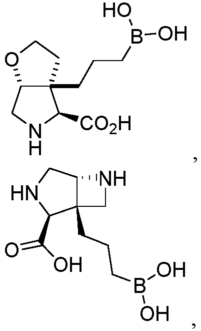 or a pharmaceutically acceptable salt thereof.

9. A compound of any one of Claims 1-8, or a pharmaceutically acceptable salt thereof for use in treating cancer.

10. A combination comprising a compound of any one of Claims 1-8, or a pharmaceutically acceptable salt thereof and other therapeutic agents for use in treating cancer.

11. A combination comprising a compound of any one of Claims 1-8, or a pharmaceutically acceptable salt thereof and a PD-1 antagonist for use in treating cancer.

12. A pharmaceutical composition comprising a compound of any one of Claims 1-8, or a pharmaceutically acceptable salt thereof, and a pharmaceutically acceptable carrier.

13. A compound of any one Claims 1-8, or a pharmaceutically acceptable salt thereof for use in therapy.

## Patentansprüche

1. Eine Verbindung der Formel I: 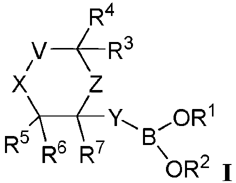 oder ein pharmazeutisch annehmbares Salz davon, wobei:
Y ein gerades oder verzweigtes (C₁-C₅)Alkylen oder Cycloalkyl(C₁-C₅)alkylen ist, wobei eine oder mehrere -CH₂-Gruppen in Y gegebenenfalls und unabhängig durch einen Rest, ausgewählt aus der Gruppe bestehend aus O, S und NH, ersetzt sind,
Z eine Bindung oder CH₂ ist,
V O, NR⁸ oder CR⁹R¹⁰ ist, wobei X und V nicht gleichzeitig O oder S und O bzw. NR⁸ sein können,
X O, S, NR¹¹, CH₂ oder CR¹²R¹³ ist, wobei V und X nicht gleichzeitig O und S, O bzw. NR¹¹ sein können,
R¹ Wasserstoff, C₃-C₆-Cycloalkyl oder C₁-C₆-Alkyl ist oder zusammen mit R² ein C₃-C₈-Cycloalkyl bildet, wobei das C₃-C₈-Cycloalkyl unsubstituiert oder substituiert ist mit einem bis vier Substituenten, ausgewählt aus der Gruppe bestehend aus Halogen,
C₁-C₆-Alkyl oder OH,
R² Wasserstoff, C₃-C₆-Cycloalkyl oder C₁-C₆-Alkyl ist oder zusammen mit R¹ ein C₃-C₈-Cycloalkyl bildet, wobei das C₃-C₈-Cycloalkyl unsubstituiert oder substituiert ist mit einem bis vier Substituenten, ausgewählt aus der Gruppe bestehend aus Halogen,
C₁-C₆-Alkyl oder OH,
R³ Wasserstoff, Halogen, C₁-C₆-Alkyl, Halogen-C₁-C₆-alkyl, OH, C₁-C₆-Alkyl-OH, C₁-C₆-Alkyl-OC₁-C₆-alkyl, C₁-C₆-Alkoxy, COOH, NH₂ oder COOC₁-C₆-Alkyl ist,
R⁴ zusammen mit R⁷ einen Heterocyclus bildet, wobei der Heterocyclus Pyrrolidin, Azetidin, Thiolan, Thietan, Oxetan, Tetrahydrofuran oder Piperidin ist,
R⁵ COOH ist,
R⁶ Wasserstoff, Halogen, C₁-C₆-Alkyl, Halogen-C₁-C₆-alkyl, OH, C₁-C₆-Alkyl-OH, C₁-C₆-Alkyl-O-C₁-C₆-alkyl, COOH, C₁-C₆-Alkoxy, COOH oder COOC₁-C₆-Alkyl ist,
R⁸ Wasserstoff,C₃-C₆-Cycloalkyl, C₁-C₆-Alkyl, C₁-C₆-Alkylaryl, C₁-C₆-Alkyl-NH₂, COC₁-C₆-Alkyl-NH₂, COC₁-C₆-Alkyl-NH(C₁-C₆-alkyl), COC₁-C₆-Alkyl-N(C₁-C₆-alkyl)₂, C₁-C₆-Halogenalkyl, C₁-C₆-Alkyl-OH oder COC₁-C₆-Alkyl ist,
R⁹ Wasserstoff, Halogen, C₁-C₆-Alkyl, Halogen-C₁-C₆-alkyl, OH, C₁-C₆-Alkyl-OH, C₁-C₆-Alkyl-OC₁-C₆-alkyl, C₁-C₆-Alkoxy, COOH oder COOC₁-C₆-Alkyl ist,
R¹⁰ Wasserstoff, Halogen, C₁-C₆-Alkyl, Halogen-C₁-C₆-alkyl, OH, C₁-C₆-Alkyl-OH, C₁-C₆-Alkyl-OC₁-C₆-alkyl, COOH, C₁-C₆-Alkoxy, COOH oder COOC₁-C₆-Alkyl ist oder zusammen mit R¹² ein C₃-C₆-Cycloalkyl oder einen Heterocyclus bildet, wobei das C₃-C₆-Cycloalkyl oder der Heterocyclus unsubstituiert oder substituiert ist mit 1 bis 3 Substituenten, ausgewählt aus der Gruppe bestehend aus Halogen, OH, C₁-C₆-Alkyl-OH, Oxo, C₁-C₆-Alkyl, C₁-C₆-Halogenalkyl, COC₁-C₆-Alkyl, C₁-C₆-Alkoxy, COOH, NH₂, C₁-C₆-Alkyl-NH₂, C₁-C₆-Alkylphenyl, CO(C₁-C₆-Alkyl)NH₂ und COOC₁-C₆-Alkyl, oder zusammen mit R⁶ eine (C₁-C₅)Alkylen-Brücke bildet, wobei eine oder mehrere -CH₂-Gruppen gegebenenfalls und unabhängig durch einen Rest ersetzt sind, ausgewählt aus der Gruppe bestehend aus O, S und NH,
R¹¹ Wasserstoff, C₃-C₆-Cycloalkyl, C₁-C₆-Alkyl, C₁-C₆-Alkylaryl, C₁-C₆-Alkyl-NH₂, COC₁-C₆-Alkyl-NH₂, COC₁-C₆-Alkyl-NH(C₁-C₆-alkyl), COC₁-C₆-Alkyl-N(C₁-C₆-alkyl)₂, C₁-C₆-Halogenalkyl, C₁-C₆-Alkyl-OH oder COC₁-C₆-Alkyl ist,
R¹² Halogen, C₁-C₆-Alkyl, Halogen-C₁-C₆-alkyl, OH, C₁-C₆-Alkyl-OH, C₁-C₆-Alkyl-OC₁-C₆-alkyl, COOH, C₁-C₆-Alkoxy, COOH oder COOC₁-C₆-Alkyl ist oder zusammen mit R⁶ oder R¹⁰ ein C₃-C₆-Cycloalkyl oder einen Heterocyclus bildet, wobei das C₃-C₆-Cycloalkyl oder der Heterocyclus unsubstituiert oder substituiert ist mit 1 bis 3 Substituenten, ausgewählt aus der Gruppe bestehend aus Halogen, OH, C₁-C₆-Alkyl-OH, Oxo, C₁-C₆-Alkyl, C₁-C₆-Halogenalkyl, COC₁-C₆-Alkyl, C₁-C₆-Alkoxy, COOH, NH₂, C₁-C₆-Alkyl-NH₂, C₁-C₆-Alkylphenyl, CO(C₁-C₆-Alkyl)NH₂ und COOC₁-C₆-Alkyl, und
R¹³ Halogen, C₁-C₆-Alkyl, Halogen-C₁-C₆-alkyl, OH, C₁-C₆-Alkyl-OH, C₁-C₆-Alkyl-OC₁-C₆-alkyl, C₁-C₆-Alkoxy, COOH oder COOC₁-C₆-Alkyl ist.

2. Die Verbindung nach Anspruch 1 oder ein pharmazeutisch annehmbares Salz davon, wobei R¹ und R² beides Wasserstoff sind.

3. Die Verbindung nach Anspruch 1 oder 2 oder ein pharmazeutisch annehmbares Salz davon, wobei Y Propylenyl ist.

4. Die Verbindung nach Anspruch 1 oder 2 oder ein pharmazeutisch annehmbares Salz davon, wobei Y 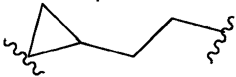 ist.

5. Die Verbindung nach einem der Ansprüche 1-3 oder ein pharmazeutisch annehmbares Salz davon, wobei R³ Wasserstoff oder COOH ist.

6. Die Verbindung nach einem der Ansprüche 1-5 oder ein pharmazeutisch annehmbares Salz davon, wobei Z eine Bindung ist.

7. Die Verbindung nach einem der Ansprüche 1-5 oder ein pharmazeutisch annehmbares Salz davon, wobei X CR¹²R¹³ oder NR¹¹ ist.

8. Eine Verbindung nach Anspruch 1, die ist: 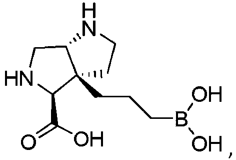 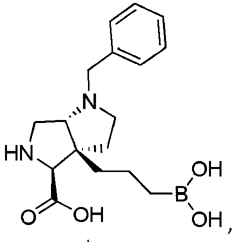 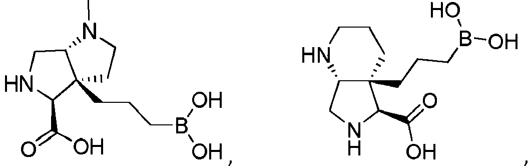 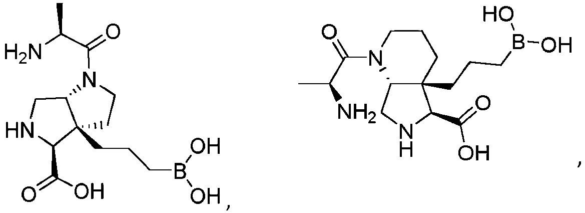 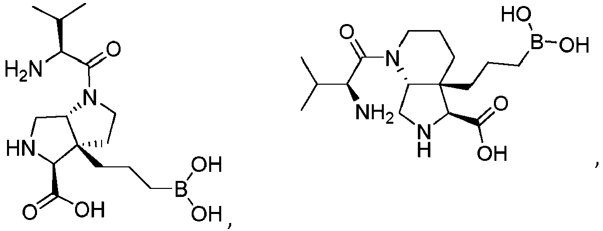 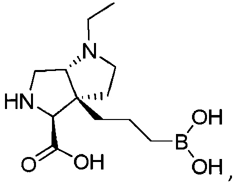 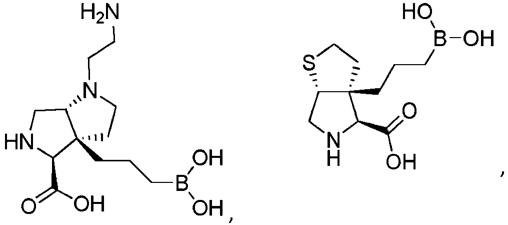 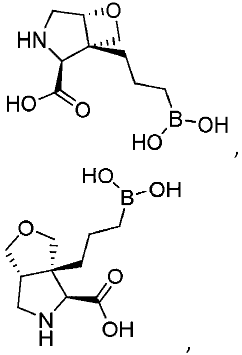 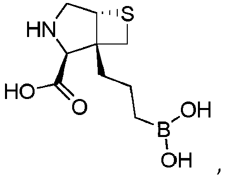 und 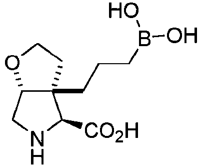 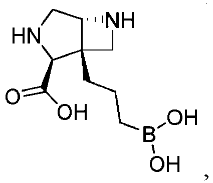 oder ein pharmazeutisch annehmbares Salz davon.

9. Eine Verbindung nach einem der Ansprüche 1-8 oder ein pharmazeutisch annehmbares Salz davon zur Verwendung bei der Behandlung von Krebs.

10. Eine Kombination, die eine Verbindung nach einem der Ansprüche 1-8 oder ein pharmazeutisch annehmbares Salz davon und andere therapeutische Mittel umfasst, zur Verwendung bei der Behandlung von Krebs.

11. Eine Kombination, die eine Verbindung nach einem der Ansprüche 1-8 oder ein pharmazeutisch annehmbares Salz davon und einen PD-1-Antagonisten umfasst, zur Verwendung bei der Behandlung von Krebs.

12. Eine pharmazeutische Zusammensetzung, die eine Verbindung nach einem der Ansprüche 1-8 oder ein pharmazeutisch annehmbares Salz davon und einen pharmazeutisch annehmbaren Träger umfasst.

13. Eine Verbindung nach einem der Ansprüche 1-8 oder ein pharmazeutisch annehmbares Salz davon zur Verwendung in der Therapie.

## Revendications

1. Composé de la Formule I : 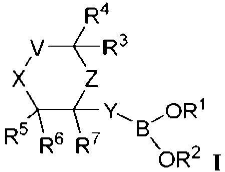 ou sel pharmaceutiquement acceptable de celui-ci, dans lequel :
Y est (C₁-C₅)alkylène ou cycloalkyl (C₁-C₅) alkylène linéaire ou ramifié, dans lequel un ou plusieurs groupes -CH₂- dans Y sont éventuellement et indépendamment remplacés par un groupement choisi dans le groupe constitué de O, S et NH ;
Z est une liaison, ou CH₂ ;
V est O, NR⁸ ou CR⁹R¹⁰, dans lequel X et V ne peuvent pas être simultanément O ou S et O ou NR⁸, respectivement ;
X est O, S, NR¹¹, CH₂, ou CR¹²R¹³, V et X ne peut pas être simultanément O et S, O ou NR¹¹, respectivement ;
R¹ est hydrogène, C₃-C₆cycloalkyle ou C₁-C₆alkyle ou, pris avec R², forme un C₃-C₈cycloalkyle, dans lequel le C₃-C₈cycloalkyle est non substitué ou substitué par un à quatre substituants choisis parmi le groupe constitué d'halogène, C₁-C₆alkyle ou OH ;
R² est hydrogène, C₃-C₆cycloalkyle ou C₁-C₆alkyle ou, pris avec R¹, forme un C₃-C₈cycloalkyle, dans lequel le C₃-C₈cycloalkyle est non substitué ou substitué par un à quatre substituants choisis parmi le groupe constitué d'halogène, C₁-C₆alkyle ou OH ;
R³ est hydrogène, halogène, C₁-C₆alkyle, halogénoC₁-C₆alkyle, OH, C₁-C₆alkylOH, C₁-C₆alkylOC₁-C₆alkyle, C₁-C₆alcoxy, COOH, NH₂ ou COOC₁-C₆alkyle ;
R⁴ est pris avec R⁷ pour former un hétérocycle, dans lequel l'hétérocycle est pyrrolidine, azétidine, thiolane, thiétane, oxétane, tétrahydrofurane ou pipéridine ;
R⁵ est COOH ;
R⁶ est hydrogène, halogène, C₁-C₆alkyle, halogénoC₁-C₆alkyle, OH, C₁-C₆alkylOH, C₁-C₆alkylOC₁-C₆alkyle, COOH, C₁-C₆alcoxy, COOH ou COOC₁-C₆alkyle ;
R⁸ est hydrogène, C₃-C₆cycloalkyle, C₁-C₆alkyle, C₁-C₆alkylaryle, C₁-C₆alkylNH₂, COC₁-C₆alkylNH₂, COC₁-C₆alkylNH(C₁-C₆alkyle), COC₁-C₆alkylN(C₁-C₆alkyl)₂, C₁-C₆halogénoalkyle, C₁-C₆alkylOH ou COC₁-C₆alkyle ;
R⁹ est hydrogène, halogène, C₁-C₆alkyle, halogénoC₁-C₆alkyle, OH, C₁-C₆alkylOH, C₁-C₆alkylOC₁-C₆alkyle, C₁-C₆alcoxy, COOH ou COOC₁-C₆alkyle ;
R¹⁰ est hydrogène, halogène, C₁-C₆alkyle, halogénoC₁-C₆alkyle, OH, C₁-C₆alkylOH, C₁-C₆alkylOC₁-C₆alkyle, COOH, C₁-C₆alcoxy, COOH ou COOC₁-C₆alkyle ou, pris avec R¹², forme un C₃-C₆cycloalkyle ou hétérocycle, le C₃-C₆cycloalkyle ou hétérocycle étant non substitué ou substitué par 1 à 3 substituants choisis dans le groupe constitué d'halogène, OH, C₁-C₆alkylOH, oxo, C₁-C₆alkyle, C₁-C₆halogénoalkyle, COC₁-C₆alkyle, C₁-C₆alcoxy, COOH, NH₂, C₁-C₆alkylNH₂, C₁-C₆alkylphényle, CO(C₁-C₆alkyl)NH₂ et COOC₁-C₆alkyle, ou, pris avec R⁶, forme un pont (C₁-C₅)alkylène, un ou plusieurs groupes -CH₂- étant éventuellement et indépendamment remplacés par un groupement choisi dans le groupe constitué de O, S et NH ;
R¹¹ est hydrogène, C₃-C₆cycloalkyle, C₁-C₆alkyle, C₁-C₆alkylaryle, C₁-C₆alkylNH₂, COC₁-C₆alkylNH₂, COC₁-C₆alkylNH(C₁-C₆alkyle), COC₁-C₆alkylN(C₁-C₆alkyl)₂, C₁-C₆halogénoalkyle, C₁-C₆alkylOH ou COC₁-C₆alkyle ;
R¹² est halogène, C₁-C₆alkyle, halogénoC₁-C₆alkyle, OH, C₁-C₆alkylOH, C₁-C₆alkylOC₁-C₆alkyle, COOH, C₁-C₆alcoxy, COOH ou COOC₁-C₆alkyle ou est pris avec R⁶ ou R¹⁰ pour former un C₃-C₆cycloalkyle ou hétérocycle, dans lequel le C₃-C₆cycloalkyle ou hétérocycle est non substitué ou substitué par 1 à 3 substituants choisis dans le groupe constitué d'halogène, OH, C₁-C₆alkylOH, oxo, C₁-C₆alkyle, C₁-C₆halogénoalkyle, COC₁-C₆alkyle, C₁-C₆alcoxy, COOH, NH₂, C₁-C₆alkylNH₂, C₁-C₆alkylphényle, CO(C₁-C₆alkyl)NH₂ et COOC₁-C₆alkyle ; et
R¹³ est halogène, C₁-C₆alkyle, halogénoC₁-C₆alkyle, OH, C₁-C₆alkylOH, C₁-C₆alkylOC₁-C₆alkyle, C₁-Cealcoxy, COOH ou COOC₁-C₆alkyle.

2. Composé selon la revendication 1, ou sel pharmaceutiquement acceptable de celui-ci, dans lequel R¹ et R² sont tous deux hydrogène.

3. Composé selon la revendication 1 ou 2, ou sel pharmaceutiquement acceptable de celui-ci, dans lequel Y est propylényle.

4. Composé selon la revendication 1 ou 2, ou sel pharmaceutiquement acceptable de celui-ci, dans lequel Y est

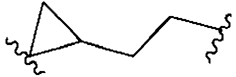

5. Composé selon l'une quelconque des revendications 1 à 3, ou sel pharmaceutiquement acceptable de celui-ci, dans lequel R³ est hydrogène ou COOH.

6. Composé selon l'une quelconque des revendications 1 à 5, ou sel pharmaceutiquement acceptable de celui-ci, dans lequel Z est une liaison.

7. Composé selon l'une quelconque des revendications 1 à 5, ou sel pharmaceutiquement acceptable de celui-ci, dans lequel X est CR¹²R¹³ ou NR¹¹.

8. Composé selon la revendication 1, qui est : 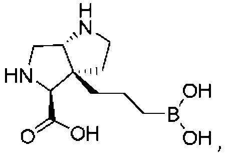 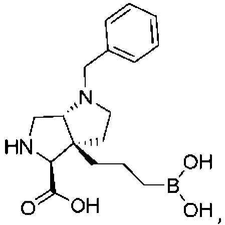 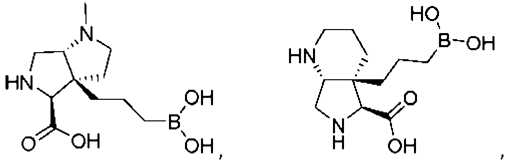 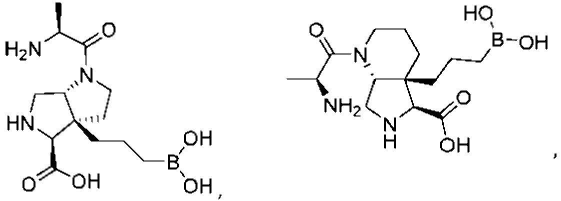 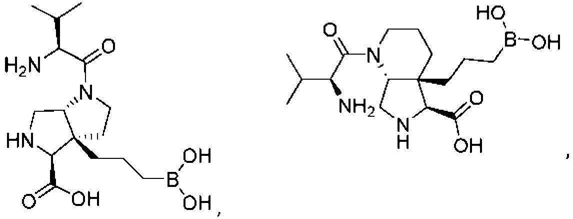 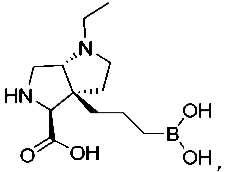 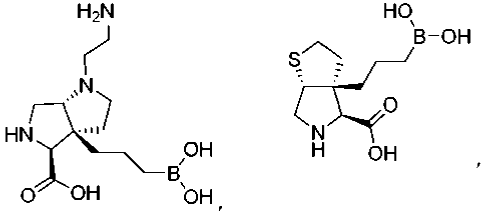 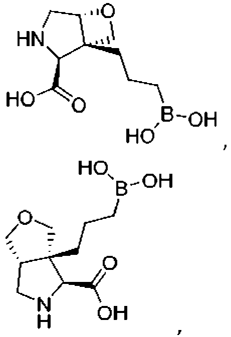 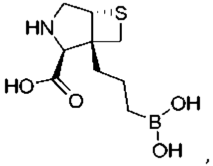 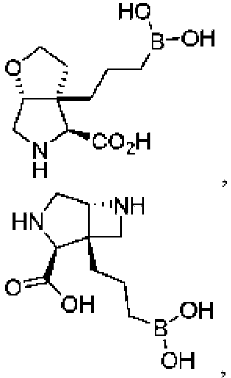 et
ou sel pharmaceutiquement acceptable de celui-ci.

9. Composé selon l'une quelconque des revendications 1 à 8, ou sel pharmaceutiquement acceptable de celui-ci, pour une utilisation dans le traitement d'un cancer.

10. Combinaison comprenant un composé selon l'une quelconque des revendications 1 à 8, ou un sel pharmaceutiquement acceptable de celui-ci et un autre agent thérapeutique, pour une utilisation dans le traitement d'un cancer.

11. Combinaison comprenant un composé selon l'une quelconque des revendications 1 à 8, ou un sel pharmaceutiquement acceptable de celui-ci et un antagoniste de PD-1 pour une utilisation dans le traitement d'un cancer.

12. Composition pharmaceutique comprenant un composé selon l'une quelconque des revendications 1 à 8, ou un sel pharmaceutiquement acceptable de celui-ci, et un support pharmaceutiquement acceptable.

13. Composé selon l'une quelconque des revendications 1 à 8, ou sel pharmaceutiquement acceptable de celui-ci, pour une utilisation dans une thérapie.
